# EUROPEAN PATENT APPLICATION

(11) **EP 1 217 066 A1**
(43) Date of publication of application: **26.06.2002**
(21) Application number: 00870316.7
(22) Date of filing: 21.12.2000
(51) Int. Cl.: C12N 15/11, C07K 14/705, A61K 38/17, G01N 33/68

(54) **Modulation of ATP-binding cassette transporter activity**

(71) Applicant: UNIVERSITEIT GENT, 9000 Gent (BE)
(72) Inventor: The designation of the inventor has not yet been filed
(74) Representative: De Clercq, Ann

(57) **Abstract**

The invention relates to the field of ATP-Binding Cassette (ABC) transporter molecules. and to molecules selectively modulating the activity of said ABC transporters. Herein are provided molecules and compounds which selectively modulate the activity of specifc ABC transporters. The invention also relates to molecules, compounds and compositions for preventing, treating or alleviating cancer or diseases related to bacterial, fungal or protozoal infections.

## Description

### FIELD OF THE INVENTION

The invention relates to the field of ATP-Binding Cassette (ABC) transporter molecules and to molecules selectively modulating the activity of said ABC transporters.

### BACKGROUND OF THE INVENTION

The ATP-binding cassette (ABC)-transporters constitute one of the largest and most highly conserved protein super families, which are found in large numbers in all organisms (Holland and Blight, 1999). These transmembrane proteins transport a wide range of compounds through biological membranes. The ABC transport proteins can import essential nutrients into cells, such as ions, sugars, amino acids etc... ABC-transporters can further protect cells by exporting a wide range of toxic compounds, signal the presence of infectious agents, and regulate development in microorganisms and mammals (Higgins, 1992). ABC-transporters participate in the regulation of several tissues such as the liver, lungs, retina and the immune system (Holland and Blight, 1999; Higgins, 1992) . Consequently, mutations affecting ABC-transporters are associated with a variety of human inherited diseases, including the cystic fibrosis transmembrane conductance regulator (CFTR) linked to cystic fibrosis (Sheppard and Welsh, 1999), and the ABC-A1 transporter linked to Tangier Disease (Rust *et al*., 1999; Bodzioch *et al.,* 1999; Brooks-Wilson *et al.,* 1999). Disease treatment is also dependent upon the function of ABC-transporters. For example in cancer treatment, expression of the P-glycoprotein or MDR1 (multidrug resistance gene product) in cancer cells, confers multidrug resistance against chemotherapeutic agents and decreases the efficacy of treatment (Borst *et al.,* 1986; Hipfner *et al.,* 1999). The resistance of some bacteria to certain classes of antibiotics can be attributed to the activity of transmembrane ABC transporters (Higgins, 1992).

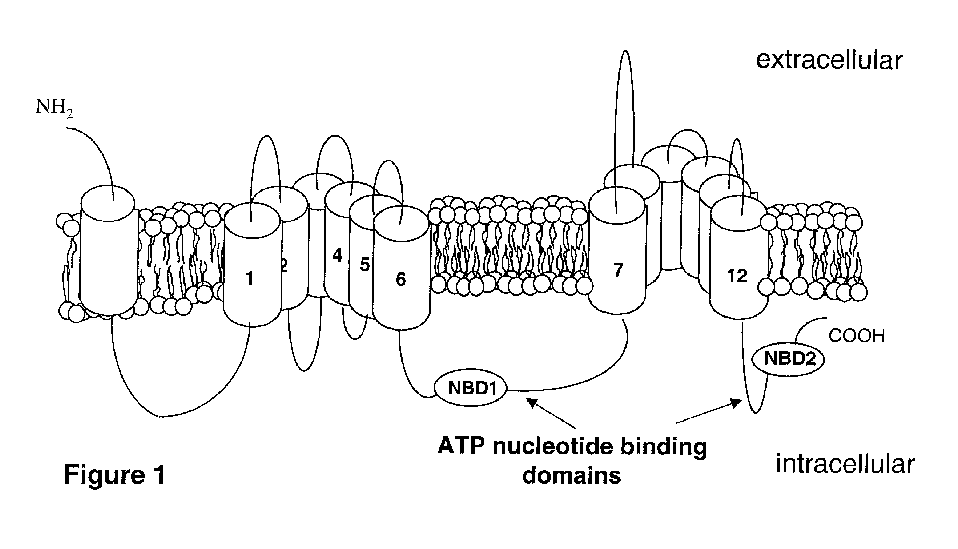

ABC-transporters consist of at least two basic subunits : an ATPase domain (also named NBD (domain) or (nucleotide binding domain)) which provides the energy required for the transport function, and a domain composed of six membrane-spanning helices, which form a channel and confer substrate specificity. Most ABC-transporters function as oligomers consisting of two ATPase and two transmembrane domains, which are either encoded separately, or tandemly replicated within a single polypeptide (Fig. 1). The transport of compounds across the membrane is accompanied by ATP hydrolysis. The ATPase domain becomes activated by binding of the allocrite and provides energy for transmembrane transport. It is thus obvious that there is strong cooperativity between the TM and ATPase domains of the ABC-transporters and they cannot function independently.

ATPase domains, homologous to those of the ABC-transporters have recently been identified in DNA repair proteins such as in Rad50, where it was found associated to a DNA binding domain (Hopfner *et al*., 2000). ATP hydrolysis by the ATPase domain of Rad50 provides the energy required for DNA binding and dissociation. In ABC-transporters, the two ATPase domains or NBD's do not function separately but rather show cooperative ATP hydrolysis, allosterically regulated by ligand binding (Higgins, 1992).

The crystal structure recently reported for a dimer of the ATPase domains of the Rad50, protein, shows that conserved motifs, in the ATPase domains form a dimerization interface. This interface holds both the ATP molecules and the two NBD monomers in an optimal conformation for the function of the transporter (Hopfner *et al*., 2000).

### Sequence and structure of the ATPase domains of ABC-transporters.

At the sequence level, ABC-ATPases are well conserved, displaying 30 % or more identity between different ABC-transporters. This identity is concentrated in several motifs, which have been used for the recognition of new ABC-transporters. The following sequence elements are typical for ABC-transporter NBD domains: the P-loop or Walker A motif: GAXXGXGKS/TT, where X can be any residue, which is critical for the binding of the beta-phosphate of the ATP nucleotide; the Walker B motif (consensus : HyHyHyHyDE where Hy is a hydrophobic residue). Upstream of the Walker B motif there is a signature motif, SXG where X is mostly G, which is typical for ATPases in ABC-transporters. Except for the Walker A motif, the functional significance of the other structural motifs was unclear until the crystal structure of several ABC transporters became available.

The crystal structure of HisP from *Salmonella typhimurium* (Hung et al., 1998), showed that the overall shape of the NBD domain is that of an "L", with two arms or lobes (I and II). Lobe I consists primarily of an β/α/β fold formed by the packing of helix A between two β-sheets consisting of six hydrogen-bonded (3 strands. Lobe II is generated by the packing of three helices against a five-stranded mixed β-sheet. The two lobes are joined into a single folded domain by a central beta-sheet II. The nucleotide binding site formed by the Walker A motif (P loop), is located in Lobe I near the interface of both lobes.

Several studies have shown that the two NBD domains, NBD1 and NBD2, of ABC-transporters function cooperatively and that inactivation of one catalytic site completely abolishes ATPase activity and transport function (Holland and Blight, 1999; Higgins, 1992). This can be achieved either by mutations in the Walker A or Walker B site, or by vanadate trapping (Holland and Blight, 1999) of ADP in the catalytic sites. An allosteric regulation of the cooperativity between the two NBD domains is probably a mode of "fine" regulation of these transporters.

The ABC-transporter family represents a class of proteins with widespread distribution in the human organism sufficing a variety of functions. Moreover, related ABC-transporters are prominent in other eukaryotes and bacteria. A possible way to interfere with the function of these transporters would be to prevent the binding of ATP. Alignment of the nucleotide binding domains of different ABC transporters from mammal and bacterial origin already enabled the identification and localization of the structural elements in the NBD domains of these transporters. Since these elements and especially the P loop are well conserved, problems arise when searching for means of blocking specific ABC-transporters without interfering with the action of other vital members of this protein family. Therefore it is an aim of the present invention to identify molecules and compounds which selectively modulate the activity of ABC transporters.

It is furthermore an aim of the present invention to provide methods for identifying and inhibiting the dimeric interfaces between the two ATPase domains of ABC-transporters. It is another aim of the invention to provide molecules, compounds and compositions for preventing, treating or alleviating cancer or diseases related to bacterial, fungal and protozoal infections.

### SUMMARY OF THE INVENTION

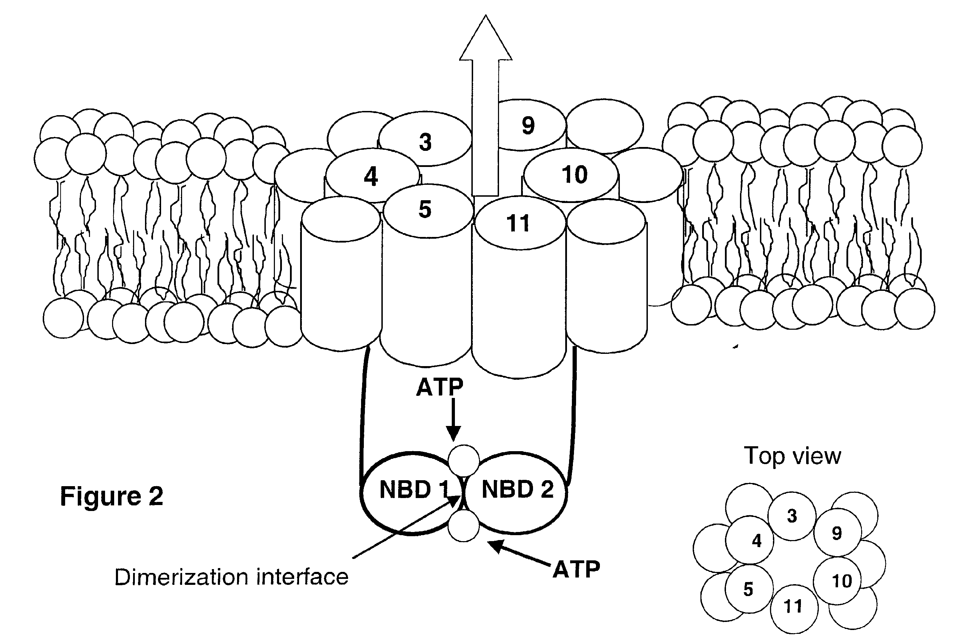

Three stretches of sequence are crucial for the heterodimeric NBD1-NBD2 structure of ABC-transporters: the residues corresponding to the signature motif, the Walker B motif and the D loop. The present inventors have identified the D-loop as the third important sequence which is conserved amongst ABC transporters. The D-loop immediately follows the Walker B motif and has been named as such by the inventors because they surprisingly found that the last amino acid (Aspartic acid, D) at the end of the conserved loop is very conserved among known ABC-transporter molecules. From several studies supported by extensive molecular modelling of the nucleotide binding domains of HisP, Rad50 and ABC-A1, the present inventors found that this D-loop is also structurally conserved amongst ABC-transporters and forms a central protein-protein dimerization interface (Figure 2). Contacts between the central residues of the D loop may contribute to the optimal dimer interface configuration. A central residue in the D loop could also be involved in the nucleophile attack on the ATP γ phosphate, through hydrogen bonding of an attacking water molecule.

As described above, the signature motif and Walker A motif, which are part of the dimer interface, belong to the best-conserved elements of the ATPase of ABC-transporters. Until now, the sequence and structure conservation of the D loop had not been described yet for the ABC-transporters. Using the sequence multiple alignment programs, the present inventors identified and analyzed the sequence conservation of these motifs in the ABC-transporters family. Consensus sequences for the D loop in different families of human ABC transporters and in bacterial, protozoal, fungal, and yeast ABC transporters are listed in Tables 1 and 2.

### The D-loop as target for inhibition of dimerization of ABC-transporters

Assembly of different proteins or of different domains within the same protein is a widespread mechanism used for growth and cellular control (Zutshi *et al.,* 1998). Many enzymes, viral proteins, and receptor-ligand interactions are comprised of oligomeric protein complexes (Jones and Thornton, 1996). Assembly of entire proteins or of protein domains are essential elements in allosteric control (Frieden, 1971), signal transduction, viral assembly and replication (Gibson, 1996). The ubiquitous nature of protein-protein interactions in essential cellular processes provides the possibility of developing novel control mechanisms based on inhibition of active protein assemblies. In the past five years, protein-protein interactions in viral enzymes and receptors were inhibited by peptides and small molecules, which led to the development of new antiviral drugs (Brickner and Chmielewski, 1998).

In the structure of the NBD1-NDB2 heterodimer, only the D-loop provides both efficiency and selectivity for inhibition or enhancement of dimerization of selected transporters. The D-loop residues ensures protein-protein interactions which are specific for sub-families of transporters, whereas dimerization via the signature and Walker A motif involves ATP binding and hydrolysis, a mechanism common to all transporters in the entire ABC family.

### DETAILED DESCRIPTION OF THE INVENTION

The present invention relates to a method for selectively modulating the activity of ABC transporters. One of the possible ways to modulate the activity of ABC transporters is by influencing the dimerization of the nucleotide binding domains.

Therefore, according to a first embodiment the present invention relates to a method for selectively modulating the activity of ABC transporters by influencing the dimerization of the nucleotide binding domains comprising the use of:
a) a polypeptide consisting of 6 to 50 amino acids comprising the D loop sequence of an ABC transporter,
b) a polypeptide consisting of the D loop sequence of an ABC transporter,
c) a peptide mimetic of any of the polypeptides of a) or b), or,
d) an antisense peptide of the polypeptides of a) or b).

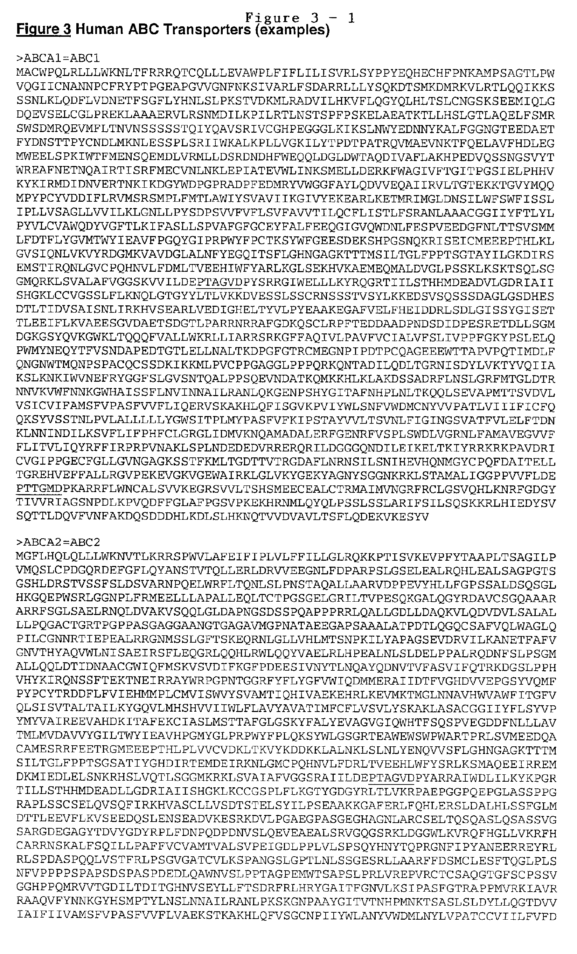

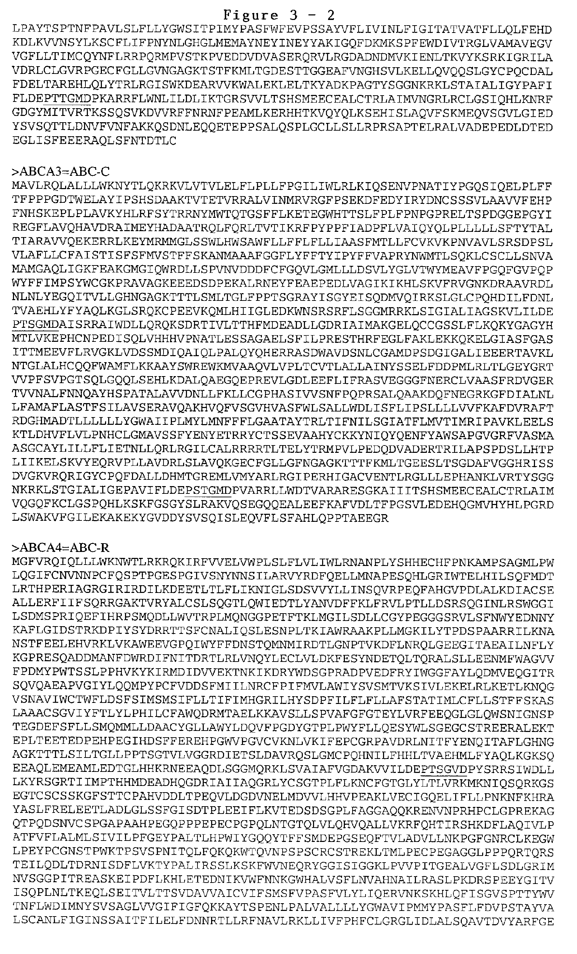

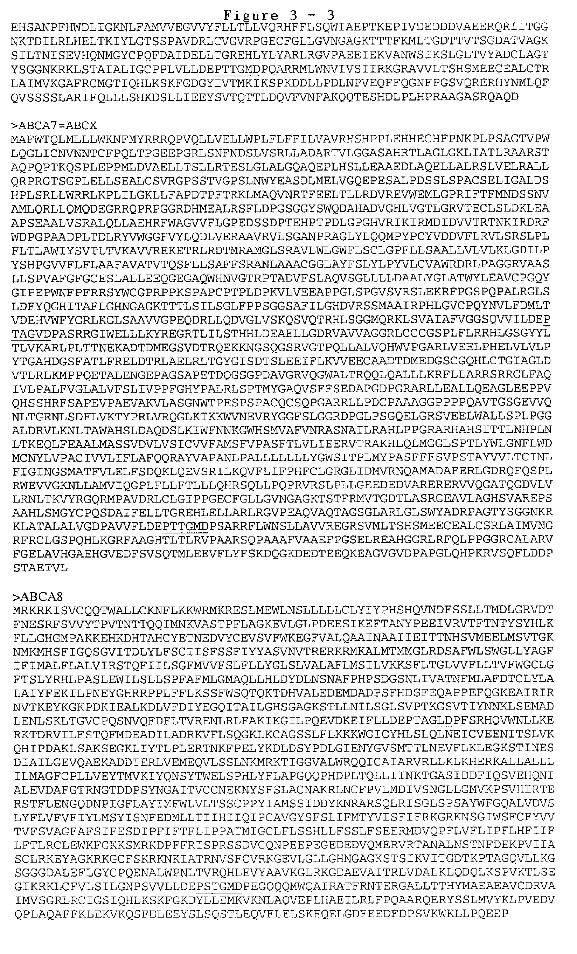

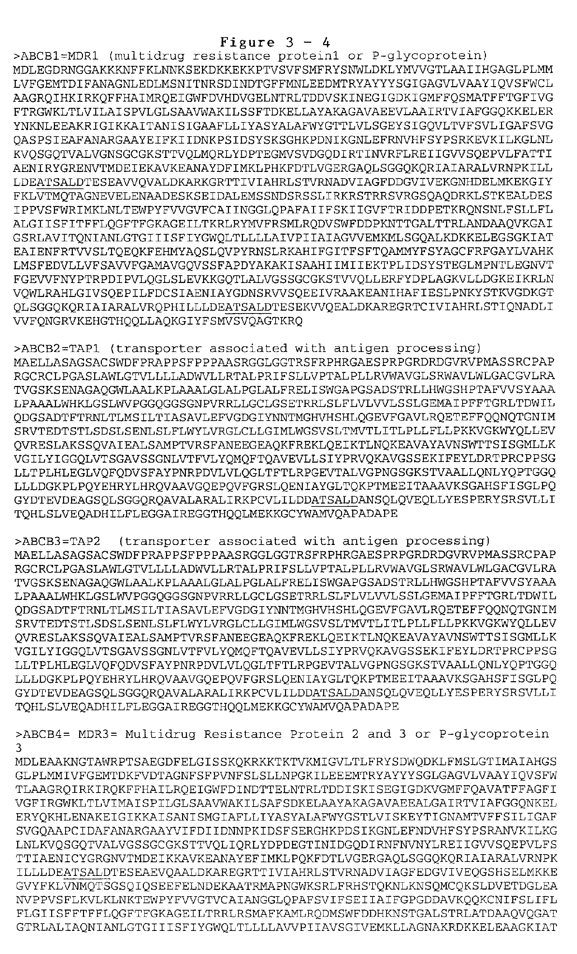

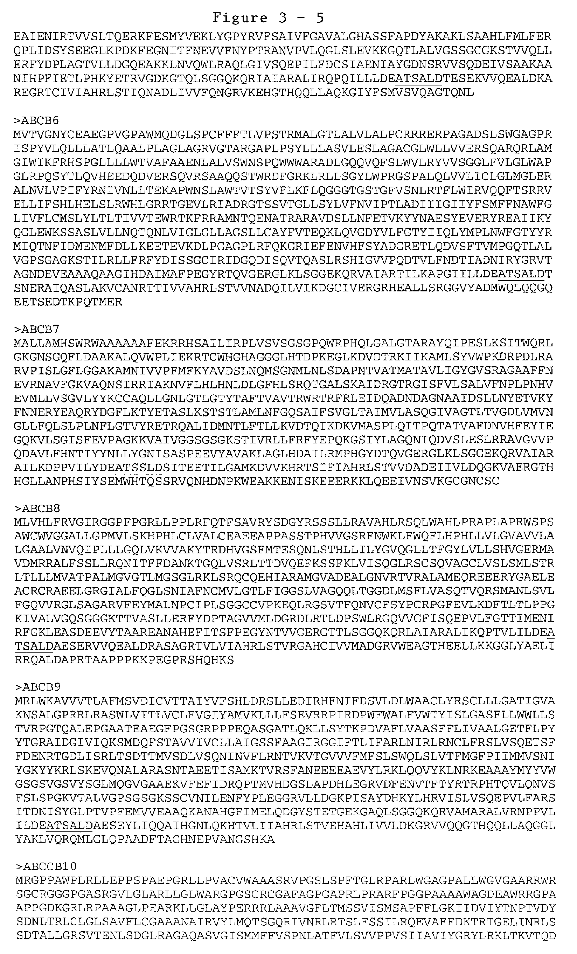

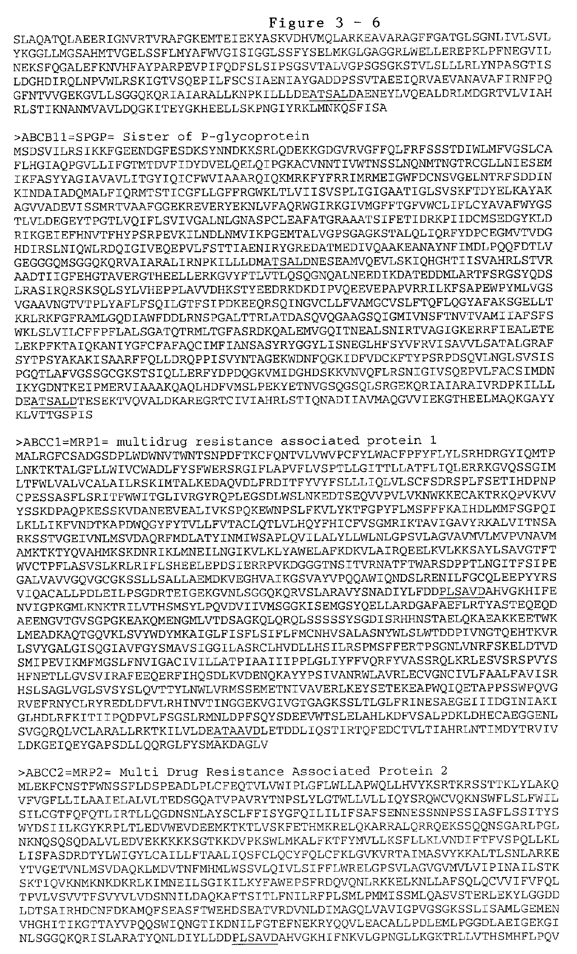

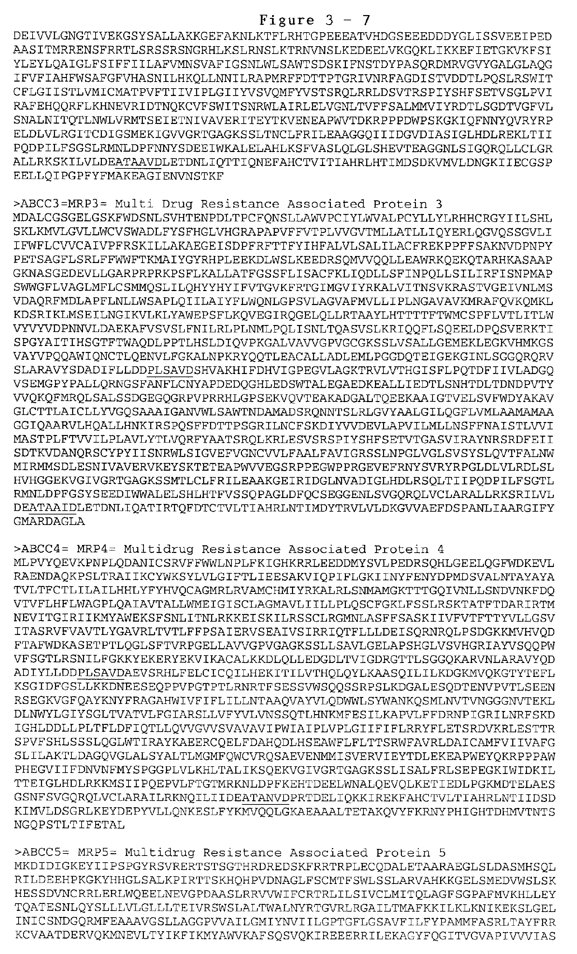

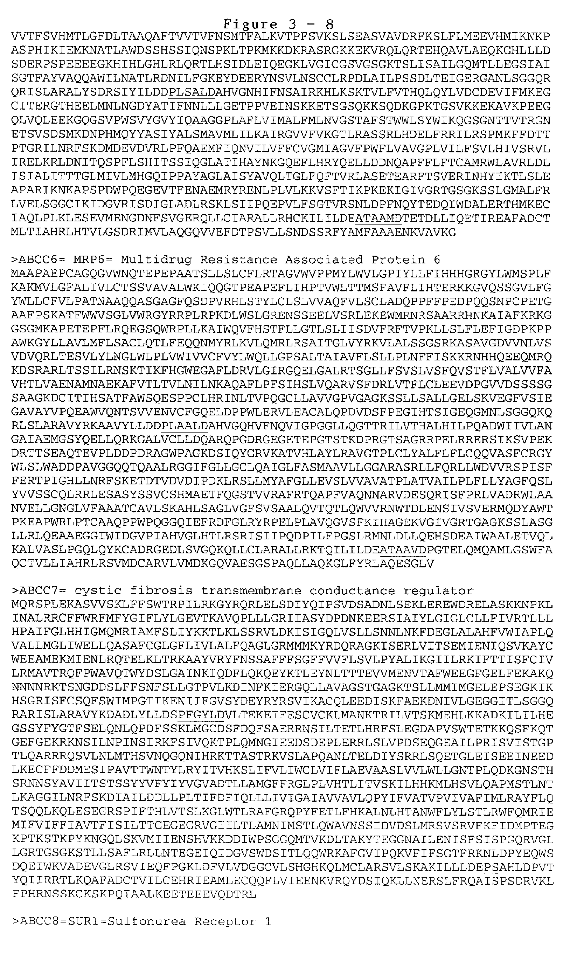

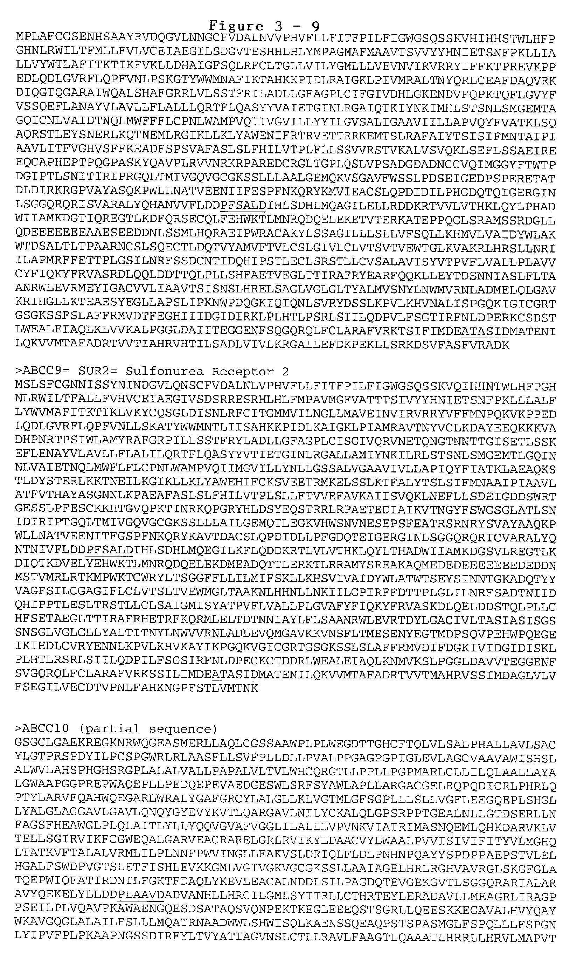

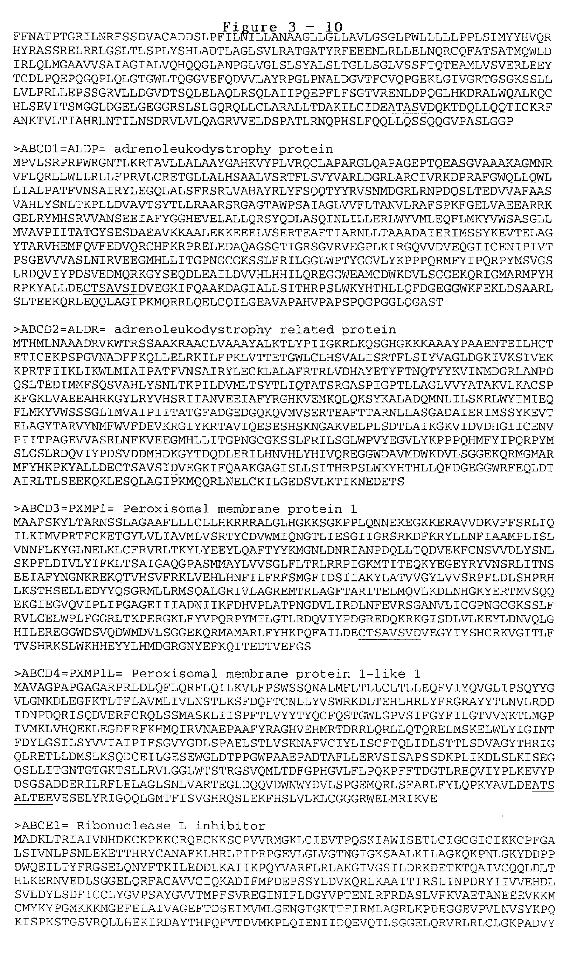

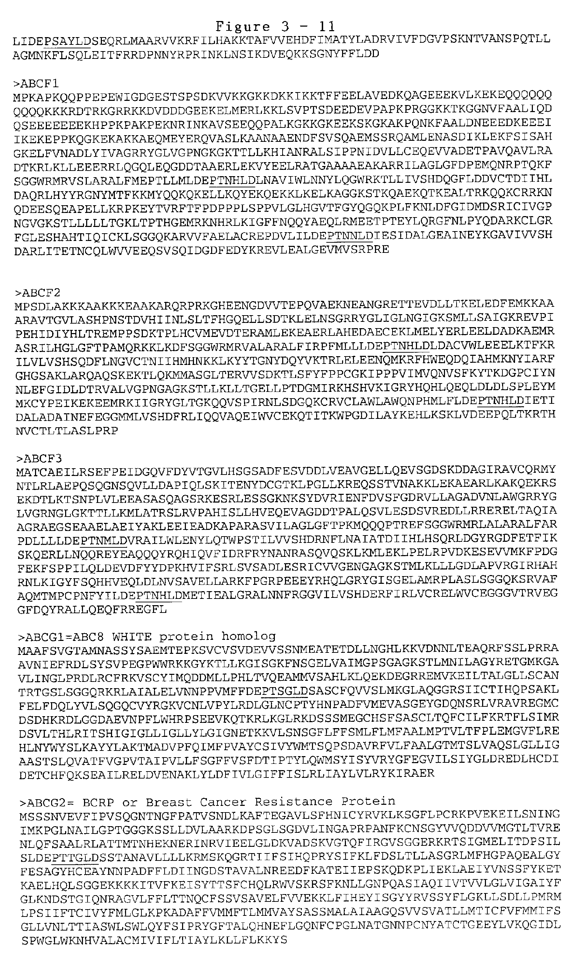

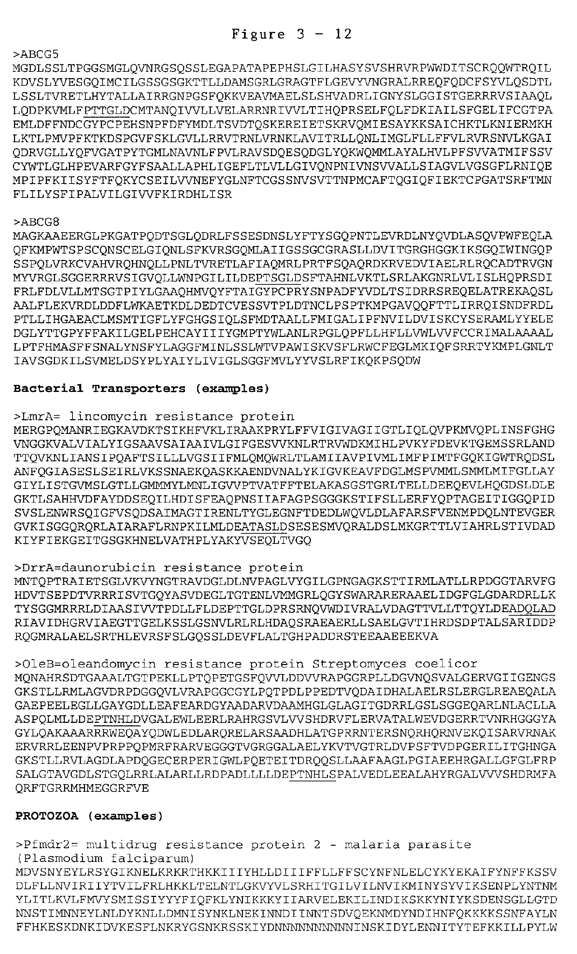

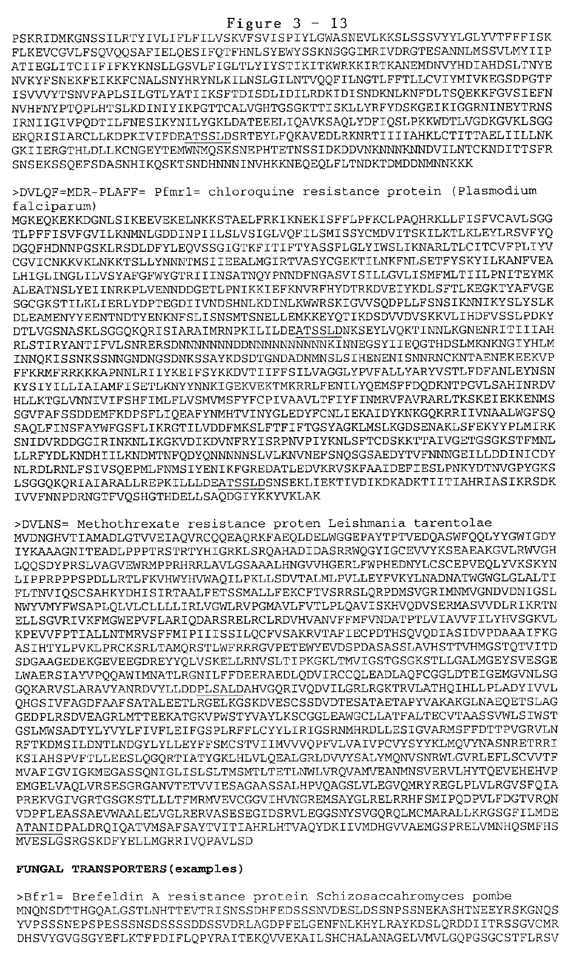

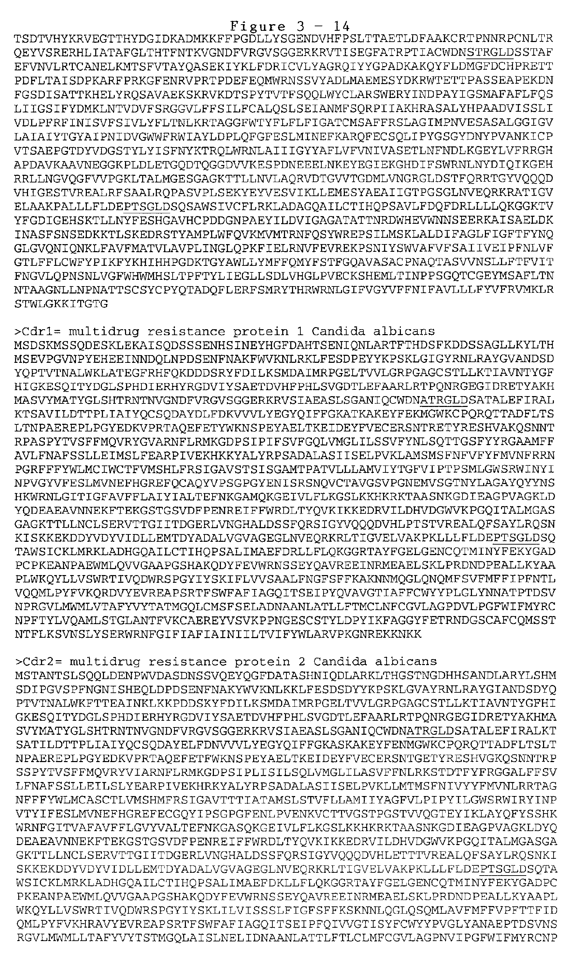

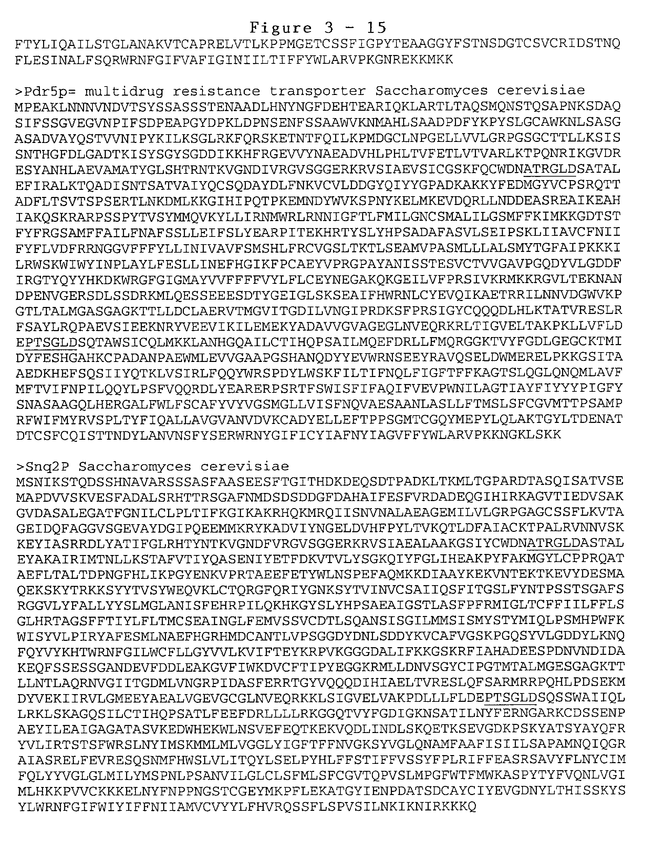

A list of known human ABC transporter molecules organized by family is provided in Table 1. In Table 2 bacterial, fungal and protozoal ABC transporters are listed. The amino acid sequences of some known examples of ABC transporters are listed in Figure 3.

The human ABC transporters have been organized in subfamilies, trivially named from ABCA to ABCF transporters and recently reviewed by Klein et al. (1999). Some of these transporters are generally known by their common names, as additionally noted in Table 1 and Figure 3. For instance, the multidrug resistance proteins or P-glycoproteins, now belonging to the ABCB transporters, have been long known as MDR proteins or belonging to the MDR/TAP subfamily.

In Table 1 and 2, the amino acid sequences of the D loop (in NBD1 and NBD2) are given for each member of the ABC transporter family listed. The D loop sequences are represented in SEQ ID NOs 1 to 43. In cases where only one nucleotide binding domain is present in the protein, for instance when the ABC transporter consists of at least two monomers or subunits, only one D loop sequence is noted in the table(s).

The expression "ABC transporter(s)", "ABC transporter protein(s)" and "ABC transporter molecule(s)" as used herein are interchangeable.

As used herein the terms "peptides" and "polypeptides" are interchangeable.

The term "modulating" relates to increasing, decreasing, inhibiting, abolishing or blocking the activity of selected transporters or groups of transporters within the ABC transporter family. For instance inhibiting the activity results at least in preventing the NBD1-NBD2 hetero-dimerization in such a way that the overall function of the ABC transporter in transporting molecules from one side to the other side of the cellular membrane is affected.

The expression "selectively modulating the activity" means that only the activity of one specific or at most a few very closely related ABC transporter molecules will be modulated in such a way that it influences the normal activity of said molecule. The "selectivity" resides in the amino acid sequence of the D loop of the nucleotide binding domain(s) of each particular ABC transporter molecule.

With the expression "D loop" is meant a sequence of 6 to 8 amino acids (ending with an aspartic acid (D)) which immediately follows the highly conserved Walker B motif in the primary structure of the ABC transporters. In each NBD domain of an ABC transporter, a D loop is present at the dimerization interface between the nucleotide binding domains. Interactions between the two D loops or between residues from one D loop and the ATP molecule bound by the second NBD play a key role in the dimerization of NBD's, and as such in the activity of the ABC transporter.

Therefore, in a more preferred embodiment the invention relates to a method for selectively modulating the activity of ABC transporters by influencing the dimerization of the nucleotide binding domains comprising the use of:
a) a polypeptide consisting of 6 to 50 amino acids, preferably 8, 10, 12, 14, 16, 18, 20, 25, 30, 35, 40, 45 or 50 amino acids, comprising the amino acid sequence represented in any of SEQ ID NOs 1 to 43,
b) a polypeptide consisting of the amino acid sequence as represented in any of SEQ ID NOs 1 to 43 or a functional homologue thereof,
c) a peptide mimetic of any of the polypeptides of a) or b), or,
d) an antisense peptide of the polypeptides of a) or b).

The expression "influencing the dimerization" can be used for inhibiting or blocking the formation of dimeric interfaces between the two ATPase domains of an ABC-transporter molecule.

The expression "functional homologue" relates to the corresponding sequences identifiable in other related ABC transporter molecules or relates to the homologous ABC transporter molecule from other organisms. As can be seen from Tables 1 and 2, some of the human ABC transporters have a bacterial or protozoal homologue with an identical D loop sequence. For instance, the D loop sequence of the human ABC transporter B7, belonging to the group of multidrug resistance proteins, is identical to the Pfmdr2 protein of *Plasmodium falciparum,* also known as a multidrug resistance protein. Therefore, it should also be understood that the person skilled in the art from the information herewith provided will know which D-loop sequence and molecules derived therefrom can be used for modulation of specific ABC transporters.

The expression "peptide mimetic" relates to a molecule that mimics the biological activity of a peptide but is no loner peptidic in chemical structure (Moore, 1996) The term mimetic is sometimes used to describe molecules that are no longer completely peptidic in nature, such as pseudopeptides and peptoids, but a strict definition is a molecule that no longer contains any peptide bonds and has a molecular weight of less than 700 daltons. The production and use of peptide mimetics is known to the one skilled in the art (see for instance Zutshi et al. (1997).

The term "antisense peptide" is reviewed by Blalock (1990) and by Roubos (1990). In this respect, the molecular recognition theory (Blalock, 1990) states that not only the complementary nucleic acid sequences interact but that, in addition, interacting sites in proteins are composed of complementary amino acid sequences (sense-receptor ligand or sense-antisense peptides). Thus, two peptides derived from complementary nucleic acid sequences in the same reading frame will show a total interchange of their hydrophobic and hydrophilic amino acids when the amino terminus of one is aligned with the carboxy terminus of the other. This inverted hydropathic pattern might allow two such peptides to assume complementary conformations responsible for specific interaction.

The present inventors found that the D loop is highly conserved in amino acid sequence as well as in structure among all members of ABC transporter family. Nevertheless, said D loop still displays sufficient variability between subfamilies and even between members of a single subfamily to serve as a target for selective interaction with inhibitory peptides or peptide mimetics. In some ABC transporter subfamilies (e.g. ABCB transporters) the sequence of the D loop seems to be conserved in all members of this subfamily for NBD2. In other families, a consensus sequence can be deducted for the D loops (in NBD1 and/or NBD2), as represented in Table 1. Therefore, in some applications it is possible to modulate or block the activity of all members of a specific ABC transporter subfamily using only one polypeptide prototype. Otherwise, in other ABC transporter subfamilies, the activity of specific members can be modulated because sufficient variability in the amino acid sequences of the respective D loops exists. Additionally, in the latter case is it also possible to modulate the activity of all members of said specific ABC transporter subfamily, provided that the consensus D-loop sequence (Tables 1 and 2) is used as a prototype polypeptide.

The term "prototype polypeptide" should be interpreted as the consensus sequence for the D-loop (e.g. for the ABCG transporter family, the consensus sequence represented in SEQ ID NO 36) on which all possible variants are patterned (e.g. the amino acid sequences represented in SEQ ID NOs 34 and 35).

"Very closely related" ABC transporter molecules for instance are molecules belonging to the same subfamily.

Furthermore, it is known that different isoforms exist for particular ABC transporter molecules. The invention thus also relates to possible variants of the D loop between isoforms of the same ABC transporter molecule.

It should be understood that the possible function and importance of the D loop has not been recognized until the present invention. Also, not all the sequences of ABC transporters are known so that also the consensus sequences for the NBD domain(s) of the transporter(s) may change. Nevertheless it should be recognized that the present invention relates to a general concept and applications of the D loop, which was recognized for the first time by the present inventors. It should therefore, be understood that the invention also relates to all applications and research tools wherein the existence, and the duality between "conserved" and at the same time "variability within the sequence" of the D-loop is used in any possible way already known in the art.

Furthermore, it should be understood that according to the present invention, molecules comprising the D loop sequences itself can be used or the D loop sequences can be used as a target for modulation or blocking.

In a preferred embodiment, the invention thus relates to a method for selectively decreasing (or increasing) the activity of an ABC transporter.

One way of modulating the activity of an ABC transporter is by blocking (or inhibiting) one of the D loops in the dimerization event for instance by a molecule comprising the corresponding amino acid sequence of the second D loop.

In several diseases associated with the functionality of ABC transporters, inhibition or blocking of the activity of ABC transporters can be beneficial for therapy. Some examples of such ABC transporters are given in bold in Table 1.

According to the invention, the activity of specific members of the ABC transporter family or groups (e;g. subfamilies) of ABC transporters can be modulated using specific prototype polypeptides as a target.

Therefore according to a preferred embodiment, the invention relates to a method for selectively modulating, preferably inhibiting or blocking, the activity of an ABC transporter, wherein said ABC transporter belongs to the group of multidrug transporter/P-glycoproteins comprising the use of:
a) a polypeptide consisting of 6 to 50 amino acids, preferably 8, 10, 12, 14, 16, 18, 20, 25, 30, 35, 40, 45 or 50 amino acids, comprising the amino acid sequence represented in any of SEQ ID NO 1 to 3, more preferably SEQ ID NO 1 and SEQ ID NO 2,
b) a polypeptide consisting of the amino acid sequence represented in any of SEQ ID NO 1 to 3, or a functional homologue thereof,
c) a peptide mimetic of any of the polypeptides of a) or b), or,
d) an antisense peptide of the polypeptide of a) or b).

Furthermore, the invention also relates to a method for selectively modulating, preferably inhibiting or blocking, the activity of an ABC transporter wherein said ABC transporter belongs to the group of the multidrug resistance associated proteins comprising the use of:
a) a polypeptide consisting of 6 to 50 amino acids, preferably 8, 10, 12, 14, 16, 18, 20, 25, 30, 35, 40, 45 or 50 amino acids, comprising the amino acid sequence represented in any of SEQ ID NOs 4 to 15, more preferably SEQ ID NOs 4, 5, 7, 8, 9, 10, 11, 12, 13, 14 or 15,
b) a polypeptide consisting of the amino acid sequence represented in any of SEQ ID NOs 4 to 15, more preferably SEQ ID NOs 4, 5, 7, 8, 9, 10, 11, 12, 13, 14 or 15, or a functional homologue thereof,
c) a peptide mimetic of any of the polypeptides of a) or b), or,
d) an antisense peptide of the polypeptide of a) or b).

The ABC transporter molecules belonging to the group of the multidrug transporter/P-glycoproteins and/or multidrug resistance associated proteins are very important in diseases such as cancer.

The multidrug resistance protein or P-glycoprotein (MDR1 or Pgp1) was the first human ABC protein cloned and is still one of the most intensively studied proteins of the family of the ABCB transporters. This special attention was attracted by the fact that multidrug resistance of cancer cells was found to be caused by this protein.

Other important candidate ABC transporters belonging to this group are the TAP1 and TAP2 transporters which are associated with antigen processing which activity needs to be suppressed upon transplantation of organs. For the moment, blocking of the activity of said transporters needs the administration of high doses of drugs such as cyclosporine. Reduction in the use of this cyclosporine and avoiding the rejection of the transplant by inhibition of the TAP transporter would increase the success of the transplant.

Additionally, also the human multidrug resistance associated protein (belonging to the MRP/CFTR or ABCC transporters) confers multidrug resistant phenotype to tumor cells. The majority of non-P-glycoprotein mediated multidrug resistance is due to the over-expression of hMRP1. HMRP1 transports both hydrophobic anticancer agents and anionic (e.g. glutathione) drug conjugates. Its physiological functioning may provide a wide range of cellular xenobiotic resistance. Therefore ABC transporters belonging to this family are especially envisaged in several applications of the present invention.

Alternatively, the modulation of the activity of an ABC transporter can also result in an improvement of the binding or dimerization of the nucleotide binding domains. Said improvement can be the result of an increase in length of the binding-time period or can be the result of an increase in frequency of dimerization events per time period between the nucleotide binding domains. A positive effect on the dimerization can for instance be achieved by the activity of small peptides or peptide mimetics which directly or indirectly interact with the D loop in a structural (conformational) sense or in an interaction between or with the amino acids constituting the D-loop motif.

One example of an ABC transporter which is envisaged to benefit from enhancement of activity or of increasing the dimerization event is for instance the CFTR transporter (cystic fibrosis transmembrane conductance regulator. The CFTR transporter is involved with the transport of chloride ions through the membrane. Increasing the activity of said transporter would be beneficial for the treatment of cystic fibrosis patients in which said transporter is defective.

Aso the activity of other ABC transporters can be modulated in a way that increasing the activity is beneficial for therapy. Some examples of such ABC transporters are underlined in Table 1.

According to a further preferred embodiment the invention relates to a method for selectively modulating, preferably enhancing, the activity of an ABC transporter wherein said ABC transporter is the cystic fibrosis transmembrane conductance regulator (CFTR) comprising the use of:
a) a polypeptide consisting of 6 to 50 amino acids, preferably 8, 10, 12, 14, 16, 18, 20, 25, 30, 35, 40, 45 or 50 amino acids, comprising the amino acid sequence represented in any of SEQ ID NOs 11 or 12,
b) a polypeptide consisting of the amino acid sequence represented in any of SEQ ID NOs 11 or 12, or a functional homologue thereof,
c) a peptide mimetic of any of the polypeptides of a) or b), or,
d) an antisense peptide of the peptide of a) or b).
(claims 24-28) Therefore the present invention also relates to the use of a (poly)peptide, antisense peptide or peptide mimetic as defined above or a compound obtainable by one of the compound screening methods described further for treatment of cancer, optionally in combination with chemotherapy. Said (poly)peptides, antisense peptide, peptide mimetic or compound can also be used for treating resistance to drugs in mammals. Said (poly)peptide, antisense peptide, peptide mimetic or compound can also be used for the preparation of a medicine for treating cancer or for preventing, treating or alleviating diseases associated with drug resistance in a mammal.

ABC transporters are not only important in humans or higher eukaryotes but also bacterial, fungal and protozoal ABC transporters are known wherein a D loop can be recognized as an important structural feature for dimerization and/or functionality and/or activity of said transporter.

Therefore according to yet another preferred embodiment, the invention relates to a method for selectively modulating the activity of an ABC transporter wherein said ABC transporter is a bacterial transporter comprising the use of:
a) a polypeptide consisting of 6 to 50 amino acids, preferably 8, 10, 12, 14, 16, 18, 20, 25, 30, 35, 40, 45 or 50 amino acids, comprising the amino acid sequence represented in any of SEQ ID NOs 27, 37 to 39,
b) a polypeptide consisting of the amino acid sequence represented in any of SEQ ID NOs 27, 37 to 39, or a functional homologue thereof,
c) a peptide mimetic of any of the polypeptides of a) or b), or,
d) an antisense peptide of the peptide of a) or b).

According to yet another preferred embodiment, the invention relates to a method for selectively modulating the activity of an ABC transporter wherein said ABC transporter is a fungal ABC transporter, comprising the use of:
a) a polypeptide consisting of 6 to 50 amino acids, preferably 8, 10, 12, 14, 16, 18, 20, 25, 30, 35, 40, 45 or 50 amino acids, comprising the amino acid sequence represented in any of SEQ ID NOs 40 to 42,
b) a polypeptide consisting of the amino acid sequence represented in any of SEQ ID NOs 40 to 42, or a functional homologue thereof,
c) a peptide mimetic of any of the polypeptides of a) or b), or,
d) an antisense peptide of the peptide of a) or b).

According to yet another preferred embodiment, the invention relates to a method for selectively modulating the activity of an ABC transporter wherein said ABC transporter is a protozoal ABC transporter, comprising the use of:
a) a polypeptide consisting of 6 to 50 amino acids, preferably 8, 10, 12, 14, 16, 18, 20, 25, 30, 35, 40, 45 or 50 amino acids, comprising the amino acid sequence represented in any of SEQ ID NO 2, 8 or 43,
b) a polypeptide consisting of the amino acid sequence represented in any of SEQ ID NOs 2, 8 or 43, or a functional homologue thereof,
c) a peptide mimetic of any of the polypeptides of a) or b), or,
d) an antisense peptide of the peptide of a) or b).

Preferably, said bacterial, fungal or protozoal ABC transporter(s) is involved in bacterial, fungal or protozoal infection of a mammal.

According to a further preferred embodiment, said bacterial, fungal or protozoal ABC transporter is involved in the induction of resistance to antibiotics or other drugs in mammals.

The activity of bacterial, fungal or protozoal ABC transporters can be explained in a way that they transport antibiotics (or certain classes of antibiotics, or other drugs) which are administered to a human or other mammal in need thereof, to the outside of the bacterial or fungal or protozoal cell wall so that said antibiotics can not exert there anti-bacterial or anti-fungal or anti-protozoal action. Therefore, the (poly)peptides or antisense peptides, or peptide mimetics or compounds that specifically block the ABC transporters in bacteria, fungi and protozoa could potentially be used for the treatment of infections caused by these pathogens. For instance blocking the D loops in the ABC transporters of said pathogens might result in a specific treatment method for bacterial, fungal or protozoal infections. The resulting inhibition of ABC transporter function in these pathogens will cause the death of said pathogen and will be beneficial to the patients. As such these (poly)peptides antisense peptides, peptide mimetics or compounds can be considered as an alternative for antibiotics, antifungicide or anti-protozoal treatment for instance in cases in which a number of organisms have developed already a drug resistance.

Also co-administration of (poly)peptides or antisense peptides, or peptide mimetic which inhibit or block the activity of bacterial ABC transporters which are involved in such processes or compounds obtainable by one of the compound screening methods described further together with the antibiotic (or drug) would be beneficial to the anti-bacterial action of said antibiotic (or drug).

Therefore, according to another embodiment the invention also relates to the use of a molecule selected from :
a) a polypeptide consisting of 6 to 50 amino acids, preferably 8, 10, 12, 14, 16, 18, 20, 25, 30, 35, 40, 45 or 50 amino acids, comprising the amino acid sequence represented in any of SEQ ID NOs 2, 8, 29 and 37 to 43,
b) a polypeptide consisting of the amino acid sequence represented in any of SEQ ID NOs 2, 8, 29 and 37 to 43, or a functional homologue thereof,
c) a peptide mimetic of any of the polypeptides of a) or b), or,
d) an antisense peptide of the peptide of a) or b),
as a anti-bacterial or ant-fungal or anti-protozoal agent.

It should be noted that SEQ ID NOs 2, 8, and 29 have a homologue in human ABC transporters. However the person skilled in the art perfectly knows which molecules or sequences represented by their SEQ ID NOs to choose when the activity of only bacterial and/or fungal and/or protozoal ABC transporters needs to be modulated. For instance in case an anti-bacterial agent is used, molecules based on SEQ ID NO 43 will be used and not for instance based on SEQ ID NO 2 or 8.

The recognition of the D-loop as a tool for selectively modulating the activity of ABC transporters can be further exploited in therapy for instance for treatment or for preparation of medicaments.

Therefore the invention also relates to a method for preventing, treating or alleviating diseases associated with the functionality of a human ABC-transporter comprising the use of:
a) a polypeptide consisting of 5 to 50 amino acids comprising the amino acid sequence represented in any of SEQ ID NOs 1 to 36,
b) a polypeptide consisting of the amino acid sequence represented in any of SEQ ID NOs 1 to 36, or a functional homologue thereof,
c) a peptide mimetic of any of the polypeptides of a) or b), or,
d) an antisense peptide of the peptide of a) or b).

Alternatively the invention also relates to a method for the preparation of a medicament for the prevention, treatment or alleviation of diseases associated with the functionality of a human ABC-transporter comprising the use of:
a) a polypeptide consisting of 5 to 50 amino acids comprising the amino acid sequence represented in any of SEQ ID NOs 1 to 36,
b) a polypeptide consisting of the amino acid sequence represented in any of SEQ ID NOs 1 to 36, or a functional homologue thereof,
c) a peptide mimetic of any of the polypeptides of a) or b), or,
d) an antisense peptide of the peptide of a) or b).

The invention furthermore relates to a method for preventing, treating or alleviating diseases related with bacterial infections comprising the use of:
a) a polypeptide consisting of 6 to 50 amino acids, preferably 8, 10, 12, 14, 16, 18, 20, 25, 30, 35, 40, 45 or 50 amino acids, comprising the amino acid sequence represented in any of SEQ ID NOs 29, 37, 38 or 39,
b) a polypeptide consisting of the amino acid sequence represented in any of SEQ ID NOs 29, 37, 38 or 39, or a functional homologue thereof,
c) a peptide mimetic of any of the polypeptides of a) or b), or,
d) an antisense peptide of the peptide of a) or b).

Alternatively the invention also relates to a method for the preparation of a medicament for the prevention, treatment or alleviation of diseases associated with bacterial infections comprising the use of:
a) a polypeptide consisting of 6 to 50 amino acids, preferably 8, 10, 12, 14,16,18, 20, 25, 30, 35, 40, 45 or 50 amino acids, comprising the amino acid sequence represented in any of SEQ ID NOs 29, 37, 38 or 39,
b) a polypeptide consisting of the amino acid sequence represented in any of SEQ ID NOs 29, 37, 38 or 39, or a functional homologue thereof,
c) a peptide mimetic of any of the polypeptides of a) or b), or,
d) an antisense peptide of the peptide of a) or b).

The invention furthermore relates to a method for preventing, treating or alleviating diseases related with fungal infections comprising the use of:
a) a polypeptide consisting of 6 to 50 amino acids, preferably 8, 10, 12, 14, 16, 18, 20, 25, 30, 35, 40, 45 or 50 amino acids, comprising the amino acid sequence represented in any of SEQ ID NOs 40 to 42,
b) a polypeptide consisting of the amino acid sequence represented in any of SEQ ID NOs 40 to 42, or a functional homologue thereof,
c) a peptide mimetic of any of the polypeptides of a) or b), or,
d) an antisense peptide of the peptide of a) or b).

Alternatively the invention also relates to a method for the preparation of a medicament for the prevention, treatment or alleviation of diseases associated with fungal infections comprising the use of:
a) a polypeptide consisting of 6 to 50 amino acids, preferably 8, 10, 12, 14, 16, 18, 20, 25, 30, 35, 40, 45 or 50 amino acids, comprising the amino acid sequence represented in any of SEQ ID NOs 40 to 42,
b) a polypeptide consisting of the amino acid sequence represented in any of SEQ ID NOs 40 to 42, or a functional homologue thereof,
c) a peptide mimetic of any of the polypeptides of a) or b), or,
d) an antisense peptide of the peptide of a) or b).

The invention furthermore relates to a method for preventing, treating or alleviating diseases related with protozoal infections comprising the use of:
a) a polypeptide consisting of 6 to 50 amino acids, preferably 8, 10, 12, 14, 16, 18, 20, 25, 30, 35, 40, 45 or 50 amino acids, comprising the amino acid sequence represented in any of SEQ ID NOs 2, 8, or 43,
b) a polypeptide consisting of the amino acid sequence represented in any of SEQ ID NOs 2, 8 or 43, or a functional homologue thereof,
c) a peptide mimetic of any of the polypeptides of a) or b), or,
d) an antisense peptide of the peptide of a) or b).

Alternatively the invention also relates to a method for the preparation of a medicament for the prevention, treatment or alleviation of diseases associated with protozoal infections comprising the use of:
a) a polypeptide consisting of 6 to 50 amino acids, preferably 8, 10, 12, 14, 16, 18, 20, 25, 30, 35, 40, 45 or 50 amino acids, comprising the amino acid sequence represented in any of SEQ ID NOs 2, 8, or 43,
b) a polypeptide consisting of the amino acid sequence represented in any of SEQ ID NOs 2, 8 or 43, or a functional homologue thereof,
c) a peptide mimetic of any of the polypeptides of a) or b), or,
d) an antisense peptide of the peptide of a) or b).

The present invention also relates to the use of any of the molecules as defined above or a compound obtainable by any of the compound screening methods described further for preventing, treating or alleviating diseases associated with bacterial, fungal or protozoal infections or for the preparation of a medicament for preventing, treating or alleviating diseases associated with bacterial, fungal or protozoal infections. Furthermore, these molecules or compounds may be used for treating resistance to antibiotics in a mammal or for preparing a medicament for treating resistance to antibiotics or other drugs in a mammal.

According to another embodiment, the present invention provides methods of identifying compounds which selectively modulate, inhibit, activate or interfere with the properties of ABC transporters. Compounds may carry agonistic or antagonistic properties. The compounds to be screened may be of extracellular, intracellular, biologic or chemical origin.

Such a screening method may comprise the following steps (a) contacting a compound to be tested with at least one of the polypeptide as defined above under any of a) to d) or with a polypeptide corresponding to the D loop or a nucleotide binding domain of an ABC transporter, (b) detecting a diminution or inhibition of the activity of said ABC transporter, and, (c) identifying said compound.

Alternatively, in step (b) of the above mentioned method, the effectiveness of said compound can also be investigated by measurement of the ATPase activity in case the compound is contacted with a functional ATPase domain (nucleotide binding domain). Methods to measure ATPase activity are known in the art but are also described further in the examples section.

The polypeptides according to the invention employed in such a method may be for example in solution or coated on suspended beads. Alternatively, these can be affixed to a solid support, borne on a cell or phage surface or located intracellularly.

When polypeptide fragments are coated on solid supports, they can be tested for their binding affinity for large numbers of compounds. These can be used in different kinds of high throughput screenings in order to identify compounds having suitable binding affinity to the polypeptides according to the invention. Platform technologies or technologies based on SPR (surface plasmon resonance) can be applied.

The invention also relates to methods for identifying compounds which selectively bind to or selectively modulate the properties of ABC transporters, which method comprises:
a) providing a yeast two-hybrid system wherein the nucleotide binding domains NBD1 and NBD2 of an ABC transporter are expressed, or,
b) providing a mammalian expression system wherein the nucleotide binding domains NBD1 and NBD2 of an ABC transporter are expressed, or,
c) providing a bacterial expression system wherein the nucleotide binding domains NBD1 and NBD2 of an ABC transporter are expressed (and/) or secreted, and,
d) interacting said compound with the complex formed by the expressed polypeptides as defined in any of a) to c),
e) inferring from the interaction between said compound and one of the nucleotide binding domains a modulation of the properties of said ABC transporter, and,
f) identifying said compound.

Compounds found using this approach and modulating the activity of a selected ABC transporter may additionally be tested on their efficiency to modulate other ABC transporter in order to avoid undesired cross-activity of said compounds on non selected ABC transporters.

Alternatively additional tests can be performed to test the influence of the compound onto protein stability, post-translational modification, precursor processing and protein translocation. All these aspects influence the concentration and/or activity of corresponding proteins and consequenly influence the effect of these onto the metabolism of the cell. Also here, medium or low throughput systems can be used to confirm results obtained by the high throughput assays.

Compounds obtainable by one of the methods described above or the use of said compounds as a medicament also form part of the invention.

The invention also relates to an isolated nucleic acid encoding at least one of the polypeptides defined above in a) to d) comprising an ABC transporter D loop represented in any of SEQ ID NOs 1 to 43.

The invention further relates to a polypeptide encodable by and isolated nucleic acid as defined above.

The invention also relates to a composition, preferably a pharmaceutical composition, comprising at least one polypeptides of the invention and to the use of said polypeptide or of the composition comprising said polypeptide as a medicament.

The invention also relates to a cellular host for use in a method described above, said cellular host transformed with a nucleic acid encoding at least one nucleotide binding domain of an ABC transporter protein or a nucleic acid comprising a nucleic acid as described above, said nucleic acid in an expressible format.

The cellular hosts used in the invention can be from bacterial, fungal, vegetal or mammalian origin. There are numerous vectors, expression systems and methods known in the art to allow the skilled in the art for transforming, transfecting or infecting the desired host cell with the desired nucleic acid in order to obtain desired expression of any of the polypeptides of the invention.

The invention, now being generally described, will be more readily understood by reference to the following examples, which are included merely for purposes of illustration of certain aspects and embodiments of the present invention and are not intended to limit the invention. All of the references_mentioned herein are incorporated by reference.

### BRIEF DESCRIPTION OF TABLES AND FIGURES

- **Figure 1.**: Schematic representation of the ABC transporters.
- **Figure 2.**: Schematic presentation of positioning of the ATP-binding site and the dimerization-interface in the ABC transporter molecule.
- **Figure 3.**: Amino acid sequences of human, bacterial, protozoal and fungal transporters: examples. The underlined seqeunces refer to the D loops in these sequences. The names of the sequences given after each ">" refer to the names given in Tables 1 and 3.
- **Table 1.**: Sequences of the D loops in different human ABC transporters, and derived consensus sequence for each family. Marked in ***bold** are the candidate transporters for inhibition of activity. Marked underlined are the candidate transporters for increase of activity.
- **Table 2:**: Sequences of the D loops in different ABC transporters from bacteria fungi and protozoa. All transporters are candidate transporters for inhibition.

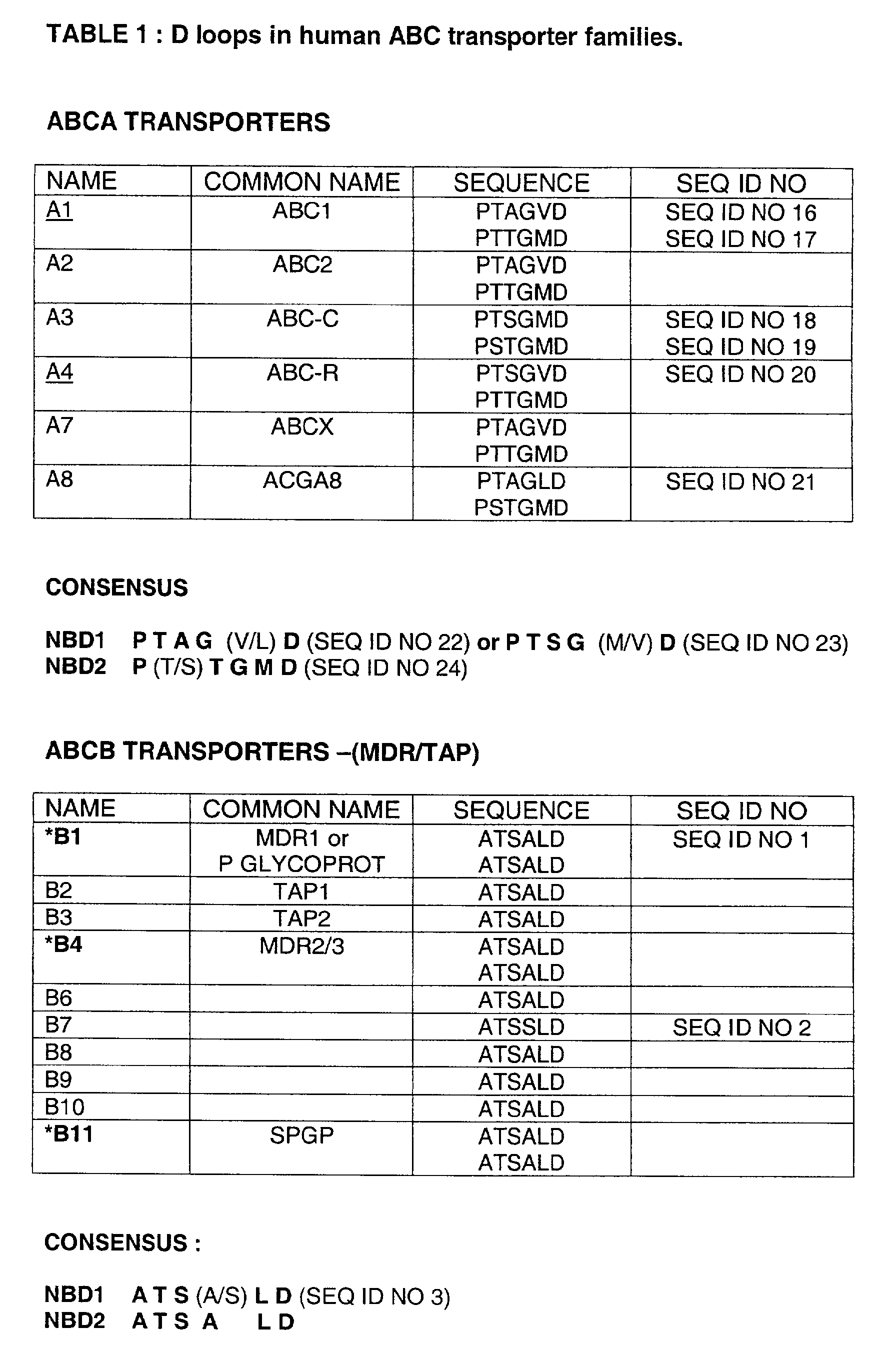

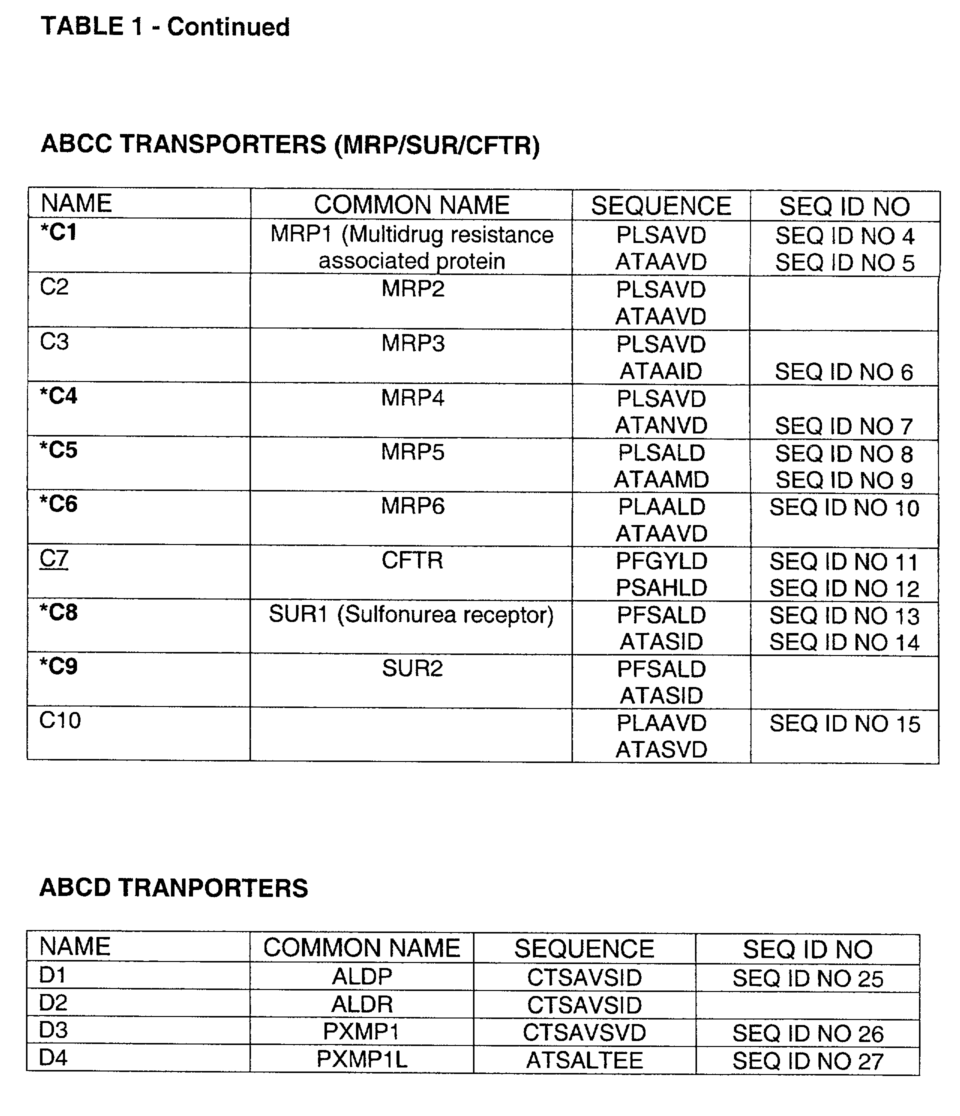

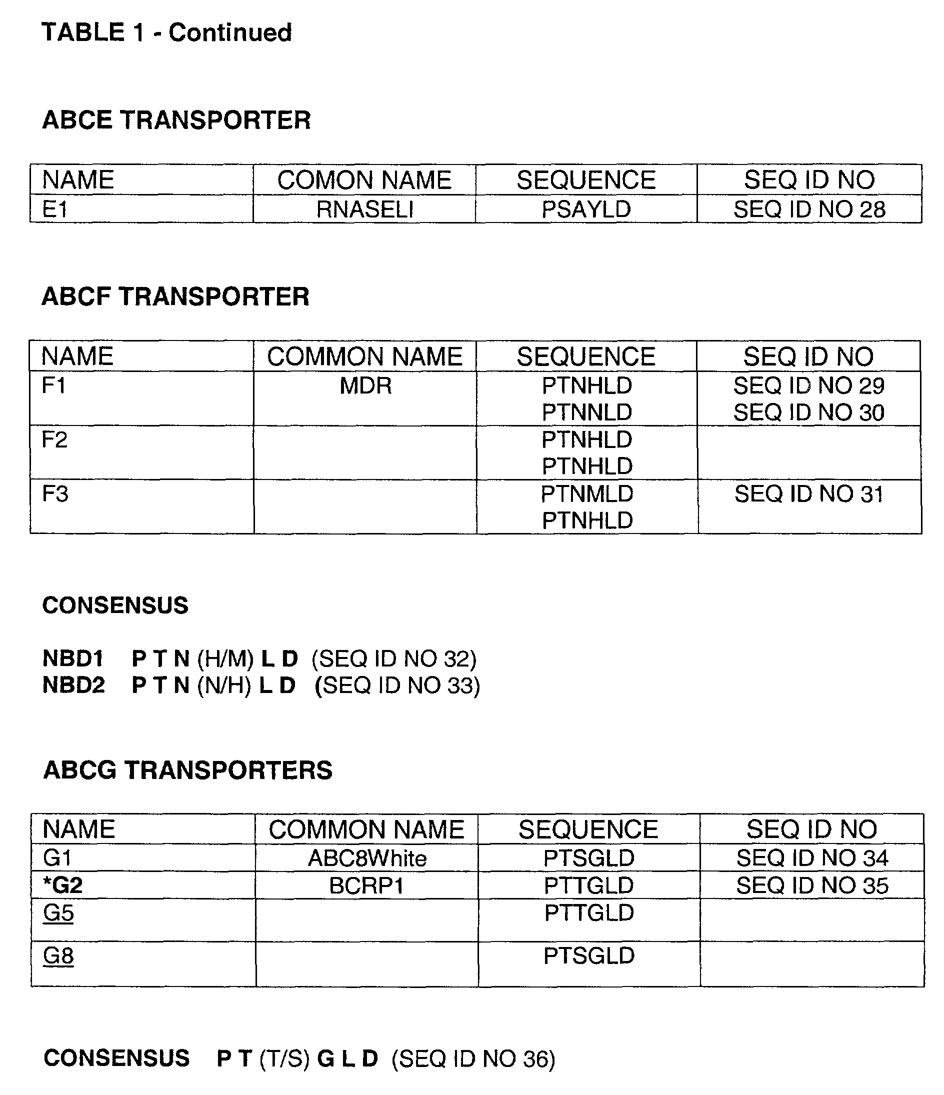

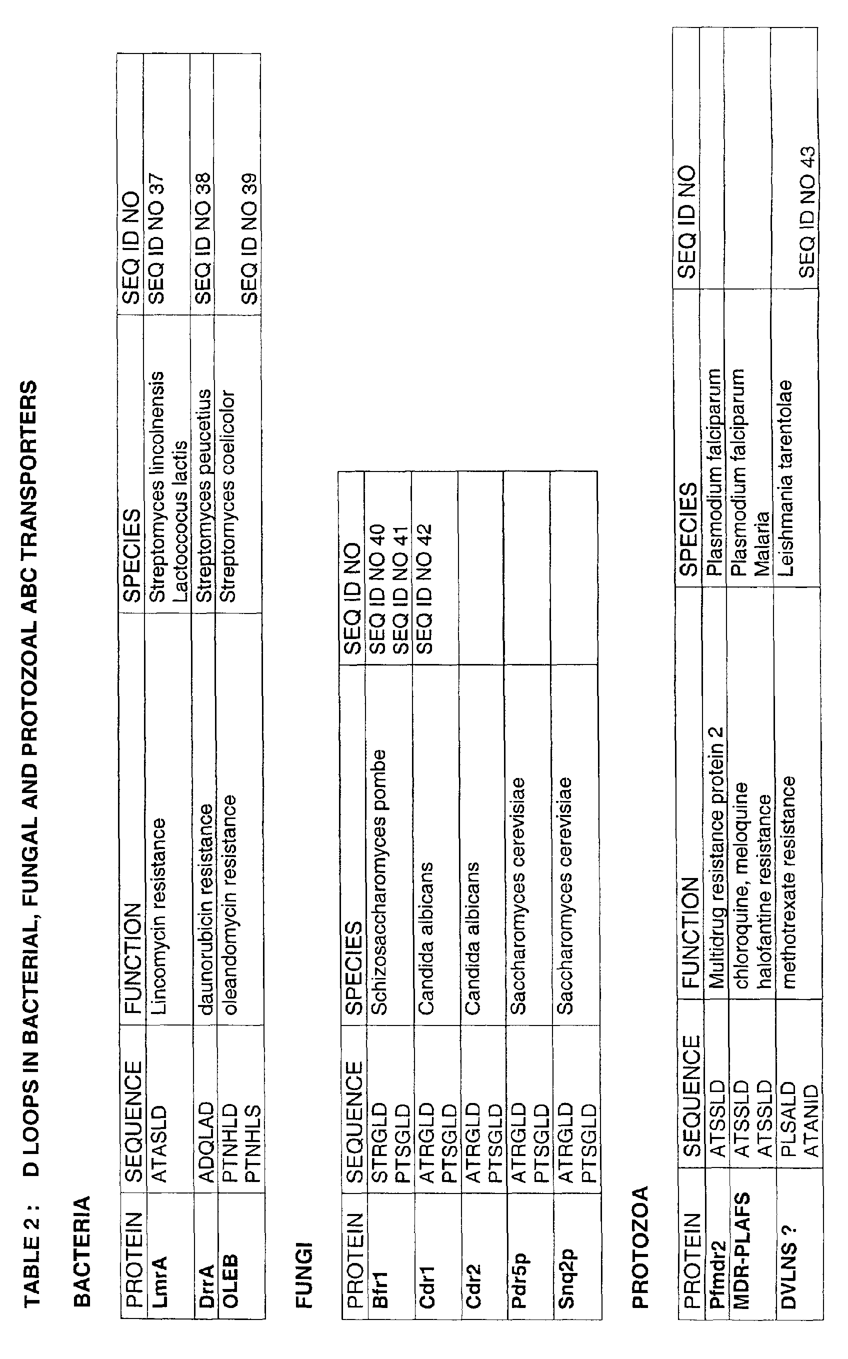

### EXAMPLES

### Example 1

### 1. Molecular modeling of the ATP binding cassette (ABC) domain.

The ABC-1 transporter contains two different nucleotide binding domains (NBD1 and NBD2), involved in the hydrolysis of ATP. The first structure of a nucleotide binding domain of an ABC transporter that has been determined by crystallization and X-ray diffraction analysis is the ATP binding cassette HisP of the *Salmonella typhimurium* histidine permease. There is significant sequence homology between HisP and both nucleotide binding domains of ABCA1, and the major structure elements of HisP are conserved The two nucleotide binding domains (NBD) of ABCA1 can therefore be modeled, based on the coordinates of the crystal structure of HisP.

The structure of the ATPase domain of the Rad50 protein, determined by crystallisation and X-ray diffraction is very similar to the HisP structure. In the presence of a non-hydrolyzable ATP analogue, the Rad50 ATPase crystallised as a dimer. This dimeric structure forms a reliable template to model dimerization of two HisP monomers and to model the putative dimeric configuration of NBD1 and NBD2 of ABCA1 and of other related ABC transporters. From these models, the actual dimer interface is be studied in closer detail and mutations impairing dimerization of the nucleotide binding domains are proposed.

### 2. Homology modeling

Sequence homology and alignments between the NBD domains of different human and bacterial ABC transporters were analyzed using BLAST, CLUSTALX and DIALIGN. Secondary structure prediction were carried out using the PHD, JPRED softwares. 3D homology calculations and model building were performed using MSI Insight 2000 software on a Silicon Graphics 02 computer, combined with SCWRL. Models are checked using Procheck, Prosa II, WHATIFF and 3D- profiles.

The built models for the two ABCA1 ATP nucleotide binding domains allow identification and characterization of the ATP binding site and dimerization site, and the design of ABCA1 mutants that lose either ATP binding, or ATPase activity or without significant structure perturbations. The models also help identify residues involved in protein-protein interactions between NBD1 and NBD2 of ABCA1.

### Example 2

### 1. Cloning of the nucleotide binding domains and transformation of E. coli.

The ABCA1 cDNA is cloned in the pcDNA3.1 plasmid under control of the T7 promotor. Because no unique restriction sites are present close to the boundaries of the NBD domains the individual nucleotide binding domains are generated by direct PCR of these regions (Taq polymerase). These PCR products are introduced in the pTrcHis TOPO plasmid (Invitrogen) that introduces directly a 6X His tail at the N-terminus and is under control of a Trc promotor. After purification, the His tag can be removed by treatment with enterokinase. Point mutations in these domains are introduced by the Quickchange mutagenesis kit (Stratagene).

Transformation of TOP10 Oneshot competent *E.coli* is performed by 'heat-shock' at 42°C during 30 seconds. The transformed *E.coli* are grown overnight at 37°C in Luria-Bertani (LB) medium. Plasmid DNA is isolated using the MiniPrep kit (Invitrogen) and checked by sequencing.

### 2. Expression and purification of the nucleotide binding domains

The optimal conditions for growth and expression are determined using standard proceduresOnce the optimal conditions for expression are determined, a larger scale culture is prepared.

The expressed NBD proteins are isolated from the *E.coli* after lysis of the bacteria by sonication. Nucleic acids are precipitated with streptomycine sulphate (10%) and removed by centrifugation. The supernatant containing the NBD-His proteins is purified by affinity chromatography using a Ni²⁺⁻agarosematrix (ProBond). The presence of the NBD protein in the eluate is verified by SDS-PAGE and Coomassie staining. The N-terminal 6xHis tag is removed by treatment with enterokinase Max.

### Example 3: ATP binding and hydrolysis after incubation with the NBD's.

### 1. ATP binding using a fluorescent labeled ATP-analogue: 2'-O-(2,4,6-trinitrophenyl) adenosine 5'-trifosfaat (TNP-ATP).

Unbound TNP nucleotides (Molecular Probes) dissolved in water display no fluorescence emission but become fluorescent once bound to nucleotide binding proteins such as ATPases. 150 µg/ml NBD1 are incubated at 25°C with 2µM TNP-ATP, 0,8 mM EDTA in a 40 mM Tris-HCI buffer (pH 7,4). Fluorescence emission is measured on an Aminco Bowman fluorescence spectrophotometer (excitation wavelength 405 nm, emission 546 nm). The concentration of the TNP-ATP is determined spectrophotometrically at 408 nm using an extinction coefficient of 2,64 x 10⁴ M⁻¹cm⁻¹.

### 2. ATP hydrolysis measured using radiolabeled ATP.

ATP-ase activity is measured as described by Gradia et al. (1997). The NBD proteins (± 100 nM) are incubated in 40 mM HEPES (pH 7,8), 75 mM NaCI, 10 mM MgCl₂, 1,75 mM DTT, 0,075 mM EDTA, 15% glycerol, 75 µg/ml acetylated BSA (Promega) and 500 mM ATP supplemented with 1 µCi γ-³²P-ATP. This mixture is incubated during 30 min at 37°C and stopped by adding an excess 10% activated charcoal dissolved in 1 mM EDTA. After removal of the charcoal by centrifugation the ³²P-orthophosphate is measured by liquid scintillation counting.

### 3. ATP hydrolysis measured by following the amount of anorganic phosphate formed using a colorimetric assay.

The EnzChek phosphate assay kit (Molecular Probes) permits the measurement of ATPase activity measurements. During the enzymatic reaction the substrate MESG (2-amino-6-mercapto-7-methylpurineribonucleoside absorbance 330 nm) is converted, in the presence of inorganic phosphate and purine nucleoside phosphorylase (PNP), to ribose-1-phosphate and 2-amino-6mercapto7-methylpurine which absorbs at 355 nm. The change in the absorption maximum of the MESG and the methylpurine allows quantification of the inorganic phosphate consumed in the reaction mixture. The NBD proteins are incubated in 50 mM HEPES (pH 7.3) containing 150 mM KCI, 10 mM MgCl2, 1 mM DTT and 400 µM ATP. The reaction mixture is then mixed with the substrates and enzymes contained in the kit according to the instructions of the manufacturer.

### Example 4: Interaction between NBD1 and NBD2

### 1. Gel-filtration

50 µg NBD1 and 50µg NBD2 in 50 mM Tris pH 7,4 buffer containing 1mM EDTA, 10 mM MgCl₂ are incubated in the presence or absence of 5mM ATP. This mixture is analyzed on a Superdex 200 column (Pharmacia) in an FPLC system (same buffer). The eluted proteins are followed at 280 nm and the elution volume is used for the calculation of the molecular weight, using a calibration curve with protein standards.

### 2. Dynamic lightscattering to detemine the size of the dimer-monomer

These measurements are performed on a DLS-700 photometer (Otsuka). Samples (NBD1 or NBD 2 only or NBD1/NBD2 mixtures) are prepared at a concentration of ± 1 mg/ml in a 0.1 M Tris-HCI (pH 7.5) containing 200 mM NaCI and 10 mM MgCl₂ at 22°C. ATP at concentrations up to 10 mM can be added to the mixture to promote dimerization. Scattering intensity is recorded for 5-10 min.

### 3. Native gradient polyacrylamide gelelectroforese.

4-20% Tris-HCI Ready gels (Biorad) are run in 90 mM Tris-HCI, 2 mM EDTA, 30 mM NaN₃ en 80 mM boric acid. 15 µg NBD protein (either NBD1, NBD2 or the mixture) are incubated with 0,2 µM ATP. After equilibration of the samples they are run on the gel for 1 hour at 70V, and then overnight (with cooling) at 120 V. The gels are stained with Coomassie Brilliant Blue and destained in 40% methanol/10% acetic acid

### 4. Solid phase assay for evaluating of the binding of NBD1 and NBD2

The NBD1 protein is coated at the surface of polystyrene microtiterplates and the excess protein is washed (PBS (pH 7.5- Tween 20 0.1%) and remaining binding sites on the plate are blocked with casein (0.1% in PBS pH 7.5). The NBD2 protein is added in increasing amounts to the coated protein in the presence or absence of ATP in a PBS-0.1% casein buffer. Bound NBD2 (containing the N-terminal His tag ) is detected using an anti-His monoclonal antibody () followed by incuabtion with an anti-mouse IgG peroxidase labeled antibody. Alternatively NBD2 directly labelled with peroxidase or alkaline phosphatase can be used for detection. The amount of bound enzyme is revealed using chromogenic or fluorometric substrates. This type of assay can be adapted for evaluation in plasmon resonance technique (BIACORE system (Amersham)), where either NBD1 or NBD2 is adsorbed on the surface.

### 5. Inhibition of dimerization using competitor peptides or peptides or small compounds blocking the D-loop

The assays mentioned above are performed in the presence of synthetic peptides corresponding to the D-loop (competition) or in the presence of peptides or small compounds that sterically block access to the D-loop (cf. modeling). Peptides are prepared using standard protocols and are added to the incubation mixtures at varying concentrations.

### 6. Evaluation of mutant NBD proteins

As described in the modeling section mutations in the NBD proteins will be proposed. Special emphasis will be given to the mutations aimed at influencing the dimer interface properties of these proteins. The ATPase activity and dimerization properties of the mutant NBDs will be tested as described above.

### REFERENCES

Bodzioch M, Orso E, Klucken J, Langmann T, Bottcher A, Diederich W, Drobnik W, Barlage S, Buchler C, Porsch-Ozcurumez M, Kaminski WE, Hahmann HW, Oette K, Rothe G, Aslanidis C, Lackner KJ, Schmitz G: The gene encoding ATP-binding cassette transporter 1 is mutated in Tangier disease. Nat.Genet. 22:347,1999.
Borst P, Zelcer N, van Helvoort A: ABC transporters in lipid transport. Biochim.Biophys.Acta 2000.Jun.26.;1486.(1.):128.-44. 1486:128.
Blalock, JE: Complementarity of peptides specified by 'sense' and 'antisense' strands of DNA. Trends Biotechnol. 8(6): 140-144, 1990.
Brickner M, Chmielewski J: Inhibiting the dimeric restriction endonuclease EcoRI using interfacial helical peptides. Chem.Biol. 5:339, 1998.
Brooks-Wilson A, Marcil M, Clee SM, Zhang LH, Roomp K, van Dam M, Yu L, Brewer C, Collins JA, Molhuizen HO, Loubser O, Ouelette BF, Fichter K, Ashbourne-Excoffon KJ, Sensen CW, Scherer S, Mott S, Denis M, Martindale D, Frohlich J, Morgan K, Koop B, Pimstone S, Kastelein JJ, Hayden MR: Mutations in ABC1 in Tangier disease and familial high-density lipoprotein deficiency Nat.Genet. 22:336, 1999.
Frieden C: Protein-protein interaction and enzymatic activity. Annu.Rev.Biochem. 40:653, 1971.
Gibson W: Structure and assembly of the virion. Intervirology 39:389, 1996.
Gradia, S., Acharya, S., Fishel, R. The human mismatch recognition complex hMSH2-hMSH6 functions as a novel molecular switch. Cell, 91: 995,1997.
Holland IB, Blight MA: ABC-ATPases, adaptable energy generators fuelling transmembrane movement of a variety of molecules in organisms from bacteria to humans. J.Mol.Biol. 293:381, 1999.
Higgins CF: ABC transporters: from microorganisms to man. Annu.Rev.Cell Biol. 8:67, 1992.
Hipfner DR, Deeley RG, Cole SP: Structural, mechanistic and clinical aspects of MRP1. Biochim.Biophys.Acta 1461:359, 1999.
Hopfner KP, Karcher A, Shin DS, Craig L, Arthur LM, Carney JP, Tainer JA: Structural biology of Rad50 ATPase: ATP-driven conformational control in DNA double-strand break repair and the ABC-ATPase superfamily. Cell 2000.Jun.23.;101.(7.):789.-800. 101:789.
Hung LW, Wang IX, Nikaido K, Liu PQ, Ames GF, Kim SH: Crystal structure of the ATP-binding subunit of an ABC transporter. Nature 396:703, 1998.
Jones S, Thornton JM: Principles of protein-protein interactions. Proc.Natl.Acad.Sci.U.S.A. 93:13, 1996.
Klein I, Sarkadi B, Varad A. An inventory of human ABC proteins. Biochim Biophys Acta 1461(2): 237-262, 1999.
Roubos E. Sense-antisense complementarity of hormone-receptor interaction sites. Trends Biotechnol 8(10): 279-281, 1990.
Rust S, Rosier M, Funke H, Real J, Amoura Z, Piette JC, Deleuze JF, Brewer HB, Duverger N, Denefle P, Assmann G: Tangier disease is caused by mutations in the gene encoding ATP-binding cassette transporter 1 Nat.Genet. 22:352, 1999
Sheppard DN, Welsh MJ: Structure and function of the CFTR chloride channel. Physiol.Rev. 79:S23, 1999
Zielenski J, Tsui LC: Cystic fibrosis: genotypic and phenotypic variations. Annu.Rev.Genet. 29:777, 1995
Zutshi R, Franciskovich J, Shultz M, Schweitzer B, Bishop P, Wilson M, Chmielewski J. Targeting the dimerization interface of HIV-1 protease: inhibition with Cross-linked Interfacial peptides. J AM Chem Soc, 119: 4841-4845, 1997
Zutshi R, Brickner M, Chmielewski J: Inhibiting the assembly of protein-protein interfaces. Curr.Opin.Chem.Biol. 2:62, 1998

## Claims

1. A method for selectively modulating the activity of ABC transporters by influencing the dimerization of the nucleotide binding domains comprising the use of:
a) a polypeptide consisting of 5 to 50 amino acids comprising the D loop sequence of an ABC transporter,
b) a polypeptide consisting of the D loop sequence of an ABC transporter,
c) a peptide mimetic of any of the polypeptides of a) or b), or
d) an antisense peptide of the polypeptdes of a) or b).

2. A method for selectively modulating the activity of ABC transporters by influencing the dimerization of the nucleotide binding domains according to claim 1 comprising the use of:
a) a polypeptide consisting of 5 to 50 amino acids comprising the amino acid sequence represented in any of SEQ ID NOs 1 to 43,
b) a polypeptide consisting of the amino acid sequence as represented in any of SEQ ID NOs 1 to 43 or a functional homologue thereof,
c) a peptide mimetic of any of the polypeptides of a) or b), or
d) an antisense peptide of the polypeptides of a) or b).

3. A method for selectively modulating the activity of an ABC transporter according to claim 1 or 2, wherein said ABC transporter belongs to the group of multidrug transporter/P-glycoproteins comprising the use of:
a) a polypeptide consisting of 5 to 50 amino acids comprising the amino acid sequence represented in any of SEQ ID NOs 1 to 3,
b) a polypeptide consisting of the amino acid sequence represented in any of SEQ ID NOs 1 to 3 or a functional homologue thereof,
c) a peptide mimetic of any of the polypeptides of a) or b), or
d) an antisense peptide of the peptide of a) or b).

4. A method for selectively modulating the activity of an ABC transporter according to claim 1 or 2, wherein said ABC transporter belongs to the group of the multidrug resistance associated proteins comprising the use of:
a) a polypeptide consisting of 5 to 50 amino acids comprising the amino acid sequence represented in any of SEQ ID NOs 4 to 15,
b) a polypeptide consisting of the amino acid sequence represented in any of SEQ ID NOs 4 to 15, or a functional homologue thereof,
c) a peptide mimetic of any of the polypeptides of a) or b), or
d) an antisense peptide of the peptide of a) or b).

5. A method for selectively modulating the activity of an ABC transporter according to claim 1 or 2, wherein said ABC transporter is a bacterial transporter comprising the use of:
a) a polypeptide consisting of 5 to 50 amino acids comprising the amino acid sequence represented in any of SEQ ID NOs 29, 37, 38 or 39,
b) a polypeptide consisting of the amino acid sequence represented in any of SEQ ID NOs 29, 37, 38 or 39, or a functional homologue thereof,
c) a peptide mimetic of any of the polypeptides of a) or b), or,
d) an antisense peptide of the peptide of a) or b).

6. A method for selectively modulating the activity of an ABC transporter according to claim 1 or 2, wherein said ABC transporter is a fungal transporter comprising the use of:
a) a polypeptide consisting of 5 to 50 amino acids comprising the amino acid sequence represented in any of SEQ ID NOs 40, 41 or 42,
b) a polypeptide consisting of the amino acid sequence represented in any of SEQ ID NOs 40, 41 or 42, or a functional homologue thereof,
c) a peptide mimetic of any of the polypeptides of a) or b), or
d) an antisense peptide of the peptide of a) or b).

7. A method for selectively modulating the activity of an ABC transporter according to claim 1 or 2, wherein said ABC transporter is a protozoal transporter comprising the use of:
a) a polypeptide consisting of 5 to 50 amino acids comprising the amino acid sequence represented in any of SEQ ID NOs 2, 8 or 43,
b) a polypeptide consisting of the amino acid sequence represented in any of SEQ ID NOs 2, 8, or 43, or a functional homologue thereof,
c) a peptide mimetic of any of the polypeptides of a) or b), or,
d) an antisense peptide of the peptide of a) or b).

8. A method according to any of claims 5 to 7 wherein said ABC transporter is involved in bacterial, fungal or protozoal infection of a mammal.

9. A method according to any of claims 5 to 7 wherein said ABC transporter is involved in the induction of resistance to antibiotics or drugs in a mammal.

10. Method for preventing, treating or alleviating diseases diseases associated with the functionality of a human ABC-transporter comprising the use of:
a) a polypeptide consisting of 5 to 50 amino acids comprising the amino acid sequence represented in any of SEQ ID NOs 1 to 36,
b) a polypeptide consisting of the amino acid sequence represented in any of SEQ ID NOs 1 to 36, or a functional homologue thereof,
c) a peptide mimetic of any of the polypeptides of a) or b), or,
d) an antisense peptide of the peptide of a) or b).

11. Method for preventing, treating or alleviating diseases related with bacterial infections comprising the use of:
a) a polypeptide consisting of 5 to 50 amino acids comprising the amino acid sequence represented in any of SEQ ID NOs 29, 37, 38 or 39,
b) a polypeptide consisting of the amino acid sequence represented in any of SEQ ID NOs 29, 37, 38 or 39, or a functional homologue thereof,
c) a peptide mimetic of any of the polypeptides of a) or b), or,
d) an antisense peptide of the peptide of a) or b).

12. Method for preventing, treating or alleviating diseases related with fungal infections comprising the use of:
a) a polypeptide consisting of 5 to 50 amino acids comprising the amino acid sequence represented in any of SEQ ID NOs 40 to 42,
b) a polypeptide consisting of the amino acid sequence represented in any of SEQ ID NOs 40 to 42, or a functional homologue thereof,
c) a peptide mimetic of any of the polypeptides of a) or b), or,
d) an antisense peptide of the peptide of a) or b).

13. Method for preventing, treating or alleviating diseases related with protozoal infections comprising the use of:
a) a polypeptide consisting of 5 to 50 amino acids comprising the amino acid sequence represented in any of SEQ ID NOs 2, 8 or 43,
b) a polypeptide consisting of the amino acid sequence represented in any of SEQ ID NOs 2, 8, or 43, or a functional homologue thereof,
c) a peptide mimetic of any of the polypeptides of a) or b), or,
d) an antisense peptide of the peptide of a) or b).

14. A method for identifying compounds which selectively bind to or selectively modulate the properties of ABC transporters, which method comprises:
a) contacting a compound to be tested with a polypeptide as defined in any of claims 1 to 7, or with a polypeptide corresponding to the D loop of an ABC transporter,
b) detecting a diminution or inhibition of the activity of said ABC transporter, and,
c) identifying said compound.

15. A method for identifying compounds which selectively bind to or selectively modulate the properties of ABC transporters, which method comprises:
a) providing a yeast two-hybrid system wherein the nucleotide binding domains NBD1 and NBD2 of an ABC transporter are expressed, or
b) providing a mammalian expression system wherein the nucleotide binding domains NBD1 and NBD2 of an ABC transporter are expressed, or
c) providing a bacterial expression system wherein the nucleotide binding domains NBD1 and NBD2 of an ABC transporter are expressed, and,
d) interacting said compound with the complex formed by the expressed polypeptides as defined in any of a) to c),
e) inferring from the interaction between said compound and one of the nucleotide binding domains a modulation of the properties of said ABC transporter, and,
f) identifying said compound.

16. An isolated nucleic acid encoding a polypeptide comprising an ABC transporter D-loop as defined in claim 1 or 2.

17. A polypeptide encodable by a nucleic acid of claim 16.

18. A cellular host transformed with a nucleic acid encoding at least one nucleotide binding domain of an ABC transporter protein or a nucleic acid comprising a nucleic acid according to claim 16, said nucleic acid in an expressible format for use in a method of claim 15.

19. A pharmaceutical composition comprising at least one polypeptide of claim 1 or 2.

20. A compound obtainable by any of the methods of claims 14 or 15.

21. Use of a polypeptide as defined in claim 1 or 2 as a medicament.

22. Use of a compound according to claim 20 as a medicament.

23. Use of a polypeptide as defined in claim 17 or a compound obtainable by any of the methods of claims 14 or 15 for preventing, treating or alleviating diseases associated with the functionality of an ABC-transporter.

24. Use of a polypeptide as defined in claim 3 or 4 or a compound obtainable by any of the methods of claims 14 or 15 for treatment of cancer.

25. Use of a polypeptide according to claim 24 in combination with chemotherapy.

26. Use of a polypeptide as defined in claim 3 or 4 or a compound obtainable by any of the methods of claims 14 or 15 for the preparation of a medicine for treating cancer.

27. Use of a polypeptide as defined in claim 3 or 4 or a compound obtainable by any of the methods of claims 14 or 15 for treating resistance to drugs in a mammal.

28. Use of a polypeptide as defined in claim 3 or 4 or a compound obtainable by any of the methods of claims 14 or 15 for the preparation of a medicament for preventing, treating or alleviating diseases associated with drug resistance in a mammal.

29. Use of a molecule as defined in claim 5 or a compound obtainable by any of the methods of claims 14 or 15 for preventing, treating or alleviating diseases associated with bacterial infections.

30. Use of a molecule as defined in claim 5 or a compound obtainable by any of the methods of claims 14 or 15 for the preparation of a medicament for preventing, treating or alleviating diseases associated with bacterial infections.

31. Use of a molecule as defined in claim 5 or a compound obtainable by any of the methods of claims 14 or 15 for treating resistance to antibiotics in a mammal.

32. Use of a molecule as defined in claim 5 or a compound obtainable by any of the methods of claims 14 or 15 for the preparation of a medicament for treating antibiotic resistance in a mammal.

33. Use of a molecule as defined in claim 5 or a compound obtainable by any of the methods of claims 14 or 15 as an anti-bacterial agent.

34. Use of a molecule as defined in claim 6 or a compound obtainable by any of the methods of claims 14 or 15 for preventing, treating or alleviating diseases associated with fungal infections.

35. Use of a molecule as defined in claim 6 or a compound obtainable by any of the methods of claims 14 or 15 for the preparation of a medicament for preventing, treating or alleviating diseases associated with fungal infections.

36. Use of a molecule as defined in claim 6 or a compound obtainable by any of the methods of claims 14 or 15 as a fungicide or anti- fungal agent.

37. Use of a molecule as defined in claim 7 or a compound obtainable by any of the methods of claims 14 or 15 for preventing, treating or alleviating diseases associated with protozoal infections.

38. Use of a molecule as defined in claim 7 or a compound obtainable by any of the methods of claims 14 or 15 for the preparation of a medicament for preventing, treating or alleviating diseases associated with protozoal infections.

39. Use of a molecule as defined in claim 7 or a compound obtainable by any of the methods of claims 14 or 15 as a fungicide or anti- fungal agent.


SEQUENCE LISTING

<110> UNIVERSITEIT GENT

<120> Modulation of ATP-binding cassette transporter activity

<130> UG-013-EP

<140> EP 00870316.7
<141> 2000-12-21

<160> 92

<170> PatentIn Ver. 2.1

<210> 1
<211> 6
<212> PRT
<213> Homo sapiens

<400> 1
Ala Thr Ser Ala Leu Asp
  1               5

<210> 2
<211> 6
<212> PRT
<213> Homo sapiens

<400> 2
Ala Thr Ser Ser Leu Asp
  1               5

<210> 3
<211> 6
<212> PRT
<213> Homo sapiens

<220>
<221> VARIANT
<222> (4)
<223> Xaa = Ala or Ser

<400> 3

Ala Thr Ser Xaa Leu Asp
1               5

<210> 4
<211> 6
<212> PRT
<213> Homo sapiens

<400> 4
Pro Leu Ser Ala Val Asp
1               5

<210> 5
<211> 6
<212> PRT
<213> Homo sapiens

<400> 5
Ala Thr Ala Ala Val Asp
1               5

<210> 6
<211> 6
<212> PRT
<213> Homo sapiens

<400> 6
Ala Thr Ala Ala Ile Asp
1               5

<210> 7
<211> 6
<212> PRT
<213> Homo sapiens

<400> 7
Ala Thr Ala Asn Val Asp
1               5

<210> 8
<211> 6
<212> PRT
<213> Homo sapiens

<400> 8
Pro Leu Ser Ala Leu Asp
1               5

<210> 9
<211> 6
<212> PRT
<213> Homo sapiens

<400> 9
Ala Thr Ala Ala Met Asp
1               5

<210> 10
<211> 6
<212> PRT
<213> Homo sapiens

<400> 10
Pro Leu Ala Ala Leu Asp
1               5

<210> 11
<211> 6
<212> PRT
<213> Homo sapiens

<400> 11
Pro Phe Gly Tyr Leu Asp
1               5

<210> 12
<211> 6
<212> PRT

<213> Homo sapiens

<400> 12
Pro Ser Ala His Leu Asp
1               5

<210> 13
<211> 6
<212> PRT
<213> Homo sapiens

<400> 13
Pro Phe Ser Ala Leu Asp
1               5

<210> 14
<211> 6
<212> PRT
<213> Homo sapiens

<400> 14
Ala Thr Ala Ser Ile Asp
1               5

<210> 15
<211> 6
<212> PRT
<213> Homo sapiens

<400> 15
Pro Leu Ala Ala Val Asp
1               5

<210> 16
<211> 6
<212> PRT
<213> Homo sapiens

<400> 16
Pro Thr Ala Gly Val Asp

1                   5

<210> 17
<211> 6
<212> PRT
<213> Homo sapiens

<400> 17
Pro Thr Thr Gly Met Asp
1                   5

<210> 18
<211> 6
<212> PRT
<213> Homo sapiens

<400> 18
Pro Thr Ser Gly Met Asp
1                   5

<210> 19
<211> 6
<212> PRT
<213> Homo sapiens

<400> 19
Pro Ser Thr Gly Met Asp
1                   5

<210> 20
<211> 6
<212> PRT
<213> Homo sapiens

<400> 20
Pro Thr Ser Gly Val Asp
1                   5

<210> 21
<211> 6
<212> PRT
<213> Homo sapiens

<400> 21
Pro Thr Ala Gly Leu Asp
1               5

<210> 22
<211> 6
<212> PRT
<213> Homo sapiens

<220>
<221> VARIANT
<222> (5)
<223> Xaa = Val or Leu

<400> 22
Pro Thr Ala Gly Xaa Asp
1               5

<210> 23
<211> 6
<212> PRT
<213> Homo sapiens

<220>
<221> VARIANT
<222> (5)
<223> Xaa = Met or Val

<400> 23
Pro Thr Ser Gly Xaa Asp
1               5

<210> 24
<211> 6
<212> PRT
<213> Homo sapiens

<220>
<221> VARIANT
<222> (2)
<223> Xaa = Thr or Ser

<400> 24
Pro Xaa Thr Gly Met Asp
1               5

<210> 25
<211> 8
<212> PRT
<213> Homo sapiens

<400> 25
Cys Thr Ser Ala Val Ser Ile Asp
1               5

<210> 26
<211> 8
<212> PRT
<213> Homo sapiens

<400> 26
Cys Thr Ser Ala Val Ser Val Asp
1               5

<210> 27
<211> 8
<212> PRT
<213> Homo sapiens

<400> 27
Ala Thr Ser Ala Leu Thr Glu Glu
1               5

<210> 28
<211> 6
<212> PRT
<213> Homo sapiens

<400> 28
Pro Ser Ala Tyr Leu Asp
1               5

<210> 29
<211> 6
<212> PRT
<213> Homo sapiens

<400> 29
Pro Thr Asn His Leu Asp
1               5

<210> 30
<211> 6
<212> PRT
<213> Homo sapiens

<400> 30
Pro Thr Asn Asn Leu Asp
1               5

<210> 31
<211> 6
<212> PRT
<213> Homo sapiens

<400> 31
Pro Thr Asn Met Leu Asp
1               5

<210> 32
<211> 6
<212> PRT
<213> Homo sapiens

<220>
<221> VARIANT
<222> (4)

<223> Xaa = His or Met

<400> 32
Pro Thr Asn Xaa Leu Asp
 1               5

<210> 33
<211> 6
<212> PRT
<213> Homo sapiens

<220>
<221> VARIANT
<222> (4)
<223> Xaa = Asn or His

<400> 33
Pro Thr Asn Xaa Leu Asp
 1               5

<210> 34
<211> 6
<212> PRT
<213> Homo sapiens

<400> 34
Pro Thr Ser Gly Leu Asp
 1               5

<210> 35
<211> 6
<212> PRT
<213> Homo sapiens

<400> 35
Pro Thr Thr Gly Leu Asp
 1               5

<210> 36
<211> 6

<212> PRT
<213> Homo sapiens

<220>
<221> VARIANT
<222> (3)
<223> Xaa = Thr or Ser

<400> 36
Pro Thr Xaa Gly Leu Asp
 1               5

<210> 37
<211> 6
<212> PRT
<213> Streptomyces lincolnensis

<400> 37
Ala Thr Ala Ser Leu Asp
 1               5

<210> 38
<211> 6
<212> PRT
<213> Streptomyces peucetius

<400> 38
Ala Asp Gln Leu Ala Asp
 1               5

<210> 39
<211> 6
<212> PRT
<213> Streptomyces coelicolor

<400> 39
Pro Thr Asn His Leu Ser
 1               5

<210> 40

<211> 6
<212> PRT
<213> Schizosaccharomyces pombe

<400> 40
Ser Thr Arg Gly Leu Asp
1               5

<210> 41
<211> 6
<212> PRT
<213> Schizosaccharomyces pombe

<400> 41
Pro Thr Ser Gly Leu Asp
1               5

<210> 42
<211> 6
<212> PRT
<213> Candida albicans

<400> 42
Ala Thr Arg Gly Leu Asp
1               5

<210> 43
<211> 6
<212> PRT
<213> Leishmania tarentolae

<400> 43
Ala Thr Ala Asn Ile Asp
1               5

<210> 44
<211> 2261
<212> PRT
<213> Homo sapiens

<400> 44
Met Ala Cys Trp Pro Gln Leu Arg Leu Leu Leu Trp Lys Asn Leu Thr
1               5                   10                  15

Phe Arg Arg Arg Gln Thr Cys Gln Leu Leu Leu Glu Val Ala Trp Pro
            20                  25                  30

Leu Phe Ile Phe Leu Ile Leu Ile Ser Val Arg Leu Ser Tyr Pro Pro
        35                  40                  45

Tyr Glu Gln His Glu Cys His Phe Pro Asn Lys Ala Met Pro Ser Ala
    50                  55                  60

Gly Thr Leu Pro Trp Val Gln Gly Ile Ile Cys Asn Ala Asn Asn Pro
65                  70                  75                  80

Cys Phe Arg Tyr Pro Thr Pro Gly Glu Ala Pro Gly Val Val Gly Asn
                85                  90                  95

Phe Asn Lys Ser Ile Val Ala Arg Leu Phe Ser Asp Ala Arg Arg Leu
            100                 105                 110

Leu Leu Tyr Ser Gln Lys Asp Thr Ser Met Lys Asp Met Arg Lys Val
        115                 120                 125

Leu Arg Thr Leu Gln Gln Ile Lys Lys Ser Ser Ser Asn Leu Lys Leu
    130                 135                 140

Gln Asp Phe Leu Val Asp Asn Glu Thr Phe Ser Gly Phe Leu Tyr His
145                 150                 155                 160

Asn Leu Ser Leu Pro Lys Ser Thr Val Asp Lys Met Leu Arg Ala Asp
                165                 170                 175

Val Ile Leu His Lys Val Phe Leu Gln Gly Tyr Gln Leu His Leu Thr
            180                 185                 190

Ser Leu Cys Asn Gly Ser Lys Ser Glu Glu Met Ile Gln Leu Gly Asp
        195                 200                 205

Gln Glu Val Ser Glu Leu Cys Gly Leu Pro Arg Glu Lys Leu Ala Ala
    210                 215                 220

Ala Glu Arg Val Leu Arg Ser Asn Met Asp Ile Leu Lys Pro Ile Leu
225                 230                 235                 240

Arg Thr Leu Asn Ser Thr Ser Pro Phe Pro Ser Lys Glu Leu Ala Glu
                245                 250                 255

Ala Thr Lys Thr Leu Leu His Ser Leu Gly Thr Leu Ala Gln Glu Leu
            260                 265                 270

Phe Ser Met Arg Ser Trp Ser Asp Met Arg Gln Glu Val Met Phe Leu
        275                 280                 285

Thr Asn Val Asn Ser Ser Ser Ser Ser Thr Gln Ile Tyr Gln Ala Val
    290                 295                 300

Ser Arg Ile Val Cys Gly His Pro Glu Gly Gly Gly Leu Lys Ile Lys
305                 310                 315                 320

Ser Leu Asn Trp Tyr Glu Asp Asn Asn Tyr Lys Ala Leu Phe Gly Gly
                325                 330                 335

Asn Gly Thr Glu Glu Asp Ala Glu Thr Phe Tyr Asp Asn Ser Thr Thr
            340                 345                 350

Pro Tyr Cys Asn Asp Leu Met Lys Asn Leu Glu Ser Ser Pro Leu Ser
        355                 360                 365

Arg Ile Ile Trp Lys Ala Leu Lys Pro Leu Leu Val Gly Lys Ile Leu
    370                 375                 380

Tyr Thr Pro Asp Thr Pro Ala Thr Arg Gln Val Met Ala Glu Val Asn
385                 390                 395                 400

Lys Thr Phe Gln Glu Leu Ala Val Phe His Asp Leu Glu Gly Met Trp
                405                 410                 415

Glu Glu Leu Ser Pro Lys Ile Trp Thr Phe Met Glu Asn Ser Gln Glu
            420                 425                 430

Met Asp Leu Val Arg Met Leu Leu Asp Ser Arg Asp Asn Asp His Phe
        435                 440                 445

Trp Glu Gln Gln Leu Asp Gly Leu Asp Trp Thr Ala Gln Asp Ile Val
    450                 455                 460

Ala Phe Leu Ala Lys His Pro Glu Asp Val Gln Ser Ser Asn Gly Ser
465                 470                 475                 480

Val Tyr Thr Trp Arg Glu Ala Phe Asn Glu Thr Asn Gln Ala Ile Arg
                485                 490                 495

Thr Ile Ser Arg Phe Met Glu Cys Val Asn Leu Asn Lys Leu Glu Pro
            500                 505                 510

Ile Ala Thr Glu Val Trp Leu Ile Asn Lys Ser Met Glu Leu Leu Asp
        515                 520                 525

Glu Arg Lys Phe Trp Ala Gly Ile Val Phe Thr Gly Ile Thr Pro Gly
    530                 535                 540

Ser Ile Glu Leu Pro His His Val Lys Tyr Lys Ile Arg Met Asp Ile
545                 550                 555                 560

Asp Asn Val Glu Arg Thr Asn Lys Ile Lys Asp Gly Tyr Trp Asp Pro
                565                 570                 575

Gly Pro Arg Ala Asp Pro Phe Glu Asp Met Arg Tyr Val Trp Gly Gly
            580                 585                 590

Phe Ala Tyr Leu Gln Asp Val Val Glu Gln Ala Ile Ile Arg Val Leu
        595                 600                 605

Thr Gly Thr Glu Lys Lys Thr Gly Val Tyr Met Gln Gln Met Pro Tyr
    610                 615                 620

Pro Cys Tyr Val Asp Asp Ile Phe Leu Arg Val Met Ser Arg Ser Met
625                 630                 635                 640

Pro Leu Phe Met Thr Leu Ala Trp Ile Tyr Ser Val Ala Val Ile Ile
                645                 650                 655

Lys Gly Ile Val Tyr Glu Lys Glu Ala Arg Leu Lys Glu Thr Met Arg
            660                 665                 670

Ile Met Gly Leu Asp Asn Ser Ile Leu Trp Phe Ser Trp Phe Ile Ser
        675                 680                 685

Ser Leu Ile Pro Leu Leu Val Ser Ala Gly Leu Leu Val Val Ile Leu
    690                 695                 700

Lys Leu Gly Asn Leu Leu Pro Tyr Ser Asp Pro Ser Val Val Phe Val
705                 710                 715                 720

Phe Leu Ser Val Phe Ala Val Val Thr Ile Leu Gln Cys Phe Leu Ile
                725                 730                 735

Ser Thr Leu Phe Ser Arg Ala Asn Leu Ala Ala Ala Cys Gly Gly Ile
            740                 745                 750

Ile Tyr Phe Thr Leu Tyr Leu Pro Tyr Val Leu Cys Val Ala Trp Gln
        755                 760                 765

Asp Tyr Val Gly Phe Thr Leu Lys Ile Phe Ala Ser Leu Leu Ser Pro
    770                 775                 780

Val Ala Phe Gly Phe Gly Cys Glu Tyr Phe Ala Leu Phe Glu Glu Gln
785                 790                 795                 800

Gly Ile Gly Val Gln Trp Asp Asn Leu Phe Glu Ser Pro Val Glu Glu
                805                 810                 815

Asp Gly Phe Asn Leu Thr Thr Ser Val Ser Met Met Leu Phe Asp Thr
            820                 825                 830

Phe Leu Tyr Gly Val Met Thr Trp Tyr Ile Glu Ala Val Phe Pro Gly
        835                 840                 845

Gln Tyr Gly Ile Pro Arg Pro Trp Tyr Phe Pro Cys Thr Lys Ser Tyr
    850                 855                 860

Trp Phe Gly Glu Glu Ser Asp Glu Lys Ser His Pro Gly Ser Asn Gln
865                 870                 875                 880

Lys Arg Ile Ser Glu Ile Cys Met Glu Glu Glu Pro Thr His Leu Lys
                885                 890                 895

Leu Gly Val Ser Ile Gln Asn Leu Val Lys Val Tyr Arg Asp Gly Met
            900                 905                 910

Lys Val Ala Val Asp Gly Leu Ala Leu Asn Phe Tyr Glu Gly Gln Ile
        915                 920                 925

Thr Ser Phe Leu Gly His Asn Gly Ala Gly Lys Thr Thr Thr Met Ser
    930                 935                 940

Ile Leu Thr Gly Leu Phe Pro Pro Thr Ser Gly Thr Ala Tyr Ile Leu
945                 950                 955                 960

Gly Lys Asp Ile Arg Ser Glu Met Ser Thr Ile Arg Gln Asn Leu Gly
                965                 970                 975

Val Cys Pro Gln His Asn Val Leu Phe Asp Met Leu Thr Val Glu Glu
            980                 985                 990

His Ile Trp Phe Tyr Ala Arg Leu Lys Gly Leu Ser Glu Lys His Val
        995                 1000                1005

Lys Ala Glu Met Glu Gln Met Ala Leu Asp Val Gly Leu Pro Ser Ser
    1010                1015                1020

Lys Leu Lys Ser Lys Thr Ser Gln Leu Ser Gly Gly Met Gln Arg Lys
1025                1030                1035                1040

Leu Ser Val Ala Leu Ala Phe Val Gly Gly Ser Lys Val Val Ile Leu
                1045                1050                1055

Asp Glu Pro Thr Ala Gly Val Asp Pro Tyr Ser Arg Arg Gly Ile Trp
            1060                1065                1070

Glu Leu Leu Leu Lys Tyr Arg Gln Gly Arg Thr Ile Ile Leu Ser Thr
        1075                1080                1085

His His Met Asp Glu Ala Asp Val Leu Gly Asp Arg Ile Ala Ile Ile
    1090                1095                1100

Ser His Gly Lys Leu Cys Cys Val Gly Ser Ser Leu Phe Leu Lys Asn
1105                1110                1115                1120

Gln Leu Gly Thr Gly Tyr Tyr Leu Thr Leu Val Lys Lys Asp Val Glu
                1125                1130                1135

Ser Ser Leu Ser Ser Cys Arg Asn Ser Ser Ser Thr Val Ser Tyr Leu
            1140                1145                1150

Lys Lys Glu Asp Ser Val Ser Gln Ser Ser Ser Asp Ala Gly Leu Gly
        1155                1160                1165

Ser Asp His Glu Ser Asp Thr Leu Thr Ile Asp Val Ser Ala Ile Ser
    1170                1175                1180

Asn Leu Ile Arg Lys His Val Ser Glu Ala Arg Leu Val Glu Asp Ile
1185                1190                1195                1200

Gly His Glu Leu Thr Tyr Val Leu Pro Tyr Glu Ala Ala Lys Glu Gly
                1205                1210                1215

Ala Phe Val Glu Leu Phe His Glu Ile Asp Asp Arg Leu Ser Asp Leu
            1220                1225                1230

Gly Ile Ser Ser Tyr Gly Ile Ser Glu Thr Thr Leu Glu Glu Ile Phe
        1235                1240                1245

Leu Lys Val Ala Glu Glu Ser Gly Val Asp Ala Glu Thr Ser Asp Gly
    1250                1255                1260

Thr Leu Pro Ala Arg Arg Asn Arg Arg Ala Phe Gly Asp Lys Gln Ser
1265                1270                1275                1280

Cys Leu Arg Pro Phe Thr Glu Asp Asp Ala Ala Asp Pro Asn Asp Ser
                1285                1290                1295

Asp Ile Asp Pro Glu Ser Arg Glu Thr Asp Leu Leu Ser Gly Met Asp
            1300                1305                1310

Gly Lys Gly Ser Tyr Gln Val Lys Gly Trp Lys Leu Thr Gln Gln Gln
        1315                1320                1325

Phe Val Ala Leu Leu Trp Lys Arg Leu Leu Ile Ala Arg Arg Ser Arg
    1330                1335                1340

Lys Gly Phe Phe Ala Gln Ile Val Leu Pro Ala Val Phe Val Cys Ile
1345                1350                1355                1360

Ala Leu Val Phe Ser Leu Ile Val Pro Pro Phe Gly Lys Tyr Pro Ser
                1365                1370                1375

Leu Glu Leu Gln Pro Trp Met Tyr Asn Glu Gln Tyr Thr Phe Val Ser
            1380                1385                1390

Asn Asp Ala Pro Glu Asp Thr Gly Thr Leu Glu Leu Leu Asn Ala Leu
        1395                1400                1405

Thr Lys Asp Pro Gly Phe Gly Thr Arg Cys Met Glu Gly Asn Pro Ile
    1410                1415                1420

Pro Asp Thr Pro Cys Gln Ala Gly Glu Glu Glu Trp Thr Thr Ala Pro
1425                1430                1435                1440

Val Pro Gln Thr Ile Met Asp Leu Phe Gln Asn Gly Asn Trp Thr Met
                1445                1450                1455

Gln Asn Pro Ser Pro Ala Cys Gln Cys Ser Ser Asp Lys Ile Lys Lys
            1460                1465                1470

Met Leu Pro Val Cys Pro Pro Gly Ala Gly Gly Leu Pro Pro Pro Gln
        1475                1480                1485

Arg Lys Gln Asn Thr Ala Asp Ile Leu Gln Asp Leu Thr Gly Arg Asn
    1490                1495                1500

Ile Ser Asp Tyr Leu Val Lys Thr Tyr Val Gln Ile Ile Ala Lys Ser
1505                1510                1515                1520

Leu Lys Asn Lys Ile Trp Val Asn Glu Phe Arg Tyr Gly Gly Phe Ser
                1525                1530                1535

Leu Gly Val Ser Asn Thr Gln Ala Leu Pro Pro Ser Gln Glu Val Asn
            1540                1545                1550

Asp Ala Thr Lys Gln Met Lys Lys His Leu Lys Leu Ala Lys Asp Ser
        1555                1560                1565

Ser Ala Asp Arg Phe Leu Asn Ser Leu Gly Arg Phe Met Thr Gly Leu
    1570                1575                1580

Asp Thr Arg Asn Asn Val Lys Val Trp Phe Asn Asn Lys Gly Trp His
1585                1590                1595                1600

Ala Ile Ser Ser Phe Leu Asn Val Ile Asn Asn Ala Ile Leu Arg Ala
                1605                1610                1615

Asn Leu Gln Lys Gly Glu Asn Pro Ser His Tyr Gly Ile Thr Ala Phe
            1620                1625                1630

Asn His Pro Leu Asn Leu Thr Lys Gln Gln Leu Ser Glu Val Ala Pro
        1635                1640                1645

Met Thr Thr Ser Val Asp Val Leu Val Ser Ile Cys Val Ile Phe Ala
    1650                1655                1660

Met Ser Phe Val Pro Ala Ser Phe Val Val Phe Leu Ile Gln Glu Arg
1665                1670                1675                1680

Val Ser Lys Ala Lys His Leu Gln Phe Ile Ser Gly Val Lys Pro Val
                1685                1690                1695

Ile Tyr Trp Leu Ser Asn Phe Val Trp Asp Met Cys Asn Tyr Val Val
            1700                1705                1710

Pro Ala Thr Leu Val Ile Ile Ile Phe Ile Cys Phe Gln Gln Lys Ser
        1715                1720                1725

Tyr Val Ser Ser Thr Asn Leu Pro Val Leu Ala Leu Leu Leu Leu Leu
    1730                1735                1740

Tyr Gly Trp Ser Ile Thr Pro Leu Met Tyr Pro Ala Ser Phe Val Phe
1745                1750                1755                1760

Lys Ile Pro Ser Thr Ala Tyr Val Val Leu Thr Ser Val Asn Leu Phe
                1765                1770                1775

Ile Gly Ile Asn Gly Ser Val Ala Thr Phe Val Leu Glu Leu Phe Thr
            1780                1785                1790

Asp Asn Lys Leu Asn Asn Ile Asn Asp Ile Leu Lys Ser Val Phe Leu
        1795                1800                1805

Ile Phe Pro His Phe Cys Leu Gly Arg Gly Leu Ile Asp Met Val Lys
    1810                1815                1820

Asn Gln Ala Met Ala Asp Ala Leu Glu Arg Phe Gly Glu Asn Arg Phe
1825                1830                1835                1840

Val Ser Pro Leu Ser Trp Asp Leu Val Gly Arg Asn Leu Phe Ala Met
                1845                1850                1855

Ala Val Glu Gly Val Val Phe Phe Leu Ile Thr Val Leu Ile Gln Tyr
            1860                1865                1870

Arg Phe Phe Ile Arg Pro Arg Pro Val Asn Ala Lys Leu Ser Pro Leu
        1875                1880                1885

Asn Asp Glu Asp Glu Asp Val Arg Arg Glu Arg Gln Arg Ile Leu Asp
    1890                1895                1900

Gly Gly Gly Gln Asn Asp Ile Leu Glu Ile Lys Glu Leu Thr Lys Ile
1905                1910                1915                1920

Tyr Arg Arg Lys Arg Lys Pro Ala Val Asp Arg Ile Cys Val Gly Ile
                1925                1930                1935

Pro Pro Gly Glu Cys Phe Gly Leu Leu Gly Val Asn Gly Ala Gly Lys
            1940                1945                1950

Ser Ser Thr Phe Lys Met Leu Thr Gly Asp Thr Thr Val Thr Arg Gly
        1955                1960                1965

Asp Ala Phe Leu Asn Arg Asn Ser Ile Leu Ser Asn Ile His Glu Val
    1970                1975                1980

His Gln Asn Met Gly Tyr Cys Pro Gln Phe Asp Ala Ile Thr Glu Leu
1985                1990                1995                2000

Leu Thr Gly Arg Glu His Val Glu Phe Phe Ala Leu Leu Arg Gly Val
                2005                2010                2015

Pro Glu Lys Glu Val Gly Lys Val Gly Glu Trp Ala Ile Arg Lys Leu
            2020                2025                2030

Gly Leu Val Lys Tyr Gly Glu Lys Tyr Ala Gly Asn Tyr Ser Gly Gly
        2035                2040                2045

Asn Lys Arg Lys Leu Ser Thr Ala Met Ala Leu Ile Gly Gly Pro Pro
    2050                2055                2060

Val Val Phe Leu Asp Glu Pro Thr Thr Gly Met Asp Pro Lys Ala Arg
2065                2070                2075                2080

Arg Phe Leu Trp Asn Cys Ala Leu Ser Val Val Lys Glu Gly Arg Ser
                2085                2090                2095

Val Val Leu Thr Ser His Ser Met Glu Glu Cys Glu Ala Leu Cys Thr
            2100                2105                2110

Arg Met Ala Ile Met Val Asn Gly Arg Phe Arg Cys Leu Gly Ser Val
        2115                2120                2125

Gln His Leu Lys Asn Arg Phe Gly Asp Gly Tyr Thr Ile Val Val Arg
    2130                2135                2140

Ile Ala Gly Ser Asn Pro Asp Leu Lys Pro Val Gln Asp Phe Phe Gly
2145                2150                2155                2160

Leu Ala Phe Pro Gly Ser Val Pro Lys Glu Lys His Arg Asn Met Leu
                2165                2170                2175

Gln Tyr Gln Leu Pro Ser Ser Leu Ser Ser Leu Ala Arg Ile Phe Ser
            2180                2185                2190

Ile Leu Ser Gln Ser Lys Lys Arg Leu His Ile Glu Asp Tyr Ser Val
        2195                2200                2205

Ser Gln Thr Thr Leu Asp Gln Val Phe Val Asn Phe Ala Lys Asp Gln
    2210                2215                2220

Ser Asp Asp Asp His Leu Lys Asp Leu Ser Leu His Lys Asn Gln Thr
2225                2230                2235                2240

Val Val Asp Val Ala Val Leu Thr Ser Phe Leu Gln Asp Glu Lys Val
                2245                2250                2255

Lys Glu Ser Tyr Val
            2260

<210> 45
<211> 2436
<212> PRT

<213> Homo sapiens

<400> 45
Met Gly Phe Leu His Gln Leu Gln Leu Leu Leu Trp Lys Asn Val Thr
1               5                   10                  15

Leu Lys Arg Arg Ser Pro Trp Val Leu Ala Phe Glu Ile Phe Ile Pro
            20                  25                  30

Leu Val Leu Phe Phe Ile Leu Leu Gly Leu Arg Gln Lys Lys Pro Thr
        35                  40                  45

Ile Ser Val Lys Glu Val Pro Phe Tyr Thr Ala Ala Pro Leu Thr Ser
    50                  55                  60

Ala Gly Ile Leu Pro Val Met Gln Ser Leu Cys Pro Asp Gly Gln Arg
65                  70                  75                  80

Asp Glu Phe Gly Phe Leu Gln Tyr Ala Asn Ser Thr Val Thr Gln Leu
                85                  90                  95

Leu Glu Arg Leu Asp Arg Val Val Glu Glu Gly Asn Leu Phe Asp Pro
            100                 105                 110

Ala Arg Pro Ser Leu Gly Ser Glu Leu Glu Ala Leu Arg Gln His Leu
        115                 120                 125

Glu Ala Leu Ser Ala Gly Pro Gly Thr Ser Gly Ser His Leu Asp Arg
    130                 135                 140

Ser Thr Val Ser Ser Phe Ser Leu Asp Ser Val Ala Arg Asn Pro Gln
145                 150                 155                 160

Glu Leu Trp Arg Phe Leu Thr Gln Asn Leu Ser Leu Pro Asn Ser Thr
                165                 170                 175

Ala Gln Ala Leu Leu Ala Ala Arg Val Asp Pro Pro Glu Val Tyr His
            180                 185                 190

Leu Leu Phe Gly Pro Ser Ser Ala Leu Asp Ser Gln Ser Gly Leu His
        195                 200                 205

Lys Gly Gln Glu Pro Trp Ser Arg Leu Gly Gly Asn Pro Leu Phe Arg
    210                 215                 220

Met Glu Glu Leu Leu Leu Ala Pro Ala Leu Leu Glu Gln Leu Thr Cys
225                 230                 235                 240

Thr Pro Gly Ser Gly Glu Leu Gly Arg Ile Leu Thr Val Pro Glu Ser
                245                 250                 255

Gln Lys Gly Ala Leu Gln Gly Tyr Arg Asp Ala Val Cys Ser Gly Gln
            260                 265                 270

Ala Ala Ala Arg Ala Arg Arg Phe Ser Gly Leu Ser Ala Glu Leu Arg
        275                 280                 285

Asn Gln Leu Asp Val Ala Lys Val Ser Gln Gln Leu Gly Leu Asp Ala
    290                 295                 300

Pro Asn Gly Ser Asp Ser Ser Pro Gln Ala Pro Pro Pro Arg Arg Leu
305                 310                 315                 320

Gln Ala Leu Leu Gly Asp Leu Leu Asp Ala Gln Lys Val Leu Gln Asp
                325                 330                 335

Val Asp Val Leu Ser Ala Leu Ala Leu Leu Leu Pro Gln Gly Ala Cys
            340                 345                 350

Thr Gly Arg Thr Pro Gly Pro Pro Ala Ser Gly Ala Gly Gly Ala Ala
        355                 360                 365

Asn Gly Thr Gly Ala Gly Ala Val Met Gly Pro Asn Ala Thr Ala Glu
    370                 375                 380

Glu Gly Ala Pro Ser Ala Ala Ala Leu Ala Thr Pro Asp Thr Leu Gln
385                 390                 395                 400

Gly Gln Cys Ser Ala Phe Val Gln Leu Trp Ala Gly Leu Gln Pro Ile
                405                 410                 415

Leu Cys Gly Asn Asn Arg Thr Ile Glu Pro Glu Ala Leu Arg Arg Gly
            420                 425                 430

Asn Met Ser Ser Leu Gly Phe Thr Ser Lys Glu Gln Arg Asn Leu Gly
        435                 440                 445

Leu Leu Val His Leu Met Thr Ser Asn Pro Lys Ile Leu Tyr Ala Pro
    450                 455                 460

Ala Gly Ser Glu Val Asp Arg Val Ile Leu Lys Ala Asn Glu Thr Phe
465                 470                 475                 480

Ala Phe Val Gly Asn Val Thr His Tyr Ala Gln Val Trp Leu Asn Ile
                485                 490                 495

Ser Ala Glu Ile Arg Ser Phe Leu Glu Gln Gly Arg Leu Gln Gln His
            500                 505                 510

Leu Arg Trp Leu Gln Gln Tyr Val Ala Glu Leu Arg Leu His Pro Glu
        515                 520                 525

Ala Leu Asn Leu Ser Leu Asp Glu Leu Pro Pro Ala Leu Arg Gln Asp
    530                 535                 540

Asn Phe Ser Leu Pro Ser Gly Met Ala Leu Leu Gln Gln Leu Asp Thr
545                 550                 555                 560

Ile Asp Asn Ala Ala Cys Gly Trp Ile Gln Phe Met Ser Lys Val Ser
                565                 570                 575

Val Asp Ile Phe Lys Gly Phe Pro Asp Glu Glu Ser Ile Val Asn Tyr
            580                 585                 590

Thr Leu Asn Gln Ala Tyr Gln Asp Asn Val Thr Val Phe Ala Ser Val
        595                 600                 605

Ile Phe Gln Thr Arg Lys Asp Gly Ser Leu Pro Pro His Val His Tyr
    610                 615                 620

Lys Ile Arg Gln Asn Ser Ser Phe Thr Glu Lys Thr Asn Glu Ile Arg
625                 630                 635                 640

Arg Ala Tyr Trp Arg Pro Gly Pro Asn Thr Gly Gly Arg Phe Tyr Phe
                645                 650                 655

Leu Tyr Gly Phe Val Trp Ile Gln Asp Met Met Glu Arg Ala Ile Ile
            660                 665                 670

Asp Thr Phe Val Gly His Asp Val Val Glu Pro Gly Ser Tyr Val Gln
        675                 680                 685

Met Phe Pro Tyr Pro Cys Tyr Thr Arg Asp Asp Phe Leu Phe Val Ile
    690                 695                 700

Glu His Met Met Pro Leu Cys Met Val Ile Ser Trp Val Tyr Ser Val
705                 710                 715                 720

Ala Met Thr Ile Gln His Ile Val Ala Glu Lys Glu His Arg Leu Lys
                725                 730                 735

Glu Val Met Lys Thr Met Gly Leu Asn Asn Ala Val His Trp Val Ala
            740                 745                 750

Trp Phe Ile Thr Gly Phe Val Gln Leu Ser Ile Ser Val Thr Ala Leu
        755                 760                 765

Thr Ala Ile Leu Lys Tyr Gly Gln Val Leu Met His Ser His Val Val
    770                 775                 780

Ile Ile Trp Leu Phe Leu Ala Val Tyr Ala Val Ala Thr Ile Met Phe
785                 790                 795                 800

Cys Phe Leu Val Ser Val Leu Tyr Ser Lys Ala Lys Leu Ala Ser Ala
                805                 810                 815

Cys Gly Gly Ile Ile Tyr Phe Leu Ser Tyr Val Pro Tyr Met Tyr Val
            820                 825                 830

Ala Ile Arg Glu Glu Val Ala His Asp Lys Ile Thr Ala Phe Glu Lys
        835                 840                 845

Cys Ile Ala Ser Leu Met Ser Thr Thr Ala Phe Gly Leu Gly Ser Lys
    850                 855                 860

Tyr Phe Ala Leu Tyr Glu Val Ala Gly Val Gly Ile Gln Trp His Thr
865                 870                 875                 880

Phe Ser Gln Ser Pro Val Glu Gly Asp Asp Phe Asn Leu Leu Leu Ala
                885                 890                 895

Val Thr Met Leu Met Val Asp Ala Val Val Tyr Gly Ile Leu Thr Trp
            900                 905                 910

Tyr Ile Glu Ala Val His Pro Gly Met Tyr Gly Leu Pro Arg Pro Trp
        915                 920                 925

Tyr Phe Pro Leu Gln Lys Ser Tyr Trp Leu Gly Ser Gly Arg Thr Glu
    930                 935                 940

Ala Trp Glu Trp Ser Trp Pro Trp Ala Arg Thr Pro Arg Leu Ser Val
945                 950                 955                 960

Met Glu Glu Asp Gln Ala Cys Ala Met Glu Ser Arg Arg Phe Glu Glu
                965                 970                 975

Thr Arg Gly Met Glu Glu Glu Pro Thr His Leu Pro Leu Val Val Cys
            980                 985                 990

Val Asp Lys Leu Thr Lys Val Tyr Lys Asp Asp Lys Lys Leu Ala Leu
        995                 1000                1005

Asn Lys Leu Ser Leu Asn Leu Tyr Glu Asn Gln Val Val Ser Phe Leu
    1010                1015                1020

Gly His Asn Gly Ala Gly Lys Thr Thr Thr Met Ser Ile Leu Thr Gly
1025                1030                1035                1040

Leu Phe Pro Pro Thr Ser Gly Ser Ala Thr Ile Tyr Gly His Asp Ile
                1045                1050                1055

Arg Thr Glu Met Asp Glu Ile Arg Lys Asn Leu Gly Met Cys Pro Gln
            1060                1065                1070

His Asn Val Leu Phe Asp Arg Leu Thr Val Glu Glu His Leu Trp Phe
        1075                1080                1085

Tyr Ser Arg Leu Lys Ser Met Ala Gln Glu Glu Ile Arg Arg Glu Met
    1090                1095                1100

Asp Lys Met Ile Glu Asp Leu Glu Leu Ser Asn Lys Arg His Ser Leu
1105                1110                1115                1120

Val Gln Thr Leu Ser Gly Gly Met Lys Arg Lys Leu Ser Val Ala Ile
                1125                1130                1135

Ala Phe Val Gly Gly Ser Arg Ala Ile Ile Leu Asp Glu Pro Thr Ala
            1140                1145                1150

Gly Val Asp Pro Tyr Ala Arg Arg Ala Ile Trp Asp Leu Ile Leu Lys
        1155                1160                1165

Tyr Lys Pro Gly Arg Thr Ile Leu Leu Ser Thr His His Met Asp Glu
    1170                1175                1180

Ala Asp Leu Leu Gly Asp Arg Ile Ala Ile Ile Ser His Gly Lys Leu
1185                1190                1195                1200

Lys Cys Cys Gly Ser Pro Leu Phe Leu Lys Gly Thr Tyr Gly Asp Gly
                1205                1210                1215

Tyr Arg Leu Thr Leu Val Lys Arg Pro Ala Glu Pro Gly Gly Pro Gln
            1220                1225                1230

Glu Pro Gly Leu Ala Ser Ser Pro Pro Gly Arg Ala Pro Leu Ser Ser
        1235                1240                1245

Cys Ser Glu Leu Gln Val Ser Gln Phe Ile Arg Lys His Val Ala Ser
    1250                1255                1260

Cys Leu Leu Val Ser Asp Thr Ser Thr Glu Leu Ser Tyr Ile Leu Pro
1265                1270                1275                1280

Ser Glu Ala Ala Lys Lys Gly Ala Phe Glu Arg Leu Phe Gln His Leu
                1285                1290                1295

Glu Arg Ser Leu Asp Ala Leu His Leu Ser Ser Phe Gly Leu Met Asp
            1300                1305                1310

Thr Thr Leu Glu Glu Val Phe Leu Lys Val Ser Glu Glu Asp Gln Ser
        1315                1320                1325

Leu Glu Asn Ser Glu Ala Asp Val Lys Glu Ser Arg Lys Asp Val Leu
    1330                1335                1340

Pro Gly Ala Glu Gly Pro Ala Ser Gly Glu Gly His Ala Gly Asn Leu
1345                1350                1355                1360

Ala Arg Cys Ser Glu Leu Thr Gln Ser Gln Ala Ser Leu Gln Ser Ala
                1365                1370                1375

Ser Ser Val Gly Ser Ala Arg Gly Asp Glu Gly Ala Gly Tyr Thr Asp
            1380                1385                1390

Val Tyr Gly Asp Tyr Arg Pro Leu Phe Asp Asn Pro Gln Asp Pro Asp
        1395                1400                1405

Asn Val Ser Leu Gln Glu Val Glu Ala Glu Ala Leu Ser Arg Val Gly
    1410                1415                1420

Gln Gly Ser Arg Lys Leu Asp Gly Gly Trp Leu Lys Val Arg Gln Phe
1425                1430                1435                1440

His Gly Leu Leu Val Lys Arg Phe His Cys Ala Arg Arg Asn Ser Lys
                1445                1450                1455

Ala Leu Phe Ser Gln Ile Leu Leu Pro Ala Phe Phe Val Cys Val Ala
            1460                1465                1470

Met Thr Val Ala Leu Ser Val Pro Glu Ile Gly Asp Leu Pro Pro Leu
        1475                1480                1485

Val Leu Ser Pro Ser Gln Tyr His Asn Tyr Thr Gln Pro Arg Gly Asn
    1490                1495                1500

Phe Ile Pro Tyr Ala Asn Glu Glu Arg Arg Glu Tyr Arg Leu Arg Leu
1505                1510                1515                1520

Ser Pro Asp Ala Ser Pro Gln Gln Leu Val Ser Thr Phe Arg Leu Pro
                1525                1530                1535

Ser Gly Val Gly Ala Thr Cys Val Leu Lys Ser Pro Ala Asn Gly Ser
            1540                1545                1550

Leu Gly Pro Thr Leu Asn Leu Ser Ser Gly Glu Ser Arg Leu Leu Ala
        1555                1560                1565

Ala Arg Phe Phe Asp Ser Met Cys Leu Glu Ser Phe Thr Gln Gly Leu
    1570                1575                1580

Pro Leu Ser Asn Phe Val Pro Pro Pro Pro Ser Pro Ala Pro Ser Asp
1585                1590                1595                1600

Ser Pro Ala Ser Pro Asp Glu Asp Leu Gln Ala Trp Asn Val Ser Leu
                1605                1610                1615

Pro Pro Thr Ala Gly Pro Glu Met Trp Thr Ser Ala Pro Ser Leu Pro
            1620                1625                1630

Arg Leu Val Arg Glu Pro Val Arg Cys Thr Cys Ser Ala Gln Gly Thr
        1635                1640                1645

Gly Phe Ser Cys Pro Ser Ser Val Gly Gly His Pro Pro Gln Met Arg
    1650                1655                1660

Val Val Thr Gly Asp Ile Leu Thr Asp Ile Thr Gly His Asn Val Ser
1665                1670                1675                1680

Glu Tyr Leu Leu Phe Thr Ser Asp Arg Phe Arg Leu His Arg Tyr Gly
                1685                1690                1695

Ala Ile Thr Phe Gly Asn Val Leu Lys Ser Ile Pro Ala Ser Phe Gly
            1700                1705                1710

Thr Arg Ala Pro Pro Met Val Arg Lys Ile Ala Val Arg Arg Ala Ala
        1715                1720                1725

Gln Val Phe Tyr Asn Asn Lys Gly Tyr His Ser Met Pro Thr Tyr Leu
    1730                1735                1740

Asn Ser Leu Asn Asn Ala Ile Leu Arg Ala Asn Leu Pro Lys Ser Lys
1745                1750                1755                1760

Gly Asn Pro Ala Ala Tyr Gly Ile Thr Val Thr Asn His Pro Met Asn
                1765                1770                1775

Lys Thr Ser Ala Ser Leu Ser Leu Asp Tyr Leu Leu Gln Gly Thr Asp
            1780                1785                1790

Val Val Ile Ala Ile Phe Ile Ile Val Ala Met Ser Phe Val Pro Ala
        1795                1800                1805

Ser Phe Val Val Phe Leu Val Ala Glu Lys Ser Thr Lys Ala Lys His
    1810                1815                1820

Leu Gln Phe Val Ser Gly Cys Asn Pro Ile Ile Tyr Trp Leu Ala Asn
1825                1830                1835                1840

Tyr Val Trp Asp Met Leu Asn Tyr Leu Val Pro Ala Thr Cys Cys Val
                1845                1850                1855

Ile Ile Leu Phe Val Phe Asp Leu Pro Ala Tyr Thr Ser Pro Thr Asn
            1860                1865                1870

Phe Pro Ala Val Leu Ser Leu Phe Leu Leu Tyr Gly Trp Ser Ile Thr
        1875                1880                1885

Pro Ile Met Tyr Pro Ala Ser Phe Trp Phe Glu Val Pro Ser Ser Ala
    1890                1895                1900

Tyr Val Phe Leu Ile Val Ile Asn Leu Phe Ile Gly Ile Thr Ala Thr
1905                1910                1915                1920

Val Ala Thr Phe Leu Leu Gln Leu Phe Glu His Asp Lys Asp Leu Lys
                1925                1930                1935

Val Val Asn Ser Tyr Leu Lys Ser Cys Phe Leu Ile Phe Pro Asn Tyr
            1940                1945                1950

Asn Leu Gly His Gly Leu Met Glu Met Ala Tyr Asn Glu Tyr Ile Asn
        1955                1960                1965

Glu Tyr Tyr Ala Lys Ile Gly Gln Phe Asp Lys Met Lys Ser Pro Phe
    1970                1975                1980

Glu Trp Asp Ile Val Thr Arg Gly Leu Val Ala Met Ala Val Glu Gly
1985                1990                1995                2000

Val Val Gly Phe Leu Leu Thr Ile Met Cys Gln Tyr Asn Phe Leu Arg
                2005                2010                2015

Arg Pro Gln Arg Met Pro Val Ser Thr Lys Pro Val Glu Asp Asp Val
            2020                2025                2030

Asp Val Ala Ser Glu Arg Gln Arg Val Leu Arg Gly Asp Ala Asp Asn
        2035                2040                2045

Asp Met Val Lys Ile Glu Asn Leu Thr Lys Val Tyr Lys Ser Arg Lys
    2050                2055                2060

Ile Gly Arg Ile Leu Ala Val Asp Arg Leu Cys Leu Gly Val Arg Pro
2065                2070                2075                2080

Gly Glu Cys Phe Gly Leu Leu Gly Val Asn Gly Ala Gly Lys Thr Ser
                2085                2090                2095

Thr Phe Lys Met Leu Thr Gly Asp Glu Ser Thr Thr Gly Gly Glu Ala
            2100                2105                2110

Phe Val Asn Gly His Ser Val Leu Lys Glu Leu Leu Gln Val Gln Gln
        2115                2120                2125

Ser Leu Gly Tyr Cys Pro Gln Cys Asp Ala Leu Phe Asp Glu Leu Thr
    2130                2135                2140

Ala Arg Glu His Leu Gln Leu Tyr Thr Arg Leu Arg Gly Ile Ser Trp
2145                2150                2155                2160

Lys Asp Glu Ala Arg Val Val Lys Trp Ala Leu Glu Lys Leu Glu Leu
                2165                2170                2175

Thr Lys Tyr Ala Asp Lys Pro Ala Gly Thr Tyr Ser Gly Gly Asn Lys
            2180                2185                2190

Arg Lys Leu Ser Thr Ala Ile Ala Leu Ile Gly Tyr Pro Ala Phe Ile
        2195                2200                2205

Phe Leu Asp Glu Pro Thr Thr Gly Met Asp Pro Lys Ala Arg Arg Phe
    2210                2215                2220

Leu Trp Asn Leu Ile Leu Asp Leu Ile Lys Thr Gly Arg Ser Val Val
2225                2230                2235                2240

Leu Thr Ser His Ser Met Glu Glu Cys Glu Ala Leu Cys Thr Arg Leu
                2245                2250                2255

Ala Ile Met Val Asn Gly Arg Leu Arg Cys Leu Gly Ser Ile Gln His
            2260                2265                2270

Leu Lys Asn Arg Phe Gly Asp Gly Tyr Met Ile Thr Val Arg Thr Lys
        2275                2280                2285

Ser Ser Gln Ser Val Lys Asp Val Val Arg Phe Phe Asn Arg Asn Phe
    2290                2295                2300

Pro Glu Ala Met Leu Lys Glu Arg His His Thr Lys Val Gln Tyr Gln
2305                2310                2315                2320

Leu Lys Ser Glu His Ile Ser Leu Ala Gln Val Phe Ser Lys Met Glu
                2325                2330                2335

Gln Val Ser Gly Val Leu Gly Ile Glu Asp Tyr Ser Val Ser Gln Thr
            2340                2345                2350

Thr Leu Asp Asn Val Phe Val Asn Phe Ala Lys Lys Gln Ser Asp Asn
        2355                2360                2365

Leu Glu Gln Gln Glu Thr Glu Pro Pro Ser Ala Leu Gln Ser Pro Leu
    2370                2375                2380

Gly Cys Leu Leu Ser Leu Leu Arg Pro Arg Ser Ala Pro Thr Glu Leu
2385                2390                2395                2400

Arg Ala Leu Val Ala Asp Glu Pro Glu Asp Leu Asp Thr Glu Asp Glu
                2405                2410                2415

Gly Leu Ile Ser Phe Glu Glu Glu Arg Ala Gln Leu Ser Phe Asn Thr
            2420                2425                2430

Asp Thr Leu Cys
        2435

<210> 46
<211> 1704
<212> PRT
<213> Homo sapiens

<400> 46
Met Ala Val Leu Arg Gln Leu Ala Leu Leu Leu Trp Lys Asn Tyr Thr
  1               5                  10                  15

Leu Gln Lys Arg Lys Val Leu Val Thr Val Leu Glu Leu Phe Leu Pro
             20                  25                  30

Leu Leu Phe Pro Gly Ile Leu Ile Trp Leu Arg Leu Lys Ile Gln Ser
         35                  40                  45

Glu Asn Val Pro Asn Ala Thr Ile Tyr Pro Gly Gln Ser Ile Gln Glu

    50                  55                  60

Leu Pro Leu Phe Phe Thr Phe Pro Pro Pro Gly Asp Thr Trp Glu Leu
65                  70                  75                  80

Ala Tyr Ile Pro Ser His Ser Asp Ala Ala Lys Thr Val Thr Glu Thr
                85                  90                  95

Val Arg Arg Ala Leu Val Ile Asn Met Arg Val Arg Gly Phe Pro Ser
            100                 105                 110

Glu Lys Asp Phe Glu Asp Tyr Ile Arg Tyr Asp Asn Cys Ser Ser Ser
        115                 120                 125

Val Leu Ala Ala Val Val Phe Glu His Pro Phe Asn His Ser Lys Glu
    130                 135                 140

Pro Leu Pro Leu Ala Val Lys Tyr His Leu Arg Phe Ser Tyr Thr Arg
145                 150                 155                 160

Arg Asn Tyr Met Trp Thr Gln Thr Gly Ser Phe Phe Leu Lys Glu Thr
                165                 170                 175

Glu Gly Trp His Thr Thr Ser Leu Phe Pro Leu Phe Pro Asn Pro Gly
            180                 185                 190

Pro Arg Glu Leu Thr Ser Pro Asp Gly Gly Glu Pro Gly Tyr Ile Arg
        195                 200                 205

Glu Gly Phe Leu Ala Val Gln His Ala Val Asp Arg Ala Ile Met Glu
    210                 215                 220

Tyr His Ala Asp Ala Ala Thr Arg Gln Leu Phe Gln Arg Leu Thr Val
225                 230                 235                 240

Thr Ile Lys Arg Phe Pro Tyr Pro Pro Phe Ile Ala Asp Pro Phe Leu
                245                 250                 255

Val Ala Ile Gln Tyr Gln Leu Pro Leu Leu Leu Leu Leu Ser Phe Thr
            260                 265                 270

Tyr Thr Ala Leu Thr Ile Ala Arg Ala Val Val Gln Glu Lys Glu Arg
        275                 280                 285

Arg Leu Lys Glu Tyr Met Arg Met Met Gly Leu Ser Ser Trp Leu His
    290                 295                 300

Trp Ser Ala Trp Phe Leu Leu Phe Phe Leu Phe Leu Leu Ile Ala Ala

305                 310                 315                 320

Ser Phe Met Thr Leu Leu Phe Cys Val Lys Val Lys Pro Asn Val Ala
                325                 330                 335

Val Leu Ser Arg Ser Asp Pro Ser Leu Val Leu Ala Phe Leu Leu Cys
            340                 345                 350

Phe Ala Ile Ser Thr Ile Ser Phe Ser Phe Met Val Ser Thr Phe Phe
        355                 360                 365

Ser Lys Ala Asn Met Ala Ala Ala Phe Gly Gly Phe Leu Tyr Phe Phe
    370                 375                 380

Thr Tyr Ile Pro Tyr Phe Phe Val Ala Pro Arg Tyr Asn Trp Met Thr
385                 390                 395                 400

Leu Ser Gln Lys Leu Cys Ser Cys Leu Leu Ser Asn Val Ala Met Ala
                405                 410                 415

Met Gly Ala Gln Leu Ile Gly Lys Phe Glu Ala Lys Gly Met Gly Ile
            420                 425                 430

Gln Trp Arg Asp Leu Leu Ser Pro Val Asn Val Asp Asp Asp Phe Cys
        435                 440                 445

Phe Gly Gln Val Leu Gly Met Leu Leu Leu Asp Ser Val Leu Tyr Gly
    450                 455                 460

Leu Val Thr Trp Tyr Met Glu Ala Val Phe Pro Gly Gln Phe Gly Val
465                 470                 475                 480

Pro Gln Pro Trp Tyr Phe Phe Ile Met Pro Ser Tyr Trp Cys Gly Lys
                485                 490                 495

Pro Arg Ala Val Ala Gly Lys Glu Glu Glu Asp Ser Asp Pro Glu Lys
            500                 505                 510

Ala Leu Arg Asn Glu Tyr Phe Glu Ala Glu Pro Glu Asp Leu Val Ala
        515                 520                 525

Gly Ile Lys Ile Lys His Leu Ser Lys Val Phe Arg Val Gly Asn Lys
    530                 535                 540

Asp Arg Ala Ala Val Arg Asp Leu Asn Leu Asn Leu Tyr Glu Gly Gln
545                 550                 555                 560

Ile Thr Val Leu Leu Gly His Asn Gly Ala Gly Lys Thr Thr Thr Leu

                565                 570                 575

Ser Met Leu Thr Gly Leu Phe Pro Pro Thr Ser Gly Arg Ala Tyr Ile
            580                 585                 590

Ser Gly Tyr Glu Ile Ser Gln Asp Met Val Gln Ile Arg Lys Ser Leu
        595                 600                 605

Gly Leu Cys Pro Gln His Asp Ile Leu Phe Asp Asn Leu Thr Val Ala
    610                 615                 620

Glu His Leu Tyr Phe Tyr Ala Gln Leu Lys Gly Leu Ser Arg Gln Lys
625                 630                 635                 640

Cys Pro Glu Glu Val Lys Gln Met Leu His Ile Ile Gly Leu Glu Asp
                645                 650                 655

Lys Trp Asn Ser Arg Ser Arg Phe Leu Ser Gly Gly Met Arg Arg Lys
            660                 665                 670

Leu Ser Ile Gly Ile Ala Leu Ile Ala Gly Ser Lys Val Leu Ile Leu
        675                 680                 685

Asp Glu Pro Thr Ser Gly Met Asp Ala Ile Ser Arg Arg Ala Ile Trp
    690                 695                 700

Asp Leu Leu Gln Arg Gln Lys Ser Asp Arg Thr Ile Val Leu Thr Thr
705                 710                 715                 720

His Phe Met Asp Glu Ala Asp Leu Leu Gly Asp Arg Ile Ala Ile Met
                725                 730                 735

Ala Lys Gly Glu Leu Gln Cys Cys Gly Ser Ser Leu Phe Leu Lys Gln
            740                 745                 750

Lys Tyr Gly Ala Gly Tyr His Met Thr Leu Val Lys Glu Pro His Cys
        755                 760                 765

Asn Pro Glu Asp Ile Ser Gln Leu Val His His His Val Pro Asn Ala
    770                 775                 780

Thr Leu Glu Ser Ser Ala Gly Ala Glu Leu Ser Phe Ile Leu Pro Arg
785                 790                 795                 800

Glu Ser Thr His Arg Phe Glu Gly Leu Phe Ala Lys Leu Glu Lys Lys
                805                 810                 815

Gln Lys Glu Leu Gly Ile Ala Ser Phe Gly Ala Ser Ile Thr Thr Met

            820                 825                 830

Glu Glu Val Phe Leu Arg Val Gly Lys Leu Val Asp Ser Ser Met Asp
        835                 840                 845

Ile Gln Ala Ile Gln Leu Pro Ala Leu Gln Tyr Gln His Glu Arg Arg
    850                 855                 860

Ala Ser Asp Trp Ala Val Asp Ser Asn Leu Cys Gly Ala Met Asp Pro
865                 870                 875                 880

Ser Asp Gly Ile Gly Ala Leu Ile Glu Glu Glu Arg Thr Ala Val Lys
                885                 890                 895

Leu Asn Thr Gly Leu Ala Leu His Cys Gln Gln Phe Trp Ala Met Phe
            900                 905                 910

Leu Lys Lys Ala Ala Tyr Ser Trp Arg Glu Trp Lys Met Val Ala Ala
        915                 920                 925

Gln Val Leu Val Pro Leu Thr Cys Val Thr Leu Ala Leu Leu Ala Ile
    930                 935                 940

Asn Tyr Ser Ser Glu Leu Phe Asp Asp Pro Met Leu Arg Leu Thr Leu
945                 950                 955                 960

Gly Glu Tyr Gly Arg Thr Val Val Pro Phe Ser Val Pro Gly Thr Ser
                965                 970                 975

Gln Leu Gly Gln Gln Leu Ser Glu His Leu Lys Asp Ala Leu Gln Ala
            980                 985                 990

Glu Gly Gln Glu Pro Arg Glu Val Leu Gly Asp Leu Glu Glu Phe Leu
        995                 1000                1005

Ile Phe Arg Ala Ser Val Glu Gly Gly Gly Phe Asn Glu Arg Cys Leu
    1010                1015                1020

Val Ala Ala Ser Phe Arg Asp Val Gly Glu Arg Thr Val Val Asn Ala
1025                1030                1035                1040

Leu Phe Asn Asn Gln Ala Tyr His Ser Pro Ala Thr Ala Leu Ala Val
                1045                1050                1055

Val Asp Asn Leu Leu Phe Lys Leu Leu Cys Gly Pro His Ala Ser Ile
            1060                1065                1070

Val Val Ser Asn Phe Pro Gln Pro Arg Ser Ala Leu Gln Ala Ala Lys

1075                 1080                1085

Asp Gln Phe Asn Glu Gly Arg Lys Gly Phe Asp Ile Ala Leu Asn Leu
    1090                1095                1100

Leu Phe Ala Met Ala Phe Leu Ala Ser Thr Phe Ser Ile Leu Ala Val
1105                1110                1115                1120

Ser Glu Arg Ala Val Gln Ala Lys His Val Gln Phe Val Ser Gly Val
                1125                1130                1135

His Val Ala Ser Phe Trp Leu Ser Ala Leu Leu Trp Asp Leu Ile Ser
            1140                1145                1150

Phe Leu Ile Pro Ser Leu Leu Leu Leu Val Val Phe Lys Ala Phe Asp
        1155                1160                1165

Val Arg Ala Phe Thr Arg Asp Gly His Met Ala Asp Thr Leu Leu Leu
    1170                1175                1180

Leu Leu Leu Tyr Gly Trp Ala Ile Ile Pro Leu Met Tyr Leu Met Asn
1185                1190                1195                1200

Phe Phe Phe Leu Gly Ala Ala Thr Ala Tyr Thr Arg Leu Thr Ile Phe
                1205                1210                1215

Asn Ile Leu Ser Gly Ile Ala Thr Phe Leu Met Val Thr Ile Met Arg
            1220                1225                1230

Ile Pro Ala Val Lys Leu Glu Glu Leu Ser Lys Thr Leu Asp His Val
        1235                1240                1245

Phe Leu Val Leu Pro Asn His Cys Leu Gly Met Ala Val Ser Ser Phe
    1250                1255                1260

Tyr Glu Asn Tyr Glu Thr Arg Arg Tyr Cys Thr Ser Ser Glu Val Ala
1265                1270                1275                1280

Ala His Tyr Cys Lys Lys Tyr Asn Ile Gln Tyr Gln Glu Asn Phe Tyr
                1285                1290                1295

Ala Trp Ser Ala Pro Gly Val Gly Arg Phe Val Ala Ser Met Ala Ala
            1300                1305                1310

Ser Gly Cys Ala Tyr Leu Ile Leu Leu Phe Leu Ile Glu Thr Asn Leu
        1315                1320                1325

Leu Gln Arg Leu Arg Gly Ile Leu Cys Ala Leu Arg Arg Arg Arg Thr

1330                1335                1340

Leu Thr Glu Leu Tyr Thr Arg Met Pro Val Leu Pro Glu Asp Gln Asp
1345                1350                1355                1360

Val Ala Asp Glu Arg Thr Arg Ile Leu Ala Pro Ser Pro Asp Ser Leu
                1365                1370                1375

Leu His Thr Pro Leu Ile Ile Lys Glu Leu Ser Lys Val Tyr Glu Gln
            1380                1385                1390

Arg Val Pro Leu Leu Ala Val Asp Arg Leu Ser Leu Ala Val Gln Lys
        1395                1400                1405

Gly Glu Cys Phe Gly Leu Leu Gly Phe Asn Gly Ala Gly Lys Thr Thr
    1410                1415                1420

Thr Phe Lys Met Leu Thr Gly Glu Glu Ser Leu Thr Ser Gly Asp Ala
1425                1430                1435                1440

Phe Val Gly Gly His Arg Ile Ser Ser Asp Val Gly Lys Val Arg Gln
                1445                1450                1455

Arg Ile Gly Tyr Cys Pro Gln Phe Asp Ala Leu Leu Asp His Met Thr
            1460                1465                1470

Gly Arg Glu Met Leu Val Met Tyr Ala Arg Leu Arg Gly Ile Pro Glu
        1475                1480                1485

Arg His Ile Gly Ala Cys Val Glu Asn Thr Leu Arg Gly Leu Leu Leu
    1490                1495                1500

Glu Pro His Ala Asn Lys Leu Val Arg Thr Tyr Ser Gly Gly Asn Lys
1505                1510                1515                1520

Arg Lys Leu Ser Thr Gly Ile Ala Leu Ile Gly Glu Pro Ala Val Ile
                1525                1530                1535

Phe Leu Asp Glu Pro Ser Thr Gly Met Asp Pro Val Ala Arg Arg Leu
            1540                1545                1550

Leu Trp Asp Thr Val Ala Arg Ala Arg Glu Ser Gly Lys Ala Ile Ile
        1555                1560                1565

Ile Thr Ser His Ser Met Glu Glu Cys Glu Ala Leu Cys Thr Arg Leu
    1570                1575                1580

Ala Ile Met Val Gln Gly Gln Phe Lys Cys Leu Gly Ser Pro Gln His

1585                1590                1595                1600

Leu Lys Ser Lys Phe Gly Ser Gly Tyr Ser Leu Arg Ala Lys Val Gln
                1605                1610                1615

Ser Glu Gly Gln Gln Glu Ala Leu Glu Glu Phe Lys Ala Phe Val Asp
            1620                1625                1630

Leu Thr Phe Pro Gly Ser Val Leu Glu Asp Glu His Gln Gly Met Val
        1635                1640                1645

His Tyr His Leu Pro Gly Arg Asp Leu Ser Trp Ala Lys Val Phe Gly
    1650                1655                1660

Ile Leu Glu Lys Ala Lys Glu Lys Tyr Gly Val Asp Asp Tyr Ser Val
1665                1670                1675                1680

Ser Gln Ile Ser Leu Glu Gln Val Phe Leu Ser Phe Ala His Leu Gln
                1685                1690                1695

Pro Pro Thr Ala Glu Glu Gly Arg
            1700

<210> 47
<211> 2273
<212> PRT
<213> Homo sapiens

<400> 47
Met Gly Phe Val Arg Gln Ile Gln Leu Leu Leu Trp Lys Asn Trp Thr
  1               5                  10                  15

Leu Arg Lys Arg Gln Lys Ile Arg Phe Val Val Glu Leu Val Trp Pro
             20                  25                  30

Leu Ser Leu Phe Leu Val Leu Ile Trp Leu Arg Asn Ala Asn Pro Leu
         35                  40                  45

Tyr Ser His His Glu Cys His Phe Pro Asn Lys Ala Met Pro Ser Ala
     50                  55                  60

Gly Met Leu Pro Trp Leu Gln Gly Ile Phe Cys Asn Val Asn Asn Pro
 65                  70                  75                  80

Cys Phe Gln Ser Pro Thr Pro Gly Glu Ser Pro Gly Ile Val Ser Asn
                 85                  90                  95

Tyr Asn Asn Ser Ile Leu Ala Arg Val Tyr Arg Asp Phe Gln Glu Leu
            100                 105                 110

Leu Met Asn Ala Pro Glu Ser Gln His Leu Gly Arg Ile Trp Thr Glu
        115                 120                 125

Leu His Ile Leu Ser Gln Phe Met Asp Thr Leu Arg Thr His Pro Glu
    130                 135                 140

Arg Ile Ala Gly Arg Gly Ile Arg Ile Arg Asp Ile Leu Lys Asp Glu
145                 150                 155                 160

Glu Thr Leu Thr Leu Phe Leu Ile Lys Asn Ile Gly Leu Ser Asp Ser
                165                 170                 175

Val Val Tyr Leu Leu Ile Asn Ser Gln Val Arg Pro Glu Gln Phe Ala
            180                 185                 190

His Gly Val Pro Asp Leu Ala Leu Lys Asp Ile Ala Cys Ser Glu Ala
        195                 200                 205

Leu Leu Glu Arg Phe Ile Ile Phe Ser Gln Arg Arg Gly Ala Lys Thr
    210                 215                 220

Val Arg Tyr Ala Leu Cys Ser Leu Ser Gln Gly Thr Leu Gln Trp Ile
225                 230                 235                 240

Glu Asp Thr Leu Tyr Ala Asn Val Asp Phe Phe Lys Leu Phe Arg Val
                245                 250                 255

Leu Pro Thr Leu Leu Asp Ser Arg Ser Gln Gly Ile Asn Leu Arg Ser
            260                 265                 270

Trp Gly Gly Ile Leu Ser Asp Met Ser Pro Arg Ile Gln Glu Phe Ile
        275                 280                 285

His Arg Pro Ser Met Gln Asp Leu Leu Trp Val Thr Arg Pro Leu Met
    290                 295                 300

Gln Asn Gly Gly Pro Glu Thr Phe Thr Lys Leu Met Gly Ile Leu Ser
305                 310                 315                 320

Asp Leu Leu Cys Gly Tyr Pro Glu Gly Gly Gly Ser Arg Val Leu Ser
                325                 330                 335

Phe Asn Trp Tyr Glu Asp Asn Asn Tyr Lys Ala Phe Leu Gly Ile Asp
            340                 345                 350

Ser Thr Arg Lys Asp Pro Ile Tyr Ser Tyr Asp Arg Arg Thr Thr Ser
        355                 360                 365

Phe Cys Asn Ala Leu Ile Gln Ser Leu Glu Ser Asn Pro Leu Thr Lys
    370                 375                 380

Ile Ala Trp Arg Ala Ala Lys Pro Leu Leu Met Gly Lys Ile Leu Tyr
385                 390                 395                 400

Thr Pro Asp Ser Pro Ala Ala Arg Arg Ile Leu Lys Asn Ala Asn Ser
                405                 410                 415

Thr Phe Glu Glu Leu Glu His Val Arg Lys Leu Val Lys Ala Trp Glu
            420                 425                 430

Glu Val Gly Pro Gln Ile Trp Tyr Phe Phe Asp Asn Ser Thr Gln Met
        435                 440                 445

Asn Met Ile Arg Asp Thr Leu Gly Asn Pro Thr Val Lys Asp Phe Leu
    450                 455                 460

Asn Arg Gln Leu Gly Glu Glu Gly Ile Thr Ala Glu Ala Ile Leu Asn
465                 470                 475                 480

Phe Leu Tyr Lys Gly Pro Arg Glu Ser Gln Ala Asp Asp Met Ala Asn
                485                 490                 495

Phe Asp Trp Arg Asp Ile Phe Asn Ile Thr Asp Arg Thr Leu Arg Leu
            500                 505                 510

Val Asn Gln Tyr Leu Glu Cys Leu Val Leu Asp Lys Phe Glu Ser Tyr
        515                 520                 525

Asn Asp Glu Thr Gln Leu Thr Gln Arg Ala Leu Ser Leu Leu Glu Glu
    530                 535                 540

Asn Met Phe Trp Ala Gly Val Val Phe Pro Asp Met Tyr Pro Trp Thr
545                 550                 555                 560

Ser Ser Leu Pro Pro His Val Lys Tyr Lys Ile Arg Met Asp Ile Asp
                565                 570                 575

Val Val Glu Lys Thr Asn Lys Ile Lys Asp Arg Tyr Trp Asp Ser Gly
            580                 585                 590

Pro Arg Ala Asp Pro Val Glu Asp Phe Arg Tyr Ile Trp Gly Gly Phe
        595                 600                 605

Ala Tyr Leu Gln Asp Met Val Glu Gln Gly Ile Thr Arg Ser Gln Val
610                 615                 620

Gln Ala Glu Ala Pro Val Gly Ile Tyr Leu Gln Gln Met Pro Tyr Pro
625                 630                 635                 640

Cys Phe Val Asp Asp Ser Phe Met Ile Ile Leu Asn Arg Cys Phe Pro
                645                 650                 655

Ile Phe Met Val Leu Ala Trp Ile Tyr Ser Val Ser Met Thr Val Lys
            660                 665                 670

Ser Ile Val Leu Glu Lys Glu Leu Arg Leu Lys Glu Thr Leu Lys Asn
        675                 680                 685

Gln Gly Val Ser Asn Ala Val Ile Trp Cys Thr Trp Phe Leu Asp Ser
    690                 695                 700

Phe Ser Ile Met Ser Met Ser Ile Phe Leu Leu Thr Ile Phe Ile Met
705                 710                 715                 720

His Gly Arg Ile Leu His Tyr Ser Asp Pro Phe Ile Leu Phe Leu Phe
                725                 730                 735

Leu Leu Ala Phe Ser Thr Ala Thr Ile Met Leu Cys Phe Leu Leu Ser
            740                 745                 750

Thr Phe Phe Ser Lys Ala Ser Leu Ala Ala Ala Cys Ser Gly Val Ile
        755                 760                 765

Tyr Phe Thr Leu Tyr Leu Pro His Ile Leu Cys Phe Ala Trp Gln Asp
    770                 775                 780

Arg Met Thr Ala Glu Leu Lys Lys Ala Val Ser Leu Leu Ser Pro Val
785                 790                 795                 800

Ala Phe Gly Phe Gly Thr Glu Tyr Leu Val Arg Phe Glu Glu Gln Gly
                805                 810                 815

Leu Gly Leu Gln Trp Ser Asn Ile Gly Asn Ser Pro Thr Glu Gly Asp
            820                 825                 830

Glu Phe Ser Phe Leu Leu Ser Met Gln Met Met Leu Leu Asp Ala Ala
        835                 840                 845

Cys Tyr Gly Leu Leu Ala Trp Tyr Leu Asp Gln Val Phe Pro Gly Asp
    850                 855                 860

Tyr Gly Thr Pro Leu Pro Trp Tyr Phe Leu Leu Gln Glu Ser Tyr Trp
865                 870                 875                 880

Leu Ser Gly Glu Gly Cys Ser Thr Arg Glu Glu Arg Ala Leu Glu Lys
                885                 890                 895

Thr Glu Pro Leu Thr Glu Glu Thr Glu Asp Pro Glu His Pro Glu Gly
            900                 905                 910

Ile His Asp Ser Phe Phe Glu Arg Glu His Pro Gly Trp Val Pro Gly
        915                 920                 925

Val Cys Val Lys Asn Leu Val Lys Ile Phe Glu Pro Cys Gly Arg Pro
    930 .               935                 940

Ala Val Asp Arg Leu Asn Ile Thr Phe Tyr Glu Asn Gln Ile Thr Ala
945                 950                 955                 960

Phe Leu Gly His Asn Gly Ala Gly Lys Thr Thr Thr Leu Ser Ile Leu
                965                 970                 975

Thr Gly Leu Leu Pro Pro Thr Ser Gly Thr Val Leu Val Gly Gly Arg
            980                 985                 990

Asp Ile Glu Thr Ser Leu Asp Ala Val Arg Gln Ser Leu Gly Met Cys
        995                 1000                1005

Pro Gln His Asn Ile Leu Phe His His Leu Thr Val Ala Glu His Met
    1010                1015                1020

Leu Phe Tyr Ala Gln Leu Lys Gly Lys Ser Gln Glu Glu Ala Gln Leu
1025                1030                1035                1040

Glu Met Glu Ala Met Leu Glu Asp Thr Gly Leu His His Lys Arg Asn
                1045                1050                1055

Glu Glu Ala Gln Asp Leu Ser Gly Gly Met Gln Arg Lys Leu Ser Val
            1060                1065                1070

Ala Ile Ala Phe Val Gly Asp Ala Lys Val Val Ile Leu Asp Glu Pro
        1075                1080                1085

Thr Ser Gly Val Asp Pro Tyr Ser Arg Arg Ser Ile Trp Asp Leu Leu
    1090                1095                1100

Leu Lys Tyr Arg Ser Gly Arg Thr Ile Ile Met Pro Thr His His Met
1105                1110                1115                1120

Asp Glu Ala Asp His Gln Gly Asp Arg Ile Ala Ile Ile Ala Gln Gly
1125 1130 1135

Arg Leu Tyr Cys Ser Gly Thr Pro Leu Phe Leu Lys Asn Cys Phe Gly
1140 1145 1150

Thr Gly Leu Tyr Leu Thr Leu Val Arg Lys Met Lys Asn Ile Gln Ser
1155 1160 1165

Gln Arg Lys Gly Ser Glu Gly Thr Cys Ser Cys Ser Ser Lys Gly Phe
1170 1175 1180

Ser Thr Thr Cys Pro Ala His Val Asp Asp Leu Thr Pro Glu Gln Val
1185 1190 1195 1200

Leu Asp Gly Asp Val Asn Glu Leu Met Asp Val Val Leu His His Val
1205 1210 1215

Pro Glu Ala Lys Leu Val Glu Cys Ile Gly Gln Glu Leu Ile Phe Leu
1220 1225 1230

Leu Pro Asn Lys Asn Phe Lys His Arg Ala Tyr Ala Ser Leu Phe Arg
1235 1240 1245

Glu Leu Glu Glu Thr Leu Ala Asp Leu Gly Leu Ser Ser Phe Gly Ile
1250 1255 1260

Ser Asp Thr Pro Leu Glu Glu Ile Phe Leu Lys Val Thr Glu Asp Ser
1265 1270 1275 1280

Asp Ser Gly Pro Leu Phe Ala Gly Gly Ala Gln Gln Lys Arg Glu Asn
1285 1290 1295

Val Asn Pro Arg His Pro Cys Leu Gly Pro Arg Glu Lys Ala Gly Gln
1300 1305 1310

Thr Pro Gln Asp Ser Asn Val Cys Ser Pro Gly Ala Pro Ala Ala His
1315 1320 1325

Pro Glu Gly Gln Pro Pro Pro Glu Pro Glu Cys Pro Gly Pro Gln Leu
1330 1335 1340

Asn Thr Gly Thr Gln Leu Val Leu Gln His Val Gln Ala Leu Leu Val
1345 1350 1355 1360

Lys Arg Phe Gln His Thr Ile Arg Ser His Lys Asp Phe Leu Ala Gln
1365 1370 1375

Ile Val Leu Pro Ala Thr Phe Val Phe Leu Ala Leu Met Leu Ser Ile
1380 1385 1390

Val Ile Leu Pro Phe Gly Glu Tyr Pro Ala Leu Thr Leu His Pro Trp
1395 1400 1405

Ile Tyr Gly Gln Gln Tyr Thr Phe Phe Ser Met Asp Glu Pro Gly Ser
1410 1415 1420

Glu Gln Phe Thr Val Leu Ala Asp Val Leu Leu Asn Lys Pro Gly Phe
1425 1430 1435 1440

Gly Asn Arg Cys Leu Lys Glu Gly Trp Leu Pro Glu Tyr Pro Cys Gly
1445 1450 1455

Asn Ser Thr Pro Trp Lys Thr Pro Ser Val Ser Pro Asn Ile Thr Gln
1460 1465 1470

Leu Phe Gln Lys Gln Lys Trp Thr Gln Val Asn Pro Ser Pro Ser Cys
1475 1480 1485

Arg Cys Ser Thr Arg Glu Lys Leu Thr Met Leu Pro Glu Cys Pro Glu
1490 1495 1500

Gly Ala Gly Gly Leu Pro Pro Pro Gln Arg Thr Gln Arg Ser Thr Glu
1505 1510 1515 1520

Ile Leu Gln Asp Leu Thr Asp Arg Asn Ile Ser Asp Phe Leu Val Lys
1525 1530 1535

Thr Tyr Pro Ala Leu Ile Arg Ser Ser Leu Lys Ser Lys Phe Trp Val
1540 1545 1550

Asn Glu Gln Arg Tyr Gly Gly Ile Ser Ile Gly Gly Lys Leu Pro Val
1555 1560 1565

Val Pro Ile Thr Gly Glu Ala Leu Val Gly Phe Leu Ser Asp Leu Gly
1570 1575 1580

Arg Ile Met Asn Val Ser Gly Gly Pro Ile Thr Arg Glu Ala Ser Lys
1585 1590 1595 1600

Glu Ile Pro Asp Phe Leu Lys His Leu Glu Thr Glu Asp Asn Ile Lys
1605 1610 1615

Val Trp Phe Asn Asn Lys Gly Trp His Ala Leu Val Ser Phe Leu Asn
1620 1625 1630

Val Ala His Asn Ala Ile Leu Arg Ala Ser Leu Pro Lys Asp Arg Ser
1635 1640 1645

Pro Glu Glu Tyr Gly Ile Thr Val Ile Ser Gln Pro Leu Asn Leu Thr
1650 1655 1660

Lys Glu Gln Leu Ser Glu Ile Thr Val Leu Thr Thr Ser Val Asp Ala
1665 1670 1675 1680

Val Val Ala Ile Cys Val Ile Phe Ser Met Ser Phe Val Pro Ala Ser
1685 1690 1695

Phe Val Leu Tyr Leu Ile Gln Glu Arg Val Asn Lys Ser Lys His Leu
1700 1705 1710

Gln Phe Ile Ser Gly Val Ser Pro Thr Thr Tyr Trp Val Thr Asn Phe
1715 1720 1725

Leu Trp Asp Ile Met Asn Tyr Ser Val Ser Ala Gly Leu Val Val Gly
1730 1735 1740

Ile Phe Ile Gly Phe Gln Lys Lys Ala Tyr Thr Ser Pro Glu Asn Leu
1745 1750 1755 1760

Pro Ala Leu Val Ala Leu Leu Leu Leu Tyr Gly Trp Ala Val Ile Pro
1765 1770 1775

Met Met Tyr Pro Ala Ser Phe Leu Phe Asp Val Pro Ser Thr Ala Tyr
1780 1785 1790

Val Ala Leu Ser Cys Ala Asn Leu Phe Ile Gly Ile Asn Ser Ser Ala
1795 1800 1805

Ile Thr Phe Ile Leu Glu Leu Phe Asp Asn Asn Arg Thr Leu Leu Arg
1810 1815 1820

Phe Asn Ala Val Leu Arg Lys Leu Leu Ile Val Phe Pro His Phe Cys
1825 1830 1835 1840

Leu Gly Arg Gly Leu Ile Asp Leu Ala Leu Ser Gln Ala Val Thr Asp
1845 1850 1855

Val Tyr Ala Arg Phe Gly Glu Glu His Ser Ala Asn Pro Phe His Trp
1860 1865 1870

Asp Leu Ile Gly Lys Asn Leu Phe Ala Met Val Val Glu Gly Val Val
1875 1880 1885

Tyr Phe Leu Leu Thr Leu Leu Val Gln Arg His Phe Phe Leu Ser Gln
1890 1895 1900

Trp Ile Ala Glu Pro Thr Lys Glu Pro Ile Val Asp Glu Asp Asp Asp
1905 1910 1915 1920

Val Ala Glu Glu Arg Gln Arg Ile Ile Thr Gly Gly Asn Lys Thr Asp
1925 1930 1935

Ile Leu Arg Leu His Glu Leu Thr Lys Ile Tyr Leu Gly Thr Ser Ser
1940 1945 1950

Pro Ala Val Asp Arg Leu Cys Val Gly Val Arg Pro Gly Glu Cys Phe
1955 1960 1965

Gly Leu Leu Gly Val Asn Gly Ala Gly Lys Thr Thr Thr Phe Lys Met
1970 1975 1980

Leu Thr Gly Asp Thr Thr Val Thr Ser Gly Asp Ala Thr Val Ala Gly
1985 1990 1995 2000

Lys Ser Ile Leu Thr Asn Ile Ser Glu Val His Gln Asn Met Gly Tyr
2005 2010 2015

Cys Pro Gln Phe Asp Ala Ile Asp Glu Leu Leu Thr Gly Arg Glu His
2020 2025 2030

Leu Tyr Leu Tyr Ala Arg Leu Arg Gly Val Pro Ala Glu Glu Ile Glu
2035 2040 2045

Lys Val Ala Asn Trp Ser Ile Lys Ser Leu Gly Leu Thr Val Tyr Ala
2050 2055 2060

Asp Cys Leu Ala Gly Thr Tyr Ser Gly Gly Asn Lys Arg Lys Leu Ser
2065 2070 2075 2080

Thr Ala Ile Ala Leu Ile Gly Cys Pro Pro Leu Val Leu Leu Asp Glu
2085 2090 2095

Pro Thr Thr Gly Met Asp Pro Gln Ala Arg Arg Met Leu Trp Asn Val
2100 2105 2110

Ile Val Ser Ile Ile Arg Lys Gly Arg Ala Val Val Leu Thr Ser His
2115 2120 2125

Ser Met Glu Glu Cys Glu Ala Leu Cys Thr Arg Leu Ala Ile Met Val
2130 2135 2140

Lys Gly Ala Phe Arg Cys Met Gly Thr Ile Gln His Leu Lys Ser Lys
2145                2150                2155                2160

Phe Gly Asp Gly Tyr Ile Val Thr Met Lys Ile Lys Ser Pro Lys Asp
                2165                2170                2175

Asp Leu Leu Pro Asp Leu Asn Pro Val Glu Gln Phe Phe Gln Gly Asn
            2180                2185                2190

Phe Pro Gly Ser Val Gln Arg Glu Arg His Tyr Asn Met Leu Gln Phe
        2195                2200                2205

Gln Val Ser Ser Ser Ser Leu Ala Arg Ile Phe Gln Leu Leu Leu Ser
    2210                2215                2220

His Lys Asp Ser Leu Leu Ile Glu Glu Tyr Ser Val Thr Gln Thr Thr
2225                2230                2235                2240

Leu Asp Gln Val Phe Val Asn Phe Ala Lys Gln Gln Thr Glu Ser His
                2245                2250                2255

Asp Leu Pro Leu His Pro Arg Ala Ala Gly Ala Ser Arg Gln Ala Gln
            2260                2265                2270

Asp

<210> 48
<211> 2146
<212> PRT
<213> Homo sapiens

<400> 48
Met Ala Phe Trp Thr Gln Leu Met Leu Leu Leu Trp Lys Asn Phe Met
  1               5                  10                  15

Tyr Arg Arg Arg Gln Pro Val Gln Leu Leu Val Glu Leu Leu Trp Pro
             20                  25                  30

Leu Phe Leu Phe Phe Ile Leu Val Ala Val Arg His Ser His Pro Pro
         35                  40                  45

Leu Glu His His Glu Cys His Phe Pro Asn Lys Pro Leu Pro Ser Ala
     50                  55                  60

Gly Thr Val Pro Trp Leu Gln Gly Leu Ile Cys Asn Val Asn Asn Thr
65                  70                  75                  80

Cys Phe Pro Gln Leu Thr Pro Gly Glu Glu Pro Gly Arg Leu Ser Asn
                85                  90                  95

Phe Asn Asp Ser Leu Val Ser Arg Leu Leu Ala Asp Ala Arg Thr Val
            100                 105                 110

Leu Gly Gly Ala Ser Ala His Arg Thr Leu Ala Gly Leu Gly Lys Leu
        115                 120                 125

Ile Ala Thr Leu Arg Ala Ala Arg Ser Thr Ala Gln Pro Gln Pro Thr
    130                 135                 140

Lys Gln Ser Pro Leu Glu Pro Pro Met Leu Asp Val Ala Glu Leu Leu
145                 150                 155                 160

Thr Ser Leu Leu Arg Thr Glu Ser Leu Gly Leu Ala Leu Gly Gln Ala
                165                 170                 175

Gln Glu Pro Leu His Ser Leu Leu Glu Ala Ala Glu Asp Leu Ala Gln
            180                 185                 190

Glu Leu Leu Ala Leu Arg Ser Leu Val Glu Leu Arg Ala Leu Leu Gln
        195                 200                 205

Arg Pro Arg Gly Thr Ser Gly Pro Leu Glu Leu Leu Ser Glu Ala Leu
    210                 215                 220

Cys Ser Val Arg Gly Pro Ser Ser Thr Val Gly Pro Ser Leu Asn Trp
225                 230                 235                 240

Tyr Glu Ala Ser Asp Leu Met Glu Leu Val Gly Gln Glu Pro Glu Ser
                245                 250                 255

Ala Leu Pro Asp Ser Ser Leu Ser Pro Ala Cys Ser Glu Leu Ile Gly
            260                 265                 270

Ala Leu Asp Ser His Pro Leu Ser Arg Leu Leu Trp Arg Arg Leu Lys
        275                 280                 285

Pro Leu Ile Leu Gly Lys Leu Leu Phe Ala Pro Asp Thr Pro Phe Thr
    290                 295                 300

Arg Lys Leu Met Ala Gln Val Asn Arg Thr Phe Glu Glu Leu Thr Leu
305                 310                 315                 320

Leu Arg Asp Val Arg Glu Val Trp Glu Met Leu Gly Pro Arg Ile Phe
                325                 330                 335

Thr Phe Met Asn Asp Ser Ser Asn Val Ala Met Leu Gln Arg Leu Leu
            340                 345                 350

Gln Met Gln Asp Glu Gly Arg Arg Gln Pro Arg Pro Gly Gly Arg Asp
        355                 360                 365

His Met Glu Ala Leu Arg Ser Phe Leu Asp Pro Gly Ser Gly Gly Tyr
    370                 375                 380

Ser Trp Gln Asp Ala His Ala Asp Val Gly His Leu Val Gly Thr Leu
385                 390                 395                 400

Gly Arg Val Thr Glu Cys Leu Ser Leu Asp Lys Leu Glu Ala Ala Pro
                405                 410                 415

Ser Glu Ala Ala Leu Val Ser Arg Ala Leu Gln Leu Leu Ala Glu His
            420                 425                 430

Arg Phe Trp Ala Gly Val Val Phe Leu Gly Pro Glu Asp Ser Ser Asp
        435                 440                 445

Pro Thr Glu His Pro Thr Pro Asp Leu Gly Pro Gly His Val Arg Ile
    450                 455                 460

Lys Ile Arg Met Asp Ile Asp Val Val Thr Arg Thr Asn Lys Ile Arg
465                 470                 475                 480

Asp Arg Phe Trp Asp Pro Gly Pro Ala Ala Asp Pro Leu Thr Asp Leu
                485                 490                 495

Arg Tyr Val Trp Gly Gly Phe Val Tyr Leu Gln Asp Leu Val Glu Arg
            500                 505                 510

Ala Ala Val Arg Val Leu Ser Gly Ala Asn Pro Arg Ala Gly Leu Tyr
        515                 520                 525

Leu Gln Gln Met Pro Tyr Pro Cys Tyr Val Asp Asp Val Phe Leu Arg
    530                 535                 540

Val Leu Ser Arg Ser Leu Pro Leu Phe Leu Thr Leu Ala Trp Ile Tyr
545                 550                 555                 560

Ser Val Thr Leu Thr Val Lys Ala Val Val Arg Glu Lys Glu Thr Arg
                565                 570                 575

Leu Arg Asp Thr Met Arg Ala Met Gly Leu Ser Arg Ala Val Leu Trp
            580                 585                 590

Leu Gly Trp Phe Leu Ser Cys Leu Gly Pro Phe Leu Leu Ser Ala Ala
        595                 600                 605

Leu Leu Val Leu Val Leu Lys Leu Gly Asp Ile Leu Pro Tyr Ser His
    610                 615                 620

Pro Gly Val Val Phe Leu Phe Leu Ala Ala Phe Ala Val Ala Thr Val
625                 630                 635                 640

Thr Gln Ser Phe Leu Leu Ser Ala Phe Phe Ser Arg Ala Asn Leu Ala
                645                 650                 655

Ala Ala Cys Gly Gly Leu Ala Tyr Phe Ser Leu Tyr Leu Pro Tyr Val
            660                 665                 670

Leu Cys Val Ala Trp Arg Asp Arg Leu Pro Ala Gly Gly Arg Val Ala
        675                 680                 685

Ala Ser Leu Leu Ser Pro Val Ala Phe Gly Phe Gly Cys Glu Ser Leu
    690                 695                 700

Ala Leu Leu Glu Glu Gln Gly Glu Gly Ala Gln Trp His Asn Val Gly
705                 710                 715                 720

Thr Arg Pro Thr Ala Asp Val Phe Ser Leu Ala Gln Val Ser Gly Leu
                725                 730                 735

Leu Leu Leu Asp Ala Ala Leu Tyr Gly Leu Ala Thr Trp Tyr Leu Glu
            740                 745                 750

Ala Val Cys Pro Gly Gln Tyr Gly Ile Pro Glu Pro Trp Asn Phe Pro
        755                 760                 765

Phe Arg Arg Ser Tyr Trp Cys Gly Pro Arg Pro Pro Lys Ser Pro Ala
    770                 775                 780

Pro Cys Pro Thr Pro Leu Asp Pro Lys Val Leu Val Glu Glu Ala Pro
785                 790                 795                 800

Pro Gly Leu Ser Pro Gly Val Ser Val Arg Ser Leu Glu Lys Arg Phe
                805                 810                 815

Pro Gly Ser Pro Gln Pro Ala Leu Arg Gly Leu Ser Leu Asp Phe Tyr
            820                 825                 830

Gln Gly His Ile Thr Ala Phe Leu Gly His Asn Gly Ala Gly Lys Thr
        835                 840                 845

Thr Thr Leu Ser Ile Leu Ser Gly Leu Phe Pro Pro Ser Gly Gly Ser
    850                 855                 860

Ala Phe Ile Leu Gly His Asp Val Arg Ser Ser Met Ala Ala Ile Arg
865                 870                 875                 880

Pro His Leu Gly Val Cys Pro Gln Tyr Asn Val Leu Phe Asp Met Leu
                885                 890                 895

Thr Val Asp Glu His Val Trp Phe Tyr Gly Arg Leu Lys Gly Leu Ser
            900                 905                 910

Ala Ala Val Val Gly Pro Glu Gln Asp Arg Leu Leu Gln Asp Val Gly
        915                 920                 925

Leu Val Ser Lys Gln Ser Val Gln Thr Arg His Leu Ser Gly Gly Met
    930                 935                 940

Gln Arg Lys Leu Ser Val Ala Ile Ala Phe Val Gly Gly Ser Gln Val
945                 950                 955                 960

Val Ile Leu Asp Glu Pro Thr Ala Gly Val Asp Pro Ala Ser Arg Arg
                965                 970                 975

Gly Ile Trp Glu Leu Leu Leu Lys Tyr Arg Glu Gly Arg Thr Leu Ile
            980                 985                 990

Leu Ser Thr His His Leu Asp Glu Ala Glu Leu Leu Gly Asp Arg Val
        995                 1000                1005

Ala Val Val Ala Gly Gly Arg Leu Cys Cys Cys Gly Ser Pro Leu Phe
    1010                1015                1020

Leu Arg Arg His Leu Gly Ser Gly Tyr Tyr Leu Thr Leu Val Lys Ala
1025                1030                1035                1040

Arg Leu Pro Leu Thr Thr Asn Glu Lys Ala Asp Thr Asp Met Glu Gly
                1045                1050                1055

Ser Val Asp Thr Arg Gln Glu Lys Lys Asn Gly Ser Gln Gly Ser Arg
            1060                1065                1070

Val Gly Thr Pro Gln Leu Leu Ala Leu Val Gln His Trp Val Pro Gly
        1075                1080                1085

Ala Arg Leu Val Glu Glu Leu Pro His Glu Leu Val Leu Val Leu Pro
    1090                1095                1100

Tyr Thr Gly Ala His Asp Gly Ser Phe Ala Thr Leu Phe Arg Glu Leu
1105                1110                1115                1120

Asp Thr Arg Leu Ala Glu Leu Arg Leu Thr Gly Tyr Gly Ile Ser Asp
                1125                1130                1135

Thr Ser Leu Glu Glu Ile Phe Leu Lys Val Val Glu Glu Cys Ala Ala
            1140                1145                1150

Asp Thr Asp Met Glu Asp Gly Ser Cys Gly Gln His Leu Cys Thr Gly
        1155                1160                1165

Ile Ala Gly Leu Asp Val Thr Leu Arg Leu Lys Met Pro Pro Gln Glu
    1170                1175                1180

Thr Ala Leu Glu Asn Gly Glu Pro Ala Gly Ser Ala Pro Glu Thr Asp
1185                1190                1195                1200

Gln Gly Ser Gly Pro Asp Ala Val Gly Arg Val Gln Gly Trp Ala Leu
                1205                1210                1215

Thr Arg Gln Gln Leu Gln Ala Leu Leu Leu Lys Arg Phe Leu Leu Ala
            1220                1225                1230

Arg Arg Ser Arg Arg Gly Leu Phe Ala Gln Ile Val Leu Pro Ala Leu
        1235                1240                1245

Phe Val Gly Leu Ala Leu Val Phe Ser Leu Ile Val Pro Pro Phe Gly
    1250                1255                1260

His Tyr Pro Ala Leu Arg Leu Ser Pro Thr Met Tyr Gly Ala Gln Val
1265                1270                1275                1280

Ser Phe Phe Ser Glu Asp Ala Pro Gly Asp Pro Gly Arg Ala Arg Leu
                1285                1290                1295

Leu Glu Ala Leu Leu Gln Glu Ala Gly Leu Glu Glu Pro Pro Val Gln
            1300                1305                1310

His Ser Ser His Arg Phe Ser Ala Pro Glu Val Pro Ala Glu Val Ala
        1315                1320                1325

Lys Val Leu Ala Ser Gly Asn Trp Thr Pro Glu Ser Pro Ser Pro Ala
    1330                1335                1340

Cys Gln Cys Ser Gln Pro Gly Ala Arg Arg Leu Leu Pro Asp Cys Pro
1345                1350                1355                1360

Ala Ala Ala Gly Gly Pro Pro Pro Pro Gln Ala Val Thr Gly Ser Gly
                1365                1370                1375

Glu Val Val Gln Asn Leu Thr Gly Arg Asn Leu Ser Asp Phe Leu Val
            1380                1385                1390

Lys Thr Tyr Pro Arg Leu Val Arg Gln Gly Leu Lys Thr Lys Lys Trp
        1395                1400                1405

Val Asn Glu Val Arg Tyr Gly Gly Phe Ser Leu Gly Gly Arg Asp Pro
    1410                1415                1420

Gly Leu Pro Ser Gly Gln Glu Leu Gly Arg Ser Val Glu Glu Leu Trp
1425                1430                1435                1440

Ala Leu Leu Ser Pro Leu Pro Gly Gly Ala Leu Asp Arg Val Leu Lys
                1445                1450                1455

Asn Leu Thr Ala Trp Ala His Ser Leu Asp Ala Gln Asp Ser Leu Lys
            1460                1465                1470

Ile Trp Phe Asn Asn Lys Gly Trp His Ser Met Val Ala Phe Val Asn
        1475                1480                1485

Arg Ala Ser Asn Ala Ile Leu Arg Ala His Leu Pro Pro Gly Arg Ala
    1490                1495                1500

Arg His Ala His Ser Ile Thr Thr Leu Asn His Pro Leu Asn Leu Thr
1505                1510                1515                1520

Lys Glu Gln Leu Phe Glu Ala Ala Leu Met Ala Ser Ser Val Asp Val
                1525                1530                1535

Leu Val Ser Ile Cys Val Val Phe Ala Met Ser Phe Val Pro Ala Ser
            1540                1545                1550

Phe Thr Leu Val Leu Ile Glu Glu Arg Val Thr Arg Ala Lys His Leu
        1555                1560                1565

Gln Leu Met Gly Gly Leu Ser Pro Thr Leu Tyr Trp Leu Gly Asn Phe
    1570                1575                1580

Leu Trp Asp Met Cys Asn Tyr Leu Val Pro Ala Cys Ile Val Val Leu
1585                1590                1595                1600

Ile Phe Leu Ala Phe Gln Gln Arg Ala Tyr Val Ala Pro Ala Asn Leu
                1605                1610                1615

Pro Ala Leu Leu Leu Leu Leu Leu Leu Tyr Gly Trp Ser Ile Thr Pro
            1620                1625                1630

Leu Met Tyr Pro Ala Ser Phe Phe Phe Ser Val Pro Ser Thr Ala Tyr
        1635                1640                1645

Val Val Leu Thr Cys Ile Asn Leu Phe Ile Gly Ile Asn Gly Ser Met
    1650                1655                1660

Ala Thr Phe Val Leu Glu Leu Phe Ser Asp Gln Lys Leu Gln Glu Val
1665                1670                1675                1680

Ser Arg Ile Leu Lys Gln Val Phe Leu Ile Phe Pro His Phe Cys Leu
                1685                1690                1695

Gly Arg Gly Leu Ile Asp Met Val Arg Asn Gln Ala Met Ala Asp Ala
            1700                1705                1710

Phe Glu Arg Leu Gly Asp Arg Gln Phe Gln Ser Pro Leu Arg Trp Glu
        1715                1720                1725

Val Val Gly Lys Asn Leu Leu Ala Met Val Ile Gln Gly Pro Leu Phe
    1730                1735                1740

Leu Leu Phe Thr Leu Leu Leu Gln His Arg Ser Gln Leu Leu Pro Gln
1745                1750                1755                1760

Pro Arg Val Arg Ser Leu Pro Leu Leu Gly Glu Glu Asp Glu Asp Val
                1765                1770                1775

Ala Arg Glu Arg Glu Arg Val Val Gln Gly Ala Thr Gln Gly Asp Val
            1780                1785                1790

Leu Val Leu Arg Asn Leu Thr Lys Val Tyr Arg Gly Gln Arg Met Pro
        1795                1800                1805

Ala Val Asp Arg Leu Cys Leu Gly Ile Pro Pro Gly Glu Cys Phe Gly
    1810                1815                1820

Leu Leu Gly Val Asn Gly Ala Gly Lys Thr Ser Thr Phe Arg Met Val
1825                1830                1835                1840

Thr Gly Asp Thr Leu Ala Ser Arg Gly Glu Ala Val Leu Ala Gly His
                1845                1850                1855

Ser Val Ala Arg Glu Pro Ser Ala Ala His Leu Ser Met Gly Tyr Cys
            1860                1865                1870

Pro Gln Ser Asp Ala Ile Phe Glu Leu Leu Thr Gly Arg Glu His Leu
        1875                1880                1885

Glu Leu Leu Ala Arg Leu Arg Gly Val Pro Glu Ala Gln Val Ala Gln
    1890                1895                1900

Thr Ala Gly Ser Gly Leu Ala Arg Leu Gly Leu Ser Trp Tyr Ala Asp
1905                1910                1915                1920

Arg Pro Ala Gly Thr Tyr Ser Gly Gly Asn Lys Arg Lys Leu Ala Thr
                1925                1930                1935

Ala Leu Ala Leu Val Gly Asp Pro Ala Val Val Phe Leu Asp Glu Pro
            1940                1945                1950

Thr Thr Gly Met Asp Pro Ser Ala Arg Arg Phe Leu Trp Asn Ser Leu
        1955                1960                1965

Leu Ala Val Val Arg Glu Gly Arg Ser Val Met Leu Thr Ser His Ser
    1970                1975                1980

Met Glu Glu Cys Glu Ala Leu Cys Ser Arg Leu Ala Ile Met Val Asn
1985                1990                1995                2000

Gly Arg Phe Arg Cys Leu Gly Ser Pro Gln His Leu Lys Gly Arg Phe
                2005                2010                2015

Ala Ala Gly His Thr Leu Thr Leu Arg Val Pro Ala Ala Arg Ser Gln
            2020                2025                2030

Pro Ala Ala Ala Phe Val Ala Ala Glu Phe Pro Gly Ser Glu Leu Arg
        2035                2040                2045

Glu Ala His Gly Gly Arg Leu Arg Phe Gln Leu Pro Pro Gly Gly Arg
    2050                2055                2060

Cys Ala Leu Ala Arg Val Phe Gly Glu Leu Ala Val His Gly Ala Glu
2065                2070                2075                2080

His Gly Val Glu Asp Phe Ser Val Ser Gln Thr Met Leu Glu Glu Val
                2085                2090                2095

Phe Leu Tyr Phe Ser Lys Asp Gln Gly Lys Asp Glu Asp Thr Glu Glu
            2100                2105                2110

Gln Lys Glu Ala Gly Val Gly Val Asp Pro Ala Pro Gly Leu Gln His
        2115                2120                2125

Pro Lys Arg Val Ser Gln Phe Leu Asp Asp Pro Ser Thr Ala Glu Thr
    2130                2135                2140

Val Leu
2145

<210> 49
<211> 1581
<212> PRT
<213> Homo sapiens

<400> 49
Met Arg Lys Arg Lys Ile Ser Val Cys Gln Gln Thr Trp Ala Leu Leu
1               5                   10                  15

Cys Lys Asn Phe Leu Lys Lys Trp Arg Met Lys Arg Glu Ser Leu Met
            20                  25                  30

Glu Trp Leu Asn Ser Leu Leu Leu Leu Leu Cys Leu Tyr Ile Tyr Pro
        35                  40                  45

His Ser His Gln Val Asn Asp Phe Ser Ser Leu Leu Thr Met Asp Leu
    50                  55                  60

Gly Arg Val Asp Thr Phe Asn Glu Ser Arg Phe Ser Val Val Tyr Thr
65                  70                  75                  80

Pro Val Thr Asn Thr Thr Gln Gln Ile Met Asn Lys Val Ala Ser Thr
                85                  90                  95

Pro Phe Leu Ala Gly Lys Glu Val Leu Gly Leu Pro Asp Glu Glu Ser
            100                 105                 110

Ile Lys Glu Phe Thr Ala Asn Tyr Pro Glu Glu Ile Val Arg Val Thr
        115                 120                 125

Phe Thr Asn Thr Tyr Ser Tyr His Leu Lys Phe Leu Leu Gly His Gly
    130                 135                 140

Met Pro Ala Lys Lys Glu His Lys Asp His Thr Ala His Cys Tyr Glu
145                 150                 155                 160

Thr Asn Glu Asp Val Tyr Cys Glu Val Ser Val Phe Trp Lys Glu Gly

                165                 170                 175

Phe Val Ala Leu Gln Ala Ala Ile Asn Ala Ala Ile Ile Glu Ile Thr
            180                 185                 190

Thr Asn His Ser Val Met Glu Glu Leu Met Ser Val Thr Gly Lys Asn
        195                 200                 205

Met Lys Met His Ser Phe Ile Gly Gln Ser Gly Val Ile Thr Asp Leu
    210                 215                 220

Tyr Leu Phe Ser Cys Ile Ile Ser Phe Ser Ser Phe Ile Tyr Tyr Ala
225                 230                 235                 240

Ser Val Asn Val Thr Arg Glu Arg Lys Arg Met Lys Ala Leu Met Thr
                245                 250                 255

Met Met Gly Leu Arg Asp Ser Ala Phe Trp Leu Ser Trp Gly Leu Leu
            260                 265                 270

Tyr Ala Gly Phe Ile Phe Ile Met Ala Leu Phe Leu Ala Leu Val Ile
        275                 280                 285

Arg Ser Thr Gln Phe Ile Ile Leu Ser Gly Phe Met Val Val Phe Ser
    290                 295                 300

Leu Phe Leu Leu Tyr Gly Leu Ser Leu Val Ala Leu Ala Phe Leu Met
305                 310                 315                 320

Ser Ile Leu Val Lys Lys Ser Phe Leu Thr Gly Leu Val Val Phe Leu
                325                 330                 335

Leu Thr Val Phe Trp Gly Cys Leu Gly Phe Thr Ser Leu Tyr Arg His
            340                 345                 350

Leu Pro Ala Ser Leu Glu Trp Ile Leu Ser Leu Leu Ser Pro Phe Ala
        355                 360                 365

Phe Met Leu Gly Met Ala Gln Leu Leu His Leu Asp Tyr Asp Leu Asn
    370                 375                 380

Ser Asn Ala Phe Pro His Pro Ser Asp Gly Ser Asn Leu Ile Val Ala
385                 390                 395                 400

Thr Asn Phe Met Leu Ala Phe Asp Thr Cys Leu Tyr Leu Ala Leu Ala
                405                 410                 415

Ile Tyr Phe Glu Lys Ile Leu Pro Asn Glu Tyr Gly His Arg Arg Pro

420                 425                 430

Pro Leu Phe Phe Leu Lys Ser Ser Phe Trp Ser Gln Thr Gln Lys Thr
        435                 440                 445

Asp His Val Ala Leu Glu Asp Glu Met Asp Ala Asp Pro Ser Phe His
    450                 455                 460

Asp Ser Phe Glu Gln Ala Pro Pro Glu Phe Gln Gly Lys Glu Ala Ile
465                 470                 475                 480

Arg Ile Arg Asn Val Thr Lys Glu Tyr Lys Gly Lys Pro Asp Lys Ile
                485                 490                 495

Glu Ala Leu Lys Asp Leu Val Phe Asp Ile Tyr Glu Gly Gln Ile Thr
            500                 505                 510

Ala Ile Leu Gly His Ser Gly Ala Gly Lys Ser Thr Leu Leu Asn Ile
        515                 520                 525

Leu Ser Gly Leu Ser Val Pro Thr Lys Gly Ser Val Thr Ile Tyr Asn
    530                 535                 540

Asn Lys Leu Ser Glu Met Ala Asp Leu Glu Asn Leu Ser Lys Leu Thr
545                 550                 555                 560

Gly Val Cys Pro Gln Ser Asn Val Gln Phe Asp Phe Leu Thr Val Arg
                565                 570                 575

Glu Asn Leu Arg Leu Phe Ala Lys Ile Lys Gly Ile Leu Pro Gln Glu
            580                 585                 590

Val Asp Lys Glu Ile Phe Leu Leu Asp Glu Pro Thr Ala Gly Leu Asp
        595                 600                 605

Pro Phe Ser Arg His Gln Val Trp Asn Leu Leu Lys Glu Arg Lys Thr
    610                 615                 620

Asp Arg Val Ile Leu Phe Ser Thr Gln Phe Met Asp Glu Ala Asp Ile
625                 630                 635                 640

Leu Ala Asp Arg Lys Val Phe Leu Ser Gln Gly Lys Leu Lys Cys Ala
                645                 650                 655

Gly Ser Ser Leu Phe Leu Lys Lys Lys Trp Gly Ile Gly Tyr His Leu
            660                 665                 670

Ser Leu Gln Leu Asn Glu Ile Cys Val Glu Glu Asn Ile Thr Ser Leu

675                 680                 685

Val Lys Gln His Ile Pro Asp Ala Lys Leu Ser Ala Lys Ser Glu Gly
    690                 695                 700

Lys Leu Ile Tyr Thr Leu Pro Leu Glu Arg Thr Asn Lys Phe Pro Glu
705                 710                 715                 720

Leu Tyr Lys Asp Leu Asp Ser Tyr Pro Asp Leu Gly Ile Glu Asn Tyr
                725                 730                 735

Gly Val Ser Met Thr Thr Leu Asn Glu Val Phe Leu Lys Leu Glu Gly
            740                 745                 750

Lys Ser Thr Ile Asn Glu Ser Asp Ile Ala Ile Leu Gly Glu Val Gln
        755                 760                 765

Ala Glu Lys Ala Asp Asp Thr Glu Arg Leu Val Glu Met Glu Gln Val
    770                 775                 780

Leu Ser Ser Leu Asn Lys Met Arg Lys Thr Ile Gly Gly Val Ala Leu
785                 790                 795                 800

Trp Arg Gln Gln Ile Cys Ala Ile Ala Arg Val Arg Leu Leu Lys Leu
                805                 810                 815

Lys His Glu Arg Lys Ala Leu Leu Ala Leu Leu Leu Ile Leu Met Ala
            820                 825                 830

Gly Phe Cys Pro Leu Leu Val Glu Tyr Thr Met Val Lys Ile Tyr Gln
        835                 840                 845

Asn Ser Tyr Thr Trp Glu Leu Ser Pro His Leu Tyr Phe Leu Ala Pro
    850                 855                 860

Gly Gln Gln Pro His Asp Pro Leu Thr Gln Leu Leu Ile Ile Asn Lys
865                 870                 875                 880

Thr Gly Ala Ser Ile Asp Asp Phe Ile Gln Ser Val Glu His Gln Asn
                885                 890                 895

Ile Ala Leu Glu Val Asp Ala Phe Gly Thr Arg Asn Gly Thr Asp Asp
            900                 905                 910

Pro Ser Tyr Asn Gly Ala Ile Thr Val Cys Cys Asn Glu Lys Asn Tyr
        915                 920                 925

Ser Phe Ser Leu Ala Cys Asn Ala Lys Arg Leu Asn Cys Phe Pro Val

930                 935                 940

Leu Met Asp Ile Val Ser Asn Gly Leu Leu Gly Met Val Lys Pro Ser
945                 950                 955                 960

Val His Ile Arg Thr Glu Arg Ser Thr Phe Leu Glu Asn Gly Gln Asp
                965                 970                 975

Asn Pro Ile Gly Phe Leu Ala Tyr Ile Met Phe Trp Leu Val Leu Thr
            980                 985                 990

Ser Ser Cys Pro Pro Tyr Ile Ala Met Ser Ser Ile Asp Asp Tyr Lys
        995                 1000                1005

Asn Arg Ala Arg Ser Gln Leu Arg Ile Ser Gly Leu Ser Pro Ser Ala
    1010                1015                1020

Tyr Trp Phe Gly Gln Ala Leu Val Asp Val Ser Leu Tyr Phe Leu Val
1025                1030                1035                1040

Phe Val Phe Ile Tyr Leu Met Ser Tyr Ile Ser Asn Phe Glu Asp Met
                1045                1050                1055

Leu Leu Thr Ile Ile His Ile Ile Gln Ile Pro Cys Ala Val Gly Tyr
            1060                1065                1070

Ser Phe Ser Leu Ile Phe Met Thr Tyr Val Ile Ser Phe Ile Phe Arg
        1075                1080                1085

Lys Gly Arg Lys Asn Ser Gly Ile Trp Ser Phe Cys Phe Tyr Val Val
    1090                1095                1100

Thr Val Phe Ser Val Ala Gly Phe Ala Phe Ser Ile Phe Glu Ser Asp
1105                1110                1115                1120

Ile Pro Phe Ile Phe Thr Phe Leu Ile Pro Pro Ala Thr Met Ile Gly
                1125                1130                1135

Cys Leu Phe Leu Ser Ser His Leu Leu Phe Ser Ser Leu Phe Ser Glu
            1140                1145                1150

Glu Arg Met Asp Val Gln Pro Phe Leu Val Phe Leu Ile Pro Phe Leu
        1155                1160                1165

His Phe Ile Ile Phe Leu Phe Thr Leu Arg Cys Leu Glu Trp Lys Phe
    1170                1175                1180

Gly Lys Lys Ser Met Arg Lys Asp Pro Phe Phe Arg Ile Ser Pro Arg

1185                1190                1195                1200

Ser Ser Asp Val Cys Gln Asn Pro Glu Glu Pro Glu Gly Glu Asp Glu
                1205                1210                1215

Asp Val Gln Met Glu Arg Val Arg Thr Ala Asn Ala Leu Asn Ser Thr
            1220                1225                1230

Asn Phe Asp Glu Lys Pro Val Ile Ile Ala Ser Cys Leu Arg Lys Glu
        1235                1240                1245

Tyr Ala Gly Lys Arg Lys Gly Cys Phe Ser Lys Arg Lys Asn Lys Ile
    1250                1255                1260

Ala Thr Arg Asn Val Ser Phe Cys Val Arg Lys Gly Glu Val Leu Gly
1265                1270                1275                1280

Leu Leu Gly His Asn Gly Ala Gly Lys Ser Thr Ser Ile Lys Val Ile
                1285                1290                1295

Thr Gly Asp Thr Lys Pro Thr Ala Gly Gln Val Leu Leu Lys Gly Ser
            1300                1305                1310

Gly Gly Gly Asp Ala Leu Glu Phe Leu Gly Tyr Cys Pro Gln Glu Asn
        1315                1320                1325

Ala Leu Trp Pro Asn Leu Thr Val Arg Gln His Leu Glu Val Tyr Ala
    1330                1335                1340

Ala Val Lys Gly Leu Arg Lys Gly Asp Ala Glu Val Ala Ile Thr Arg
1345                1350                1355                1360

Leu Val Asp Ala Leu Lys Leu Gln Asp Gln Leu Lys Ser Pro Val Lys
                1365                1370                1375

Thr Leu Ser Glu Gly Ile Lys Arg Lys Leu Cys Phe Val Leu Ser Ile
            1380                1385                1390

Leu Gly Asn Pro Ser Val Val Leu Leu Asp Glu Pro Ser Thr Gly Met
        1395                1400                1405

Asp Pro Glu Gly Gln Gln Gln Met Trp Gln Ala Ile Arg Ala Thr Phe
    1410                1415                1420

Arg Asn Thr Glu Arg Gly Ala Leu Leu Thr Thr His Tyr Met Ala Glu
1425                1430                1435                1440

Ala Glu Ala Val Cys Asp Arg Val Ala Ile Met Val Ser Gly Arg Leu

1445                1450                1455

Arg Cys Ile Gly Ser Ile Gln His Leu Lys Ser Lys Phe Gly Lys Asp
        1460                1465                1470

Tyr Leu Leu Glu Met Lys Val Lys Asn Leu Ala Gln Val Glu Pro Leu
    1475                1480                1485

His Ala Glu Ile Leu Arg Leu Phe Pro Gln Ala Ala Arg Gln Glu Arg
  1490                1495                1500

Tyr Ser Ser Leu Met Val Tyr Lys Leu Pro Val Glu Asp Val Gln Pro
1505                1510                1515                1520

Leu Ala Gln Ala Phe Phe Lys Leu Glu Lys Val Lys Gln Ser Phe Asp
                1525                1530                1535

Leu Glu Glu Tyr Ser Leu Ser Gln Ser Thr Leu Glu Gln Val Phe Leu
        1540                1545                1550

Glu Leu Ser Lys Glu Gln Glu Leu Gly Asp Phe Glu Glu Asp Phe Asp
    1555                1560                1565

Pro Ser Val Lys Trp Lys Leu Leu Pro Gln Glu Glu Pro
  1570                1575                1580

<210> 50
<211> 1279
<212> PRT
<213> Homo sapiens

<400> 50
Met Asp Leu Glu Gly Asp Arg Asn Gly Gly Ala Lys Lys Lys Asn Phe
 1               5                  10                  15

Phe Lys Leu Asn Asn Lys Ser Glu Lys Asp Lys Lys Glu Lys Lys Pro
            20                  25                  30

Thr Val Ser Val Phe Ser Met Phe Arg Tyr Ser Asn Trp Leu Asp Lys
        35                  40                  45

Leu Tyr Met Val Val Gly Thr Leu Ala Ala Ile Ile His Gly Ala Gly
    50                  55                  60

Leu Pro Leu Met Met Leu Val Phe Gly Glu Met Thr Asp Ile Phe Ala
65                  70                  75                  80

Asn Ala Gly Asn Leu Glu Asp Leu Met Ser Asn Ile Thr Asn Arg Ser
                85                  90                  95

Asp Ile Asn Asp Thr Gly Phe Phe Met Asn Leu Glu Glu Asp Met Thr
            100                 105                 110

Arg Tyr Ala Tyr Tyr Tyr Ser Gly Ile Gly Ala Gly Val Leu Val Ala
        115                 120                 125

Ala Tyr Ile Gln Val Ser Phe Trp Cys Leu Ala Ala Gly Arg Gln Ile
    130                 135                 140

His Lys Ile Arg Lys Gln Phe Phe His Ala Ile Met Arg Gln Glu Ile
145                 150                 155                 160

Gly Trp Phe Asp Val His Asp Val Gly Glu Leu Asn Thr Arg Leu Thr
                165                 170                 175

Asp Asp Val Ser Lys Ile Asn Glu Gly Ile Gly Asp Lys Ile Gly Met
            180                 185                 190

Phe Phe Gln Ser Met Ala Thr Phe Phe Thr Gly Phe Ile Val Gly Phe
        195                 200                 205

Thr Arg Gly Trp Lys Leu Thr Leu Val Ile Leu Ala Ile Ser Pro Val
    210                 215                 220

Leu Gly Leu Ser Ala Ala Val Trp Ala Lys Ile Leu Ser Ser Phe Thr
225                 230                 235                 240

Asp Lys Glu Leu Leu Ala Tyr Ala Lys Ala Gly Ala Val Ala Glu Glu
                245                 250                 255

Val Leu Ala Ala Ile Arg Thr Val Ile Ala Phe Gly Gly Gln Lys Lys
            260                 265                 270

Glu Leu Glu Arg Tyr Asn Lys Asn Leu Glu Glu Ala Lys Arg Ile Gly
        275                 280                 285

Ile Lys Lys Ala Ile Thr Ala Asn Ile Ser Ile Gly Ala Ala Phe Leu
    290                 295                 300

Leu Ile Tyr Ala Ser Tyr Ala Leu Ala Phe Trp Tyr Gly Thr Thr Leu
305                 310                 315                 320

Val Leu Ser Gly Glu Tyr Ser Ile Gly Gln Val Leu Thr Val Phe Ser
                325                 330                 335

Val Leu Ile Gly Ala Phe Ser Val Gly Gln Ala Ser Pro Ser Ile Glu
            340                 345                 350

Ala Phe Ala Asn Ala Arg Gly Ala Ala Tyr Glu Ile Phe Lys Ile Ile
        355                 360                 365

Asp Asn Lys Pro Ser Ile Asp Ser Tyr Ser Lys Ser Gly His Lys Pro
    370                 375                 380

Asp Asn Ile Lys Gly Asn Leu Glu Phe Arg Asn Val His Phe Ser Tyr
385                 390                 395                 400

Pro Ser Arg Lys Glu Val Lys Ile Leu Lys Gly Leu Asn Leu Lys Val
                405                 410                 415

Gln Ser Gly Gln Thr Val Ala Leu Val Gly Asn Ser Gly Cys Gly Lys
            420                 425                 430

Ser Thr Thr Val Gln Leu Met Gln Arg Leu Tyr Asp Pro Thr Glu Gly
        435                 440                 445

Met Val Ser Val Asp Gly Gln Asp Ile Arg Thr Ile Asn Val Arg Phe
    450                 455                 460

Leu Arg Glu Ile Ile Gly Val Val Ser Gln Glu Pro Val Leu Phe Ala
465                 470                 475                 480

Thr Thr Ile Ala Glu Asn Ile Arg Tyr Gly Arg Glu Asn Val Thr Met
                485                 490                 495

Asp Glu Ile Glu Lys Ala Val Lys Glu Ala Asn Ala Tyr Asp Phe Ile
            500                 505                 510

Met Lys Leu Pro His Lys Phe Asp Thr Leu Val Gly Glu Arg Gly Ala
        515                 520                 525

Gln Leu Ser Gly Gly Gln Lys Gln Arg Ile Ala Ile Ala Arg Ala Leu
    530                 535                 540

Val Arg Asn Pro Lys Ile Leu Leu Leu Asp Glu Ala Thr Ser Ala Leu
545                 550                 555                 560

Asp Thr Glu Ser Glu Ala Val Val Gln Val Ala Leu Asp Lys Ala Arg
                565                 570                 575

Lys Gly Arg Thr Thr Ile Val Ile Ala His Arg Leu Ser Thr Val Arg
            580                 585                 590

Asn Ala Asp Val Ile Ala Gly Phe Asp Asp Gly Val Ile Val Glu Lys
        595                 600                 605

Gly Asn His Asp Glu Leu Met Lys Glu Lys Gly Ile Tyr Phe Lys Leu
    610                 615                 620

Val Thr Met Gln Thr Ala Gly Asn Glu Val Glu Leu Glu Asn Ala Ala
625                 630                 635                 640

Asp Glu Ser Lys Ser Glu Ile Asp Ala Leu Glu Met Ser Ser Asn Asp
                645                 650                 655

Ser Arg Ser Ser Leu Ile Arg Lys Arg Ser Thr Arg Arg Ser Val Arg
            660                 665                 670

Gly Ser Gln Ala Gln Asp Arg Lys Leu Ser Thr Lys Glu Ala Leu Asp
        675                 680                 685

Glu Ser Ile Pro Pro Val Ser Phe Trp Arg Ile Met Lys Leu Asn Leu
    690                 695                 700

Thr Glu Trp Pro Tyr Phe Val Val Gly Val Phe Cys Ala Ile Ile Asn
705                 710                 715                 720

Gly Gly Leu Gln Pro Ala Phe Ala Ile Ile Phe Ser Lys Ile Ile Gly
                725                 730                 735

Val Phe Thr Arg Ile Asp Asp Pro Glu Thr Lys Arg Gln Asn Ser Asn
            740                 745                 750

Leu Phe Ser Leu Leu Phe Leu Ala Leu Gly Ile Ile Ser Phe Ile Thr
        755                 760                 765

Phe Phe Leu Gln Gly Phe Thr Phe Gly Lys Ala Gly Glu Ile Leu Thr
    770                 775                 780

Lys Arg Leu Arg Tyr Met Val Phe Arg Ser Met Leu Arg Gln Asp Val
785                 790                 795                 800

Ser Trp Phe Asp Asp Pro Lys Asn Thr Thr Gly Ala Leu Thr Thr Arg
                805                 810                 815

Leu Ala Asn Asp Ala Ala Gln Val Lys Gly Ala Ile Gly Ser Arg Leu
            820                 825                 830

Ala Val Ile Thr Gln Asn Ile Ala Asn Leu Gly Thr Gly Ile Ile Ile
        835                 840                 845

Ser Phe Ile Tyr Gly Trp Gln Leu Thr Leu Leu Leu Leu Ala Ile Val
    850                 855                 860

Pro Ile Ile Ala Ile Ala Gly Val Val Glu Met Lys Met Leu Ser Gly
865                 870                 875                 880

Gln Ala Leu Lys Asp Lys Lys Glu Leu Glu Gly Ser Gly Lys Ile Ala
                885                 890                 895

Thr Glu Ala Ile Glu Asn Phe Arg Thr Val Val Ser Leu Thr Gln Glu
            900                 905                 910

Gln Lys Phe Glu His Met Tyr Ala Gln Ser Leu Gln Val Pro Tyr Arg
        915                 920                 925

Asn Ser Leu Arg Lys Ala His Ile Phe Gly Ile Thr Phe Ser Phe Thr
    930                 935                 940

Gln Ala Met Met Tyr Phe Ser Tyr Ala Gly Cys Phe Arg Phe Gly Ala
945                 950                 955                 960

Tyr Leu Val Ala His Lys Leu Met Ser Phe Glu Asp Val Leu Leu Val
                965                 970                 975

Phe Ser Ala Val Val Phe Gly Ala Met Ala Val Gly Gln Val Ser Ser
            980                 985                 990

Phe Ala Pro Asp Tyr Ala Lys Ala Lys Ile Ser Ala Ala His Ile Ile
        995                 1000                1005

Met Ile Ile Glu Lys Thr Pro Leu Ile Asp Ser Tyr Ser Thr Glu Gly
    1010                1015                1020

Leu Met Pro Asn Thr Leu Glu Gly Asn Val Thr Phe Gly Glu Val Val
1025                1030                1035                1040

Phe Asn Tyr Pro Thr Arg Pro Asp Ile Pro Val Leu Gln Gly Leu Ser
                1045                1050                1055

Leu Glu Val Lys Lys Gly Gln Thr Leu Ala Leu Val Gly Ser Ser Gly
            1060                1065                1070

Cys Gly Lys Ser Thr Val Val Gln Leu Leu Glu Arg Phe Tyr Asp Pro
        1075                1080                1085

Leu Ala Gly Lys Val Leu Leu Asp Gly Lys Glu Ile Lys Arg Leu Asn
    1090                1095                1100

Val Gln Trp Leu Arg Ala His Leu Gly Ile Val Ser Gln Glu Pro Ile
1105                1110                1115                1120

Leu Phe Asp Cys Ser Ile Ala Glu Asn Ile Ala Tyr Gly Asp Asn Ser
                1125                1130                1135

Arg Val Val Ser Gln Glu Glu Ile Val Arg Ala Ala Lys Glu Ala Asn
            1140                1145                1150

Ile His Ala Phe Ile Glu Ser Leu Pro Asn Lys Tyr Ser Thr Lys Val
        1155                1160                1165

Gly Asp Lys Gly Thr Gln Leu Ser Gly Gly Gln Lys Gln Arg Ile Ala
    1170                1175                1180

Ile Ala Arg Ala Leu Val Arg Gln Pro His Ile Leu Leu Leu Asp Glu
1185                1190                1195                1200

Ala Thr Ser Ala Leu Asp Thr Glu Ser Glu Lys Val Val Gln Glu Ala
                1205                1210                1215

Leu Asp Lys Ala Arg Glu Gly Arg Thr Cys Ile Val Ile Ala His Arg
            1220                1225                1230

Leu Ser Thr Ile Gln Asn Ala Asp Leu Ile Val Val Phe Gln Asn Gly
        1235                1240                1245

Arg Val Lys Glu His Gly Thr His Gln Gln Leu Leu Ala Gln Lys Gly
    1250                1255                1260

Ile Tyr Phe Ser Met Val Ser Val Gln Ala Gly Thr Lys Arg Gln
1265                1270                1275

<210> 51
<211> 808
<212> PRT
<213> Homo sapiens

<400> 51
Met Ala Glu Leu Leu Ala Ser Ala Gly Ser Ala Cys Ser Trp Asp Phe
1               5                   10                  15

Pro Arg Ala Pro Pro Ser Phe Pro Pro Pro Ala Ala Ser Arg Gly Gly
            20                  25                  30

Leu Gly Gly Thr Arg Ser Phe Arg Pro His Arg Gly Ala Glu Ser Pro
        35                  40                  45

Arg Pro Gly Arg Asp Arg Asp Gly Val Arg Val Pro Met Ala Ser Ser
    50                  55                  60

Arg Cys Pro Ala Pro Arg Gly Cys Arg Cys Leu Pro Gly Ala Ser Leu
65                  70                  75                  80

Ala Trp Leu Gly Thr Val Leu Leu Leu Leu Ala Asp Trp Val Leu Leu
                85                  90                  95

Arg Thr Ala Leu Pro Arg Ile Phe Ser Leu Leu Val Pro Thr Ala Leu
            100                 105                 110

Pro Leu Leu Arg Val Trp Ala Val Gly Leu Ser Arg Trp Ala Val Leu
        115                 120                 125

Trp Leu Gly Ala Cys Gly Val Leu Arg Ala Thr Val Gly Ser Lys Ser
    130                 135                 140

Glu Asn Ala Gly Ala Gln Gly Trp Leu Ala Ala Leu Lys Pro Leu Ala
145                 150                 155                 160

Ala Ala Leu Gly Leu Ala Leu Pro Gly Leu Ala Leu Phe Arg Glu Leu
                165                 170                 175

Ile Ser Trp Gly Ala Pro Gly Ser Ala Asp Ser Thr Arg Leu Leu His
            180                 185                 190

Trp Gly Ser His Pro Thr Ala Phe Val Val Ser Tyr Ala Ala Ala Leu
        195                 200                 205

Pro Ala Ala Ala Leu Trp His Lys Leu Gly Ser Leu Trp Val Pro Gly
    210                 215                 220

Gly Gln Gly Gly Ser Gly Asn Pro Val Arg Arg Leu Leu Gly Cys Leu
225                 230                 235                 240

Gly Ser Glu Thr Arg Arg Leu Ser Leu Phe Leu Val Leu Val Val Leu
                245                 250                 255

Ser Ser Leu Gly Glu Met Ala Ile Pro Phe Phe Thr Gly Arg Leu Thr
            260                 265                 270

Asp Trp Ile Leu Gln Asp Gly Ser Ala Asp Thr Phe Thr Arg Asn Leu
        275                 280                 285

Thr Leu Met Ser Ile Leu Thr Ile Ala Ser Ala Val Leu Glu Phe Val
    290                 295                 300

Gly Asp Gly Ile Tyr Asn Asn Thr Met Gly His Val His Ser His Leu
305                 310                 315                 320

Gln Gly Glu Val Phe Gly Ala Val Leu Arg Gln Glu Thr Glu Phe Phe
                325                 330                 335

Gln Gln Asn Gln Thr Gly Asn Ile Met Ser Arg Val Thr Glu Asp Thr
            340                 345                 350

Ser Thr Leu Ser Asp Ser Leu Ser Glu Asn Leu Ser Leu Phe Leu Trp
        355                 360                 365

Tyr Leu Val Arg Gly Leu Cys Leu Leu Gly Ile Met Leu Trp Gly Ser
    370                 375                 380

Val Ser Leu Thr Met Val Thr Leu Ile Thr Leu Pro Leu Leu Phe Leu
385                 390                 395                 400

Leu Pro Lys Lys Val Gly Lys Trp Tyr Gln Leu Leu Glu Val Gln Val
                405                 410                 415

Arg Glu Ser Leu Ala Lys Ser Ser Gln Val Ala Ile Glu Ala Leu Ser
            420                 425                 430

Ala Met Pro Thr Val Arg Ser Phe Ala Asn Glu Glu Gly Glu Ala Gln
        435                 440                 445

Lys Phe Arg Glu Lys Leu Gln Glu Ile Lys Thr Leu Asn Gln Lys Glu
    450                 455                 460

Ala Val Ala Tyr Ala Val Asn Ser Trp Thr Thr Ser Ile Ser Gly Met
465                 470                 475                 480

Leu Leu Lys Val Gly Ile Leu Tyr Ile Gly Gly Gln Leu Val Thr Ser
                485                 490                 495

Gly Ala Val Ser Ser Gly Asn Leu Val Thr Phe Val Leu Tyr Gln Met
            500                 505                 510

Gln Phe Thr Gln Ala Val Glu Val Leu Leu Ser Ile Tyr Pro Arg Val
        515                 520                 525

Gln Lys Ala Val Gly Ser Ser Glu Lys Ile Phe Glu Tyr Leu Asp Arg
    530                 535                 540

Thr Pro Arg Cys Pro Pro Ser Gly Leu Leu Thr Pro Leu His Leu Glu
545                 550                 555                 560

Gly Leu Val Gln Phe Gln Asp Val Ser Phe Ala Tyr Pro Asn Arg Pro
                565                 570                 575

Asp Val Leu Val Leu Gln Gly Leu Thr Phe Thr Leu Arg Pro Gly Glu
            580                 585                 590

Val Thr Ala Leu Val Gly Pro Asn Gly Ser Gly Lys Ser Thr Val Ala
        595                 600                 605

Ala Leu Leu Gln Asn Leu Tyr Gln Pro Thr Gly Gly Gln Leu Leu Leu
    610                 615                 620

Asp Gly Lys Pro Leu Pro Gln Tyr Glu His Arg Tyr Leu His Arg Gln
625                 630                 635                 640

Val Ala Ala Val Gly Gln Glu Pro Gln Val Phe Gly Arg Ser Leu Gln
                645                 650                 655

Glu Asn Ile Ala Tyr Gly Leu Thr Gln Lys Pro Thr Met Glu Glu Ile
            660                 665                 670

Thr Ala Ala Ala Val Lys Ser Gly Ala His Ser Phe Ile Ser Gly Leu
        675                 680                 685

Pro Gln Gly Tyr Asp Thr Glu Val Asp Glu Ala Gly Ser Gln Leu Ser
    690                 695                 700

Gly Gly Gln Arg Gln Ala Val Ala Leu Ala Arg Ala Leu Ile Arg Lys
705                 710                 715                 720

Pro Cys Val Leu Ile Leu Asp Asp Ala Thr Ser Ala Leu Asp Ala Asn
                725                 730                 735

Ser Gln Leu Gln Val Glu Gln Leu Leu Tyr Glu Ser Pro Glu Arg Tyr
            740                 745                 750

Ser Arg Ser Val Leu Leu Ile Thr Gln His Leu Ser Leu Val Glu Gln
        755                 760                 765

Ala Asp His Ile Leu Phe Leu Glu Gly Gly Ala Ile Arg Glu Gly Gly
    770                 775                 780

Thr His Gln Gln Leu Met Glu Lys Lys Gly Cys Tyr Trp Ala Met Val
785                 790                 795                 800

Gln Ala Pro Ala Asp Ala Pro Glu
                805

<210> 52
<211> 808
<212> PRT
<213> Homo sapiens

<400> 52
Met Ala Glu Leu Leu Ala Ser Ala Gly Ser Ala Cys Ser Trp Asp Phe
1               5                   10                  15

Pro Arg Ala Pro Pro Ser Phe Pro Pro Pro Ala Ala Ser Arg Gly Gly
            20                  25                  30

Leu Gly Gly Thr Arg Ser Phe Arg Pro His Arg Gly Ala Glu Ser Pro
        35                  40                  45

Arg Pro Gly Arg Asp Arg Asp Gly Val Arg Val Pro Met Ala Ser Ser
    50                  55                  60

Arg Cys Pro Ala Pro Arg Gly Cys Arg Cys Leu Pro Gly Ala Ser Leu
65                  70                  75                  80

Ala Trp Leu Gly Thr Val Leu Leu Leu Leu Ala Asp Trp Val Leu Leu
                85                  90                  95

Arg Thr Ala Leu Pro Arg Ile Phe Ser Leu Leu Val Pro Thr Ala Leu
            100                 105                 110

Pro Leu Leu Arg Val Trp Ala Val Gly Leu Ser Arg Trp Ala Val Leu
        115                 120                 125

Trp Leu Gly Ala Cys Gly Val Leu Arg Ala Thr Val Gly Ser Lys Ser
    130                 135                 140

Glu Asn Ala Gly Ala Gln Gly Trp Leu Ala Ala Leu Lys Pro Leu Ala
145                 150                 155                 160

Ala Ala Leu Gly Leu Ala Leu Pro Gly Leu Ala Leu Phe Arg Glu Leu
                165                 170                 175

Ile Ser Trp Gly Ala Pro Gly Ser Ala Asp Ser Thr Arg Leu Leu His
            180                 185                 190

Trp Gly Ser His Pro Thr Ala Phe Val Val Ser Tyr Ala Ala Ala Leu

195                 200                 205

Pro Ala Ala Ala Leu Trp His Lys Leu Gly Ser Leu Trp Val Pro Gly
    210                 215                 220

Gly Gln Gly Gly Ser Gly Asn Pro Val Arg Arg Leu Leu Gly Cys Leu
225                 230                 235                 240

Gly Ser Glu Thr Arg Arg Leu Ser Leu Phe Leu Val Leu Val Val Leu
                245                 250                 255

Ser Ser Leu Gly Glu Met Ala Ile Pro Phe Phe Thr Gly Arg Leu Thr
            260                 265                 270

Asp Trp Ile Leu Gln Asp Gly Ser Ala Asp Thr Phe Thr Arg Asn Leu
        275                 280                 285

Thr Leu Met Ser Ile Leu Thr Ile Ala Ser Ala Val Leu Glu Phe Val
    290                 295                 300

Gly Asp Gly Ile Tyr Asn Asn Thr Met Gly His Val His Ser His Leu
305                 310                 315                 320

Gln Gly Glu Val Phe Gly Ala Val Leu Arg Gln Glu Thr Glu Phe Phe
                325                 330                 335

Gln Gln Asn Gln Thr Gly Asn Ile Met Ser Arg Val Thr Glu Asp Thr
            340                 345                 350

Ser Thr Leu Ser Asp Ser Leu Ser Glu Asn Leu Ser Leu Phe Leu Trp
        355                 360                 365

Tyr Leu Val Arg Gly Leu Cys Leu Leu Gly Ile Met Leu Trp Gly Ser
    370                 375                 380

Val Ser Leu Thr Met Val Thr Leu Ile Thr Leu Pro Leu Leu Phe Leu
385                 390                 395                 400

Leu Pro Lys Lys Val Gly Lys Trp Tyr Gln Leu Leu Glu Val Gln Val
                405                 410                 415

Arg Glu Ser Leu Ala Lys Ser Ser Gln Val Ala Ile Glu Ala Leu Ser
            420                 425                 430

Ala Met Pro Thr Val Arg Ser Phe Ala Asn Glu Glu Gly Glu Ala Gln
        435                 440                 445

Lys Phe Arg Glu Lys Leu Gln Glu Ile Lys Thr Leu Asn Gln Lys Glu

450                 455                 460

Ala Val Ala Tyr Ala Val Asn Ser Trp Thr Thr Ser Ile Ser Gly Met
465                 470                 475                 480

Leu Leu Lys Val Gly Ile Leu Tyr Ile Gly Gly Gln Leu Val Thr Ser
                485                 490                 495

Gly Ala Val Ser Ser Gly Asn Leu Val Thr Phe Val Leu Tyr Gln Met
            500                 505                 510

Gln Phe Thr Gln Ala Val Glu Val Leu Leu Ser Ile Tyr Pro Arg Val
        515                 520                 525

Gln Lys Ala Val Gly Ser Ser Glu Lys Ile Phe Glu Tyr Leu Asp Arg
    530                 535                 540

Thr Pro Arg Cys Pro Pro Ser Gly Leu Leu Thr Pro Leu His Leu Glu
545                 550                 555                 560

Gly Leu Val Gln Phe Gln Asp Val Ser Phe Ala Tyr Pro Asn Arg Pro
                565                 570                 575

Asp Val Leu Val Leu Gln Gly Leu Thr Phe Thr Leu Arg Pro Gly Glu
            580                 585                 590

Val Thr Ala Leu Val Gly Pro Asn Gly Ser Gly Lys Ser Thr Val Ala
        595                 600                 605

Ala Leu Leu Gln Asn Leu Tyr Gln Pro Thr Gly Gly Gln Leu Leu Leu
    610                 615                 620

Asp Gly Lys Pro Leu Pro Gln Tyr Glu His Arg Tyr Leu His Arg Gln
625                 630                 635                 640

Val Ala Ala Val Gly Gln Glu Pro Gln Val Phe Gly Arg Ser Leu Gln
                645                 650                 655

Glu Asn Ile Ala Tyr Gly Leu Thr Gln Lys Pro Thr Met Glu Glu Ile
            660                 665                 670

Thr Ala Ala Ala Val Lys Ser Gly Ala His Ser Phe Ile Ser Gly Leu
        675                 680                 685

Pro Gln Gly Tyr Asp Thr Glu Val Asp Glu Ala Gly Ser Gln Leu Ser
    690                 695                 700

Gly Gly Gln Arg Gln Ala Val Ala Leu Ala Arg Ala Leu Ile Arg Lys

705                 710                 715                 720

Pro Cys Val Leu Ile Leu Asp Asp Ala Thr Ser Ala Leu Asp Ala Asn
                725                 730                 735

Ser Gln Leu Gln Val Glu Gln Leu Leu Tyr Glu Ser Pro Glu Arg Tyr
            740                 745                 750

Ser Arg Ser Val Leu Leu Ile Thr Gln His Leu Ser Leu Val Glu Gln
        755                 760                 765

Ala Asp His Ile Leu Phe Leu Glu Gly Gly Ala Ile Arg Glu Gly Gly
    770                 775                 780

Thr His Gln Gln Leu Met Glu Lys Lys Gly Cys Tyr Trp Ala Met Val
785                 790                 795                 800

Gln Ala Pro Ala Asp Ala Pro Glu
                805

<210> 53
<211> 1232
<212> PRT
<213> Homo sapiens

<400> 53
Met Asp Leu Glu Ala Ala Lys Asn Gly Thr Ala Trp Arg Pro Thr Ser
  1               5                  10                  15

Ala Glu Gly Asp Phe Glu Leu Gly Ile Ser Ser Lys Gln Lys Arg Lys
             20                  25                  30

Lys Thr Lys Thr Val Lys Met Ile Gly Val Leu Thr Leu Phe Arg Tyr
         35                  40                  45

Ser Asp Trp Gln Asp Lys Leu Phe Met Ser Leu Gly Thr Ile Met Ala
     50                  55                  60

Ile Ala His Gly Ser Gly Leu Pro Leu Met Met Ile Val Phe Gly Glu
 65                  70                  75                  80

Met Thr Asp Lys Phe Val Asp Thr Ala Gly Asn Phe Ser Phe Pro Val
                 85                  90                  95

Asn Phe Ser Leu Ser Leu Leu Asn Pro Gly Lys Ile Leu Glu Glu Glu
            100                 105                 110

Met Thr Arg Tyr Ala Tyr Tyr Tyr Ser Gly Leu Gly Ala Gly Val Leu
        115                 120                 125

Val Ala Ala Tyr Ile Gln Val Ser Phe Trp Thr Leu Ala Ala Gly Arg
    130                 135                 140

Gln Ile Arg Lys Ile Arg Gln Lys Phe Phe His Ala Ile Leu Arg Gln
145                 150                 155                 160

Glu Ile Gly Trp Phe Asp Ile Asn Asp Thr Thr Glu Leu Asn Thr Arg
                165                 170                 175

Leu Thr Asp Asp Ile Ser Lys Ile Ser Glu Gly Ile Gly Asp Lys Val
            180                 185                 190

Gly Met Phe Phe Gln Ala Val Ala Thr Phe Phe Ala Gly Phe Ile Val
        195                 200                 205

Gly Phe Ile Arg Gly Trp Lys Leu Thr Leu Val Ile Met Ala Ile Ser
    210                 215                 220

Pro Ile Leu Gly Leu Ser Ala Ala Val Trp Ala Lys Ile Leu Ser Ala
225                 230                 235                 240

Phe Ser Asp Lys Glu Leu Ala Ala Tyr Ala Lys Ala Gly Ala Val Ala
                245                 250                 255

Glu Glu Ala Leu Gly Ala Ile Arg Thr Val Ile Ala Phe Gly Gly Gln
            260                 265                 270

Asn Lys Glu Leu Glu Arg Tyr Gln Lys His Leu Glu Asn Ala Lys Glu
        275                 280                 285

Ile Gly Ile Lys Lys Ala Ile Ser Ala Asn Ile Ser Met Gly Ile Ala
    290                 295                 300

Phe Leu Leu Ile Tyr Ala Ser Tyr Ala Leu Ala Phe Trp Tyr Gly Ser
305                 310                 315                 320

Thr Leu Val Ile Ser Lys Glu Tyr Thr Ile Gly Asn Ala Met Thr Val
                325                 330                 335

Phe Phe Ser Ile Leu Ile Gly Ala Phe Ser Val Gly Gln Ala Ala Pro
            340                 345                 350

Cys Ile Asp Ala Phe Ala Asn Ala Arg Gly Ala Ala Tyr Val Ile Phe
        355                 360                 365

Asp Ile Ile Asp Asn Asn Pro Lys Ile Asp Ser Phe Ser Glu Arg Gly
    370                 375                 380

His Lys Pro Asp Ser Ile Lys Gly Asn Leu Glu Phe Asn Asp Val His
385                 390                 395                 400

Phe Ser Tyr Pro Ser Arg Ala Asn Val Lys Ile Leu Lys Gly Leu Asn
                405                 410                 415

Leu Lys Val Gln Ser Gly Gln Thr Val Ala Leu Val Gly Ser Ser Gly
            420                 425                 430

Cys Gly Lys Ser Thr Thr Val Gln Leu Ile Gln Arg Leu Tyr Asp Pro
        435                 440                 445

Asp Glu Gly Thr Ile Asn Ile Asp Gly Gln Asp Ile Arg Asn Phe Asn
    450                 455                 460

Val Asn Tyr Leu Arg Glu Ile Ile Gly Val Val Ser Gln Glu Pro Val
465                 470                 475                 480

Leu Phe Ser Thr Thr Ile Ala Glu Asn Ile Cys Tyr Gly Arg Gly Asn
                485                 490                 495

Val Thr Met Asp Glu Ile Lys Lys Ala Val Lys Glu Ala Asn Ala Tyr
            500                 505                 510

Glu Phe Ile Met Lys Leu Pro Gln Lys Phe Asp Thr Leu Val Gly Glu
        515                 520                 525

Arg Gly Ala Gln Leu Ser Gly Gly Gln Lys Gln Arg Ile Ala Ile Ala
    530                 535                 540

Arg Ala Leu Val Arg Asn Pro Lys Ile Leu Leu Leu Asp Glu Ala Thr
545                 550                 555                 560

Ser Ala Leu Asp Thr Glu Ser Glu Ala Glu Val Gln Ala Ala Leu Asp
                565                 570                 575

Lys Ala Arg Glu Gly Arg Thr Thr Ile Val Ile Ala His Arg Leu Ser
            580                 585                 590

Thr Val Arg Asn Ala Asp Val Ile Ala Gly Phe Glu Asp Gly Val Ile
        595                 600                 605

Val Glu Gln Gly Ser His Ser Glu Leu Met Lys Lys Glu Gly Val Tyr
    610                 615                 620

Phe Lys Leu Val Asn Met Gln Thr Ser Gly Ser Gln Ile Gln Ser Glu
625                 630                 635                 640

Glu Phe Glu Leu Asn Asp Glu Lys Ala Ala Thr Arg Met Ala Pro Asn
                645                 650                 655

Gly Trp Lys Ser Arg Leu Phe Arg His Ser Thr Gln Lys Asn Leu Lys
            660                 665                 670

Asn Ser Gln Met Cys Gln Lys Ser Leu Asp Val Glu Thr Asp Gly Leu
        675                 680                 685

Glu Ala Asn Val Pro Pro Val Ser Phe Leu Lys Val Leu Lys Leu Asn
    690                 695                 700

Lys Thr Glu Trp Pro Tyr Phe Val Val Gly Thr Val Cys Ala Ile Ala
705                 710                 715                 720

Asn Gly Gly Leu Gln Pro Ala Phe Ser Val Ile Phe Ser Glu Ile Ile
                725                 730                 735

Ala Ile Phe Gly Pro Gly Asp Asp Ala Val Lys Gln Gln Lys Cys Asn
            740                 745                 750

Ile Phe Ser Leu Ile Phe Leu Phe Leu Gly Ile Ile Ser Phe Phe Thr
        755                 760                 765

Phe Phe Leu Gln Gly Phe Thr Phe Gly Lys Ala Gly Glu Ile Leu Thr
    770                 775                 780

Arg Arg Leu Arg Ser Met Ala Phe Lys Ala Met Leu Arg Gln Asp Met
785                 790                 795                 800

Ser Trp Phe Asp Asp His Lys Asn Ser Thr Gly Ala Leu Ser Thr Arg
                805                 810                 815

Leu Ala Thr Asp Ala Ala Gln Val Gln Gly Ala Thr Gly Thr Arg Leu
            820                 825                 830

Ala Leu Ile Ala Gln Asn Ile Ala Asn Leu Gly Thr Gly Ile Ile Ile
        835                 840                 845

Ser Phe Ile Tyr Gly Trp Gln Leu Thr Leu Leu Leu Leu Ala Val Val
    850                 855                 860

Pro Ile Ile Ala Val Ser Gly Ile Val Glu Met Lys Leu Leu Ala Gly
865                 870                 875                 880

Asn Ala Lys Arg Asp Lys Lys Glu Leu Glu Ala Ala Gly Lys Ile Ala
                885                 890                 895

Thr Glu Ala Ile Glu Asn Ile Arg Thr Val Val Ser Leu Thr Gln Glu
            900                 905                 910

Arg Lys Phe Glu Ser Met Tyr Val Glu Lys Leu Tyr Gly Pro Tyr Arg
        915                 920                 925

Val Phe Ser Ala Ile Val Phe Gly Ala Val Ala Leu Gly His Ala Ser
    930                 935                 940

Ser Phe Ala Pro Asp Tyr Ala Lys Ala Lys Leu Ser Ala Ala His Leu
945                 950                 955                 960

Phe Met Leu Phe Glu Arg Gln Pro Leu Ile Asp Ser Tyr Ser Glu Glu
                965                 970                 975

Gly Leu Lys Pro Asp Lys Phe Glu Gly Asn Ile Thr Phe Asn Glu Val
            980                 985                 990

Val Phe Asn Tyr Pro Thr Arg Ala Asn Val Pro Val Leu Gln Gly Leu
        995                 1000                1005

Ser Leu Glu Val Lys Lys Gly Gln Thr Leu Ala Leu Val Gly Ser Ser
    1010                1015                1020

Gly Cys Gly Lys Ser Thr Val Val Gln Leu Leu Glu Arg Phe Tyr Asp
1025                1030                1035                1040

Pro Leu Ala Gly Thr Val Leu Leu Asp Gly Gln Glu Ala Lys Lys Leu
                1045                1050                1055

Asn Val Gln Trp Leu Arg Ala Gln Leu Gly Ile Val Ser Gln Glu Pro
            1060                1065                1070

Ile Leu Phe Asp Cys Ser Ile Ala Glu Asn Ile Ala Tyr Gly Asp Asn
        1075                1080                1085

Ser Arg Val Val Ser Gln Asp Glu Ile Val Ser Ala Ala Lys Ala Ala
    1090                1095                1100

Asn Ile His Pro Phe Ile Glu Thr Leu Pro His Lys Tyr Glu Thr Arg
1105                1110                1115                1120

Val Gly Asp Lys Gly Thr Gln Leu Ser Gly Gly Gln Lys Gln Arg Ile
                1125                1130                1135

Ala Ile Ala Arg Ala Leu Ile Arg Gln Pro Gln Ile Leu Leu Leu Asp
            1140                1145                1150

Glu Ala Thr Ser Ala Leu Asp Thr Glu Ser Glu Lys Val Val Gln Glu
        1155                1160                1165

Ala Leu Asp Lys Ala Arg Glu Gly Arg Thr Cys Ile Val Ile Ala His
    1170                1175                1180

Arg Leu Ser Thr Ile Gln Asn Ala Asp Leu Ile Val Val Phe Gln Asn
1185                1190                1195                1200

Gly Arg Val Lys Glu His Gly Thr His Gln Gln Leu Leu Ala Gln Lys
                1205                1210                1215

Gly Ile Tyr Phe Ser Met Val Ser Val Gln Ala Gly Thr Gln Asn Leu
            1220                1225                1230

<210> 54
<211> 842
<212> PRT
<213> Homo sapiens

<400> 54

<210> 55

<211> 752
<212> PRT
<213> Homo sapiens

<400> 55
Met Ala Leu Leu Ala Met His Ser Trp Arg Trp Ala Ala Ala Ala Ala
1               5                   10                  15

Ala Phe Glu Lys Arg Arg His Ser Ala Ile Leu Ile Arg Pro Leu Val
            20                  25                  30

Ser Val Ser Gly Ser Gly Pro Gln Trp Arg Pro His Gln Leu Gly Ala
        35                  40                  45

Leu Gly Thr Ala Arg Ala Tyr Gln Ile Pro Glu Ser Leu Lys Ser Ile
    50                  55                  60

Thr Trp Gln Arg Leu Gly Lys Gly Asn Ser Gly Gln Phe Leu Asp Ala
65                  70                  75                  80

Ala Lys Ala Leu Gln Val Trp Pro Leu Ile Glu Lys Arg Thr Cys Trp
                85                  90                  95

His Gly His Ala Gly Gly Gly Leu His Thr Asp Pro Lys Glu Gly Leu
            100                 105                 110

Lys Asp Val Asp Thr Arg Lys Ile Ile Lys Ala Met Leu Ser Tyr Val
        115                 120                 125

Trp Pro Lys Asp Arg Pro Asp Leu Arg Ala Arg Val Pro Ile Ser Leu
    130                 135                 140

Gly Phe Leu Gly Gly Ala Lys Ala Met Asn Ile Val Val Pro Phe Met
145                 150                 155                 160

Phe Lys Tyr Ala Val Asp Ser Leu Asn Gln Met Ser Gly Asn Met Leu
                165                 170                 175

Asn Leu Ser Asp Ala Pro Asn Thr Val Ala Thr Met Ala Thr Ala Val
            180                 185                 190

Leu Ile Gly Tyr Gly Val Ser Arg Ala Gly Ala Ala Phe Phe Asn Glu
        195                 200                 205

Val Arg Asn Ala Val Phe Gly Lys Val Ala Gln Asn Ser Ile Arg Arg
    210                 215                 220

Ile Ala Lys Asn Val Phe Leu His Leu His Asn Leu Asp Leu Gly Phe

225                 230                 235                 240

His Leu Ser Arg Gln Thr Gly Ala Leu Ser Lys Ala Ile Asp Arg Gly
                245                 250                 255

Thr Arg Gly Ile Ser Phe Val Leu Ser Ala Leu Val Phe Asn Pro Leu
            260                 265                 270

Pro Asn His Val Glu Val Met Leu Leu Val Ser Gly Val Leu Tyr Tyr
        275                 280                 285

Lys Cys Cys Ala Gln Leu Leu Gly Asn Leu Gly Thr Leu Gly Thr Tyr
    290                 295                 300

Thr Ala Phe Thr Val Ala Val Thr Arg Trp Arg Thr Arg Phe Arg Leu
305                 310                 315                 320

Glu Ile Asp Gln Ala Asp Asn Asp Ala Gly Asn Ala Ala Ile Asp Ser
                325                 330                 335

Leu Leu Asn Tyr Glu Thr Val Lys Tyr Phe Asn Asn Glu Arg Tyr Glu
            340                 345                 350

Ala Gln Arg Tyr Asp Gly Phe Leu Lys Thr Tyr Glu Thr Ala Ser Leu
        355                 360                 365

Lys Ser Thr Ser Thr Leu Ala Met Leu Asn Phe Gly Gln Ser Ala Ile
    370                 375                 380

Phe Ser Val Gly Leu Thr Ala Ile Met Val Leu Ala Ser Gln Gly Ile
385                 390                 395                 400

Val Ala Gly Thr Leu Thr Val Gly Asp Leu Val Met Val Asn Gly Leu
                405                 410                 415

Leu Phe Gln Leu Ser Leu Pro Leu Asn Phe Leu Gly Thr Val Tyr Arg
            420                 425                 430

Glu Thr Arg Gln Ala Leu Ile Asp Met Asn Thr Leu Phe Thr Leu Leu
        435                 440                 445

Lys Val Asp Thr Gln Ile Lys Asp Lys Val Met Ala Ser Pro Leu Gln
    450                 455                 460

Ile Thr Pro Gln Thr Ala Thr Val Ala Phe Asp Asn Val His Phe Glu
465                 470                 475                 480

Tyr Ile Glu Gly Gln Lys Val Leu Ser Gly Ile Ser Phe Glu Val Pro

                485                 490                 495

Ala Gly Lys Lys Val Ala Ile Val Gly Gly Ser Gly Ser Gly Lys Ser
            500                 505                 510

Thr Ile Val Arg Leu Leu Phe Arg Phe Tyr Glu Pro Gln Lys Gly Ser
        515                 520                 525

Ile Tyr Leu Ala Gly Gln Asn Ile Gln Asp Val Ser Leu Glu Ser Leu
    530                 535                 540

Arg Arg Ala Val Gly Val Val Pro Gln Asp Ala Val Leu Phe His Asn
545                 550                 555                 560

Thr Ile Tyr Tyr Asn Leu Leu Tyr Gly Asn Ile Ser Ala Ser Pro Glu
                565                 570                 575

Glu Val Tyr Ala Val Ala Lys Leu Ala Gly Leu His Asp Ala Ile Leu
            580                 585                 590

Arg Met Pro His Gly Tyr Asp Thr Gln Val Gly Glu Arg Gly Leu Lys
        595                 600                 605

Leu Ser Gly Gly Glu Lys Gln Arg Val Ala Ile Ala Arg Ala Ile Leu
    610                 615                 620

Lys Asp Pro Pro Val Ile Leu Tyr Asp Glu Ala Thr Ser Ser Leu Asp
625                 630                 635                 640

Ser Ile Thr Glu Glu Thr Ile Leu Gly Ala Met Lys Asp Val Val Lys
                645                 650                 655

His Arg Thr Ser Ile Phe Ile Ala His Arg Leu Ser Thr Val Val Asp
            660                 665                 670

Ala Asp Glu Ile Ile Val Leu Asp Gln Gly Lys Val Ala Glu Arg Gly
        675                 680                 685

Thr His His Gly Leu Leu Ala Asn Pro His Ser Ile Tyr Ser Glu Met
    690                 695                 700

Trp His Thr Gln Ser Ser Arg Val Gln Asn His Asp Asn Pro Lys Trp
705                 710                 715                 720

Glu Ala Lys Lys Glu Asn Ile Ser Lys Glu Glu Glu Arg Lys Lys Leu
                725                 730                 735

Gln Glu Glu Ile Val Asn Ser Val Lys Gly Cys Gly Asn Cys Ser Cys

740                 745                 750

<210> 56
<211> 718
<212> PRT
<213> Homo sapiens

<400> 56
Met Leu Val His Leu Phe Arg Val Gly Ile Arg Gly Gly Pro Phe Pro
1               5                   10                  15

Gly Arg Leu Leu Pro Pro Leu Arg Phe Gln Thr Phe Ser Ala Val Arg
            20                  25                  30

Tyr Ser Asp Gly Tyr Arg Ser Ser Ser Leu Leu Arg Ala Val Ala His
        35                  40                  45

Leu Arg Ser Gln Leu Trp Ala His Leu Pro Arg Ala Pro Leu Ala Pro
    50                  55                  60

Arg Trp Ser Pro Ser Ala Trp Cys Trp Val Gly Gly Ala Leu Leu Gly
65                  70                  75                  80

Pro Met Val Leu Ser Lys His Pro His Leu Cys Leu Val Ala Leu Cys
                85                  90                  95

Glu Ala Glu Glu Ala Pro Pro Ala Ser Ser Thr Pro His Val Val Gly
            100                 105                 110

Ser Arg Phe Asn Trp Lys Leu Phe Trp Gln Phe Leu His Pro His Leu
        115                 120                 125

Leu Val Leu Gly Val Ala Val Val Leu Ala Leu Gly Ala Ala Leu Val
    130                 135                 140

Asn Val Gln Ile Pro Leu Leu Leu Gly Gln Leu Val Lys Val Val Ala
145                 150                 155                 160

Lys Tyr Thr Arg Asp His Val Gly Ser Phe Met Thr Glu Ser Gln Asn
                165                 170                 175

Leu Ser Thr His Leu Leu Ile Leu Tyr Gly Val Gln Gly Leu Leu Thr
            180                 185                 190

Phe Gly Tyr Leu Val Leu Leu Ser His Val Gly Glu Arg Met Ala Val
        195                 200                 205

Asp Met Arg Arg Ala Leu Phe Ser Ser Leu Leu Arg Gln Asn Ile Thr
    210                 215                 220

Phe Phe Asp Ala Asn Lys Thr Gly Gln Leu Val Ser Arg Leu Thr Thr
225                 230                 235                 240

Asp Val Gln Glu Phe Lys Ser Ser Phe Lys Leu Val Ile Ser Gln Gly
                245                 250                 255

Leu Arg Ser Cys Ser Gln Val Ala Gly Cys Leu Val Ser Leu Ser Met
            260                 265                 270

Leu Ser Thr Arg Leu Thr Leu Leu Leu Met Val Ala Thr Pro Ala Leu
        275                 280                 285

Met Gly Val Gly Thr Leu Met Gly Ser Gly Leu Arg Lys Leu Ser Arg
    290                 295                 300

Gln Cys Gln Glu His Ile Ala Arg Ala Met Gly Val Ala Asp Glu Ala
305                 310                 315                 320

Leu Gly Asn Val Arg Thr Val Arg Ala Leu Ala Met Glu Gln Arg Glu
                325                 330                 335

Glu Glu Arg Tyr Gly Ala Glu Leu Glu Ala Cys Arg Cys Arg Ala Glu
            340                 345                 350

Glu Leu Gly Arg Gly Ile Ala Leu Phe Gln Gly Leu Ser Asn Ile Ala
        355                 360                 365

Phe Asn Cys Met Val Leu Gly Thr Leu Phe Ile Gly Gly Ser Leu Val
    370                 375                 380

Ala Gly Gln Gln Leu Thr Gly Gly Asp Leu Met Ser Phe Leu Val Ala
385                 390                 395                 400

Ser Gln Thr Val Gln Arg Ser Met Ala Asn Leu Ser Val Leu Phe Gly
                405                 410                 415

Gln Val Val Arg Gly Leu Ser Ala Gly Ala Arg Val Phe Glu Tyr Met
            420                 425                 430

Ala Leu Asn Pro Cys Ile Pro Leu Ser Gly Gly Cys Cys Val Pro Lys
        435                 440                 445

Glu Gln Leu Arg Gly Ser Val Thr Phe Gln Asn Val Cys Phe Ser Tyr
    450                 455                 460

Pro Cys Arg Pro Gly Phe Glu Val Leu Lys Asp Phe Thr Leu Thr Leu
465                 470                 475                 480

Pro Pro Gly Lys Ile Val Ala Leu Val Gly Gln Ser Gly Gly Gly Lys
                485                 490                 495

Thr Thr Val Ala Ser Leu Leu Glu Arg Phe Tyr Asp Pro Thr Ala Gly
            500                 505                 510

Val Val Met Leu Asp Gly Arg Asp Leu Arg Thr Leu Asp Pro Ser Trp
        515                 520                 525

Leu Arg Gly Gln Val Val Gly Phe Ile Ser Gln Glu Pro Val Leu Phe
    530                 535                 540

Gly Thr Thr Ile Met Glu Asn Ile Arg Phe Gly Lys Leu Glu Ala Ser
545                 550                 555                 560

Asp Glu Glu Val Tyr Thr Ala Ala Arg Glu Ala Asn Ala His Glu Phe
                565                 570                 575

Ile Thr Ser Phe Pro Glu Gly Tyr Asn Thr Val Val Gly Glu Arg Gly
            580                 585                 590

Thr Thr Leu Ser Gly Gly Gln Lys Gln Arg Leu Ala Ile Ala Arg Ala
        595                 600                 605

Leu Ile Lys Gln Pro Thr Val Leu Ile Leu Asp Glu Ala Thr Ser Ala
    610                 615                 620

Leu Asp Ala Glu Ser Glu Arg Val Val Gln Glu Ala Leu Asp Arg Ala
625                 630                 635                 640

Ser Ala Gly Arg Thr Val Leu Val Ile Ala His Arg Leu Ser Thr Val
                645                 650                 655

Arg Gly Ala His Cys Ile Val Val Met Ala Asp Gly Arg Val Trp Glu
            660                 665                 670

Ala Gly Thr His Glu Glu Leu Leu Lys Lys Gly Gly Leu Tyr Ala Glu
        675                 680                 685

Leu Ile Arg Arg Gln Ala Leu Asp Ala Pro Arg Thr Ala Ala Pro Pro
    690                 695                 700

Pro Lys Lys Pro Glu Gly Pro Arg Ser His Gln His Lys Ser
705                 710                 715

<210> 57
<211> 723
<212> PRT
<213> Homo sapiens

<400> 57
Met Arg Leu Trp Lys Ala Val Val Val Thr Leu Ala Phe Met Ser Val
1               5                   10                  15

Asp Ile Cys Val Thr Thr Ala Ile Tyr Val Phe Ser His Leu Asp Arg
            20                  25                  30

Ser Leu Leu Glu Asp Ile Arg His Phe Asn Ile Phe Asp Ser Val Leu
        35                  40                  45

Asp Leu Trp Ala Ala Cys Leu Tyr Arg Ser Cys Leu Leu Leu Gly Ala
    50                  55                  60

Thr Ile Gly Val Ala Lys Asn Ser Ala Leu Gly Pro Arg Arg Leu Arg
65                  70                  75                  80

Ala Ser Trp Leu Val Ile Thr Leu Val Cys Leu Phe Val Gly Ile Tyr
                85                  90                  95

Ala Met Val Lys Leu Leu Leu Phe Ser Glu Val Arg Arg Pro Ile Arg
            100                 105                 110

Asp Pro Trp Phe Trp Ala Leu Phe Val Trp Thr Tyr Ile Ser Leu Gly
        115                 120                 125

Ala Ser Phe Leu Leu Trp Trp Leu Leu Ser Thr Val Arg Pro Gly Thr
    130                 135                 140

Gln Ala Leu Glu Pro Gly Ala Ala Thr Glu Ala Glu Gly Phe Pro Gly
145                 150                 155                 160

Ser Gly Arg Pro Pro Pro Glu Gln Ala Ser Gly Ala Thr Leu Gln Lys
                165                 170                 175

Leu Leu Ser Tyr Thr Lys Pro Asp Val Ala Phe Leu Val Ala Ala Ser
            180                 185                 190

Phe Phe Leu Ile Val Ala Ala Leu Gly Glu Thr Phe Leu Pro Tyr Tyr
        195                 200                 205

Thr Gly Arg Ala Ile Asp Gly Ile Val Ile Gln Lys Ser Met Asp Gln
    210                 215                 220

Phe Ser Thr Ala Val Val Ile Val Cys Leu Leu Ala Ile Gly Ser Ser
225                 230                 235                 240

Phe Ala Ala Gly Ile Arg Gly Gly Ile Phe Thr Leu Ile Phe Ala Arg
                245                 250                 255

Leu Asn Ile Arg Leu Arg Asn Cys Leu Phe Arg Ser Leu Val Ser Gln
            260                 265                 270

Glu Thr Ser Phe Phe Asp Glu Asn Arg Thr Gly Asp Leu Ile Ser Arg
        275                 280                 285

Leu Thr Ser Asp Thr Thr Met Val Ser Asp Leu Val Ser Gln Asn Ile
    290                 295                 300

Asn Val Phe Leu Arg Asn Thr Val Lys Val Thr Gly Val Val Val Phe
305                 310                 315                 320

Met Phe Ser Leu Ser Trp Gln Leu Ser Leu Val Thr Phe Met Gly Phe
                325                 330                 335

Pro Ile Ile Met Met Val Ser Asn Ile Tyr Gly Lys Tyr Tyr Lys Arg
            340                 345                 350

Leu Ser Lys Glu Val Gln Asn Ala Leu Ala Arg Ala Ser Asn Thr Ala
        355                 360                 365

Glu Glu Thr Ile Ser Ala Met Lys Thr Val Arg Ser Phe Ala Asn Glu
    370                 375                 380

Glu Glu Glu Ala Glu Val Tyr Leu Arg Lys Leu Gln Gln Val Tyr Lys
385                 390                 395                 400

Leu Asn Arg Lys Glu Ala Ala Ala Tyr Met Tyr Tyr Val Trp Gly Ser
                405                 410                 415

Gly Ser Val Gly Ser Val Tyr Ser Gly Leu Met Gln Gly Val Gly Ala
            420                 425                 430

Ala Glu Lys Val Phe Glu Phe Ile Asp Arg Gln Pro Thr Met Val His
        435                 440                 445

Asp Gly Ser Leu Ala Pro Asp His Leu Glu Gly Arg Val Asp Phe Glu
    450                 455                 460

Asn Val Thr Phe Thr Tyr Arg Thr Arg Pro His Thr Gln Val Leu Gln
465                 470                 475                 480

Asn Val Ser Phe Ser Leu Ser Pro Gly Lys Val Thr Ala Leu Val Gly
                485                 490                 495

Pro Ser Gly Ser Gly Lys Ser Ser Cys Val Asn Ile Leu Glu Asn Phe
            500                 505                 510

Tyr Pro Leu Glu Gly Gly Arg Val Leu Leu Asp Gly Lys Pro Ile Ser
        515                 520                 525

Ala Tyr Asp His Lys Tyr Leu His Arg Val Ile Ser Leu Val Ser Gln
    530                 535                 540

Glu Pro Val Leu Phe Ala Arg Ser Ile Thr Asp Asn Ile Ser Tyr Gly
545                 550                 555                 560

Leu Pro Thr Val Pro Phe Glu Met Val Val Glu Ala Ala Gln Lys Ala
                565                 570                 575

Asn Ala His Gly Phe Ile Met Glu Leu Gln Asp Gly Tyr Ser Thr Glu
            580                 585                 590

Thr Gly Glu Lys Gly Ala Gln Leu Ser Gly Gly Gln Lys Gln Arg Val
        595                 600                 605

Ala Met Ala Arg Ala Leu Val Arg Asn Pro Pro Val Leu Ile Leu Asp
    610                 615                 620

Glu Ala Thr Ser Ala Leu Asp Ala Glu Ser Glu Tyr Leu Ile Gln Gln
625                 630                 635                 640

Ala Ile His Gly Asn Leu Gln Lys His Thr Val Leu Ile Ile Ala His
                645                 650                 655

Arg Leu Ser Thr Val Glu His Ala His Leu Ile Val Val Leu Asp Lys
            660                 665                 670

Gly Arg Val Val Gln Gln Gly Thr His Gln Gln Leu Leu Ala Gln Gly
        675                 680                 685

Gly Leu Tyr Ala Lys Leu Val Gln Arg Gln Met Leu Gly Leu Gln Pro
    690                 695                 700

Ala Ala Asp Phe Thr Ala Gly His Asn Glu Pro Val Ala Asn Gly Ser
705                 710                 715                 720

His Lys Ala

<210> 58
<211> 738
<212> PRT
<213> Homo sapiens

<400> 58
Met Arg Gly Pro Pro Ala Trp Pro Leu Arg Leu Leu Glu Pro Pro Ser
1               5                   10                  15

Pro Ala Glu Pro Gly Arg Leu Leu Pro Val Ala Cys Val Trp Ala Ala
            20                  25                  30

Ala Ser Arg Val Pro Gly Ser Leu Ser Pro Phe Thr Gly Leu Arg Pro
        35                  40                  45

Ala Arg Leu Trp Gly Ala Gly Pro Ala Leu Leu Trp Gly Val Gly Ala
    50                  55                  60

Ala Arg Arg Trp Arg Ser Gly Cys Arg Gly Gly Gly Pro Gly Ala Ser
65                  70                  75                  80

Arg Gly Val Leu Gly Leu Ala Arg Leu Leu Gly Leu Trp Ala Arg Gly
                85                  90                  95

Pro Gly Ser Cys Arg Cys Gly Ala Phe Ala Gly Pro Gly Ala Pro Arg
            100                 105                 110

Leu Pro Arg Ala Arg Phe Pro Gly Gly Pro Ala Ala Ala Ala Trp Ala
        115                 120                 125

Gly Asp Glu Ala Trp Arg Arg Gly Pro Ala Ala Pro Pro Gly Asp Lys
    130                 135                 140

Gly Arg Leu Arg Pro Ala Ala Ala Gly Leu Pro Glu Ala Arg Lys Leu
145                 150                 155                 160

Leu Gly Leu Ala Tyr Pro Glu Arg Arg Arg Leu Ala Ala Ala Val Gly
                165                 170                 175

Phe Leu Thr Met Ser Ser Val Ile Ser Met Ser Ala Pro Phe Phe Leu

            180                 185                 190

Gly Lys Ile Ile Asp Val Ile Tyr Thr Asn Pro Thr Val Asp Tyr Ser
        195                 200                 205

Asp Asn Leu Thr Arg Leu Cys Leu Gly Leu Ser Ala Val Phe Leu Cys
    210                 215                 220

Gly Ala Ala Ala Asn Ala Ile Arg Val Tyr Leu Met Gln Thr Ser Gly
225                 230                 235                 240

Gln Arg Ile Val Asn Arg Leu Arg Thr Ser Leu Phe Ser Ser Ile Leu
                245                 250                 255

Arg Gln Glu Val Ala Phe Phe Asp Lys Thr Arg Thr Gly Glu Leu Ile
            260                 265                 270

Asn Arg Leu Ser Ser Asp Thr Ala Leu Leu Gly Arg Ser Val Thr Glu
        275                 280                 285

Asn Leu Ser Asp Gly Leu Arg Ala Gly Ala Gln Ala Ser Val Gly Ile
    290                 295                 300

Ser Met Met Phe Phe Val Ser Pro Asn Leu Ala Thr Phe Val Leu Ser
305                 310                 315                 320

Val Val Pro Pro Val Ser Ile Ile Ala Val Ile Tyr Gly Arg Tyr Leu
                325                 330                 335

Arg Lys Leu Thr Lys Val Thr Gln Asp Ser Leu Ala Gln Ala Thr Gln
            340                 345                 350

Leu Ala Glu Glu Arg Ile Gly Asn Val Arg Thr Val Arg Ala Phe Gly
        355                 360                 365

Lys Glu Met Thr Glu Ile Glu Lys Tyr Ala Ser Lys Val Asp His Val
    370                 375                 380

Met Gln Leu Ala Arg Lys Glu Ala Val Ala Arg Ala Gly Phe Phe Gly
385                 390                 395                 400

Ala Thr Gly Leu Ser Gly Asn Leu Ile Val Leu Ser Val Leu Tyr Lys
                405                 410                 415

Gly Gly Leu Leu Met Gly Ser Ala His Met Thr Val Gly Glu Leu Ser
            420                 425                 430

Ser Phe Leu Met Tyr Ala Phe Trp Val Gly Ile Ser Ile Gly Gly Leu

        435                 440                 445

Ser Ser Phe Tyr Ser Glu Leu Met Lys Gly Leu Gly Ala Gly Gly Arg
    450                 455                 460

Leu Trp Glu Leu Leu Glu Arg Glu Pro Lys Leu Pro Phe Asn Glu Gly
465                 470                 475                 480

Val Ile Leu Asn Glu Lys Ser Phe Gln Gly Ala Leu Glu Phe Lys Asn
                485                 490                 495

Val His Phe Ala Tyr Pro Ala Arg Pro Glu Val Pro Ile Phe Gln Asp
            500                 505                 510

Phe Ser Leu Ser Ile Pro Ser Gly Ser Val Thr Ala Leu Val Gly Pro
        515                 520                 525

Ser Gly Ser Gly Lys Ser Thr Val Leu Ser Leu Leu Leu Arg Leu Tyr
    530                 535                 540

Asn Pro Ala Ser Gly Thr Ile Ser Leu Asp Gly His Asp Ile Arg Gln
545                 550                 555                 560

Leu Asn Pro Val Trp Leu Arg Ser Lys Ile Gly Thr Val Ser Gln Glu
                565                 570                 575

Pro Ile Leu Phe Ser Cys Ser Ile Ala Glu Asn Ile Ala Tyr Gly Ala
            580                 585                 590

Asp Asp Pro Ser Ser Val Thr Ala Glu Glu Ile Gln Arg Val Ala Glu
        595                 600                 605

Val Ala Asn Ala Val Ala Phe Ile Arg Asn Phe Pro Gln Gly Phe Asn
    610                 615                 620

Thr Val Val Gly Glu Lys Gly Val Leu Leu Ser Gly Gly Gln Lys Gln
625                 630                 635                 640

Arg Ile Ala Ile Ala Arg Ala Leu Leu Lys Asn Pro Lys Ile Leu Leu
                645                 650                 655

Leu Asp Glu Ala Thr Ser Ala Leu Asp Ala Glu Asn Glu Tyr Leu Val
            660                 665                 670

Gln Glu Ala Leu Asp Arg Leu Met Asp Gly Arg Thr Val Leu Val Ile
        675                 680                 685

Ala His Arg Leu Ser Thr Ile Lys Asn Ala Asn Met Val Ala Val Leu

690                 695                 700

Asp Gln Gly Lys Ile Thr Glu Tyr Gly Lys His Glu Glu Leu Leu Ser
705                 710                 715                 720

Lys Pro Asn Gly Ile Tyr Arg Lys Leu Met Asn Lys Gln Ser Phe Ile
                725                 730                 735

Ser Ala

<210> 59
<211> 1321
<212> PRT
<213> Homo sapiens

<400> 59

<210> 60
<211> 1481
<212> PRT
<213> Homo sapiens

<400> 60
Met Ala Leu Arg Gly Phe Cys Ser Ala Asp Gly Ser Asp Pro Leu Trp
  1               5                  10                  15

Asp Trp Asn Val Thr Trp Asn Thr Ser Asn Pro Asp Phe Thr Lys Cys
             20                  25                  30

Phe Gln Asn Thr Val Leu Val Trp Val Pro Cys Phe Tyr Leu Trp Ala
         35                  40                  45

Cys Phe Pro Phe Tyr Phe Leu Tyr Leu Ser Arg His Asp Arg Gly Tyr
     50                  55                  60

Ile Gln Met Thr Pro Leu Asn Lys Thr Lys Thr Ala Leu Gly Phe Leu
65                  70                  75                  80

Leu Trp Ile Val Cys Trp Ala Asp Leu Phe Tyr Ser Phe Trp Glu Arg
                85                  90                  95

Ser Arg Gly Ile Phe Leu Ala Pro Val Phe Leu Val Ser Pro Thr Leu
            100                 105                 110

Leu Gly Ile Thr Thr Leu Leu Ala Thr Phe Leu Ile Gln Leu Glu Arg
        115                 120                 125

Arg Lys Gly Val Gln Ser Ser Gly Ile Met Leu Thr Phe Trp Leu Val
    130                 135                 140

Ala Leu Val Cys Ala Leu Ala Ile Leu Arg Ser Lys Ile Met Thr Ala
145                 150                 155                 160

Leu Lys Glu Asp Ala Gln Val Asp Leu Phe Arg Asp Ile Thr Phe Tyr
                165                 170                 175

Val Tyr Phe Ser Leu Leu Leu Ile Gln Leu Val Leu Ser Cys Phe Ser
            180                 185                 190

Asp Arg Ser Pro Leu Phe Ser Glu Thr Ile His Asp Pro Asn Pro Cys
        195                 200                 205

Pro Glu Ser Ser Ala Ser Phe Leu Ser Arg Ile Thr Phe Trp Trp Ile
    210                 215                 220

Thr Gly Leu Ile Val Arg Gly Tyr Arg Gln Pro Leu Glu Gly Ser Asp
225                 230                 235                 240

Leu Trp Ser Leu Asn Lys Glu Asp Thr Ser Glu Gln Val Val Pro Val
                245                 250                 255

Leu Val Lys Asn Trp Lys Lys Glu Cys Ala Lys Thr Arg Lys Gln Pro
            260                 265                 270

Val Lys Val Val Tyr Ser Ser Lys Asp Pro Ala Gln Pro Lys Glu Ser
        275                 280                 285

Ser Lys Val Asp Ala Asn Glu Glu Val Glu Ala Leu Ile Val Lys Ser
    290                 295                 300

Pro Gln Lys Glu Trp Asn Pro Ser Leu Phe Lys Val Leu Tyr Lys Thr
305                 310                 315                 320

Phe Gly Pro Tyr Phe Leu Met Ser Phe Phe Phe Lys Ala Ile His Asp
                325                 330                 335

Leu Met Met Phe Ser Gly Pro Gln Ile Leu Lys Leu Leu Ile Lys Phe
            340                 345                 350

Val Asn Asp Thr Lys Ala Pro Asp Trp Gln Gly Tyr Phe Tyr Thr Val
        355                 360                 365

Leu Leu Phe Val Thr Ala Cys Leu Gln Thr Leu Val Leu His Gln Tyr
    370                 375                 380

Phe His Ile Cys Phe Val Ser Gly Met Arg Ile Lys Thr Ala Val Ile
385                 390                 395                 400

Gly Ala Val Tyr Arg Lys Ala Leu Val Ile Thr Asn Ser Ala Arg Lys
                405                 410                 415

Ser Ser Thr Val Gly Glu Ile Val Asn Leu Met Ser Val Asp Ala Gln
            420                 425                 430

Arg Phe Met Asp Leu Ala Thr Tyr Ile Asn Met Ile Trp Ser Ala Pro
        435                 440                 445

Leu Gln Val Ile Leu Ala Leu Tyr Leu Leu Trp Leu Asn Leu Gly Pro
    450                 455                 460

Ser Val Leu Ala Gly Val Ala Val Met Val Leu Met Val Pro Val Asn
465                 470                 475                 480

Ala Val Met Ala Met Lys Thr Lys Thr Tyr Gln Val Ala His Met Lys
                485                 490                 495

Ser Lys Asp Asn Arg Ile Lys Leu Met Asn Glu Ile Leu Asn Gly Ile
            500                 505                 510

Lys Val Leu Lys Leu Tyr Ala Trp Glu Leu Ala Phe Lys Asp Lys Val
        515                 520                 525

Leu Ala Ile Arg Gln Glu Glu Leu Lys Val Leu Lys Lys Ser Ala Tyr
    530                 535                 540

Leu Ser Ala Val Gly Thr Phe Thr Trp Val Cys Thr Pro Phe Leu Ala
545                 550                 555                 560

Ser Val Ser Leu Lys Arg Leu Arg Ile Phe Leu Ser His Glu Glu Leu
                565                 570                 575

Glu Pro Asp Ser Ile Glu Arg Arg Pro Val Lys Asp Gly Gly Gly Thr
            580                 585                 590

Asn Ser Ile Thr Val Arg Asn Ala Thr Phe Thr Trp Ala Arg Ser Asp
        595                 600                 605

Pro Pro Thr Leu Asn Gly Ile Thr Phe Ser Ile Pro Glu Gly Ala Leu
    610                 615                 620

Val Ala Val Val Gly Gln Val Gly Cys Gly Lys Ser Ser Leu Leu Ser
625                 630                 635                 640

Ala Leu Leu Ala Glu Met Asp Lys Val Glu Gly His Val Ala Ile Lys
                645                 650                 655

Gly Ser Val Ala Tyr Val Pro Gln Gln Ala Trp Ile Gln Asn Asp Ser
            660                 665                 670

Leu Arg Glu Asn Ile Leu Phe Gly Cys Gln Leu Glu Glu Pro Tyr Tyr
        675                 680                 685

Arg Ser Val Ile Gln Ala Cys Ala Leu Leu Pro Asp Leu Glu Ile Leu
    690                 695                 700

Pro Ser Gly Asp Arg Thr Glu Ile Gly Glu Lys Gly Val Asn Leu Ser
705                 710                 715                 720

Gly Gly Gln Lys Gln Arg Val Ser Leu Ala Arg Ala Val Tyr Ser Asn
                725                 730                 735

Ala Asp Ile Tyr Leu Phe Asp Asp Pro Leu Ser Ala Val Asp Ala His
            740                 745                 750

Val Gly Lys His Ile Phe Glu Asn Val Ile Gly Pro Lys Gly Met Leu
        755                 760                 765

Lys Asn Lys Thr Arg Ile Leu Val Thr His Ser Met Ser Tyr Leu Pro
    770                 775                 780

Gln Val Asp Val Ile Ile Val Met Ser Gly Gly Lys Ile Ser Glu Met
785                 790                 795                 800

Gly Ser Tyr Gln Glu Leu Leu Ala Arg Asp Gly Ala Phe Ala Glu Phe
                805                 810                 815

Leu Arg Thr Tyr Ala Ser Thr Glu Gln Glu Gln Asp Ala Glu Glu Asn
            820                 825                 830

Gly Val Thr Gly Val Ser Gly Pro Gly Lys Glu Ala Lys Gln Met Glu
        835                 840                 845

Asn Gly Met Leu Val Thr Asp Ser Ala Gly Lys Gln Leu Gln Arg Gln
    850                 855                 860

Leu Ser Ser Ser Ser Ser Tyr Ser Gly Asp Ile Ser Arg His His Asn
865                 870                 875                 880

Ser Thr Ala Glu Leu Gln Lys Ala Glu Ala Lys Lys Glu Glu Thr Trp
                885                 890                 895

Lys Leu Met Glu Ala Asp Lys Ala Gln Thr Gly Gln Val Lys Leu Ser
            900                 905                 910

Val Tyr Trp Asp Tyr Met Lys Ala Ile Gly Leu Phe Ile Ser Phe Leu
        915                 920                 925

Ser Ile Phe Leu Phe Met Cys Asn His Val Ser Ala Leu Ala Ser Asn
    930                 935                 940

Tyr Trp Leu Ser Leu Trp Thr Asp Asp Pro Ile Val Asn Gly Thr Gln
945                 950                 955                 960

Glu His Thr Lys Val Arg Leu Ser Val Tyr Gly Ala Leu Gly Ile Ser
                965                 970                 975

Gln Gly Ile Ala Val Phe Gly Tyr Ser Met Ala Val Ser Ile Gly Gly
            980                 985                 990

Ile Leu Ala Ser Arg Cys Leu His Val Asp Leu Leu His Ser Ile Leu
        995                 1000                1005

Arg Ser Pro Met Ser Phe Phe Glu Arg Thr Pro Ser Gly Asn Leu Val
    1010                1015                1020

Asn Arg Phe Ser Lys Glu Leu Asp Thr Val Asp Ser Met Ile Pro Glu
1025                1030                1035                1040

Val Ile Lys Met Phe Met Gly Ser Leu Phe Asn Val Ile Gly Ala Cys
                1045                1050                1055

Ile Val Ile Leu Leu Ala Thr Pro Ile Ala Ala Ile Ile Ile Pro Pro
            1060                1065                1070

Leu Gly Leu Ile Tyr Phe Phe Val Gln Arg Phe Tyr Val Ala Ser Ser
        1075                1080                1085

Arg Gln Leu Lys Arg Leu Glu Ser Val Ser Arg Ser Pro Val Tyr Ser
    1090                1095                1100

His Phe Asn Glu Thr Leu Leu Gly Val Ser Val Ile Arg Ala Phe Glu
1105                1110                1115                1120

Glu Gln Glu Arg Phe Ile His Gln Ser Asp Leu Lys Val Asp Glu Asn
                1125                1130                1135

Gln Lys Ala Tyr Tyr Pro Ser Ile Val Ala Asn Arg Trp Leu Ala Val
            1140                1145                1150

Arg Leu Glu Cys Val Gly Asn Cys Ile Val Leu Phe Ala Ala Leu Phe
        1155                1160                1165

Ala Val Ile Ser Arg His Ser Leu Ser Ala Gly Leu Val Gly Leu Ser
    1170                1175                1180

Val Ser Tyr Ser Leu Gln Val Thr Thr Tyr Leu Asn Trp Leu Val Arg
1185                1190                1195                1200

Met Ser Ser Glu Met Glu Thr Asn Ile Val Ala Val Glu Arg Leu Lys
                1205                1210                1215

Glu Tyr Ser Glu Thr Glu Lys Glu Ala Pro Trp Gln Ile Gln Glu Thr
            1220                1225                1230

Ala Pro Pro Ser Ser Trp Pro Gln Val Gly Arg Val Glu Phe Arg Asn
        1235                1240                1245

Tyr Cys Leu Arg Tyr Arg Glu Asp Leu Asp Phe Val Leu Arg His Ile
    1250                1255                1260

Asn Val Thr Ile Asn Gly Gly Glu Lys Val Gly Ile Val Gly Thr Gly
1265                1270                1275                1280

Ala Gly Lys Ser Ser Leu Thr Leu Gly Leu Phe Arg Ile Asn Glu Ser
                1285                1290                1295

Ala Glu Gly Glu Ile Ile Ile Asp Gly Ile Asn Ile Ala Lys Ile Gly
            1300                1305                1310

Leu His Asp Leu Arg Phe Lys Ile Thr Ile Ile Pro Gln Asp Pro Val
        1315                1320                1325

Leu Phe Ser Gly Ser Leu Arg Met Asn Leu Asp Pro Phe Ser Gln Tyr
    1330                1335                1340

Ser Asp Glu Glu Val Trp Thr Ser Leu Glu Leu Ala His Leu Lys Asp
1345                1350                1355                1360

Phe Val Ser Ala Leu Pro Asp Lys Leu Asp His Glu Cys Ala Glu Gly
                1365                1370                1375

Gly Glu Asn Leu Ser Val Gly Gln Arg Gln Leu Val Cys Leu Ala Arg
            1380                1385                1390

Ala Leu Leu Arg Lys Thr Lys Ile Leu Val Leu Asp Glu Ala Thr Ala
        1395                1400                1405

Ala Val Asp Leu Glu Thr Asp Asp Leu Ile Gln Ser Thr Ile Arg Thr
    1410                1415                1420

Gln Phe Glu Asp Cys Thr Val Leu Thr Ile Ala His Arg Leu Asn Thr
1425                1430                1435                1440

Ile Met Asp Tyr Thr Arg Val Ile Val Leu Asp Lys Gly Glu Ile Gln
                1445                1450                1455

Glu Tyr Gly Ala Pro Ser Asp Leu Leu Gln Gln Arg Gly Leu Phe Tyr
            1460                1465                1470

Ser Met Ala Lys Asp Ala Gly Leu Val
        1475                1480

<210> 61
<211> 1545
<212> PRT
<213> Homo sapiens

<400> 61
Met Leu Glu Lys Phe Cys Asn Ser Thr Phe Trp Asn Ser Ser Phe Leu
  1               5                  10                  15

Asp Ser Pro Glu Ala Asp Leu Pro Leu Cys Phe Glu Gln Thr Val Leu
             20                  25                  30

Val Trp Ile Pro Leu Gly Phe Leu Trp Leu Leu Ala Pro Trp Gln Leu
         35                  40                  45

Leu His Val Tyr Lys Ser Arg Thr Lys Arg Ser Ser Thr Thr Lys Leu
     50                  55                  60

Tyr Leu Ala Lys Gln Val Phe Val Gly Phe Leu Leu Ile Leu Ala Ala

65                  70                  75                  80

Ile Glu Leu Ala Leu Val Leu Thr Glu Asp Ser Gly Gln Ala Thr Val
                85                  90                  95

Pro Ala Val Arg Tyr Thr Asn Pro Ser Leu Tyr Leu Gly Thr Trp Leu
            100                 105                 110

Leu Val Leu Leu Ile Gln Tyr Ser Arg Gln Trp Cys Val Gln Lys Asn
        115                 120                 125

Ser Trp Phe Leu Ser Leu Phe Trp Ile Leu Ser Ile Leu Cys Gly Thr
    130                 135                 140

Phe Gln Phe Gln Thr Leu Ile Arg Thr Leu Leu Gln Gly Asp Asn Ser
145                 150                 155                 160

Asn Leu Ala Tyr Ser Cys Leu Phe Phe Ile Ser Tyr Gly Phe Gln Ile
                165                 170                 175

Leu Ile Leu Ile Phe Ser Ala Phe Ser Glu Asn Asn Glu Ser Ser Asn
            180                 185                 190

Asn Pro Ser Ser Ile Ala Ser Phe Leu Ser Ser Ile Thr Tyr Ser Trp
        195                 200                 205

Tyr Asp Ser Ile Ile Leu Lys Gly Tyr Lys Arg Pro Leu Thr Leu Glu
    210                 215                 220

Asp Val Trp Glu Val Asp Glu Glu Met Lys Thr Lys Thr Leu Val Ser
225                 230                 235                 240

Lys Phe Glu Thr His Met Lys Arg Glu Leu Gln Lys Ala Arg Arg Ala
                245                 250                 255

Leu Gln Arg Arg Gln Glu Lys Ser Ser Gln Gln Asn Ser Gly Ala Arg
            260                 265                 270

Leu Pro Gly Leu Asn Lys Asn Gln Ser Gln Ser Gln Asp Ala Leu Val
        275                 280                 285

Leu Glu Asp Val Glu Lys Lys Lys Lys Lys Ser Gly Thr Lys Lys Asp
    290                 295                 300

Val Pro Lys Ser Trp Leu Met Lys Ala Leu Phe Lys Thr Phe Tyr Met
305                 310                 315                 320

Val Leu Leu Lys Ser Phe Leu Leu Lys Leu Val Asn Asp Ile Phe Thr

325                 330                 335

Phe Val Ser Pro Gln Leu Leu Lys Leu Leu Ile Ser Phe Ala Ser Asp
            340                 345                 350

Arg Asp Thr Tyr Leu Trp Ile Gly Tyr Leu Cys Ala Ile Leu Leu Phe
        355                 360                 365

Thr Ala Ala Leu Ile Gln Ser Phe Cys Leu Gln Cys Tyr Phe Gln Leu
    370                 375                 380

Cys Phe Lys Leu Gly Val Lys Val Arg Thr Ala Ile Met Ala Ser Val
385                 390                 395                 400

Tyr Lys Lys Ala Leu Thr Leu Ser Asn Leu Ala Arg Lys Glu Tyr Thr
                405                 410                 415

Val Gly Glu Thr Val Asn Leu Met Ser Val Asp Ala Gln Lys Leu Met
            420                 425                 430

Asp Val Thr Asn Phe Met His Met Leu Trp Ser Ser Val Leu Gln Ile
        435                 440                 445

Val Leu Ser Ile Phe Phe Leu Trp Arg Glu Leu Gly Pro Ser Val Leu
    450                 455                 460

Ala Gly Val Gly Val Met Val Leu Val Ile Pro Ile Asn Ala Ile Leu
465                 470                 475                 480

Ser Thr Lys Ser Lys Thr Ile Gln Val Lys Asn Met Lys Asn Lys Asp
                485                 490                 495

Lys Arg Leu Lys Ile Met Asn Glu Ile Leu Ser Gly Ile Lys Ile Leu
            500                 505                 510

Lys Tyr Phe Ala Trp Glu Pro Ser Phe Arg Asp Gln Val Gln Asn Leu
        515                 520                 525

Arg Lys Lys Glu Leu Lys Asn Leu Leu Ala Phe Ser Gln Leu Gln Cys
    530                 535                 540

Val Val Ile Phe Val Phe Gln Leu Thr Pro Val Leu Val Ser Val Val
545                 550                 555                 560

Thr Phe Ser Val Tyr Val Leu Val Asp Ser Asn Asn Ile Leu Asp Ala
                565                 570                 575

Gln Lys Ala Phe Thr Ser Ile Thr Leu Phe Asn Ile Leu Arg Phe Pro

580                 585                 590

Leu Ser Met Leu Pro Met Met Ile Ser Ser Met Leu Gln Ala Ser Val
        595                 600                 605

Ser Thr Glu Arg Leu Glu Lys Tyr Leu Gly Gly Asp Asp Leu Asp Thr
    610                 615                 620

Ser Ala Ile Arg His Asp Cys Asn Phe Asp Lys Ala Met Gln Phe Ser
625                 630                 635                 640

Glu Ala Ser Phe Thr Trp Glu His Asp Ser Glu Ala Thr Val Arg Asp
                645                 650                 655

Val Asn Leu Asp Ile Met Ala Gly Gln Leu Val Ala Val Ile Gly Pro
            660                 665                 670

Val Gly Ser Gly Lys Ser Ser Leu Ile Ser Ala Met Leu Gly Glu Met
        675                 680                 685

Glu Asn Val His Gly His Ile Thr Ile Lys Gly Thr Thr Ala Tyr Val
    690                 695                 700

Pro Gln Gln Ser Trp Ile Gln Asn Gly Thr Ile Lys Asp Asn Ile Leu
705                 710                 715                 720

Phe Gly Thr Glu Phe Asn Glu Lys Arg Tyr Gln Gln Val Leu Glu Ala
                725                 730                 735

Cys Ala Leu Leu Pro Asp Leu Glu Met Leu Pro Gly Gly Asp Leu Ala
            740                 745                 750

Glu Ile Gly Glu Lys Gly Ile Asn Leu Ser Gly Gly Gln Lys Gln Arg
        755                 760                 765

Ile Ser Leu Ala Arg Ala Thr Tyr Gln Asn Leu Asp Ile Tyr Leu Leu
    770                 775                 780

Asp Asp Pro Leu Ser Ala Val Asp Ala His Val Gly Lys His Ile Phe
785                 790                 795                 800

Asn Lys Val Leu Gly Pro Asn Gly Leu Leu Lys Gly Lys Thr Arg Leu
                805                 810                 815

Leu Val Thr His Ser Met His Phe Leu Pro Gln Val Asp Glu Ile Val
            820                 825                 830

Val Leu Gly Asn Gly Thr Ile Val Glu Lys Gly Ser Tyr Ser Ala Leu

        835                 840                 845

Leu Ala Lys Lys Gly Glu Phe Ala Lys Asn Leu Lys Thr Phe Leu Arg
    850                 855                 860

His Thr Gly Pro Glu Glu Glu Ala Thr Val His Asp Gly Ser Glu Glu
865                 870                 875                 880

Glu Asp Asp Asp Tyr Gly Leu Ile Ser Ser Val Glu Glu Ile Pro Glu
                885                 890                 895

Asp Ala Ala Ser Ile Thr Met Arg Arg Glu Asn Ser Phe Arg Arg Thr
            900                 905                 910

Leu Ser Arg Ser Ser Arg Ser Asn Gly Arg His Leu Lys Ser Leu Arg
        915                 920                 925

Asn Ser Leu Lys Thr Arg Asn Val Asn Ser Leu Lys Glu Asp Glu Glu
    930                 935                 940

Leu Val Lys Gly Gln Lys Leu Ile Lys Lys Glu Phe Ile Glu Thr Gly
945                 950                 955                 960

Lys Val Lys Phe Ser Ile Tyr Leu Glu Tyr Leu Gln Ala Ile Gly Leu
                965                 970                 975

Phe Ser Ile Phe Phe Ile Ile Leu Ala Phe Val Met Asn Ser Val Ala
            980                 985                 990

Phe Ile Gly Ser Asn Leu Trp Leu Ser Ala Trp Thr Ser Asp Ser Lys
        995                 1000                1005

Ile Phe Asn Ser Thr Asp Tyr Pro Ala Ser Gln Arg Asp Met Arg Val
    1010                1015                1020

Gly Val Tyr Gly Ala Leu Gly Leu Ala Gln Gly Ile Phe Val Phe Ile
1025                1030                1035                1040

Ala His Phe Trp Ser Ala Phe Gly Phe Val His Ala Ser Asn Ile Leu
                1045                1050                1055

His Lys Gln Leu Leu Asn Asn Ile Leu Arg Ala Pro Met Arg Phe Phe
            1060                1065                1070

Asp Thr Thr Pro Thr Gly Arg Ile Val Asn Arg Phe Ala Gly Asp Ile
        1075                1080                1085

Ser Thr Val Asp Asp Thr Leu Pro Gln Ser Leu Arg Ser Trp Ile Thr

1090                1095                1100

Cys Phe Leu Gly Ile Ile Ser Thr Leu Val Met Ile Cys Met Ala Thr
1105                1110                1115                1120

Pro Val Phe Thr Ile Ile Val Ile Pro Leu Gly Ile Ile Tyr Val Ser
                1125                1130                1135

Val Gln Met Phe Tyr Val Ser Thr Ser Arg Gln Leu Arg Arg Leu Asp
            1140                1145                1150

Ser Val Thr Arg Ser Pro Ile Tyr Ser His Phe Ser Glu Thr Val Ser
        1155                1160                1165

Gly Leu Pro Val Ile Arg Ala Phe Glu His Gln Gln Arg Phe Leu Lys
    1170                1175                1180

His Asn Glu Val Arg Ile Asp Thr Asn Gln Lys Cys Val Phe Ser Trp
1185                1190                1195                1200

Ile Thr Ser Asn Arg Trp Leu Ala Ile Arg Leu Glu Leu Val Gly Asn
                1205                1210                1215

Leu Thr Val Phe Phe Ser Ala Leu Met Met Val Ile Tyr Arg Asp Thr
            1220                1225                1230

Leu Ser Gly Asp Thr Val Gly Phe Val Leu Ser Asn Ala Leu Asn Ile
        1235                1240                1245

Thr Gln Thr Leu Asn Trp Leu Val Arg Met Thr Ser Glu Ile Glu Thr
    1250                1255                1260

Asn Ile Val Ala Val Glu Arg Ile Thr Glu Tyr Thr Lys Val Glu Asn
1265                1270                1275                1280

Glu Ala Pro Trp Val Thr Asp Lys Arg Pro Pro Pro Asp Trp Pro Ser
                1285                1290                1295

Lys Gly Lys Ile Gln Phe Asn Asn Tyr Gln Val Arg Tyr Arg Pro Glu
            1300                1305                1310

Leu Asp Leu Val Leu Arg Gly Ile Thr Cys Asp Ile Gly Ser Met Glu
        1315                1320                1325

Lys Ile Gly Val Val Gly Arg Thr Gly Ala Gly Lys Ser Ser Leu Thr
    1330                1335                1340

Asn Cys Leu Phe Arg Ile Leu Glu Ala Ala Gly Gly Gln Ile Ile Ile

1345                1350                1355                1360

Asp Gly Val Asp Ile Ala Ser Ile Gly Leu His Asp Leu Arg Glu Lys
                1365                1370                1375

Leu Thr Ile Ile Pro Gln Asp Pro Ile Leu Phe Ser Gly Ser Leu Arg
            1380                1385                1390

Met Asn Leu Asp Pro Phe Asn Asn Tyr Ser Asp Glu Glu Ile Trp Lys
        1395                1400                1405

Ala Leu Glu Leu Ala His Leu Lys Ser Phe Val Ala Ser Leu Gln Leu
    1410                1415                1420

Gly Leu Ser His Glu Val Thr Glu Ala Gly Gly Asn Leu Ser Ile Gly
1425                1430                1435                1440

Gln Arg Gln Leu Leu Cys Leu Gly Arg Ala Leu Leu Arg Lys Ser Lys
                1445                1450                1455

Ile Leu Val Leu Asp Glu Ala Thr Ala Ala Val Asp Leu Glu Thr Asp
            1460                1465                1470

Asn Leu Ile Gln Thr Thr Ile Gln Asn Glu Phe Ala His Cys Thr Val
        1475                1480                1485

Ile Thr Ile Ala His Arg Leu His Thr Ile Met Asp Ser Asp Lys Val
    1490                1495                1500

Met Val Leu Asp Asn Gly Lys Ile Ile Glu Cys Gly Ser Pro Glu Glu
1505                1510                1515                1520

Leu Leu Gln Ile Pro Gly Pro Phe Tyr Phe Met Ala Lys Glu Ala Gly
                1525                1530                1535

Ile Glu Asn Val Asn Ser Thr Lys Phe
            1540                1545

<210> 62
<211> 1527
<212> PRT
<213> Homo sapiens

<400> 62
Met Asp Ala Leu Cys Gly Ser Gly Glu Leu Gly Ser Lys Phe Trp Asp
1               5                   10                  15

Ser Asn Leu Ser Val His Thr Glu Asn Pro Asp Leu Thr Pro Cys Phe
            20                  25                  30

Gln Asn Ser Leu Leu Ala Trp Val Pro Cys Ile Tyr Leu Trp Val Ala
        35                  40                  45

Leu Pro Cys Tyr Leu Leu Tyr Leu Arg His His Cys Arg Gly Tyr Ile
    50                  55                  60

Ile Leu Ser His Leu Ser Lys Leu Lys Met Val Leu Gly Val Leu Leu
65                  70                  75                  80

Trp Cys Val Ser Trp Ala Asp Leu Phe Tyr Ser Phe His Gly Leu Val
                85                  90                  95

His Gly Arg Ala Pro Ala Pro Val Phe Phe Val Thr Pro Leu Val Val
            100                 105                 110

Gly Val Thr Met Leu Leu Ala Thr Leu Leu Ile Gln Tyr Glu Arg Leu
        115                 120                 125

Gln Gly Val Gln Ser Ser Gly Val Leu Ile Ile Phe Trp Phe Leu Cys
    130                 135                 140

Val Val Cys Ala Ile Val Pro Phe Arg Ser Lys Ile Leu Leu Ala Lys
145                 150                 155                 160

Ala Glu Gly Glu Ile Ser Asp Pro Phe Arg Phe Thr Thr Phe Tyr Ile
                165                 170                 175

His Phe Ala Leu Val Leu Ser Ala Leu Ile Leu Ala Cys Phe Arg Glu
            180                 185                 190

Lys Pro Pro Phe Phe Ser Ala Lys Asn Val Asp Pro Asn Pro Tyr Pro
        195                 200                 205

Glu Thr Ser Ala Gly Phe Leu Ser Arg Leu Phe Phe Trp Trp Phe Thr
    210                 215                 220

Lys Met Ala Ile Tyr Gly Tyr Arg His Pro Leu Glu Glu Lys Asp Leu
225                 230                 235                 240

Trp Ser Leu Lys Glu Glu Asp Arg Ser Gln Met Val Val Gln Gln Leu
                245                 250                 255

Leu Glu Ala Trp Arg Lys Gln Glu Lys Gln Thr Ala Arg His Lys Ala
            260                 265                 270

Ser Ala Ala Pro Gly Lys Asn Ala Ser Gly Glu Asp Glu Val Leu Leu
        275                 280                 285

Gly Ala Arg Pro Arg Pro Arg Lys Pro Ser Phe Leu Lys Ala Leu Leu
    290                 295                 300

Ala Thr Phe Gly Ser Ser Phe Leu Ile Ser Ala Cys Phe Lys Leu Ile
305                 310                 315                 320

Gln Asp Leu Leu Ser Phe Ile Asn Pro Gln Leu Leu Ser Ile Leu Ile
                325                 330                 335

Arg Phe Ile Ser Asn Pro Met Ala Pro Ser Trp Trp Gly Phe Leu Val
            340                 345                 350

Ala Gly Leu Met Phe Leu Cys Ser Met Met Gln Ser Leu Ile Leu Gln
        355                 360                 365

His Tyr Tyr His Tyr Ile Phe Val Thr Gly Val Lys Phe Arg Thr Gly
    370                 375                 380

Ile Met Gly Val Ile Tyr Arg Lys Ala Leu Val Ile Thr Asn Ser Val
385                 390                 395                 400

Lys Arg Ala Ser Thr Val Gly Glu Ile Val Asn Leu Met Ser Val Asp
                405                 410                 415

Ala Gln Arg Phe Met Asp Leu Ala Pro Phe Leu Asn Leu Leu Trp Ser
            420                 425                 430

Ala Pro Leu Gln Ile Ile Leu Ala Ile Tyr Phe Leu Trp Gln Asn Leu
        435                 440                 445

Gly Pro Ser Val Leu Ala Gly Val Ala Phe Met Val Leu Leu Ile Pro
    450                 455                 460

Leu Asn Gly Ala Val Ala Val Lys Met Arg Ala Phe Gln Val Lys Gln
465                 470                 475                 480

Met Lys Leu Lys Asp Ser Arg Ile Lys Leu Met Ser Glu Ile Leu Asn
                485                 490                 495

Gly Ile Lys Val Leu Lys Leu Tyr Ala Trp Glu Pro Ser Phe Leu Lys
            500                 505                 510

Gln Val Glu Gly Ile Arg Gln Gly Glu Leu Gln Leu Leu Arg Thr Ala
        515                 520                 525

Ala Tyr Leu His Thr Thr Thr Thr Phe Thr Trp Met Cys Ser Pro Phe
    530                 535                 540

Leu Val Thr Leu Ile Thr Leu Trp Val Tyr Val Tyr Val Asp Pro Asn
545                 550                 555                 560

Asn Val Leu Asp Ala Glu Lys Ala Phe Val Ser Val Ser Leu Phe Asn
                565                 570                 575

Ile Leu Arg Leu Pro Leu Asn Met Leu Pro Gln Leu Ile Ser Asn Leu
            580                 585                 590

Thr Gln Ala Ser Val Ser Leu Lys Arg Ile Gln Gln Phe Leu Ser Gln
        595                 600                 605

Glu Glu Leu Asp Pro Gln Ser Val Glu Arg Lys Thr Ile Ser Pro Gly
    610                 615                 620

Tyr Ala Ile Thr Ile His Ser Gly Thr Phe Thr Trp Ala Gln Asp Leu
625                 630                 635                 640

Pro Pro Thr Leu His Ser Leu Asp Ile Gln Val Pro Lys Gly Ala Leu
                645                 650                 655

Val Ala Val Val Gly Pro Val Gly Cys Gly Lys Ser Ser Leu Val Ser
            660                 665                 670

Ala Leu Leu Gly Glu Met Glu Lys Leu Glu Gly Lys Val His Met Lys
        675                 680                 685

Gly Ser Val Ala Tyr Val Pro Gln Gln Ala Trp Ile Gln Asn Cys Thr
    690                 695                 700

Leu Gln Glu Asn Val Leu Phe Gly Lys Ala Leu Asn Pro Lys Arg Tyr
705                 710                 715                 720

Gln Gln Thr Leu Glu Ala Cys Ala Leu Leu Ala Asp Leu Glu Met Leu
                725                 730                 735

Pro Gly Gly Asp Gln Thr Glu Ile Gly Glu Lys Gly Ile Asn Leu Ser
            740                 745                 750

Gly Gly Gln Arg Gln Arg Val Ser Leu Ala Arg Ala Val Tyr Ser Asp
        755                 760                 765

Ala Asp Ile Phe Leu Leu Asp Asp Pro Leu Ser Ala Val Asp Ser His
    770                 775                 780

Val Ala Lys His Ile Phe Asp His Val Ile Gly Pro Glu Gly Val Leu
785                 790                 795                 800

Ala Gly Lys Thr Arg Val Leu Val Thr His Gly Ile Ser Phe Leu Pro
                805                 810                 815

Gln Thr Asp Phe Ile Ile Val Leu Ala Asp Gly Gln Val Ser Glu Met
            820                 825                 830

Gly Pro Tyr Pro Ala Leu Leu Gln Arg Asn Gly Ser Phe Ala Asn Phe
        835                 840                 845

Leu Cys Asn Tyr Ala Pro Asp Glu Asp Gln Gly His Leu Glu Asp Ser
    850                 855                 860

Trp Thr Ala Leu Glu Gly Ala Glu Asp Lys Glu Ala Leu Leu Ile Glu
865                 870                 875                 880

Asp Thr Leu Ser Asn His Thr Asp Leu Thr Asp Asn Asp Pro Val Thr
                885                 890                 895

Tyr Val Val Gln Lys Gln Phe Met Arg Gln Leu Ser Ala Leu Ser Ser
            900                 905                 910

Asp Gly Glu Gly Gln Gly Arg Pro Val Pro Arg Arg His Leu Gly Pro
        915                 920                 925

Ser Glu Lys Val Gln Val Thr Glu Ala Lys Ala Asp Gly Ala Leu Thr
    930                 935                 940

Gln Glu Glu Lys Ala Ala Ile Gly Thr Val Glu Leu Ser Val Phe Trp
945                 950                 955                 960

Asp Tyr Ala Lys Ala Val Gly Leu Cys Thr Thr Leu Ala Ile Cys Leu
                965                 970                 975

Leu Tyr Val Gly Gln Ser Ala Ala Ala Ile Gly Ala Asn Val Trp Leu
            980                 985                 990

Ser Ala Trp Thr Asn Asp Ala Met Ala Asp Ser Arg Gln Asn Asn Thr
        995                 1000                1005

Ser Leu Arg Leu Gly Val Tyr Ala Ala Leu Gly Ile Leu Gln Gly Phe
    1010                1015                1020

Leu Val Met Leu Ala Ala Met Ala Met Ala Ala Gly Gly Ile Gln Ala
1025                1030                1035                1040

Ala Arg Val Leu His Gln Ala Leu Leu His Asn Lys Ile Arg Ser Pro
                1045                1050                1055

Gln Ser Phe Phe Asp Thr Thr Pro Ser Gly Arg Ile Leu Asn Cys Phe
            1060                1065                1070

Ser Lys Asp Ile Tyr Val Val Asp Glu Val Leu Ala Pro Val Ile Leu
        1075                1080                1085

Met Leu Leu Asn Ser Phe Phe Asn Ala Ile Ser Thr Leu Val Val Ile
    1090                1095                1100

Met Ala Ser Thr Pro Leu Phe Thr Val Val Ile Leu Pro Leu Ala Val
1105                1110                1115                1120

Leu Tyr Thr Leu Val Gln Arg Phe Tyr Ala Ala Thr Ser Arg Gln Leu
                1125                1130                1135

Lys Arg Leu Glu Ser Val Ser Arg Ser Pro Ile Tyr Ser His Phe Ser
            1140                1145                1150

Glu Thr Val Thr Gly Ala Ser Val Ile Arg Ala Tyr Asn Arg Ser Arg
        1155                1160                1165

Asp Phe Glu Ile Ile Ser Asp Thr Lys Val Asp Ala Asn Gln Arg Ser
    1170                1175                1180

Cys Tyr Pro Tyr Ile Ile Ser Asn Arg Trp Leu Ser Ile Gly Val Glu
1185                1190                1195                1200

Phe Val Gly Asn Cys Val Val Leu Phe Ala Ala Leu Phe Ala Val Ile
                1205                1210                1215

Gly Arg Ser Ser Leu Asn Pro Gly Leu Val Gly Leu Ser Val Ser Tyr
            1220                1225                1230

Ser Leu Gln Val Thr Phe Ala Leu Asn Trp Met Ile Arg Met Met Ser
        1235                1240                1245

Asp Leu Glu Ser Asn Ile Val Ala Val Glu Arg Val Lys Glu Tyr Ser
    1250                1255                1260

Lys Thr Glu Thr Glu Ala Pro Trp Val Val Glu Gly Ser Arg Pro Pro
1265                1270                1275                1280

Glu Gly Trp Pro Pro Arg Gly Glu Val Glu Phe Arg Asn Tyr Ser Val
                1285                1290                1295

Arg Tyr Arg Pro Gly Leu Asp Leu Val Leu Arg Asp Leu Ser Leu His
            1300                1305                1310

Val His Gly Gly Glu Lys Val Gly Ile Val Gly Arg Thr Gly Ala Gly
        1315                1320                1325

Lys Ser Ser Met Thr Leu Cys Leu Phe Arg Ile Leu Glu Ala Ala Lys
    1330                1335                1340

Gly Glu Ile Arg Ile Asp Gly Leu Asn Val Ala Asp Ile Gly Leu His
1345                1350                1355                1360

Asp Leu Arg Ser Gln Leu Thr Ile Ile Pro Gln Asp Pro Ile Leu Phe
                1365                1370                1375

Ser Gly Thr Leu Arg Met Asn Leu Asp Pro Phe Gly Ser Tyr Ser Glu
            1380                1385                1390

Glu Asp Ile Trp Trp Ala Leu Glu Leu Ser His Leu His Thr Phe Val
        1395                1400                1405

Ser Ser Gln Pro Ala Gly Leu Asp Phe Gln Cys Ser Glu Gly Gly Glu
    1410                1415                1420

Asn Leu Ser Val Gly Gln Arg Gln Leu Val Cys Leu Ala Arg Ala Leu
1425                1430                1435                1440

Leu Arg Lys Ser Arg Ile Leu Val Leu Asp Glu Ala Thr Ala Ala Ile
                1445                1450                1455

Asp Leu Glu Thr Asp Asn Leu Ile Gln Ala Thr Ile Arg Thr Gln Phe
            1460                1465                1470

Asp Thr Cys Thr Val Leu Thr Ile Ala His Arg Leu Asn Thr Ile Met
        1475                1480                1485

Asp Tyr Thr Arg Val Leu Val Leu Asp Lys Gly Val Val Ala Glu Phe
    1490                1495                1500

Asp Ser Pro Ala Asn Leu Ile Ala Ala Arg Gly Ile Phe Tyr Gly Met
1505                1510                1515                1520

Ala Arg Asp Ala Gly Leu Ala
                1525

<210> 63
<211> 1325
<212> PRT
<213> Homo sapiens

<400> 63
Met Leu Pro Val Tyr Gln Glu Val Lys Pro Asn Pro Leu Gln Asp Ala
1               5                   10                  15

Asn Ile Cys Ser Arg Val Phe Phe Trp Trp Leu Asn Pro Leu Phe Lys
            20                  25                  30

Ile Gly His Lys Arg Arg Leu Glu Glu Asp Asp Met Tyr Ser Val Leu
        35                  40                  45

Pro Glu Asp Arg Ser Gln His Leu Gly Glu Glu Leu Gln Gly Phe Trp
    50                  55                  60

Asp Lys Glu Val Leu Arg Ala Glu Asn Asp Ala Gln Lys Pro Ser Leu
65                  70                  75                  80

Thr Arg Ala Ile Ile Lys Cys Tyr Trp Lys Ser Tyr Leu Val Leu Gly
                85                  90                  95

Ile Phe Thr Leu Ile Glu Glu Ser Ala Lys Val Ile Gln Pro Ile Phe
            100                 105                 110

Leu Gly Lys Ile Ile Asn Tyr Phe Glu Asn Tyr Asp Pro Met Asp Ser
        115                 120                 125

Val Ala Leu Asn Thr Ala Tyr Ala Tyr Ala Thr Val Leu Thr Phe Cys
    130                 135                 140

Thr Leu Ile Leu Ala Ile Leu His His Leu Tyr Phe Tyr His Val Gln
145                 150                 155                 160

Cys Ala Gly Met Arg Leu Arg Val Ala Met Cys His Met Ile Tyr Arg
                165                 170                 175

Lys Ala Leu Arg Leu Ser Asn Met Ala Met Gly Lys Thr Thr Thr Gly
            180                 185                 190

Gln Ile Val Asn Leu Leu Ser Asn Asp Val Asn Lys Phe Asp Gln Val
        195                 200                 205

Thr Val Phe Leu His Phe Leu Trp Ala Gly Pro Leu Gln Ala Ile Ala
    210                 215                 220

Val Thr Ala Leu Leu Trp Met Glu Ile Gly Ile Ser Cys Leu Ala Gly
225                 230                 235                 240

Met Ala Val Leu Ile Ile Leu Leu Pro Leu Gln Ser Cys Phe Gly Lys
                245                 250                 255

Leu Phe Ser Ser Leu Arg Ser Lys Thr Ala Thr Phe Thr Asp Ala Arg
            260                 265                 270

Ile Arg Thr Met Asn Glu Val Ile Thr Gly Ile Arg Ile Ile Lys Met
        275                 280                 285

Tyr Ala Trp Glu Lys Ser Phe Ser Asn Leu Ile Thr Asn Leu Arg Lys
    290                 295                 300

Lys Glu Ile Ser Lys Ile Leu Arg Ser Ser Cys Leu Arg Gly Met Asn
305                 310                 315                 320

Leu Ala Ser Phe Phe Ser Ala Ser Lys Ile Ile Val Phe Val Thr Phe
                325                 330                 335

Thr Thr Tyr Val Leu Leu Gly Ser Val Ile Thr Ala Ser Arg Val Phe
            340                 345                 350

Val Ala Val Thr Leu Tyr Gly Ala Val Arg Leu Thr Val Thr Leu Phe
        355                 360                 365

Phe Pro Ser Ala Ile Glu Arg Val Ser Glu Ala Ile Val Ser Ile Arg
    370                 375                 380

Arg Ile Gln Thr Phe Leu Leu Leu Asp Glu Ile Ser Gln Arg Asn Arg
385                 390                 395                 400

Gln Leu Pro Ser Asp Gly Lys Lys Met Val His Val Gln Asp Phe Thr
                405                 410                 415

Ala Phe Trp Asp Lys Ala Ser Glu Thr Pro Thr Leu Gln Gly Leu Ser
            420                 425                 430

Phe Thr Val Arg Pro Gly Glu Leu Leu Ala Val Val Gly Pro Val Gly
        435                 440                 445

Ala Gly Lys Ser Ser Leu Leu Ser Ala Val Leu Gly Glu Leu Ala Pro
    450                 455                 460

Ser His Gly Leu Val Ser Val His Gly Arg Ile Ala Tyr Val Ser Gln
465                 470                 475                 480

Gln Pro Trp Val Phe Ser Gly Thr Leu Arg Ser Asn Ile Leu Phe Gly
                485                 490                 495

Lys Lys Tyr Glu Lys Glu Arg Tyr Glu Lys Val Ile Lys Ala Cys Ala
            500                 505                 510

Leu Lys Lys Asp Leu Gln Leu Leu Glu Asp Gly Asp Leu Thr Val Ile
        515                 520                 525

Gly Asp Arg Gly Thr Thr Leu Ser Gly Gly Gln Lys Ala Arg Val Asn
    530                 535                 540

Leu Ala Arg Ala Val Tyr Gln Asp Ala Asp Ile Tyr Leu Leu Asp Asp
545                 550                 555                 560

Pro Leu Ser Ala Val Asp Ala Glu Val Ser Arg His Leu Phe Glu Leu
                565                 570                 575

Cys Ile Cys Gln Ile Leu His Glu Lys Ile Thr Ile Leu Val Thr His
            580                 585                 590

Gln Leu Gln Tyr Leu Lys Ala Ala Ser Gln Ile Leu Ile Leu Lys Asp
        595                 600                 605

Gly Lys Met Val Gln Lys Gly Thr Tyr Thr Glu Phe Leu Lys Ser Gly
    610                 615                 620

Ile Asp Phe Gly Ser Leu Leu Lys Lys Asp Asn Glu Glu Ser Glu Gln
625                 630                 635                 640

Pro Pro Val Pro Gly Thr Pro Thr Leu Arg Asn Arg Thr Phe Ser Glu
                645                 650                 655

Ser Ser Val Trp Ser Gln Gln Ser Ser Arg Pro Ser Leu Lys Asp Gly
            660                 665                 670

Ala Leu Glu Ser Gln Asp Thr Glu Asn Val Pro Val Thr Leu Ser Glu
        675                 680                 685

Glu Asn Arg Ser Glu Gly Lys Val Gly Phe Gln Ala Tyr Lys Asn Tyr
    690                 695                 700

Phe Arg Ala Gly Ala His Trp Ile Val Phe Ile Phe Leu Ile Leu Leu
705                 710                 715                 720

Asn Thr Ala Ala Gln Val Ala Tyr Val Leu Gln Asp Trp Trp Leu Ser
                725                 730                 735

Tyr Trp Ala Asn Lys Gln Ser Met Leu Asn Val Thr Val Asn Gly Gly
            740                 745                 750

Gly Asn Val Thr Glu Lys Leu Asp Leu Asn Trp Tyr Leu Gly Ile Tyr
        755                 760                 765

Ser Gly Leu Thr Val Ala Thr Val Leu Phe Gly Ile Ala Arg Ser Leu
    770                 775                 780

Leu Val Phe Tyr Val Leu Val Asn Ser Ser Gln Thr Leu His Asn Lys
785                 790                 795                 800

Met Phe Glu Ser Ile Leu Lys Ala Pro Val Leu Phe Phe Asp Arg Asn
                805                 810                 815

Pro Ile Gly Arg Ile Leu Asn Arg Phe Ser Lys Asp Ile Gly His Leu
            820                 825                 830

Asp Asp Leu Leu Pro Leu Thr Phe Leu Asp Phe Ile Gln Thr Leu Leu
        835                 840                 845

Gln Val Val Gly Val Val Ser Val Ala Val Ala Val Ile Pro Trp Ile
    850                 855                 860

Ala Ile Pro Leu Val Pro Leu Gly Ile Ile Phe Ile Phe Leu Arg Arg
865                 870                 875                 880

Tyr Phe Leu Glu Thr Ser Arg Asp Val Lys Arg Leu Glu Ser Thr Thr
                885                 890                 895

Arg Ser Pro Val Phe Ser His Leu Ser Ser Ser Leu Gln Gly Leu Trp
            900                 905                 910

Thr Ile Arg Ala Tyr Lys Ala Glu Glu Arg Cys Gln Glu Leu Phe Asp
        915                 920                 925

Ala His Gln Asp Leu His Ser Glu Ala Trp Phe Leu Phe Leu Thr Thr
    930                 935                 940

Ser Arg Trp Phe Ala Val Arg Leu Asp Ala Ile Cys Ala Met Phe Val
945                 950                 955                 960

Ile Ile Val Ala Phe Gly Ser Leu Ile Leu Ala Lys Thr Leu Asp Ala
                965                 970                 975

Gly Gln Val Gly Leu Ala Leu Ser Tyr Ala Leu Thr Leu Met Gly Met
            980                 985                 990

Phe Gln Trp Cys Val Arg Gln Ser Ala Glu Val Glu Asn Met Met Ile
        995                 1000                1005

Ser Val Glu Arg Val Ile Glu Tyr Thr Asp Leu Glu Lys Glu Ala Pro
    1010                1015                1020

Trp Glu Tyr Gln Lys Arg Pro Pro Pro Ala Trp Pro His Glu Gly Val
1025                1030                1035                1040

Ile Ile Phe Asp Asn Val Asn Phe Met Tyr Ser Pro Gly Gly Pro Leu
                1045                1050                1055

Val Leu Lys His Leu Thr Ala Leu Ile Lys Ser Gln Glu Lys Val Gly
            1060                1065                1070

Ile Val Gly Arg Thr Gly Ala Gly Lys Ser Ser Leu Ile Ser Ala Leu
        1075                1080                1085

Phe Arg Leu Ser Glu Pro Glu Gly Lys Ile Trp Ile Asp Lys Ile Leu
    1090                1095                1100

Thr Thr Glu Ile Gly Leu His Asp Leu Arg Lys Lys Met Ser Ile Ile
1105                1110                1115                1120

Pro Gln Glu Pro Val Leu Phe Thr Gly Thr Met Arg Lys Asn Leu Asp
                1125                1130                1135

Pro Phe Lys Glu His Thr Asp Glu Glu Leu Trp Asn Ala Leu Gln Glu
            1140                1145                1150

Val Gln Leu Lys Glu Thr Ile Glu Asp Leu Pro Gly Lys Met Asp Thr
        1155                1160                1165

Glu Leu Ala Glu Ser Gly Ser Asn Phe Ser Val Gly Gln Arg Gln Leu
    1170                1175                1180

Val Cys Leu Ala Arg Ala Ile Leu Arg Lys Asn Gln Ile Leu Ile Ile
1185                1190                1195                1200

Asp Glu Ala Thr Ala Asn Val Asp Pro Arg Thr Asp Glu Leu Ile Gln
                1205                1210                1215

Lys Lys Ile Arg Glu Lys Phe Ala His Cys Thr Val Leu Thr Ile Ala
            1220                1225                1230

His Arg Leu Asn Thr Ile Ile Asp Ser Asp Lys Ile Met Val Leu Asp
        1235                1240                1245

Ser Gly Arg Leu Lys Glu Tyr Asp Glu Pro Tyr Val Leu Leu Gln Asn
1250                1255                1260

Lys Glu Ser Leu Phe Tyr Lys Met Val Gln Gln Leu Gly Lys Ala Glu
1265                1270                1275                1280

Ala Ala Ala Leu Thr Glu Thr Ala Lys Gln Val Tyr Phe Lys Arg Asn
                1285                1290                1295

Tyr Pro His Ile Gly His Thr Asp His Met Val Thr Asn Thr Ser Asn
            1300                1305                1310

Gly Gln Pro Ser Thr Leu Thr Ile Phe Glu Thr Ala Leu
        1315                1320                1325

<210> 64
<211> 1437
<212> PRT
<213> Homo sapiens

<400> 64
Met Lys Asp Ile Asp Ile Gly Lys Glu Tyr Ile Ile Pro Ser Pro Gly
1               5                   10                  15

Tyr Arg Ser Val Arg Glu Arg Thr Ser Thr Ser Gly Thr His Arg Asp
            20                  25                  30

Arg Glu Asp Ser Lys Phe Arg Arg Thr Arg Pro Leu Glu Cys Gln Asp
        35                  40                  45

Ala Leu Glu Thr Ala Ala Arg Ala Glu Gly Leu Ser Leu Asp Ala Ser
    50                  55                  60

Met His Ser Gln Leu Arg Ile Leu Asp Glu Glu His Pro Lys Gly Lys
65                  70                  75                  80

Tyr His His Gly Leu Ser Ala Leu Lys Pro Ile Arg Thr Thr Ser Lys
                85                  90                  95

His Gln His Pro Val Asp Asn Ala Gly Leu Phe Ser Cys Met Thr Phe
            100                 105                 110

Ser Trp Leu Ser Ser Leu Ala Arg Val Ala His Lys Lys Gly Glu Leu
        115                 120                 125

Ser Met Glu Asp Val Trp Ser Leu Ser Lys His Glu Ser Ser Asp Val

130                 135                 140

Asn Cys Arg Arg Leu Glu Arg Leu Trp Gln Glu Glu Leu Asn Glu Val
145                 150                 155                 160

Gly Pro Asp Ala Ala Ser Leu Arg Arg Val Val Trp Ile Phe Cys Arg
                165                 170                 175

Thr Arg Leu Ile Leu Ser Ile Val Cys Leu Met Ile Thr Gln Leu Ala
            180                 185                 190

Gly Phe Ser Gly Pro Ala Phe Met Val Lys His Leu Leu Glu Tyr Thr
        195                 200                 205

Gln Ala Thr Glu Ser Asn Leu Gln Tyr Ser Leu Leu Leu Val Leu Gly
    210                 215                 220

Leu Leu Leu Thr Glu Ile Val Arg Ser Trp Ser Leu Ala Leu Thr Trp
225                 230                 235                 240

Ala Leu Asn Tyr Arg Thr Gly Val Arg Leu Arg Gly Ala Ile Leu Thr
                245                 250                 255

Met Ala Phe Lys Lys Ile Leu Lys Leu Lys Asn Ile Lys Glu Lys Ser
            260                 265                 270

Leu Gly Glu Leu Ile Asn Ile Cys Ser Asn Asp Gly Gln Arg Met Phe
        275                 280                 285

Glu Ala Ala Ala Val Gly Ser Leu Leu Ala Gly Gly Pro Val Val Ala
    290                 295                 300

Ile Leu Gly Met Ile Tyr Asn Val Ile Ile Leu Gly Pro Thr Gly Phe
305                 310                 315                 320

Leu Gly Ser Ala Val Phe Ile Leu Phe Tyr Pro Ala Met Met Phe Ala
                325                 330                 335

Ser Arg Leu Thr Ala Tyr Phe Arg Arg Lys Cys Val Ala Ala Thr Asp
            340                 345                 350

Glu Arg Val Gln Lys Met Asn Glu Val Leu Thr Tyr Ile Lys Phe Ile
        355                 360                 365

Lys Met Tyr Ala Trp Val Lys Ala Phe Ser Gln Ser Val Gln Lys Ile
    370                 375                 380

Arg Glu Glu Glu Arg Arg Ile Leu Glu Lys Ala Gly Tyr Phe Gln Gly

385                 390                 395                 400

Ile Thr Val Gly Val Ala Pro Ile Val Val Val Ile Ala Ser Val Val
                405                 410                 415

Thr Phe Ser Val His Met Thr Leu Gly Phe Asp Leu Thr Ala Ala Gln
            420                 425                 430

Ala Phe Thr Val Val Thr Val Phe Asn Ser Met Thr Phe Ala Leu Lys
        435                 440                 445

Val Thr Pro Phe Ser Val Lys Ser Leu Ser Glu Ala Ser Val Ala Val
    450                 455                 460

Asp Arg Phe Lys Ser Leu Phe Leu Met Glu Glu Val His Met Ile Lys
465                 470                 475                 480

Asn Lys Pro Ala Ser Pro His Ile Lys Ile Glu Met Lys Asn Ala Thr
                485                 490                 495

Leu Ala Trp Asp Ser Ser His Ser Ser Ile Gln Asn Ser Pro Lys Leu
            500                 505                 510

Thr Pro Lys Met Lys Lys Asp Lys Arg Ala Ser Arg Gly Lys Lys Glu
        515                 520                 525

Lys Val Arg Gln Leu Gln Arg Thr Glu His Gln Ala Val Leu Ala Glu
    530                 535                 540

Gln Lys Gly His Leu Leu Leu Asp Ser Asp Glu Arg Pro Ser Pro Glu
545                 550                 555                 560

Glu Glu Glu Gly Lys His Ile His Leu Gly His Leu Arg Leu Gln Arg
                565                 570                 575

Thr Leu His Ser Ile Asp Leu Glu Ile Gln Glu Gly Lys Leu Val Gly
            580                 585                 590

Ile Cys Gly Ser Val Gly Ser Gly Lys Thr Ser Leu Ile Ser Ala Ile
        595                 600                 605

Leu Gly Gln Met Thr Leu Leu Glu Gly Ser Ile Ala Ile Ser Gly Thr
    610                 615                 620

Phe Ala Tyr Val Ala Gln Gln Ala Trp Ile Leu Asn Ala Thr Leu Arg
625                 630                 635                 640

Asp Asn Ile Leu Phe Gly Lys Glu Tyr Asp Glu Glu Arg Tyr Asn Ser

                645                 650                 655

Val Leu Asn Ser Cys Cys Leu Arg Pro Asp Leu Ala Ile Leu Pro Ser
            660                 665                 670

Ser Asp Leu Thr Glu Ile Gly Glu Arg Gly Ala Asn Leu Ser Gly Gly
        675                 680                 685

Gln Arg Gln Arg Ile Ser Leu Ala Arg Ala Leu Tyr Ser Asp Arg Ser
    690                 695                 700

Ile Tyr Ile Leu Asp Asp Pro Leu Ser Ala Leu Asp Ala His Val Gly
705                 710                 715                 720

Asn His Ile Phe Asn Ser Ala Ile Arg Lys His Leu Lys Ser Lys Thr
                725                 730                 735

Val Leu Phe Val Thr His Gln Leu Gln Tyr Leu Val Asp Cys Asp Glu
            740                 745                 750

Val Ile Phe Met Lys Glu Gly Cys Ile Thr Glu Arg Gly Thr His Glu
        755                 760                 765

Glu Leu Met Asn Leu Asn Gly Asp Tyr Ala Thr Ile Phe Asn Asn Leu
    770                 775                 780

Leu Leu Gly Glu Thr Pro Pro Val Glu Ile Asn Ser Lys Lys Glu Thr
785                 790                 795                 800

Ser Gly Ser Gln Lys Lys Ser Gln Asp Lys Gly Pro Lys Thr Gly Ser
                805                 810                 815

Val Lys Lys Glu Lys Ala Val Lys Pro Glu Glu Gly Gln Leu Val Gln
            820                 825                 830

Leu Glu Glu Lys Gly Gln Gly Ser Val Pro Trp Ser Val Tyr Gly Val
        835                 840                 845

Tyr Ile Gln Ala Ala Gly Gly Pro Leu Ala Phe Leu Val Ile Met Ala
    850                 855                 860

Leu Phe Met Leu Asn Val Gly Ser Thr Ala Phe Ser Thr Trp Trp Leu
865                 870                 875                 880

Ser Tyr Trp Ile Lys Gln Gly Ser Gly Asn Thr Thr Val Thr Arg Gly
                885                 890                 895

Asn Glu Thr Ser Val Ser Asp Ser Met Lys Asp Asn Pro His Met Gln

            900                 905                 910

Tyr Tyr Ala Ser Ile Tyr Ala Leu Ser Met Ala Val Met Leu Ile Leu
        915                 920                 925

Lys Ala Ile Arg Gly Val Val Phe Val Lys Gly Thr Leu Arg Ala Ser
    930                 935                 940

Ser Arg Leu His Asp Glu Leu Phe Arg Arg Ile Leu Arg Ser Pro Met
945                 950                 955                 960

Lys Phe Phe Asp Thr Thr Pro Thr Gly Arg Ile Leu Asn Arg Phe Ser
                965                 970                 975

Lys Asp Met Asp Glu Val Asp Val Arg Leu Pro Phe Gln Ala Glu Met
            980                 985                 990

Phe Ile Gln Asn Val Ile Leu Val Phe Phe Cys Val Gly Met Ile Ala
        995                 1000                1005

Gly Val Phe Pro Trp Phe Leu Val Ala Val Gly Pro Leu Val Ile Leu
    1010                1015                1020

Phe Ser Val Leu His Ile Val Ser Arg Val Leu Ile Arg Glu Leu Lys
1025                1030                1035                1040

Arg Leu Asp Asn Ile Thr Gln Ser Pro Phe Leu Ser His Ile Thr Ser
                1045                1050                1055

Ser Ile Gln Gly Leu Ala Thr Ile His Ala Tyr Asn Lys Gly Gln Glu
            1060                1065                1070

Phe Leu His Arg Tyr Gln Glu Leu Leu Asp Asp Asn Gln Ala Pro Phe
        1075                1080                1085

Phe Leu Phe Thr Cys Ala Met Arg Trp Leu Ala Val Arg Leu Asp Leu
    1090                1095                1100

Ile Ser Ile Ala Leu Ile Thr Thr Thr Gly Leu Met Ile Val Leu Met
1105                1110                1115                1120

His Gly Gln Ile Pro Pro Ala Tyr Ala Gly Leu Ala Ile Ser Tyr Ala
                1125                1130                1135

Val Gln Leu Thr Gly Leu Phe Gln Phe Thr Val Arg Leu Ala Ser Glu
            1140                1145                1150

Thr Glu Ala Arg Phe Thr Ser Val Glu Arg Ile Asn His Tyr Ile Lys

1155                 1160                 1165

Thr Leu Ser Leu Glu Ala Pro Ala Arg Ile Lys Asn Lys Ala Pro Ser
    1170                 1175                 1180

Pro Asp Trp Pro Gln Glu Gly Glu Val Thr Phe Glu Asn Ala Glu Met
1185                 1190                 1195                 1200

Arg Tyr Arg Glu Asn Leu Pro Leu Val Leu Lys Lys Val Ser Phe Thr
                 1205                 1210                 1215

Ile Lys Pro Lys Glu Lys Ile Gly Ile Val Gly Arg Thr Gly Ser Gly
             1220                 1225                 1230

Lys Ser Ser Leu Gly Met Ala Leu Phe Arg Leu Val Glu Leu Ser Gly
        1235                 1240                 1245

Gly Cys Ile Lys Ile Asp Gly Val Arg Ile Ser Asp Ile Gly Leu Ala
    1250                 1255                 1260

Asp Leu Arg Ser Lys Leu Ser Ile Ile Pro Gln Glu Pro Val Leu Phe
1265                 1270                 1275                 1280

Ser Gly Thr Val Arg Ser Asn Leu Asp Pro Phe Asn Gln Tyr Thr Glu
                 1285                 1290                 1295

Asp Gln Ile Trp Asp Ala Leu Glu Arg Thr His Met Lys Glu Cys Ile
             1300                 1305                 1310

Ala Gln Leu Pro Leu Lys Leu Glu Ser Glu Val Met Glu Asn Gly Asp
        1315                 1320                 1325

Asn Phe Ser Val Gly Glu Arg Gln Leu Leu Cys Ile Ala Arg Ala Leu
    1330                 1335                 1340

Leu Arg His Cys Lys Ile Leu Ile Leu Asp Glu Ala Thr Ala Ala Met
1345                 1350                 1355                 1360

Asp Thr Glu Thr Asp Leu Leu Ile Gln Glu Thr Ile Arg Glu Ala Phe
                 1365                 1370                 1375

Ala Asp Cys Thr Met Leu Thr Ile Ala His Arg Leu His Thr Val Leu
             1380                 1385                 1390

Gly Ser Asp Arg Ile Met Val Leu Ala Gln Gly Gln Val Val Glu Phe
        1395                 1400                 1405

Asp Thr Pro Ser Val Leu Leu Ser Asn Asp Ser Ser Arg Phe Tyr Ala

1410                1415                1420

Met Phe Ala Ala Ala Glu Asn Lys Val Ala Val Lys Gly
1425                1430                1435

<210> 65
<211> 1503
<212> PRT
<213> Homo sapiens

<400> 65
Met Ala Ala Pro Ala Glu Pro Cys Ala Gly Gln Gly Val Trp Asn Gln
1               5                   10                  15

Thr Glu Pro Glu Pro Ala Ala Thr Ser Leu Leu Ser Leu Cys Phe Leu
            20                  25                  30

Arg Thr Ala Gly Val Trp Val Pro Pro Met Tyr Leu Trp Val Leu Gly
        35                  40                  45

Pro Ile Tyr Leu Leu Phe Ile His His His Gly Arg Gly Tyr Leu Trp
    50                  55                  60

Met Ser Pro Leu Phe Lys Ala Lys Met Val Leu Gly Phe Ala Leu Ile
65                  70                  75                  80

Val Leu Cys Thr Ser Ser Val Ala Val Ala Leu Trp Lys Ile Gln Gln
                85                  90                  95

Gly Thr Pro Glu Ala Pro Glu Phe Leu Ile His Pro Thr Val Trp Leu
            100                 105                 110

Thr Thr Met Ser Phe Ala Val Phe Leu Ile His Thr Glu Arg Lys Lys
        115                 120                 125

Gly Val Gln Ser Ser Gly Val Leu Phe Gly Tyr Trp Leu Leu Cys Phe
    130                 135                 140

Val Leu Pro Ala Thr Asn Ala Ala Gln Gln Ala Ser Gly Ala Gly Phe
145                 150                 155                 160

Gln Ser Asp Pro Val Arg His Leu Ser Thr Tyr Leu Cys Leu Ser Leu
                165                 170                 175

Val Val Ala Gln Phe Val Leu Ser Cys Leu Ala Asp Gln Pro Pro Phe
            180                 185                 190

Phe Pro Glu Asp Pro Gln Gln Ser Asn Pro Cys Pro Glu Thr Gly Ala
        195                 200                 205

Ala Phe Pro Ser Lys Ala Thr Phe Trp Trp Val Ser Gly Leu Val Trp
    210                 215                 220

Arg Gly Tyr Arg Arg Pro Leu Arg Pro Lys Asp Leu Trp Ser Leu Gly
225                 230                 235                 240

Arg Glu Asn Ser Ser Glu Glu Leu Val Ser Arg Leu Glu Lys Glu Trp
                245                 250                 255

Met Arg Asn Arg Ser Ala Ala Arg Arg His Asn Lys Ala Ile Ala Phe
            260                 265                 270

Lys Arg Lys Gly Gly Ser Gly Met Lys Ala Pro Glu Thr Glu Pro Phe
        275                 280                 285

Leu Arg Gln Glu Gly Ser Gln Trp Arg Pro Leu Leu Lys Ala Ile Trp
    290                 295                 300

Gln Val Phe His Ser Thr Phe Leu Leu Gly Thr Leu Ser Leu Ile Ile
305                 310                 315                 320

Ser Asp Val Phe Arg Phe Thr Val Pro Lys Leu Leu Ser Leu Phe Leu
                325                 330                 335

Glu Phe Ile Gly Asp Pro Lys Pro Pro Ala Trp Lys Gly Tyr Leu Leu
            340                 345                 350

Ala Val Leu Met Phe Leu Ser Ala Cys Leu Gln Thr Leu Phe Glu Gln
        355                 360                 365

Gln Asn Met Tyr Arg Leu Lys Val Leu Gln Met Arg Leu Arg Ser Ala
    370                 375                 380

Ile Thr Gly Leu Val Tyr Arg Lys Val Leu Ala Leu Ser Ser Gly Ser
385                 390                 395                 400

Arg Lys Ala Ser Ala Val Gly Asp Val Val Asn Leu Val Ser Val Asp
                405                 410                 415

Val Gln Arg Leu Thr Glu Ser Val Leu Tyr Leu Asn Gly Leu Trp Leu
            420                 425                 430

Pro Leu Val Trp Ile Val Val Cys Phe Val Tyr Leu Trp Gln Leu Leu
        435                 440                 445

Gly Pro Ser Ala Leu Thr Ala Ile Ala Val Phe Leu Ser Leu Leu Pro
    450                 455                 460

Leu Asn Phe Phe Ile Ser Lys Lys Arg Asn His His Gln Glu Glu Gln
465                 470                 475                 480

Met Arg Gln Lys Asp Ser Arg Ala Arg Leu Thr Ser Ser Ile Leu Arg
                485                 490                 495

Asn Ser Lys Thr Ile Lys Phe His Gly Trp Glu Gly Ala Phe Leu Asp
            500                 505                 510

Arg Val Leu Gly Ile Arg Gly Gln Glu Leu Gly Ala Leu Arg Thr Ser
        515                 520                 525

Gly Leu Leu Phe Ser Val Ser Leu Val Ser Phe Gln Val Ser Thr Phe
    530                 535                 540

Leu Val Ala Leu Val Val Phe Ala Val His Thr Leu Val Ala Glu Asn
545                 550                 555                 560

Ala Met Asn Ala Glu Lys Ala Phe Val Thr Leu Thr Val Leu Asn Ile
                565                 570                 575

Leu Asn Lys Ala Gln Ala Phe Leu Pro Phe Ser Ile His Ser Leu Val
            580                 585                 590

Gln Ala Arg Val Ser Phe Asp Arg Leu Val Thr Phe Leu Cys Leu Glu
        595                 600                 605

Glu Val Asp Pro Gly Val Val Asp Ser Ser Ser Ser Gly Ser Ala Ala
    610                 615                 620

Gly Lys Asp Cys Ile Thr Ile His Ser Ala Thr Phe Ala Trp Ser Gln
625                 630                 635                 640

Glu Ser Pro Pro Cys Leu His Arg Ile Asn Leu Thr Val Pro Gln Gly
                645                 650                 655

Cys Leu Leu Ala Val Val Gly Pro Val Gly Ala Gly Lys Ser Ser Leu
            660                 665                 670

Leu Ser Ala Leu Leu Gly Glu Leu Ser Lys Val Glu Gly Phe Val Ser
        675                 680                 685

Ile Glu Gly Ala Val Ala Tyr Val Pro Gln Glu Ala Trp Val Gln Asn
    690                 695                 700

Thr Ser Val Val Glu Asn Val Cys Phe Gly Gln Glu Leu Asp Pro Pro
705                 710                 715                 720

Trp Leu Glu Arg Val Leu Glu Ala Cys Ala Leu Gln Pro Asp Val Asp
                725                 730                 735

Ser Phe Pro Glu Gly Ile His Thr Ser Ile Gly Glu Gln Gly Met Asn
            740                 745                 750

Leu Ser Gly Gly Gln Lys Gln Arg Leu Ser Leu Ala Arg Ala Val Tyr
        755                 760                 765

Arg Lys Ala Ala Val Tyr Leu Leu Asp Asp Pro Leu Ala Ala Leu Asp
    770                 775                 780

Ala His Val Gly Gln His Val Phe Asn Gln Val Ile Gly Pro Gly Gly
785                 790                 795                 800

Leu Leu Gln Gly Thr Thr Arg Ile Leu Val Thr His Ala Leu His Ile
                805                 810                 815

Leu Pro Gln Ala Asp Trp Ile Ile Val Leu Ala Asn Gly Ala Ile Ala
            820                 825                 830

Glu Met Gly Ser Tyr Gln Glu Leu Leu Gln Arg Lys Gly Ala Leu Val
        835                 840                 845

Cys Leu Leu Asp Gln Ala Arg Gln Pro Gly Asp Arg Gly Glu Gly Glu
    850                 855                 860

Thr Glu Pro Gly Thr Ser Thr Lys Asp Pro Arg Gly Thr Ser Ala Gly
865                 870                 875                 880

Arg Arg Pro Glu Leu Arg Arg Glu Arg Ser Ile Lys Ser Val Pro Glu
                885                 890                 895

Lys Asp Arg Thr Thr Ser Glu Ala Gln Thr Glu Val Pro Leu Asp Asp
            900                 905                 910

Pro Asp Arg Ala Gly Trp Pro Ala Gly Lys Asp Ser Ile Gln Tyr Gly
        915                 920                 925

Arg Val Lys Ala Thr Val His Leu Ala Tyr Leu Arg Ala Val Gly Thr
    930                 935                 940

Pro Leu Cys Leu Tyr Ala Leu Phe Leu Phe Leu Cys Gln Gln Val Ala
945                 950                 955                 960

Ser Phe Cys Arg Gly Tyr Trp Leu Ser Leu Trp Ala Asp Asp Pro Ala
                965                 970                 975

Val Gly Gly Gln Gln Thr Gln Ala Ala Leu Arg Gly Gly Ile Phe Gly
            980                 985                 990

Leu Leu Gly Cys Leu Gln Ala Ile Gly Leu Phe Ala Ser Met Ala Ala
        995                 1000                1005

Val Leu Leu Gly Gly Ala Arg Ala Ser Arg Leu Leu Phe Gln Arg Leu
    1010                1015                1020

Leu Trp Asp Val Val Arg Ser Pro Ile Ser Phe Phe Glu Arg Thr Pro
1025                1030                1035                1040

Ile Gly His Leu Leu Asn Arg Phe Ser Lys Glu Thr Asp Thr Val Asp
                1045                1050                1055

Val Asp Ile Pro Asp Lys Leu Arg Ser Leu Leu Met Tyr Ala Phe Gly
            1060                1065                1070

Leu Leu Glu Val Ser Leu Val Val Ala Val Ala Thr Pro Leu Ala Thr
        1075                1080                1085

Val Ala Ile Leu Pro Leu Phe Leu Leu Tyr Ala Gly Phe Gln Ser Leu
    1090                1095                1100

Tyr Val Val Ser Ser Cys Gln Leu Arg Arg Leu Glu Ser Ala Ser Tyr
1105                1110                1115                1120

Ser Ser Val Cys Ser His Met Ala Glu Thr Phe Gln Gly Ser Thr Val
                1125                1130                1135

Val Arg Ala Phe Arg Thr Gln Ala Pro Phe Val Ala Gln Asn Asn Ala
            1140                1145                1150

Arg Val Asp Glu Ser Gln Arg Ile Ser Phe Pro Arg Leu Val Ala Asp
        1155                1160                1165

Arg Trp Leu Ala Ala Asn Val Glu Leu Leu Gly Asn Gly Leu Val Phe
    1170                1175                1180

Ala Ala Ala Thr Cys Ala Val Leu Ser Lys Ala His Leu Ser Ala Gly
1185                1190                1195                1200

Leu Val Gly Phe Ser Val Ser Ala Ala Leu Gln Val Thr Gln Thr Leu
                1205                1210                1215

Gln Trp Val Val Arg Asn Trp Thr Asp Leu Glu Asn Ser Ile Val Ser
            1220                1225                1230

Val Glu Arg Met Gln Asp Tyr Ala Trp Thr Pro Lys Glu Ala Pro Trp
        1235                1240                1245

Arg Leu Pro Thr Cys Ala Ala Gln Pro Pro Trp Pro Gln Gly Gly Gln
    1250                1255                1260

Ile Glu Phe Arg Asp Phe Gly Leu Arg Tyr Arg Pro Glu Leu Pro Leu
1265                1270                1275                1280

Ala Val Gln Gly Val Ser Phe Lys Ile His Ala Gly Glu Lys Val Gly
                1285                1290                1295

Ile Val Gly Arg Thr Gly Ala Gly Lys Ser Ser Leu Ala Ser Gly Leu
            1300                1305                1310

Leu Arg Leu Gln Glu Ala Ala Glu Gly Gly Ile Trp Ile Asp Gly Val
        1315                1320                1325

Pro Ile Ala His Val Gly Leu His Thr Leu Arg Ser Arg Ile Ser Ile
    1330                1335                1340

Ile Pro Gln Asp Pro Ile Leu Phe Pro Gly Ser Leu Arg Met Asn Leu
1345                1350                1355                1360

Asp Leu Leu Gln Glu His Ser Asp Glu Ala Ile Trp Ala Ala Leu Glu
                1365                1370                1375

Thr Val Gln Leu Lys Ala Leu Val Ala Ser Leu Pro Gly Gln Leu Gln
            1380                1385                1390

Tyr Lys Cys Ala Asp Arg Gly Glu Asp Leu Ser Val Gly Gln Lys Gln
        1395                1400                1405

Leu Leu Cys Leu Ala Arg Ala Leu Leu Arg Lys Thr Gln Ile Leu Ile
    1410                1415                1420

Leu Asp Glu Ala Thr Ala Ala Val Asp Pro Gly Thr Glu Leu Gln Met
1425                1430                1435                1440

Gln Ala Met Leu Gly Ser Trp Phe Ala Gln Cys Thr Val Leu Leu Ile
                1445                1450                1455

Ala His Arg Leu Arg Ser Val Met Asp Cys Ala Arg Val Leu Val Met
            1460                1465                1470

Asp Lys Gly Gln Val Ala Glu Ser Gly Ser Pro Ala Gln Leu Leu Ala
        1475                1480                1485

Gln Lys Gly Leu Phe Tyr Arg Leu Ala Gln Glu Ser Gly Leu Val
    1490                1495                1500

<210> 66
<211> 1480
<212> PRT
<213> Homo sapiens

<400> 66
Met Gln Arg Ser Pro Leu Glu Lys Ala Ser Val Val Ser Lys Leu Phe
  1               5                  10                  15

Phe Ser Trp Thr Arg Pro Ile Leu Arg Lys Gly Tyr Arg Gln Arg Leu
             20                  25                  30

Glu Leu Ser Asp Ile Tyr Gln Ile Pro Ser Val Asp Ser Ala Asp Asn
         35                  40                  45

Leu Ser Glu Lys Leu Glu Arg Glu Trp Asp Arg Glu Leu Ala Ser Lys
     50                  55                  60

Lys Asn Pro Lys Leu Ile Asn Ala Leu Arg Arg Cys Phe Phe Trp Arg
 65                  70                  75                  80

Phe Met Phe Tyr Gly Ile Phe Leu Tyr Leu Gly Glu Val Thr Lys Ala
                 85                  90                  95

Val Gln Pro Leu Leu Leu Gly Arg Ile Ile Ala Ser Tyr Asp Pro Asp
            100                 105                 110

Asn Lys Glu Glu Arg Ser Ile Ala Ile Tyr Leu Gly Ile Gly Leu Cys
        115                 120                 125

Leu Leu Phe Ile Val Arg Thr Leu Leu Leu His Pro Ala Ile Phe Gly
    130                 135                 140

Leu His His Ile Gly Met Gln Met Arg Ile Ala Met Phe Ser Leu Ile
145                 150                 155                 160

Tyr Lys Lys Thr Leu Lys Leu Ser Ser Arg Val Leu Asp Lys Ile Ser
                165                 170                 175

Ile Gly Gln Leu Val Ser Leu Leu Ser Asn Asn Leu Asn Lys Phe Asp
            180                 185                 190

Glu Gly Leu Ala Leu Ala His Phe Val Trp Ile Ala Pro Leu Gln Val
        195                 200                 205

Ala Leu Leu Met Gly Leu Ile Trp Glu Leu Leu Gln Ala Ser Ala Phe
    210                 215                 220

Cys Gly Leu Gly Phe Leu Ile Val Leu Ala Leu Phe Gln Ala Gly Leu
225                 230                 235                 240

Gly Arg Met Met Met Lys Tyr Arg Asp Gln Arg Ala Gly Lys Ile Ser
                245                 250                 255

Glu Arg Leu Val Ile Thr Ser Glu Met Ile Glu Asn Ile Gln Ser Val
            260                 265                 270

Lys Ala Tyr Cys Trp Glu Glu Ala Met Glu Lys Met Ile Glu Asn Leu
        275                 280                 285

Arg Gln Thr Glu Leu Lys Leu Thr Arg Lys Ala Ala Tyr Val Arg Tyr
    290                 295                 300

Phe Asn Ser Ser Ala Phe Phe Phe Ser Gly Phe Phe Val Val Phe Leu
305                 310                 315                 320

Ser Val Leu Pro Tyr Ala Leu Ile Lys Gly Ile Ile Leu Arg Lys Ile
                325                 330                 335

Phe Thr Thr Ile Ser Phe Cys Ile Val Leu Arg Met Ala Val Thr Arg
            340                 345                 350

Gln Phe Pro Trp Ala Val Gln Thr Trp Tyr Asp Ser Leu Gly Ala Ile
        355                 360                 365

Asn Lys Ile Gln Asp Phe Leu Gln Lys Gln Glu Tyr Lys Thr Leu Glu
    370                 375                 380

Tyr Asn Leu Thr Thr Thr Glu Val Val Met Glu Asn Val Thr Ala Phe
385                 390                 395                 400

Trp Glu Glu Gly Phe Gly Glu Leu Phe Glu Lys Ala Lys Gln Asn Asn
                405                 410                 415

Asn Asn Arg Lys Thr Ser Asn Gly Asp Asp Ser Leu Phe Phe Ser Asn
            420                 425                 430

Phe Ser Leu Leu Gly Thr Pro Val Leu Lys Asp Ile Asn Phe Lys Ile
      435                 440                 445

Glu Arg Gly Gln Leu Leu Ala Val Ala Gly Ser Thr Gly Ala Gly Lys
    450                 455                 460

Thr Ser Leu Leu Met Met Ile Met Gly Glu Leu Glu Pro Ser Glu Gly
465                 470                 475                 480

Lys Ile Lys His Ser Gly Arg Ile Ser Phe Cys Ser Gln Phe Ser Trp
                485                 490                 495

Ile Met Pro Gly Thr Ile Lys Glu Asn Ile Ile Phe Gly Val Ser Tyr
            500                 505                 510

Asp Glu Tyr Arg Tyr Arg Ser Val Ile Lys Ala Cys Gln Leu Glu Glu
      515                 520                 525

Asp Ile Ser Lys Phe Ala Glu Lys Asp Asn Ile Val Leu Gly Glu Gly
    530                 535                 540

Gly Ile Thr Leu Ser Gly Gly Gln Arg Ala Arg Ile Ser Leu Ala Arg
545                 550                 555                 560

Ala Val Tyr Lys Asp Ala Asp Leu Tyr Leu Leu Asp Ser Pro Phe Gly
                565                 570                 575

Tyr Leu Asp Val Leu Thr Glu Lys Glu Ile Phe Glu Ser Cys Val Cys
            580                 585                 590

Lys Leu Met Ala Asn Lys Thr Arg Ile Leu Val Thr Ser Lys Met Glu
      595                 600                 605

His Leu Lys Lys Ala Asp Lys Ile Leu Ile Leu His Glu Gly Ser Ser
    610                 615                 620

Tyr Phe Tyr Gly Thr Phe Ser Glu Leu Gln Asn Leu Gln Pro Asp Phe
625                 630                 635                 640

Ser Ser Lys Leu Met Gly Cys Asp Ser Phe Asp Gln Phe Ser Ala Glu
                645                 650                 655

Arg Arg Asn Ser Ile Leu Thr Glu Thr Leu His Arg Phe Ser Leu Glu
            660                 665                 670

Gly Asp Ala Pro Val Ser Trp Thr Glu Thr Lys Lys Gln Ser Phe Lys
      675                 680                 685

Gln Thr Gly Glu Phe Gly Glu Lys Arg Lys Asn Ser Ile Leu Asn Pro
    690                 695                 700

Ile Asn Ser Ile Arg Lys Phe Ser Ile Val Gln Lys Thr Pro Leu Gln
705                 710                 715                 720

Met Asn Gly Ile Glu Glu Asp Ser Asp Glu Pro Leu Glu Arg Arg Leu
                725                 730                 735

Ser Leu Val Pro Asp Ser Glu Gln Gly Glu Ala Ile Leu Pro Arg Ile
            740                 745                 750

Ser Val Ile Ser Thr Gly Pro Thr Leu Gln Ala Arg Arg Arg Gln Ser
        755                 760                 765

Val Leu Asn Leu Met Thr His Ser Val Asn Gln Gly Gln Asn Ile His
    770                 775                 780

Arg Lys Thr Thr Ala Ser Thr Arg Lys Val Ser Leu Ala Pro Gln Ala
785                 790                 795                 800

Asn Leu Thr Glu Leu Asp Ile Tyr Ser Arg Arg Leu Ser Gln Glu Thr
                805                 810                 815

Gly Leu Glu Ile Ser Glu Glu Ile Asn Glu Glu Asp Leu Lys Glu Cys
            820                 825                 830

Phe Phe Asp Asp Met Glu Ser Ile Pro Ala Val Thr Thr Trp Asn Thr
        835                 840                 845

Tyr Leu Arg Tyr Ile Thr Val His Lys Ser Leu Ile Phe Val Leu Ile
    850                 855                 860

Trp Cys Leu Val Ile Phe Leu Ala Glu Val Ala Ala Ser Leu Val Val
865                 870                 875                 880

Leu Trp Leu Leu Gly Asn Thr Pro Leu Gln Asp Lys Gly Asn Ser Thr
                885                 890                 895

His Ser Arg Asn Asn Ser Tyr Ala Val Ile Ile Thr Ser Thr Ser Ser
            900                 905                 910

Tyr Tyr Val Phe Tyr Ile Tyr Val Gly Val Ala Asp Thr Leu Leu Ala
        915                 920                 925

Met Gly Phe Phe Arg Gly Leu Pro Leu Val His Thr Leu Ile Thr Val
    930                 935                 940

Ser Lys Ile Leu His His Lys Met Leu His Ser Val Leu Gln Ala Pro
945                 950                 955                 960

Met Ser Thr Leu Asn Thr Leu Lys Ala Gly Gly Ile Leu Asn Arg Phe
                965                 970                 975

Ser Lys Asp Ile Ala Ile Leu Asp Asp Leu Leu Pro Leu Thr Ile Phe
            980                 985                 990

Asp Phe Ile Gln Leu Leu Leu Ile Val Ile Gly Ala Ile Ala Val Val
        995                 1000                1005

Ala Val Leu Gln Pro Tyr Ile Phe Val Ala Thr Val Pro Val Ile Val
    1010                1015                1020

Ala Phe Ile Met Leu Arg Ala Tyr Phe Leu Gln Thr Ser Gln Gln Leu
1025                1030                1035                1040

Lys Gln Leu Glu Ser Glu Gly Arg Ser Pro Ile Phe Thr His Leu Val
                1045                1050                1055

Thr Ser Leu Lys Gly Leu Trp Thr Leu Arg Ala Phe Gly Arg Gln Pro
            1060                1065                1070

Tyr Phe Glu Thr Leu Phe His Lys Ala Leu Asn Leu His Thr Ala Asn
        1075                1080                1085

Trp Phe Leu Tyr Leu Ser Thr Leu Arg Trp Phe Gln Met Arg Ile Glu
    1090                1095                1100

Met Ile Phe Val Ile Phe Phe Ile Ala Val Thr Phe Ile Ser Ile Leu
1105                1110                1115                1120

Thr Thr Gly Glu Gly Glu Gly Arg Val Gly Ile Ile Leu Thr Leu Ala
                1125                1130                1135

Met Asn Ile Met Ser Thr Leu Gln Trp Ala Val Asn Ser Ser Ile Asp
            1140                1145                1150

Val Asp Ser Leu Met Arg Ser Val Ser Arg Val Phe Lys Phe Ile Asp
        1155                1160                1165

Met Pro Thr Glu Gly Lys Pro Thr Lys Ser Thr Lys Pro Tyr Lys Asn
    1170                1175                1180

Gly Gln Leu Ser Lys Val Met Ile Ile Glu Asn Ser His Val Lys Lys
1185                1190                1195                1200

Asp Asp Ile Trp Pro Ser Gly Gly Gln Met Thr Val Lys Asp Leu Thr
                1205                1210                1215

Ala Lys Tyr Thr Glu Gly Gly Asn Ala Ile Leu Glu Asn Ile Ser Phe
            1220                1225                1230

Ser Ile Ser Pro Gly Gln Arg Val Gly Leu Leu Gly Arg Thr Gly Ser
        1235                1240                1245

Gly Lys Ser Thr Leu Leu Ser Ala Phe Leu Arg Leu Leu Asn Thr Glu
    1250                1255                1260

Gly Glu Ile Gln Ile Asp Gly Val Ser Trp Asp Ser Ile Thr Leu Gln
1265                1270                1275                1280

Gln Trp Arg Lys Ala Phe Gly Val Ile Pro Gln Lys Val Phe Ile Phe
                1285                1290                1295

Ser Gly Thr Phe Arg Lys Asn Leu Asp Pro Tyr Glu Gln Trp Ser Asp
            1300                1305                1310

Gln Glu Ile Trp Lys Val Ala Asp Glu Val Gly Leu Arg Ser Val Ile
        1315                1320                1325

Glu Gln Phe Pro Gly Lys Leu Asp Phe Val Leu Val Asp Gly Gly Cys
    1330                1335                1340

Val Leu Ser His Gly His Lys Gln Leu Met Cys Leu Ala Arg Ser Val
1345                1350                1355                1360

Leu Ser Lys Ala Lys Ile Leu Leu Leu Asp Glu Pro Ser Ala His Leu
                1365                1370                1375

Asp Pro Val Thr Tyr Gln Ile Ile Arg Arg Thr Leu Lys Gln Ala Phe
            1380                1385                1390

Ala Asp Cys Thr Val Ile Leu Cys Glu His Arg Ile Glu Ala Met Leu
        1395                1400                1405

Glu Cys Gln Gln Phe Leu Val Ile Glu Glu Asn Lys Val Arg Gln Tyr
    1410                1415                1420

Asp Ser Ile Gln Lys Leu Leu Asn Glu Arg Ser Leu Phe Arg Gln Ala
1425                1430                1435                1440

Ile Ser Pro Ser Asp Arg Val Lys Leu Phe Pro His Arg Asn Ser Ser
                1445                1450                1455

Lys Cys Lys Ser Lys Pro Gln Ile Ala Ala Leu Lys Glu Glu Thr Glu
            1460                1465                1470

Glu Glu Val Gln Asp Thr Arg Leu
        1475                1480

<210> 67
<211> 1581
<212> PRT
<213> Homo sapiens

<400> 67
Met Pro Leu Ala Phe Cys Gly Ser Glu Asn His Ser Ala Ala Tyr Arg
1               5                   10                  15

Val Asp Gln Gly Val Leu Asn Asn Gly Cys Phe Val Asp Ala Leu Asn
            20                  25                  30

Val Val Pro His Val Phe Leu Leu Phe Ile Thr Phe Pro Ile Leu Phe
        35                  40                  45

Ile Gly Trp Gly Ser Gln Ser Ser Lys Val His Ile His His Ser Thr
    50                  55                  60

Trp Leu His Phe Pro Gly His Asn Leu Arg Trp Ile Leu Thr Phe Met
65                  70                  75                  80

Leu Leu Phe Val Leu Val Cys Glu Ile Ala Glu Gly Ile Leu Ser Asp
                85                  90                  95

Gly Val Thr Glu Ser His His Leu His Leu Tyr Met Pro Ala Gly Met
            100                 105                 110

Ala Phe Met Ala Ala Val Thr Ser Val Val Tyr Tyr His Asn Ile Glu
        115                 120                 125

Thr Ser Asn Phe Pro Lys Leu Leu Ile Ala Leu Leu Val Tyr Trp Thr
    130                 135                 140

Leu Ala Phe Ile Thr Lys Thr Ile Lys Phe Val Lys Leu Leu Asp His
145                 150                 155                 160

Ala Ile Gly Phe Ser Gln Leu Arg Phe Cys Leu Thr Gly Leu Leu Val
                165                 170                 175

Ile Leu Tyr Gly Met Leu Leu Leu Val Glu Val Asn Val Ile Arg Val

180                 185                 190

Arg Arg Tyr Ile Phe Phe Lys Thr Pro Arg Glu Val Lys Pro Pro Glu
        195                 200                 205

Asp Leu Gln Asp Leu Gly Val Arg Phe Leu Gln Pro Phe Val Asn Leu
    210                 215                 220

Pro Ser Lys Gly Thr Tyr Trp Trp Met Asn Ala Phe Ile Lys Thr Ala
225                 230                 235                 240

His Lys Lys Pro Ile Asp Leu Arg Ala Ile Gly Lys Leu Pro Ile Val
                245                 250                 255

Met Arg Ala Leu Thr Asn Tyr Gln Arg Leu Cys Glu Ala Phe Asp Ala
            260                 265                 270

Gln Val Arg Lys Asp Ile Gln Gly Thr Gln Gly Ala Arg Ala Ile Trp
        275                 280                 285

Gln Ala Leu Ser His Ala Phe Gly Arg Arg Leu Val Leu Ser Ser Thr
    290                 295                 300

Phe Arg Ile Leu Ala Asp Leu Leu Gly Phe Ala Gly Pro Leu Cys Ile
305                 310                 315                 320

Phe Gly Ile Val Asp His Leu Gly Lys Glu Asn Asp Val Phe Gln Pro
                325                 330                 335

Lys Thr Gln Phe Leu Gly Val Tyr Phe Val Ser Ser Gln Glu Phe Leu
            340                 345                 350

Ala Asn Ala Tyr Val Leu Ala Val Leu Leu Phe Leu Ala Leu Leu Leu
        355                 360                 365

Gln Arg Thr Phe Leu Gln Ala Ser Tyr Tyr Val Ala Ile Glu Thr Gly
    370                 375                 380

Ile Asn Leu Arg Gly Ala Ile Gln Thr Lys Ile Tyr Asn Lys Ile Met
385                 390                 395                 400

His Leu Ser Thr Ser Asn Leu Ser Met Gly Glu Met Thr Ala Gly Gln
                405                 410                 415

Ile Cys Asn Leu Val Ala Ile Asp Thr Asn Gln Leu Met Trp Phe Phe
            420                 425                 430

Phe Leu Cys Pro Asn Leu Trp Ala Met Pro Val Gln Ile Ile Val Gly

435                 440                 445

Val Ile Leu Leu Tyr Tyr Ile Leu Gly Val Ser Ala Leu Ile Gly Ala
    450                 455                 460

Ala Val Ile Ile Leu Leu Ala Pro Val Gln Tyr Phe Val Ala Thr Lys
465                 470                 475                 480

Leu Ser Gln Ala Gln Arg Ser Thr Leu Glu Tyr Ser Asn Glu Arg Leu
                485                 490                 495

Lys Gln Thr Asn Glu Met Leu Arg Gly Ile Lys Leu Leu Lys Leu Tyr
            500                 505                 510

Ala Trp Glu Asn Ile Phe Arg Thr Arg Val Glu Thr Thr Arg Arg Lys
        515                 520                 525

Glu Met Thr Ser Leu Arg Ala Phe Ala Ile Tyr Thr Ser Ile Ser Ile
    530                 535                 540

Phe Met Asn Thr Ala Ile Pro Ile Ala Ala Val Leu Ile Thr Phe Val
545                 550                 555                 560

Gly His Val Ser Phe Phe Lys Glu Ala Asp Phe Ser Pro Ser Val Ala
                565                 570                 575

Phe Ala Ser Leu Ser Leu Phe His Ile Leu Val Thr Pro Leu Phe Leu
            580                 585                 590

Leu Ser Ser Val Val Arg Ser Thr Val Lys Ala Leu Val Ser Val Gln
        595                 600                 605

Lys Leu Ser Glu Phe Leu Ser Ser Ala Glu Ile Arg Glu Glu Gln Cys
    610                 615                 620

Ala Pro His Glu Pro Thr Pro Gln Gly Pro Ala Ser Lys Tyr Gln Ala
625                 630                 635                 640

Val Pro Leu Arg Val Val Asn Arg Lys Arg Pro Ala Arg Glu Asp Cys
                645                 650                 655

Arg Gly Leu Thr Gly Pro Leu Gln Ser Leu Val Pro Ser Ala Asp Gly
            660                 665                 670

Asp Ala Asp Asn Cys Cys Val Gln Ile Met Gly Gly Tyr Phe Thr Trp
        675                 680                 685

Thr Pro Asp Gly Ile Pro Thr Leu Ser Asn Ile Thr Ile Arg Ile Pro

690                 695                 700

Arg Gly Gln Leu Thr Met Ile Val Gly Gln Val Gly Cys Gly Lys Ser
705                 710                 715                 720

Ser Leu Leu Leu Ala Ala Leu Gly Glu Met Gln Lys Val Ser Gly Ala
                725                 730                 735

Val Phe Trp Ser Ser Leu Pro Asp Ser Glu Ile Gly Glu Asp Pro Ser
            740                 745                 750

Pro Glu Arg Glu Thr Ala Thr Asp Leu Asp Ile Arg Lys Arg Gly Pro
        755                 760                 765

Val Ala Tyr Ala Ser Gln Lys Pro Trp Leu Leu Asn Ala Thr Val Glu
    770                 775                 780

Glu Asn Ile Ile Phe Glu Ser Pro Phe Asn Lys Gln Arg Tyr Lys Met
785                 790                 795                 800

Val Ile Glu Ala Cys Ser Leu Gln Pro Asp Ile Asp Ile Leu Pro His
                805                 810                 815

Gly Asp Gln Thr Gln Ile Gly Glu Arg Gly Ile Asn Leu Ser Gly Gly
            820                 825                 830

Gln Arg Gln Arg Ile Ser Val Ala Arg Ala Leu Tyr Gln His Ala Asn
        835                 840                 845

Val Val Phe Leu Asp Asp Pro Phe Ser Ala Leu Asp Ile His Leu Ser
    850                 855                 860

Asp His Leu Met Gln Ala Gly Ile Leu Glu Leu Leu Arg Asp Asp Lys
865                 870                 875                 880

Arg Thr Val Val Leu Val Thr His Lys Leu Gln Tyr Leu Pro His Ala
                885                 890                 895

Asp Trp Ile Ile Ala Met Lys Asp Gly Thr Ile Gln Arg Glu Gly Thr
            900                 905                 910

Leu Lys Asp Phe Gln Arg Ser Glu Cys Gln Leu Phe Glu His Trp Lys
        915                 920                 925

Thr Leu Met Asn Arg Gln Asp Gln Glu Leu Glu Lys Glu Thr Val Thr
    930                 935                 940

Glu Arg Lys Ala Thr Glu Pro Pro Gln Gly Leu Ser Arg Ala Met Ser

945                 950                 955                 960

Ser Arg Asp Gly Leu Leu Gln Asp Glu Glu Glu Glu Glu Glu Glu Ala
                965                 970                 975

Ala Glu Ser Glu Glu Asp Asp Asn Leu Ser Ser Met Leu His Gln Arg
            980                 985                 990

Ala Glu Ile Pro Trp Arg Ala Cys Ala Lys Tyr Leu Ser Ser Ala Gly
        995                 1000                1005

Ile Leu Leu Leu Ser Leu Leu Val Phe Ser Gln Leu Leu Lys His Met
    1010                1015                1020

Val Leu Val Ala Ile Asp Tyr Trp Leu Ala Lys Trp Thr Asp Ser Ala
1025                1030                1035                1040

Leu Thr Leu Thr Pro Ala Ala Arg Asn Cys Ser Leu Ser Gln Glu Cys
                1045                1050                1055

Thr Leu Asp Gln Thr Val Tyr Ala Met Val Phe Thr Val Leu Cys Ser
            1060                1065                1070

Leu Gly Ile Val Leu Cys Leu Val Thr Ser Val Thr Val Glu Trp Thr
        1075                1080                1085

Gly Leu Lys Val Ala Lys Arg Leu His Arg Ser Leu Leu Asn Arg Ile
    1090                1095                1100

Ile Leu Ala Pro Met Arg Phe Phe Glu Thr Thr Pro Leu Gly Ser Ile
1105                1110                1115                1120

Leu Asn Arg Phe Ser Ser Asp Cys Asn Thr Ile Asp Gln His Ile Pro
                1125                1130                1135

Ser Thr Leu Glu Cys Leu Ser Arg Ser Thr Leu Leu Cys Val Ser Ala
            1140                1145                1150

Leu Ala Val Ile Ser Tyr Val Thr Pro Val Phe Leu Val Ala Leu Leu
        1155                1160                1165

Pro Leu Ala Val Val Cys Tyr Phe Ile Gln Lys Tyr Phe Arg Val Ala
    1170                1175                1180

Ser Arg Asp Leu Gln Gln Leu Asp Asp Thr Thr Gln Leu Pro Leu Leu
1185                1190                1195                1200

Ser His Phe Ala Glu Thr Val Glu Gly Leu Thr Thr Ile Arg Ala Phe

1205                 1210                 1215

Arg Tyr Glu Ala Arg Phe Gln Gln Lys Leu Leu Glu Tyr Thr Asp Ser
            1220                 1225                 1230

Asn Asn Ile Ala Ser Leu Phe Leu Thr Ala Ala Asn Arg Trp Leu Glu
        1235                 1240                 1245

Val Arg Met Glu Tyr Ile Gly Ala Cys Val Val Leu Ile Ala Ala Val
    1250                 1255                 1260

Thr Ser Ile Ser Asn Ser Leu His Arg Glu Leu Ser Ala Gly Leu Val
1265                 1270                 1275                 1280

Gly Leu Gly Leu Thr Tyr Ala Leu Met Val Ser Asn Tyr Leu Asn Trp
                1285                 1290                 1295

Met Val Arg Asn Leu Ala Asp Met Glu Leu Gln Leu Gly Ala Val Lys
            1300                 1305                 1310

Arg Ile His Gly Leu Leu Lys Thr Glu Ala Glu Ser Tyr Glu Gly Leu
        1315                 1320                 1325

Leu Ala Pro Ser Leu Ile Pro Lys Asn Trp Pro Asp Gln Gly Lys Ile
    1330                 1335                 1340

Gln Ile Gln Asn Leu Ser Val Arg Tyr Asp Ser Ser Leu Lys Pro Val
1345                 1350                 1355                 1360

Leu Lys His Val Asn Ala Leu Ile Ser Pro Gly Gln Lys Ile Gly Ile
                1365                 1370                 1375

Cys Gly Arg Thr Gly Ser Gly Lys Ser Ser Phe Ser Leu Ala Phe Phe
            1380                 1385                 1390

Arg Met Val Asp Thr Phe Glu Gly His Ile Ile Ile Asp Gly Ile Asp
        1395                 1400                 1405

Ile Arg Lys Leu Pro Leu His Thr Leu Pro Ser Arg Leu Ser Ile Ile
    1410                 1415                 1420

Leu Gln Asp Pro Val Leu Phe Ser Gly Thr Ile Arg Phe Asn Leu Asp
1425                 1430                 1435                 1440

Pro Glu Arg Lys Cys Ser Asp Ser Thr Leu Trp Glu Ala Leu Glu Ile
                1445                 1450                 1455

Ala Gln Leu Lys Leu Val Val Lys Ala Leu Pro Gly Gly Leu Asp Ala

1460                    1465                    1470

Ile Ile Thr Glu Gly Gly Glu Asn Phe Ser Gln Gly Gln Arg Gln Leu
        1475                    1480                    1485

Phe Cys Leu Ala Arg Ala Phe Val Arg Lys Thr Ser Ile Phe Ile Met
    1490                    1495                    1500

Asp Glu Ala Thr Ala Ser Ile Asp Met Ala Thr Glu Asn Ile Leu Gln
1505                    1510                    1515                    1520

Lys Val Val Met Thr Ala Phe Ala Asp Arg Thr Val Val Thr Ile Ala
                1525                    1530                    1535

His Arg Val His Thr Ile Leu Ser Ala Asp Leu Val Ile Val Leu Lys
            1540                    1545                    1550

Arg Gly Ala Ile Leu Glu Phe Asp Lys Pro Glu Lys Leu Leu Ser Arg
        1555                    1560                    1565

Lys Asp Ser Val Phe Ala Ser Phe Val Arg Ala Asp Lys
    1570                    1575                    1580

<210> 68
<211> 1549
<212> PRT
<213> Homo sapiens

<400> 68
Met Ser Leu Ser Phe Cys Gly Asn Asn Ile Ser Ser Tyr Asn Ile Asn
1               5                   10                  15

Asp Gly Val Leu Gln Asn Ser Cys Phe Val Asp Ala Leu Asn Leu Val
            20                  25                  30

Pro His Val Phe Leu Leu Phe Ile Thr Phe Pro Ile Leu Phe Ile Gly
        35                  40                  45

Trp Gly Ser Gln Ser Ser Lys Val Gln Ile His His Asn Thr Trp Leu
    50                  55                  60

His Phe Pro Gly His Asn Leu Arg Trp Ile Leu Thr Phe Ala Leu Leu
65                  70                  75                  80

Phe Val His Val Cys Glu Ile Ala Glu Gly Ile Val Ser Asp Ser Arg
                85                  90                  95

Arg Glu Ser Arg His Leu His Leu Phe Met Pro Ala Val Met Gly Phe
            100                 105                 110

Val Ala Thr Thr Thr Ser Ile Val Tyr Tyr His Asn Ile Glu Thr Ser
        115                 120                 125

Asn Phe Pro Lys Leu Leu Leu Ala Leu Phe Leu Tyr Trp Val Met Ala
    130                 135                 140

Phe Ile Thr Lys Thr Ile Lys Leu Val Lys Tyr Cys Gln Ser Gly Leu
145                 150                 155                 160

Asp Ile Ser Asn Leu Arg Phe Cys Ile Thr Gly Met Met Val Ile Leu
                165                 170                 175

Asn Gly Leu Leu Met Ala Val Glu Ile Asn Val Ile Arg Val Arg Arg
            180                 185                 190

Tyr Val Phe Phe Met Asn Pro Gln Lys Val Lys Pro Pro Glu Asp Leu
        195                 200                 205

Gln Asp Leu Gly Val Arg Phe Leu Gln Pro Phe Val Asn Leu Leu Ser
    210                 215                 220

Lys Ala Thr Tyr Trp Trp Met Asn Thr Leu Ile Ile Ser Ala His Lys
225                 230                 235                 240

Lys Pro Ile Asp Leu Lys Ala Ile Gly Lys Leu Pro Ile Ala Met Arg
                245                 250                 255

Ala Val Thr Asn Tyr Val Cys Leu Lys Asp Ala Tyr Glu Glu Gln Lys
            260                 265                 270

Lys Lys Val Ala Asp His Pro Asn Arg Thr Pro Ser Ile Trp Leu Ala
        275                 280                 285

Met Tyr Arg Ala Phe Gly Arg Pro Ile Leu Leu Ser Ser Thr Phe Arg
    290                 295                 300

Tyr Leu Ala Asp Leu Leu Gly Phe Ala Gly Pro Leu Cys Ile Ser Gly
305                 310                 315                 320

Ile Val Gln Arg Val Asn Glu Thr Gln Asn Gly Thr Asn Asn Thr Thr
                325                 330                 335

Gly Ile Ser Glu Thr Leu Ser Ser Lys Glu Phe Leu Glu Asn Ala Tyr
            340                 345                 350

Val Leu Ala Val Leu Leu Phe Leu Ala Leu Ile Leu Gln Arg Thr Phe
        355                 360                 365

Leu Gln Ala Ser Tyr Tyr Val Thr Ile Glu Thr Gly Ile Asn Leu Arg
    370                 375                 380

Gly Ala Leu Leu Ala Met Ile Tyr Asn Lys Ile Leu Arg Leu Ser Thr
385                 390                 395                 400

Ser Asn Leu Ser Met Gly Glu Met Thr Leu Gly Gln Ile Asn Asn Leu
                405                 410                 415

Val Ala Ile Glu Thr Asn Gln Leu Met Trp Phe Leu Phe Leu Cys Pro
            420                 425                 430

Asn Leu Trp Ala Met Pro Val Gln Ile Ile Met Gly Val Ile Leu Leu
        435                 440                 445

Tyr Asn Leu Leu Gly Ser Ser Ala Leu Val Gly Ala Ala Val Ile Val
    450                 455                 460

Leu Leu Ala Pro Ile Gln Tyr Phe Ile Ala Thr Lys Leu Ala Glu Ala
465                 470                 475                 480

Gln Lys Ser Thr Leu Asp Tyr Ser Thr Glu Arg Leu Lys Lys Thr Asn
                485                 490                 495

Glu Ile Leu Lys Gly Ile Lys Leu Leu Lys Leu Tyr Ala Trp Glu His
            500                 505                 510

Ile Phe Cys Lys Ser Val Glu Glu Thr Arg Met Lys Glu Leu Ser Ser
        515                 520                 525

Leu Lys Thr Phe Ala Leu Tyr Thr Ser Leu Ser Ile Phe Met Asn Ala
    530                 535                 540

Ala Ile Pro Ile Ala Ala Val Leu Ala Thr Phe Val Thr His Ala Tyr
545                 550                 555                 560

Ala Ser Gly Asn Asn Leu Lys Pro Ala Glu Ala Phe Ala Ser Leu Ser
                565                 570                 575

Leu Phe His Ile Leu Val Thr Pro Leu Ser Leu Leu Phe Thr Val Val
            580                 585                 590

Arg Phe Ala Val Lys Ala Ile Ile Ser Val Gln Lys Leu Asn Glu Phe
        595                 600                 605

Leu Leu Ser Asp Glu Ile Gly Asp Asp Ser Trp Arg Thr Gly Glu Ser
    610                 615                 620

Ser Leu Pro Phe Glu Ser Cys Lys Lys His Thr Gly Val Gln Pro Lys
625                 630                 635                 640

Thr Ile Asn Arg Lys Gln Pro Gly Arg Tyr His Leu Asp Ser Tyr Glu
                645                 650                 655

Gln Ser Thr Arg Arg Leu Arg Pro Ala Glu Thr Glu Asp Ile Ala Ile
            660                 665                 670

Lys Val Thr Asn Gly Tyr Phe Ser Trp Gly Ser Gly Leu Ala Thr Leu
        675                 680                 685

Ser Asn Ile Asp Ile Arg Ile Pro Thr Gly Gln Leu Thr Met Ile Val
    690                 695                 700

Gly Gln Val Gly Cys Gly Lys Ser Ser Leu Leu Leu Ala Ile Leu Gly
705                 710                 715                 720

Glu Met Gln Thr Leu Glu Gly Lys Val His Trp Ser Asn Val Asn Glu
                725                 730                 735

Ser Glu Pro Ser Phe Glu Ala Thr Arg Ser Arg Asn Arg Tyr Ser Val
            740                 745                 750

Ala Tyr Ala Ala Gln Lys Pro Trp Leu Leu Asn Ala Thr Val Glu Glu
        755                 760                 765

Asn Ile Thr Phe Gly Ser Pro Phe Asn Lys Gln Arg Tyr Lys Ala Val
    770                 775                 780

Thr Asp Ala Cys Ser Leu Gln Pro Asp Ile Asp Leu Leu Pro Phe Gly
785                 790                 795                 800

Asp Gln Thr Glu Ile Gly Glu Arg Gly Ile Asn Leu Ser Gly Gly Gln
                805                 810                 815

Arg Gln Arg Ile Cys Val Ala Arg Ala Leu Tyr Gln Asn Thr Asn Ile
            820                 825                 830

Val Phe Leu Asp Asp Pro Phe Ser Ala Leu Asp Ile His Leu Ser Asp
        835                 840                 845

His Leu Met Gln Glu Gly Ile Leu Lys Phe Leu Gln Asp Asp Lys Arg
    850                 855                 860

Thr Leu Val Leu Val Thr His Lys Leu Gln Tyr Leu Thr His Ala Asp
865                 870                 875                 880

Trp Ile Ile Ala Met Lys Asp Gly Ser Val Leu Arg Glu Gly Thr Leu
                885                 890                 895

Lys Asp Ile Gln Thr Lys Asp Val Glu Leu Tyr Glu His Trp Lys Thr
            900                 905                 910

Leu Met Asn Arg Gln Asp Gln Glu Leu Glu Lys Asp Met Glu Ala Asp
        915                 920                 925

Gln Thr Thr Leu Glu Arg Lys Thr Leu Arg Arg Ala Met Tyr Ser Arg
    930                 935                 940

Glu Ala Lys Ala Gln Met Glu Asp Glu Asp Glu Glu Glu Glu Glu Glu
945                 950                 955                 960

Glu Asp Glu Asp Asp Asn Met Ser Thr Val Met Arg Leu Arg Thr Lys
                965                 970                 975

Met Pro Trp Lys Thr Cys Trp Arg Tyr Leu Thr Ser Gly Gly Phe Phe
            980                 985                 990

Leu Leu Ile Leu Met Ile Phe Ser Lys Leu Leu Lys His Ser Val Ile
        995                 1000                1005

Val Ala Ile Asp Tyr Trp Leu Ala Thr Trp Thr Ser Glu Tyr Ser Ile
    1010                1015                1020

Asn Asn Thr Gly Lys Ala Asp Gln Thr Tyr Tyr Val Ala Gly Phe Ser
1025                1030                1035                1040

Ile Leu Cys Gly Ala Gly Ile Phe Leu Cys Leu Val Thr Ser Leu Thr
                1045                1050                1055

Val Glu Trp Met Gly Leu Thr Ala Ala Lys Asn Leu His His Asn Leu
            1060                1065                1070

Leu Asn Lys Ile Ile Leu Gly Pro Ile Arg Phe Phe Asp Thr Thr Pro
        1075                1080                1085

Leu Gly Leu Ile Leu Asn Arg Phe Ser Ala Asp Thr Asn Ile Ile Asp
    1090                1095                1100

Gln His Ile Pro Pro Thr Leu Glu Ser Leu Thr Arg Ser Thr Leu Leu
1105                1110                1115                1120

Cys Leu Ser Ala Ile Gly Met Ile Ser Tyr Ala Thr Pro Val Phe Leu
                1125                1130                1135

Val Ala Leu Leu Pro Leu Gly Val Ala Phe Tyr Phe Ile Gln Lys Tyr
            1140                1145                1150

Phe Arg Val Ala Ser Lys Asp Leu Gln Glu Leu Asp Asp Ser Thr Gln
        1155                1160                1165

Leu Pro Leu Leu Cys His Phe Ser Glu Thr Ala Glu Gly Leu Thr Thr
    1170                1175                1180

Ile Arg Ala Phe Arg His Glu Thr Arg Phe Lys Gln Arg Met Leu Glu
1185                1190                1195                1200

Leu Thr Asp Thr Asn Asn Ile Ala Tyr Leu Phe Leu Ser Ala Ala Asn
                1205                1210                1215

Arg Trp Leu Glu Val Arg Thr Asp Tyr Leu Gly Ala Cys Ile Val Leu
            1220                1225                1230

Thr Ala Ser Ile Ala Ser Ile Ser Gly Ser Ser Asn Ser Gly Leu Val
        1235                1240                1245

Gly Leu Gly Leu Leu Tyr Ala Leu Thr Ile Thr Asn Tyr Leu Asn Trp
    1250                1255                1260

Val Val Arg Asn Leu Ala Asp Leu Glu Val Gln Met Gly Ala Val Lys
1265                1270                1275                1280

Lys Val Asn Ser Phe Leu Thr Met Glu Ser Glu Asn Tyr Glu Gly Thr
                1285                1290                1295

Met Asp Pro Ser Gln Val Pro Glu His Trp Pro Gln Glu Gly Glu Ile
            1300                1305                1310

Lys Ile His Asp Leu Cys Val Arg Tyr Glu Asn Asn Leu Lys Pro Val
        1315                1320                1325

Leu Lys His Val Lys Ala Tyr Ile Lys Pro Gly Gln Lys Val Gly Ile
    1330                1335                1340

Cys Gly Arg Thr Gly Ser Gly Lys Ser Ser Leu Ser Leu Ala Phe Phe
1345                1350                1355                1360

Arg Met Val Asp Ile Phe Asp Gly Lys Ile Val Ile Asp Gly Ile Asp
                1365                1370                1375

Ile Ser Lys Leu Pro Leu His Thr Leu Arg Ser Arg Leu Ser Ile Ile
        1380                1385                1390

Leu Gln Asp Pro Ile Leu Phe Ser Gly Ser Ile Arg Phe Asn Leu Asp
    1395                1400                1405

Pro Glu Cys Lys Cys Thr Asp Asp Arg Leu Trp Glu Ala Leu Glu Ile
  1410                1415                1420

Ala Gln Leu Lys Asn Met Val Lys Ser Leu Pro Gly Gly Leu Asp Ala
1425                1430                1435                1440

Val Val Thr Glu Gly Gly Glu Asn Phe Ser Val Gly Gln Arg Gln Leu
                1445                1450                1455

Phe Cys Leu Ala Arg Ala Phe Val Arg Lys Ser Ser Ile Leu Ile Met
        1460                1465                1470

Asp Glu Ala Thr Ala Ser Ile Asp Met Ala Thr Glu Asn Ile Leu Gln
    1475                1480                1485

Lys Val Val Met Thr Ala Phe Ala Asp Arg Thr Val Val Thr Met Ala
  1490                1495                1500

His Arg Val Ser Ser Ile Met Asp Ala Gly Leu Val Leu Val Phe Ser
1505                1510                1515                1520

Glu Gly Ile Leu Val Glu Cys Asp Thr Val Pro Asn Leu Phe Ala His
                1525                1530                1535

Lys Asn Gly Pro Phe Ser Thr Leu Val Met Thr Asn Lys
        1540                1545

<210> 69
<211> 1513
<212> PRT
<213> Homo sapiens

<400> 69
Gly Ser Gly Cys Leu Gly Ala Glu Lys Arg Glu Gly Lys Asn Arg Trp
 1               5                  10                  15

Gln Gly Glu Ala Ser Met Glu Arg Leu Leu Ala Gln Leu Cys Gly Ser
            20                  25                  30

Ser Ala Ala Trp Pro Leu Pro Leu Trp Glu Gly Asp Thr Thr Gly His
        35                  40                  45

Cys Phe Thr Gln Leu Val Leu Ser Ala Leu Pro His Ala Leu Leu Ala
    50                  55                  60

Val Leu Ser Ala Cys Tyr Leu Gly Thr Pro Arg Ser Pro Asp Tyr Ile
65                  70                  75                  80

Leu Pro Cys Ser Pro Gly Trp Arg Leu Arg Leu Ala Ala Ser Phe Leu
                85                  90                  95

Leu Ser Val Phe Pro Leu Leu Asp Leu Leu Pro Val Ala Leu Pro Pro
            100                 105                 110

Gly Ala Gly Pro Gly Pro Ile Gly Leu Glu Val Leu Ala Gly Cys Val
        115                 120                 125

Ala Ala Val Ala Trp Ile Ser His Ser Leu Ala Leu Trp Val Leu Ala
    130                 135                 140

His Ser Pro His Gly His Ser Arg Gly Pro Leu Ala Leu Ala Leu Val
145                 150                 155                 160

Ala Leu Leu Pro Ala Pro Ala Leu Val Leu Thr Val Leu Trp His Cys
                165                 170                 175

Gln Arg Gly Thr Leu Leu Pro Pro Leu Leu Pro Gly Pro Met Ala Arg
            180                 185                 190

Leu Cys Leu Leu Ile Leu Gln Leu Ala Ala Leu Leu Ala Tyr Ala Leu
        195                 200                 205

Gly Trp Ala Ala Pro Gly Gly Pro Arg Glu Pro Trp Ala Gln Glu Pro
    210                 215                 220

Leu Leu Pro Glu Asp Gln Glu Pro Glu Val Ala Glu Asp Gly Glu Ser
225                 230                 235                 240

Trp Leu Ser Arg Phe Ser Tyr Ala Trp Leu Ala Pro Leu Leu Ala Arg
                245                 250                 255

Gly Ala Cys Gly Glu Leu Arg Gln Pro Gln Asp Ile Cys Arg Leu Pro
            260                 265                 270

His Arg Leu Gln Pro Thr Tyr Leu Ala Arg Val Phe Gln Ala His Trp
        275                 280                 285

Gln Glu Gly Ala Arg Leu Trp Arg Ala Leu Tyr Gly Ala Phe Gly Arg
    290                 295                 300

Cys Tyr Leu Ala Leu Gly Leu Leu Lys Leu Val Gly Thr Met Leu Gly
305                 310                 315                 320

Phe Ser Gly Pro Leu Leu Leu Ser Leu Leu Val Gly Phe Leu Glu Glu
                325                 330                 335

Gly Gln Glu Pro Leu Ser His Gly Leu Leu Tyr Ala Leu Gly Leu Ala
            340                 345                 350

Gly Gly Ala Val Leu Gly Ala Val Leu Gln Asn Gln Tyr Gly Tyr Glu
        355                 360                 365

Val Tyr Lys Val Thr Leu Gln Ala Arg Gly Ala Val Leu Asn Ile Leu
    370                 375                 380

Tyr Cys Lys Ala Leu Gln Leu Gly Pro Ser Arg Pro Pro Thr Gly Glu
385                 390                 395                 400

Ala Leu Asn Leu Leu Gly Thr Asp Ser Glu Arg Leu Leu Asn Phe Ala
                405                 410                 415

Gly Ser Phe His Glu Ala Trp Gly Leu Pro Leu Gln Leu Ala Ile Thr
            420                 425                 430

Leu Tyr Leu Leu Tyr Gln Gln Val Gly Val Ala Phe Val Gly Gly Leu
        435                 440                 445

Ile Leu Ala Leu Leu Leu Val Pro Val Asn Lys Val Ile Ala Thr Arg
    450                 455                 460

Ile Met Ala Ser Asn Gln Glu Met Leu Gln His Lys Asp Ala Arg Val
465                 470                 475                 480

Lys Leu Val Thr Glu Leu Leu Ser Gly Ile Arg Val Ile Lys Phe Cys
                485                 490                 495

Gly Trp Glu Gln Ala Leu Gly Ala Arg Val Glu Ala Cys Arg Ala Arg
            500                 505                 510

Glu Leu Gly Arg Leu Arg Val Ile Lys Tyr Leu Asp Ala Ala Cys Val
        515                 520                 525

Tyr Leu Trp Ala Ala Leu Pro Val Val Ile Ser Ile Val Ile Phe Ile
    530                 535                 540

Thr Tyr Val Leu Met Gly His Gln Leu Thr Ala Thr Lys Val Phe Thr
545                 550                 555                 560

Ala Leu Ala Leu Val Arg Met Leu Ile Leu Pro Leu Asn Asn Phe Pro
                565                 570                 575

Trp Val Ile Asn Gly Leu Leu Glu Ala Lys Val Ser Leu Asp Arg Ile
            580                 585                 590

Gln Leu Phe Leu Asp Leu Pro Asn His Asn Pro Gln Ala Tyr Tyr Ser
        595                 600                 605

Pro Asp Pro Pro Ala Glu Pro Ser Thr Val Leu Glu Leu His Gly Ala
    610                 615                 620

Leu Phe Ser Trp Asp Pro Val Gly Thr Ser Leu Glu Thr Phe Ile Ser
625                 630                 635                 640

His Leu Glu Val Lys Lys Gly Met Leu Val Gly Ile Val Gly Lys Val
                645                 650                 655

Gly Cys Gly Lys Ser Ser Leu Leu Ala Ala Ile Ala Gly Glu Leu His
            660                 665                 670

Arg Leu Arg Gly His Val Ala Val Arg Gly Leu Ser Lys Gly Phe Gly
        675                 680                 685

Leu Ala Thr Gln Glu Pro Trp Ile Gln Phe Ala Thr Ile Arg Asp Asn
    690                 695                 700

Ile Leu Phe Gly Lys Thr Phe Asp Ala Gln Leu Tyr Lys Glu Val Leu
705                 710                 715                 720

Glu Ala Cys Ala Leu Asn Asp Asp Leu Ser Ile Leu Pro Ala Gly Asp
                725                 730                 735

Gln Thr Glu Val Gly Glu Lys Gly Val Thr Leu Ser Gly Gly Gln Arg
            740                 745                 750

Ala Arg Ile Ala Leu Ala Arg Ala Val Tyr Gln Glu Lys Glu Leu Tyr
        755                 760                 765

Leu Leu Asp Asp Pro Leu Ala Ala Val Asp Ala Asp Val Ala Asn His
    770                 775                 780

Leu Leu His Arg Cys Ile Leu Gly Met Leu Ser Tyr Thr Thr Arg Leu
785                 790                 795                 800

Leu Cys Thr His Arg Thr Glu Tyr Leu Glu Arg Ala Asp Ala Val Leu
                805                 810                 815

Leu Met Glu Ala Gly Arg Leu Ile Arg Ala Gly Pro Pro Ser Glu Ile
            820                 825                 830

Leu Pro Leu Val Gln Ala Val Pro Lys Ala Trp Ala Glu Asn Gly Gln
        835                 840                 845

Glu Ser Asp Ser Ala Thr Ala Gln Ser Val Gln Asn Pro Glu Lys Thr
    850                 855                 860

Lys Glu Gly Leu Glu Glu Glu Gln Ser Thr Ser Gly Arg Leu Leu Gln
865                 870                 875                 880

Glu Glu Ser Lys Lys Glu Gly Ala Val Ala Leu His Val Tyr Gln Ala
                885                 890                 895

Tyr Trp Lys Ala Val Gly Gln Gly Leu Ala Leu Ala Ile Leu Phe Ser
            900                 905                 910

Leu Leu Leu Met Gln Ala Thr Arg Asn Ala Ala Asp Trp Trp Leu Ser
        915                 920                 925

His Trp Ile Ser Gln Leu Lys Ala Glu Asn Ser Ser Gln Glu Ala Gln
    930                 935                 940

Pro Ser Thr Ser Pro Ala Ser Met Gly Leu Phe Ser Pro Gln Leu Leu
945                 950                 955                 960

Leu Phe Ser Pro Gly Asn Leu Tyr Ile Pro Val Phe Pro Leu Pro Lys
                965                 970                 975

Ala Ala Pro Asn Gly Ser Ser Asp Ile Arg Phe Tyr Leu Thr Val Tyr
            980                 985                 990

Ala Thr Ile Ala Gly Val Asn Ser Leu Cys Thr Leu Leu Arg Ala Val
        995                 1000                1005

Leu Phe Ala Ala Gly Thr Leu Gln Ala Ala Ala Thr Leu His Arg Arg
    1010                1015                1020

Leu Leu His Arg Val Leu Met Ala Pro Val Thr Phe Phe Asn Ala Thr
1025                1030                1035                1040

Pro Thr Gly Arg Ile Leu Asn Arg Phe Ser Ser Asp Val Ala Cys Ala
                1045                1050                1055

Asp Asp Ser Leu Pro Phe Ile Leu Asn Ile Leu Leu Ala Asn Ala Ala
            1060                1065                1070

Gly Leu Leu Gly Leu Leu Ala Val Leu Gly Ser Gly Leu Pro Trp Leu
        1075                1080                1085

Leu Leu Leu Leu Pro Pro Leu Ser Ile Met Tyr Tyr His Val Gln Arg
    1090                1095                1100

His Tyr Arg Ala Ser Ser Arg Glu Leu Arg Arg Leu Gly Ser Leu Thr
1105                1110                1115                1120

Leu Ser Pro Leu Tyr Ser His Leu Ala Asp Thr Leu Ala Gly Leu Ser
                1125                1130                1135

Val Leu Arg Ala Thr Gly Ala Thr Tyr Arg Phe Glu Glu Glu Asn Leu
            1140                1145                1150

Arg Leu Leu Glu Leu Asn Gln Arg Cys Gln Phe Ala Thr Ser Ala Thr
        1155                1160                1165

Met Gln Trp Leu Asp Ile Arg Leu Gln Leu Met Gly Ala Ala Val Val
    1170                1175                1180

Ser Ala Ile Ala Gly Ile Ala Leu Val Gln His Gln Gln Gly Leu Ala
1185                1190                1195                1200

Asn Pro Gly Leu Val Gly Leu Ser Leu Ser Tyr Ala Leu Ser Leu Thr
                1205                1210                1215

Gly Leu Leu Ser Gly Leu Val Ser Ser Phe Thr Gln Thr Glu Ala Met
            1220                1225                1230

Leu Val Ser Val Glu Arg Leu Glu Glu Tyr Thr Cys Asp Leu Pro Gln
        1235                1240                1245

Glu Pro Gln Gly Gln Pro Leu Gln Leu Gly Thr Gly Trp Leu Thr Gln
    1250                1255                1260

Gly Gly Val Glu Phe Gln Asp Val Val Leu Ala Tyr Arg Pro Gly Leu
1265                1270                1275                1280

Pro Asn Ala Leu Asp Gly Val Thr Phe Cys Val Gln Pro Gly Glu Lys
                1285                1290                1295

Leu Gly Ile Val Gly Arg Thr Gly Ser Gly Lys Ser Ser Leu Leu Leu
            1300                1305                1310

Val Leu Phe Arg Leu Leu Glu Pro Ser Ser Gly Arg Val Leu Leu Asp
        1315                1320                1325

Gly Val Asp Thr Ser Gln Leu Glu Leu Ala Gln Leu Arg Ser Gln Leu
    1330                1335                1340

Ala Ile Ile Pro Gln Glu Pro Phe Leu Phe Ser Gly Thr Val Arg Glu
1345                1350                1355                1360

Asn Leu Asp Pro Gln Gly Leu His Lys Asp Arg Ala Leu Trp Gln Ala
                1365                1370                1375

Leu Lys Gln Cys His Leu Ser Glu Val Ile Thr Ser Met Gly Gly Leu
            1380                1385                1390

Asp Gly Glu Leu Gly Glu Gly Gly Arg Ser Leu Ser Leu Gly Gln Arg
        1395                1400                1405

Gln Leu Leu Cys Leu Ala Arg Ala Leu Leu Thr Asp Ala Lys Ile Leu
    1410                1415                1420

Cys Ile Asp Glu Ala Thr Ala Ser Val Asp Gln Lys Thr Asp Gln Leu
1425                1430                1435                1440

Leu Gln Gln Thr Ile Cys Lys Arg Phe Ala Asn Lys Thr Val Leu Thr
                1445                1450                1455

Ile Ala His Arg Leu Asn Thr Ile Leu Asn Ser Asp Arg Val Leu Val
            1460                1465                1470

Leu Gln Ala Gly Arg Val Val Glu Leu Asp Ser Pro Ala Thr Leu Arg
        1475                1480                1485

Asn Gln Pro His Ser Leu Phe Gln Gln Leu Leu Gln Ser Ser Gln Gln
    1490                1495                1500

Gly Val Pro Ala Ser Leu Gly Gly Pro
1505                1510

<210> 70
<211> 745
<212> PRT
<213> Homo sapiens

<400> 70

<210> 71
<211> 740

<212> PRT
<213> Homo sapiens

<400> 71
Met Thr His Met Leu Asn Ala Ala Ala Asp Arg Val Lys Trp Thr Arg
1               5                   10                  15

Ser Ser Ala Ala Lys Arg Ala Ala Cys Leu Val Ala Ala Ala Tyr Ala
            20                  25                  30

Leu Lys Thr Leu Tyr Pro Ile Ile Gly Lys Arg Leu Lys Gln Ser Gly
        35                  40                  45

His Gly Lys Lys Lys Ala Ala Ala Tyr Pro Ala Ala Glu Asn Thr Glu
    50                  55                  60

Ile Leu His Cys Thr Glu Thr Ile Cys Glu Lys Pro Ser Pro Gly Val
65                  70                  75                  80

Asn Ala Asp Phe Phe Lys Gln Leu Leu Glu Leu Arg Lys Ile Leu Phe
                85                  90                  95

Pro Lys Leu Val Thr Thr Glu Thr Gly Trp Leu Cys Leu His Ser Val
            100                 105                 110

Ala Leu Ile Ser Arg Thr Phe Leu Ser Ile Tyr Val Ala Gly Leu Asp
        115                 120                 125

Gly Lys Ile Val Lys Ser Ile Val Glu Lys Lys Pro Arg Thr Phe Ile
    130                 135                 140

Ile Lys Leu Ile Lys Trp Leu Met Ile Ala Ile Pro Ala Thr Phe Val
145                 150                 155                 160

Asn Ser Ala Ile Arg Tyr Leu Glu Cys Lys Leu Ala Leu Ala Phe Arg
                165                 170                 175

Thr Arg Leu Val Asp His Ala Tyr Glu Thr Tyr Phe Thr Asn Gln Thr
            180                 185                 190

Tyr Tyr Lys Val Ile Asn Met Asp Gly Arg Leu Ala Asn Pro Asp Gln
        195                 200                 205

Ser Leu Thr Glu Asp Ile Met Met Phe Ser Gln Ser Val Ala His Leu
    210                 215                 220

Tyr Ser Asn Leu Thr Lys Pro Ile Leu Asp Val Met Leu Thr Ser Tyr
225                 230                 235                 240

Thr Leu Ile Gln Thr Ala Thr Ser Arg Gly Ala Ser Pro Ile Gly Pro
                245                 250                 255

Thr Leu Leu Ala Gly Leu Val Val Tyr Ala Thr Ala Lys Val Leu Lys
            260                 265                 270

Ala Cys Ser Pro Lys Phe Gly Lys Leu Val Ala Glu Glu Ala His Arg
        275                 280                 285

Lys Gly Tyr Leu Arg Tyr Val His Ser Arg Ile Ile Ala Asn Val Glu
    290                 295                 300

Glu Ile Ala Phe Tyr Arg Gly His Lys Val Glu Met Lys Gln Leu Gln
305                 310                 315                 320

Lys Ser Tyr Lys Ala Leu Ala Asp Gln Met Asn Leu Ile Leu Ser Lys
                325                 330                 335

Arg Leu Trp Tyr Ile Met Ile Glu Gln Phe Leu Met Lys Tyr Val Trp
            340                 345                 350

Ser Ser Ser Gly Leu Ile Met Val Ala Ile Pro Ile Ile Thr Ala Thr
        355                 360                 365

Gly Phe Ala Asp Gly Glu Asp Gly Gln Lys Gln Val Met Val Ser Glu
    370                 375                 380

Arg Thr Glu Ala Phe Thr Thr Ala Arg Asn Leu Leu Ala Ser Gly Ala
385                 390                 395                 400

Asp Ala Ile Glu Arg Ile Met Ser Ser Tyr Lys Glu Val Thr Glu Leu
                405                 410                 415

Ala Gly Tyr Thr Ala Arg Val Tyr Asn Met Phe Trp Val Phe Asp Glu
            420                 425                 430

Val Lys Arg Gly Ile Tyr Lys Arg Thr Ala Val Ile Gln Glu Ser Glu
        435                 440                 445

Ser His Ser Lys Asn Gly Ala Lys Val Glu Leu Pro Leu Ser Asp Thr
    450                 455                 460

Leu Ala Ile Lys Gly Lys Val Ile Asp Val Asp His Gly Ile Ile Cys
465                 470                 475                 480

Glu Asn Val Pro Ile Ile Thr Pro Ala Gly Glu Val Val Ala Ser Arg
                485                 490                 495

Leu Asn Phe Lys Val Glu Glu Gly Met His Leu Leu Ile Thr Gly Pro
            500                 505                 510

Asn Gly Cys Gly Lys Ser Ser Leu Phe Arg Ile Leu Ser Gly Leu Trp
        515                 520                 525

Pro Val Tyr Glu Gly Val Leu Tyr Lys Pro Pro Pro Gln His Met Phe
    530                 535                 540

Tyr Ile Pro Gln Arg Pro Tyr Met Ser Leu Gly Ser Leu Arg Asp Gln
545                 550                 555                 560

Val Ile Tyr Pro Asp Ser Val Asp Asp Met His Asp Lys Gly Tyr Thr
                565                 570                 575

Asp Gln Asp Leu Glu Arg Ile Leu His Asn Val His Leu Tyr His Ile
            580                 585                 590

Val Gln Arg Glu Gly Gly Trp Asp Ala Val Met Asp Trp Lys Asp Val
        595                 600                 605

Leu Ser Gly Gly Glu Lys Gln Arg Met Gly Met Ala Arg Met Phe Tyr
    610                 615                 620

His Lys Pro Lys Tyr Ala Leu Leu Asp Glu Cys Thr Ser Ala Val Ser
625                 630                 635                 640

Ile Asp Val Glu Gly Lys Ile Phe Gln Ala Ala Lys Gly Ala Gly Ile
                645                 650                 655

Ser Leu Leu Ser Ile Thr His Arg Pro Ser Leu Trp Lys Tyr His Thr
            660                 665                 670

His Leu Leu Gln Phe Asp Gly Glu Gly Gly Trp Arg Phe Glu Gln Leu
        675                 680                 685

Asp Thr Ala Ile Arg Leu Thr Leu Ser Glu Glu Lys Gln Lys Leu Glu
    690                 695                 700

Ser Gln Leu Ala Gly Ile Pro Lys Met Gln Gln Arg Leu Asn Glu Leu
705                 710                 715                 720

Cys Lys Ile Leu Gly Glu Asp Ser Val Leu Lys Thr Ile Lys Asn Glu
                725                 730                 735

Asp Glu Thr Ser
            740

<210> 72
<211> 659
<212> PRT
<213> Homo sapiens

<400> 72
Met Ala Ala Phe Ser Lys Tyr Leu Thr Ala Arg Asn Ser Ser Leu Ala
1               5                   10                  15

Gly Ala Ala Phe Leu Leu Leu Cys Leu Leu His Lys Arg Arg Arg Ala
            20                  25                  30

Leu Gly Leu His Gly Lys Lys Ser Gly Lys Pro Pro Leu Gln Asn Asn
        35                  40                  45

Glu Lys Glu Gly Lys Lys Glu Arg Ala Val Val Asp Lys Val Phe Phe
    50                  55                  60

Ser Arg Leu Ile Gln Ile Leu Lys Ile Met Val Pro Arg Thr Phe Cys
65                  70                  75                  80

Lys Glu Thr Gly Tyr Leu Val Leu Ile Ala Val Met Leu Val Ser Arg
                85                  90                  95

Thr Tyr Cys Asp Val Trp Met Ile Gln Asn Gly Thr Leu Ile Glu Ser
            100                 105                 110

Gly Ile Ile Gly Arg Ser Arg Lys Asp Phe Lys Arg Tyr Leu Leu Asn
        115                 120                 125

Phe Ile Ala Ala Met Pro Leu Ile Ser Leu Val Asn Asn Phe Leu Lys
    130                 135                 140

Tyr Gly Leu Asn Glu Leu Lys Leu Cys Phe Arg Val Arg Leu Thr Lys
145                 150                 155                 160

Tyr Leu Tyr Glu Glu Tyr Leu Gln Ala Phe Thr Tyr Tyr Lys Met Gly
                165                 170                 175

Asn Leu Asp Asn Arg Ile Ala Asn Pro Asp Gln Leu Leu Thr Gln Asp
            180                 185                 190

Val Glu Lys Phe Cys Asn Ser Val Val Asp Leu Tyr Ser Asn Leu Ser
        195                 200                 205

Lys Pro Phe Leu Asp Ile Val Leu Tyr Ile Phe Lys Leu Thr Ser Ala
    210                 215                 220

Ile Gly Ala Gln Gly Pro Ala Ser Met Met Ala Tyr Leu Val Val Ser
225                 230                 235                 240

Gly Leu Phe Leu Thr Arg Leu Arg Arg Pro Ile Gly Lys Met Thr Ile
                245                 250                 255

Thr Glu Gln Lys Tyr Glu Gly Glu Tyr Arg Tyr Val Asn Ser Arg Leu
            260                 265                 270

Ile Thr Asn Ser Glu Glu Ile Ala Phe Tyr Asn Gly Asn Lys Arg Glu
        275                 280                 285

Lys Gln Thr Val His Ser Val Phe Arg Lys Leu Val Glu His Leu His
    290                 295                 300

Asn Phe Ile Leu Phe Arg Phe Ser Met Gly Phe Ile Asp Ser Ile Ile
305                 310                 315                 320

Ala Lys Tyr Leu Ala Thr Val Val Gly Tyr Leu Val Val Ser Arg Pro
                325                 330                 335

Phe Leu Asp Leu Ser His Pro Arg His Leu Lys Ser Thr His Ser Glu
            340                 345                 350

Leu Leu Glu Asp Tyr Tyr Gln Ser Gly Arg Met Leu Leu Arg Met Ser
        355                 360                 365

Gln Ala Leu Gly Arg Ile Val Leu Ala Gly Arg Glu Met Thr Arg Leu
    370                 375                 380

Ala Gly Phe Thr Ala Arg Ile Thr Glu Leu Met Gln Val Leu Lys Asp
385                 390                 395                 400

Leu Asn His Gly Lys Tyr Glu Arg Thr Met Val Ser Gln Gln Glu Lys
                405                 410                 415

Gly Ile Glu Gly Val Gln Val Ile Pro Leu Ile Pro Gly Ala Gly Glu
            420                 425                 430

Ile Ile Ile Ala Asp Asn Ile Ile Lys Phe Asp His Val Pro Leu Ala
        435                 440                 445

Thr Pro Asn Gly Asp Val Leu Ile Arg Asp Leu Asn Phe Glu Val Arg
    450                 455                 460

Ser Gly Ala Asn Val Leu Ile Cys Gly Pro Asn Gly Cys Gly Lys Ser
465                 470                 475                 480

Ser Leu Phe Arg Val Leu Gly Glu Leu Trp Pro Leu Phe Gly Gly Arg
                485                 490                 495

Leu Thr Lys Pro Glu Arg Gly Lys Leu Phe Tyr Val Pro Gln Arg Pro
            500                 505                 510

Tyr Met Thr Leu Gly Thr Leu Arg Asp Gln Val Ile Tyr Pro Asp Gly
        515                 520                 525

Arg Glu Asp Gln Lys Arg Lys Gly Ile Ser Asp Leu Val Leu Lys Glu
    530                 535                 540

Tyr Leu Asp Asn Val Gln Leu Gly His Ile Leu Glu Arg Glu Gly Gly
545                 550                 555                 560

Trp Asp Ser Val Gln Asp Trp Met Asp Val Leu Ser Gly Gly Glu Lys
                565                 570                 575

Gln Arg Met Ala Met Ala Arg Leu Phe Tyr His Lys Pro Gln Phe Ala
            580                 585                 590

Ile Leu Asp Glu Cys Thr Ser Ala Val Ser Val Asp Val Glu Gly Tyr
        595                 600                 605

Ile Tyr Ser His Cys Arg Lys Val Gly Ile Thr Leu Phe Thr Val Ser
    610                 615                 620

His Arg Lys Ser Leu Trp Lys His His Glu Tyr Tyr Leu His Met Asp
625                 630                 635                 640

Gly Arg Gly Asn Tyr Glu Phe Lys Gln Ile Thr Glu Asp Thr Val Glu
                645                 650                 655

Phe Gly Ser

<210> 73
<211> 606
<212> PRT
<213> Homo sapiens

<400> 73

<210> 74
<211> 599
<212> PRT
<213> Homo sapiens

<400> 74
Met Ala Asp Lys Leu Thr Arg Ile Ala Ile Val Asn His Asp Lys Cys
  1               5                  10                  15

Lys Pro Lys Lys Cys Arg Gln Glu Cys Lys Lys Ser Cys Pro Val Val
             20                  25                  30

Arg Met Gly Lys Leu Cys Ile Glu Val Thr Pro Gln Ser Lys Ile Ala
         35                  40                  45

Trp Ile Ser Glu Thr Leu Cys Ile Gly Cys Gly Ile Cys Ile Lys Lys
     50                  55                  60

Cys Pro Phe Gly Ala Leu Ser Ile Val Asn Leu Pro Ser Asn Leu Glu
 65                  70                  75                  80

Lys Glu Thr Thr His Arg Tyr Cys Ala Asn Ala Phe Lys Leu His Arg
                 85                  90                  95

Leu Pro Ile Pro Arg Pro Gly Glu Val Leu Gly Leu Val Gly Thr Asn
            100                 105                 110

Gly Ile Gly Lys Ser Ala Ala Leu Lys Ile Leu Ala Gly Lys Gln Lys
        115                 120                 125

Pro Asn Leu Gly Lys Tyr Asp Asp Pro Pro Asp Trp Gln Glu Ile Leu
    130                 135                 140

Thr Tyr Phe Arg Gly Ser Glu Leu Gln Asn Tyr Phe Thr Lys Ile Leu
145                 150                 155                 160

Glu Asp Asp Leu Lys Ala Ile Ile Lys Pro Gln Tyr Val Ala Arg Phe
                165                 170                 175

Leu Arg Leu Ala Lys Gly Thr Val Gly Ser Ile Leu Asp Arg Lys Asp
            180                 185                 190

Glu Thr Lys Thr Gln Ala Ile Val Cys Gln Gln Leu Asp Leu Thr His
        195                 200                 205

Leu Lys Glu Arg Asn Val Glu Asp Leu Ser Gly Gly Glu Leu Gln Arg
    210                 215                 220

Phe Ala Cys Ala Val Val Cys Ile Gln Lys Ala Asp Ile Phe Met Phe
225                 230                 235                 240

Asp Glu Pro Ser Ser Tyr Leu Asp Val Lys Gln Arg Leu Lys Ala Ala
                245                 250                 255

Ile Thr Ile Arg Ser Leu Ile Asn Pro Asp Arg Tyr Ile Ile Val Val
            260                 265                 270

Glu His Asp Leu Ser Val Leu Asp Tyr Leu Ser Asp Phe Ile Cys Cys
        275                 280                 285

Leu Tyr Gly Val Pro Ser Ala Tyr Gly Val Val Thr Met Pro Phe Ser
    290                 295                 300

Val Arg Glu Gly Ile Asn Ile Phe Leu Asp Gly Tyr Val Pro Thr Glu
305                 310                 315                 320

Asn Leu Arg Phe Arg Asp Ala Ser Leu Val Phe Lys Val Ala Glu Thr
                325                 330                 335

Ala Asn Glu Glu Glu Val Lys Lys Met Cys Met Tyr Lys Tyr Pro Gly
            340                 345                 350

Met Lys Lys Lys Met Gly Glu Phe Glu Leu Ala Ile Val Ala Gly Glu
        355                 360                 365

Phe Thr Asp Ser Glu Ile Met Val Met Leu Gly Glu Asn Gly Thr Gly
    370                 375                 380

Lys Thr Thr Phe Ile Arg Met Leu Ala Gly Arg Leu Lys Pro Asp Glu
385                 390                 395                 400

Gly Gly Glu Val Pro Val Leu Asn Val Ser Tyr Lys Pro Gln Lys Ile
                405                 410                 415

Ser Pro Lys Ser Thr Gly Ser Val Arg Gln Leu Leu His Glu Lys Ile
            420                 425                 430

Arg Asp Ala Tyr Thr His Pro Gln Phe Val Thr Asp Val Met Lys Pro
        435                 440                 445

Leu Gln Ile Glu Asn Ile Ile Asp Gln Glu Val Gln Thr Leu Ser Gly
    450                 455                 460

Gly Glu Leu Gln Arg Val Arg Leu Arg Leu Cys Leu Gly Lys Pro Ala
465                 470                 475                 480

Asp Val Tyr Leu Ile Asp Glu Pro Ser Ala Tyr Leu Asp Ser Glu Gln
                485                 490                 495

Arg Leu Met Ala Ala Arg Val Val Lys Arg Phe Ile Leu His Ala Lys
            500                 505                 510

Lys Thr Ala Phe Val Val Glu His Asp Phe Ile Met Ala Thr Tyr Leu
        515                 520                 525

Ala Asp Arg Val Ile Val Phe Asp Gly Val Pro Ser Lys Asn Thr Val
    530                 535                 540

Ala Asn Ser Pro Gln Thr Leu Leu Ala Gly Met Asn Lys Phe Leu Ser
545                 550                 555                 560

Gln Leu Glu Ile Thr Phe Arg Arg Asp Pro Asn Asn Tyr Arg Pro Arg
                565                 570                 575

Ile Asn Lys Leu Asn Ser Ile Lys Asp Val Glu Gln Lys Lys Ser Gly
            580                 585                 590

Asn Tyr Phe Phe Leu Asp Asp
        595

<210> 75
<211> 807
<212> PRT

<213> Homo sapiens

<400> 75

Met Pro Lys Ala Pro Lys Gln Gln Pro Pro Glu Pro Glu Trp Ile Gly
1               5                   10                  15

Asp Gly Glu Ser Thr Ser Pro Ser Asp Lys Val Val Lys Lys Gly Lys
            20                  25                  30

Lys Asp Lys Lys Ile Lys Lys Thr Phe Phe Glu Glu Leu Ala Val Glu
        35                  40                  45

Asp Lys Gln Ala Gly Glu Glu Glu Lys Val Leu Lys Glu Lys Glu Gln
    50                  55                  60

Gln Gln Gln Gln Gln Gln Gln Gln Gln Lys Lys Lys Arg Asp Thr Arg
65                  70                  75                  80

Lys Gly Arg Arg Lys Lys Asp Val Asp Asp Asp Gly Glu Glu Lys Glu
                85                  90                  95

Leu Met Glu Arg Leu Lys Lys Leu Ser Val Pro Thr Ser Asp Glu Glu
            100                 105                 110

Asp Glu Val Pro Ala Pro Lys Pro Arg Gly Gly Lys Lys Thr Lys Gly
        115                 120                 125

Gly Asn Val Phe Ala Ala Leu Ile Gln Asp Gln Ser Glu Glu Glu Glu
    130                 135                 140

Glu Glu Glu Lys His Pro Pro Lys Pro Ala Lys Pro Glu Lys Asn Arg
145                 150                 155                 160

Ile Asn Lys Ala Val Ser Glu Glu Gln Gln Pro Ala Leu Lys Gly Lys
                165                 170                 175

Lys Gly Lys Glu Glu Lys Ser Lys Gly Lys Ala Lys Pro Gln Asn Lys
            180                 185                 190

Phe Ala Ala Leu Asp Asn Glu Glu Glu Asp Lys Glu Glu Glu Ile Ile
        195                 200                 205

Lys Glu Lys Glu Pro Pro Lys Gln Gly Lys Glu Lys Ala Lys Lys Ala
    210                 215                 220

Glu Gln Met Glu Tyr Glu Arg Gln Val Ala Ser Leu Lys Ala Ala Asn
225                 230                 235                 240

Ala Ala Glu Asn Asp Phe Ser Val Ser Gln Ala Glu Met Ser Ser Arg
                245                 250                 255

Gln Ala Met Leu Glu Asn Ala Ser Asp Ile Lys Leu Glu Lys Phe Ser
            260                 265                 270

Ile Ser Ala His Gly Lys Glu Leu Phe Val Asn Ala Asp Leu Tyr Ile
        275                 280                 285

Val Ala Gly Arg Arg Tyr Gly Leu Val Gly Pro Asn Gly Lys Gly Lys
    290                 295                 300

Thr Thr Leu Leu Lys His Ile Ala Asn Arg Ala Leu Ser Ile Pro Pro
305                 310                 315                 320

Asn Ile Asp Val Leu Leu Cys Glu Gln Glu Val Val Ala Asp Glu Thr
                325                 330                 335

Pro Ala Val Gln Ala Val Leu Arg Ala Asp Thr Lys Arg Leu Lys Leu
            340                 345                 350

Leu Glu Glu Glu Arg Arg Leu Gln Gly Gln Leu Glu Gln Gly Asp Asp
        355                 360                 365

Thr Ala Ala Glu Arg Leu Glu Lys Val Tyr Glu Glu Leu Arg Ala Thr
    370                 375                 380

Gly Ala Ala Ala Ala Glu Ala Lys Ala Arg Arg Ile Leu Ala Gly Leu
385                 390                 395                 400

Gly Phe Asp Pro Glu Met Gln Asn Arg Pro Thr Gln Lys Phe Ser Gly
                405                 410                 415

Gly Trp Arg Met Arg Val Ser Leu Ala Arg Ala Leu Phe Met Glu Pro
            420                 425                 430

Thr Leu Leu Met Leu Asp Glu Pro Thr Asn His Leu Asp Leu Asn Ala
        435                 440                 445

Val Ile Trp Leu Asn Asn Tyr Leu Gln Gly Trp Arg Lys Thr Leu Leu
    450                 455                 460

Ile Val Ser His Asp Gln Gly Phe Leu Asp Asp Val Cys Thr Asp Ile
465                 470                 475                 480

Ile His Leu Asp Ala Gln Arg Leu His Tyr Tyr Arg Gly Asn Tyr Met
                485                 490                 495

Thr Phe Lys Lys Met Tyr Gln Gln Lys Gln Lys Glu Leu Leu Lys Gln
            500                 505                 510

Tyr Glu Lys Gln Glu Lys Lys Leu Lys Glu Leu Lys Ala Gly Gly Lys
        515                 520                 525

Ser Thr Lys Gln Ala Glu Lys Gln Thr Lys Glu Ala Leu Thr Arg Lys
    530                 535                 540

Gln Gln Lys Cys Arg Arg Lys Asn Gln Asp Glu Glu Ser Gln Glu Ala
545                 550                 555                 560

Pro Glu Leu Leu Lys Arg Pro Lys Glu Tyr Thr Val Arg Phe Thr Phe
                565                 570                 575

Pro Asp Pro Pro Pro Leu Ser Pro Pro Val Leu Gly Leu His Gly Val
            580                 585                 590

Thr Phe Gly Tyr Gln Gly Gln Lys Pro Leu Phe Lys Asn Leu Asp Phe
        595                 600                 605

Gly Ile Asp Met Asp Ser Arg Ile Cys Ile Val Gly Pro Asn Gly Val
    610                 615                 620

Gly Lys Ser Thr Leu Leu Leu Leu Leu Thr Gly Lys Leu Thr Pro Thr
625                 630                 635                 640

His Gly Glu Met Arg Lys Asn His Arg Leu Lys Ile Gly Phe Phe Asn
                645                 650                 655

Gln Gln Tyr Ala Glu Gln Leu Arg Met Glu Glu Thr Pro Thr Glu Tyr
            660                 665                 670

Leu Gln Arg Gly Phe Asn Leu Pro Tyr Gln Asp Ala Arg Lys Cys Leu
        675                 680                 685

Gly Arg Phe Gly Leu Glu Ser His Ala His Thr Ile Gln Ile Cys Lys
    690                 695                 700

Leu Ser Gly Gly Gln Lys Ala Arg Val Val Phe Ala Glu Leu Ala Cys
705                 710                 715                 720

Arg Glu Pro Asp Val Leu Ile Leu Asp Glu Pro Thr Asn Asn Leu Asp
                725                 730                 735

Ile Glu Ser Ile Asp Ala Leu Gly Glu Ala Ile Asn Glu Tyr Lys Gly
            740                 745                 750

Ala Val Ile Val Val Ser His Asp Ala Arg Leu Ile Thr Glu Thr Asn
        755                 760                 765

Cys Gln Leu Trp Val Val Glu Glu Gln Ser Val Ser Gln Ile Asp Gly
    770                 775                 780

Asp Phe Glu Asp Tyr Lys Arg Glu Val Leu Glu Ala Leu Gly Glu Val
785                 790                 795                 800

Met Val Ser Arg Pro Arg Glu
                805

<210> 76
<211> 634
<212> PRT
<213> Homo sapiens

<400> 76
Met Pro Ser Asp Leu Ala Lys Lys Lys Ala Ala Lys Lys Lys Glu Ala
1               5                   10                  15

Ala Lys Ala Arg Gln Arg Pro Arg Lys Gly His Glu Glu Asn Gly Asp
            20                  25                  30

Val Val Thr Glu Pro Gln Val Ala Glu Lys Asn Glu Ala Asn Gly Arg
        35                  40                  45

Glu Thr Thr Glu Val Asp Leu Leu Thr Lys Glu Leu Glu Asp Phe Glu
    50                  55                  60

Met Lys Lys Ala Ala Ala Arg Ala Val Thr Gly Val Leu Ala Ser His
65                  70                  75                  80

Pro Asn Ser Thr Asp Val His Ile Ile Asn Leu Ser Leu Thr Phe His
                85                  90                  95

Gly Gln Glu Leu Leu Ser Asp Thr Lys Leu Glu Leu Asn Ser Gly Arg
            100                 105                 110

Arg Tyr Gly Leu Ile Gly Leu Asn Gly Ile Gly Lys Ser Met Leu Leu
        115                 120                 125

Ser Ala Ile Gly Lys Arg Glu Val Pro Ile Pro Glu His Ile Asp Ile
    130                 135                 140

Tyr His Leu Thr Arg Glu Met Pro Pro Ser Asp Lys Thr Pro Leu His

145                 150                 155                 160

Cys Val Met Glu Val Asp Thr Glu Arg Ala Met Leu Glu Lys Glu Ala
                165                 170                 175

Glu Arg Leu Ala His Glu Asp Ala Glu Cys Glu Lys Leu Met Glu Leu
            180                 185                 190

Tyr Glu Arg Leu Glu Glu Leu Asp Ala Asp Lys Ala Glu Met Arg Ala
        195                 200                 205

Ser Arg Ile Leu His Gly Leu Gly Phe Thr Pro Ala Met Gln Arg Lys
    210                 215                 220

Lys Leu Lys Asp Phe Ser Gly Gly Trp Arg Met Arg Val Ala Leu Ala
225                 230                 235                 240

Arg Ala Leu Phe Ile Arg Pro Phe Met Leu Leu Leu Asp Glu Pro Thr
                245                 250                 255

Asn His Leu Asp Leu Asp Ala Cys Val Trp Leu Glu Glu Glu Leu Lys
            260                 265                 270

Thr Phe Lys Arg Ile Leu Val Leu Val Ser His Ser Gln Asp Phe Leu
        275                 280                 285

Asn Gly Val Cys Thr Asn Ile Ile His Met His Asn Lys Lys Leu Lys
    290                 295                 300

Tyr Tyr Thr Gly Asn Tyr Asp Gln Tyr Val Lys Thr Arg Leu Glu Leu
305                 310                 315                 320

Glu Glu Asn Gln Met Lys Arg Phe His Trp Glu Gln Asp Gln Ile Ala
                325                 330                 335

His Met Lys Asn Tyr Ile Ala Arg Phe Gly His Gly Ser Ala Lys Leu
            340                 345                 350

Ala Arg Gln Ala Gln Ser Lys Glu Lys Thr Leu Gln Lys Met Met Ala
        355                 360                 365

Ser Gly Leu Thr Glu Arg Val Val Ser Asp Lys Thr Leu Ser Phe Tyr
    370                 375                 380

Phe Pro Pro Cys Gly Lys Ile Pro Pro Pro Val Ile Met Val Gln Asn
385                 390                 395                 400

Val Ser Phe Lys Tyr Thr Lys Asp Gly Pro Cys Ile Tyr Asn Asn Leu

                405                 410                 415

Glu Phe Gly Ile Asp Leu Asp Thr Arg Val Ala Leu Val Gly Pro Asn
            420                 425                 430

Gly Ala Gly Lys Ser Thr Leu Leu Lys Leu Leu Thr Gly Glu Leu Leu
        435                 440                 445

Pro Thr Asp Gly Met Ile Arg Lys His Ser His Val Lys Ile Gly Arg
    450                 455                 460

Tyr His Gln His Leu Gln Glu Gln Leu Asp Leu Asp Leu Ser Pro Leu
465                 470                 475                 480

Glu Tyr Met Met Lys Cys Tyr Pro Glu Ile Lys Glu Lys Glu Glu Met
                485                 490                 495

Arg Lys Ile Ile Gly Arg Tyr Gly Leu Thr Gly Lys Gln Gln Val Ser
            500                 505                 510

Pro Ile Arg Asn Leu Ser Asp Gly Gln Lys Cys Arg Val Cys Leu Ala
        515                 520                 525

Trp Leu Ala Trp Gln Asn Pro His Met Leu Phe Leu Asp Glu Pro Thr
    530                 535                 540

Asn His Leu Asp Ile Glu Thr Ile Asp Ala Leu Ala Asp Ala Ile Asn
545                 550                 555                 560

Glu Phe Glu Gly Gly Met Met Leu Val Ser His Asp Phe Arg Leu Ile
                565                 570                 575

Gln Gln Val Ala Gln Glu Ile Trp Val Cys Glu Lys Gln Thr Ile Thr
            580                 585                 590

Lys Trp Pro Gly Asp Ile Leu Ala Tyr Lys Glu His Leu Lys Ser Lys
        595                 600                 605

Leu Val Asp Glu Glu Pro Gln Leu Thr Lys Arg Thr His Asn Val Cys
    610                 615                 620

Thr Leu Thr Leu Ala Ser Leu Pro Arg Pro
625                 630

<210> 77
<211> 709

<212> PRT
<213> Homo sapiens

<400> 77
Met Ala Thr Cys Ala Glu Ile Leu Arg Ser Glu Phe Pro Glu Ile Asp
1               5                   10                  15

Gly Gln Val Phe Asp Tyr Val Thr Gly Val Leu His Ser Gly Ser Ala
            20                  25                  30

Asp Phe Glu Ser Val Asp Asp Leu Val Glu Ala Val Gly Glu Leu Leu
        35                  40                  45

Gln Glu Val Ser Gly Asp Ser Lys Asp Asp Ala Gly Ile Arg Ala Val
    50                  55                  60

Cys Gln Arg Met Tyr Asn Thr Leu Arg Leu Ala Glu Pro Gln Ser Gln
65                  70                  75                  80

Gly Asn Ser Gln Val Leu Leu Asp Ala Pro Ile Gln Leu Ser Lys Ile
                85                  90                  95

Thr Glu Asn Tyr Asp Cys Gly Thr Lys Leu Pro Gly Leu Leu Lys Arg
            100                 105                 110

Glu Gln Ser Ser Thr Val Asn Ala Lys Lys Leu Glu Lys Ala Glu Ala
        115                 120                 125

Arg Leu Lys Ala Lys Gln Glu Lys Arg Ser Glu Lys Asp Thr Leu Lys
    130                 135                 140

Thr Ser Asn Pro Leu Val Leu Glu Glu Ala Ser Ala Ser Gln Ala Gly
145                 150                 155                 160

Ser Arg Lys Glu Ser Arg Leu Glu Ser Ser Gly Lys Asn Lys Ser Tyr
                165                 170                 175

Asp Val Arg Ile Glu Asn Phe Asp Val Ser Phe Gly Asp Arg Val Leu
            180                 185                 190

Leu Ala Gly Ala Asp Val Asn Leu Ala Trp Gly Arg Arg Tyr Gly Leu
        195                 200                 205

Val Gly Arg Asn Gly Leu Gly Lys Thr Thr Leu Leu Lys Met Leu Ala
    210                 215                 220

Thr Arg Ser Leu Arg Val Pro Ala His Ile Ser Leu Leu His Val Glu
225                 230                 235                 240

Gln Glu Val Ala Gly Asp Asp Thr Pro Ala Leu Gln Ser Val Leu Glu
                245                 250                 255

Ser Asp Ser Val Arg Glu Asp Leu Leu Arg Arg Glu Arg Glu Leu Thr
            260                 265                 270

Ala Gln Ile Ala Ala Gly Arg Ala Glu Gly Ser Glu Ala Ala Glu Leu
        275                 280                 285

Ala Glu Ile Tyr Ala Lys Leu Glu Glu Ile Glu Ala Asp Lys Ala Pro
    290                 295                 300

Ala Arg Ala Ser Val Ile Leu Ala Gly Leu Gly Phe Thr Pro Lys Met
305                 310                 315                 320

Gln Gln Gln Pro Thr Arg Glu Phe Ser Gly Gly Trp Arg Met Arg Leu
                325                 330                 335

Ala Leu Ala Arg Ala Leu Phe Ala Arg Pro Asp Leu Leu Leu Leu Asp
            340                 345                 350

Glu Pro Thr Asn Met Leu Asp Val Arg Ala Ile Leu Trp Leu Glu Asn
        355                 360                 365

Tyr Leu Gln Thr Trp Pro Ser Thr Ile Leu Val Val Ser His Asp Arg
    370                 375                 380

Asn Phe Leu Asn Ala Ile Ala Thr Asp Ile Ile His Leu His Ser Gln
385                 390                 395                 400

Arg Leu Asp Gly Tyr Arg Gly Asp Phe Glu Thr Phe Ile Lys Ser Lys
                405                 410                 415

Gln Glu Arg Leu Leu Asn Gln Gln Arg Glu Tyr Glu Ala Gln Gln Gln
            420                 425                 430

Tyr Arg Gln His Ile Gln Val Phe Ile Asp Arg Phe Arg Tyr Asn Ala
        435                 440                 445

Asn Arg Ala Ser Gln Val Gln Ser Lys Leu Lys Met Leu Glu Lys Leu
    450                 455                 460

Pro Glu Leu Arg Pro Val Asp Lys Glu Ser Glu Val Val Met Lys Phe
465                 470                 475                 480

Pro Asp Gly Phe Glu Lys Phe Ser Pro Pro Ile Leu Gln Leu Asp Glu
                485                 490                 495

Val Asp Phe Tyr Tyr Asp Pro Lys His Val Ile Phe Ser Arg Leu Ser
            500                 505                 510

Val Ser Ala Asp Leu Glu Ser Arg Ile Cys Val Val Gly Glu Asn Gly
        515                 520                 525

Ala Gly Lys Ser Thr Met Leu Lys Leu Leu Leu Gly Asp Leu Ala Pro
    530                 535                 540

Val Arg Gly Ile Arg His Ala His Arg Asn Leu Lys Ile Gly Tyr Phe
545                 550                 555                 560

Ser Gln His His Val Glu Gln Leu Asp Leu Asn Val Ser Ala Val Glu
                565                 570                 575

Leu Leu Ala Arg Lys Phe Pro Gly Arg Pro Glu Glu Glu Tyr Arg His
            580                 585                 590

Gln Leu Gly Arg Tyr Gly Ile Ser Gly Glu Leu Ala Met Arg Pro Leu
        595                 600                 605

Ala Ser Leu Ser Gly Gly Gln Lys Ser Arg Val Ala Phe Ala Gln Met
    610                 615                 620

Thr Met Pro Cys Pro Asn Phe Tyr Ile Leu Asp Glu Pro Thr Asn His
625                 630                 635                 640

Leu Asp Met Glu Thr Ile Glu Ala Leu Gly Arg Ala Leu Asn Asn Phe
                645                 650                 655

Arg Gly Gly Val Ile Leu Val Ser His Asp Glu Arg Phe Ile Arg Leu
            660                 665                 670

Val Cys Arg Glu Leu Trp Val Cys Glu Gly Gly Gly Val Thr Arg Val
        675                 680                 685

Glu Gly Gly Phe Asp Gln Tyr Arg Ala Leu Leu Gln Glu Gln Phe Arg
    690                 695                 700

Arg Glu Gly Phe Leu
705

<210> 78
<211> 674
<212> PRT

<213> Homo sapiens

<400> 78
Met Ala Ala Phe Ser Val Gly Thr Ala Met Asn Ala Ser Ser Tyr Ser
1               5                   10                  15

Ala Glu Met Thr Glu Pro Lys Ser Val Cys Val Ser Val Asp Glu Val
            20                  25                  30

Val Ser Ser Asn Met Glu Ala Thr Glu Thr Asp Leu Leu Asn Gly His
        35                  40                  45

Leu Lys Lys Val Asp Asn Asn Leu Thr Glu Ala Gln Arg Phe Ser Ser
    50                  55                  60

Leu Pro Arg Arg Ala Ala Val Asn Ile Glu Phe Arg Asp Leu Ser Tyr
65                  70                  75                  80

Ser Val Pro Glu Gly Pro Trp Trp Arg Lys Lys Gly Tyr Lys Thr Leu
                85                  90                  95

Leu Lys Gly Ile Ser Gly Lys Phe Asn Ser Gly Glu Leu Val Ala Ile
            100                 105                 110

Met Gly Pro Ser Gly Ala Gly Lys Ser Thr Leu Met Asn Ile Leu Ala
        115                 120                 125

Gly Tyr Arg Glu Thr Gly Met Lys Gly Ala Val Leu Ile Asn Gly Leu
    130                 135                 140

Pro Arg Asp Leu Arg Cys Phe Arg Lys Val Ser Cys Tyr Ile Met Gln
145                 150                 155                 160

Asp Asp Met Leu Leu Pro His Leu Thr Val Gln Glu Ala Met Met Val
                165                 170                 175

Ser Ala His Leu Lys Leu Gln Glu Lys Asp Glu Gly Arg Arg Glu Met
            180                 185                 190

Val Lys Glu Ile Leu Thr Ala Leu Gly Leu Leu Ser Cys Ala Asn Thr
        195                 200                 205

Arg Thr Gly Ser Leu Ser Gly Gly Gln Arg Lys Arg Leu Ala Ile Ala
    210                 215                 220

Leu Glu Leu Val Asn Asn Pro Pro Val Met Phe Phe Asp Glu Pro Thr
225                 230                 235                 240

Ser Gly Leu Asp Ser Ala Ser Cys Phe Gln Val Val Ser Leu Met Lys
                245                 250                 255

Gly Leu Ala Gln Gly Gly Arg Ser Ile Ile Cys Thr Ile His Gln Pro
            260                 265                 270

Ser Ala Lys Leu Phe Glu Leu Phe Asp Gln Leu Tyr Val Leu Ser Gln
        275                 280                 285

Gly Gln Cys Val Tyr Arg Gly Lys Val Cys Asn Leu Val Pro Tyr Leu
    290                 295                 300

Arg Asp Leu Gly Leu Asn Cys Pro Thr Tyr His Asn Pro Ala Asp Phe
305                 310                 315                 320

Val Met Glu Val Ala Ser Gly Glu Tyr Gly Asp Gln Asn Ser Arg Leu
                325                 330                 335

Val Arg Ala Val Arg Glu Gly Met Cys Asp Ser Asp His Lys Arg Asp
            340                 345                 350

Leu Gly Gly Asp Ala Glu Val Asn Pro Phe Leu Trp His Arg Pro Ser
        355                 360                 365

Glu Glu Val Lys Gln Thr Lys Arg Leu Lys Gly Leu Arg Lys Asp Ser
    370                 375                 380

Ser Ser Met Glu Gly Cys His Ser Phe Ser Ala Ser Cys Leu Thr Gln
385                 390                 395                 400

Phe Cys Ile Leu Phe Lys Arg Thr Phe Leu Ser Ile Met Arg Asp Ser
                405                 410                 415

Val Leu Thr His Leu Arg Ile Thr Ser His Ile Gly Ile Gly Leu Leu
            420                 425                 430

Ile Gly Leu Leu Tyr Leu Gly Ile Gly Asn Glu Thr Lys Lys Val Leu
        435                 440                 445

Ser Asn Ser Gly Phe Leu Phe Phe Ser Met Leu Phe Leu Met Phe Ala
    450                 455                 460

Ala Leu Met Pro Thr Val Leu Thr Phe Pro Leu Glu Met Gly Val Phe
465                 470                 475                 480

Leu Arg Glu His Leu Asn Tyr Trp Tyr Ser Leu Lys Ala Tyr Tyr Leu
                485                 490                 495

Ala Lys Thr Met Ala Asp Val Pro Phe Gln Ile Met Phe Pro Val Ala
            500                 505                 510

Tyr Cys Ser Ile Val Tyr Trp Met Thr Ser Gln Pro Ser Asp Ala Val
        515                 520                 525

Arg Phe Val Leu Phe Ala Ala Leu Gly Thr Met Thr Ser Leu Val Ala
    530                 535                 540

Gln Ser Leu Gly Leu Leu Ile Gly Ala Ala Ser Thr Ser Leu Gln Val
545                 550                 555                 560

Ala Thr Phe Val Gly Pro Val Thr Ala Ile Pro Val Leu Leu Phe Ser
                565                 570                 575

Gly Phe Phe Val Ser Phe Asp Thr Ile Pro Thr Tyr Leu Gln Trp Met
            580                 585                 590

Ser Tyr Ile Ser Tyr Val Arg Tyr Gly Phe Glu Gly Val Ile Leu Ser
        595                 600                 605

Ile Tyr Gly Leu Asp Arg Glu Asp Leu His Cys Asp Ile Asp Glu Thr
    610                 615                 620

Cys His Phe Gln Lys Ser Glu Ala Ile Leu Arg Glu Leu Asp Val Glu
625                 630                 635                 640

Asn Ala Lys Leu Tyr Leu Asp Phe Ile Val Leu Gly Ile Phe Phe Ile
                645                 650                 655

Ser Leu Arg Leu Ile Ala Tyr Leu Val Leu Arg Tyr Lys Ile Arg Ala
            660                 665                 670

Glu Arg

<210> 79
<211> 655
<212> PRT
<213> Homo sapiens

<400> 79
Met Ser Ser Ser Asn Val Glu Val Phe Ile Pro Val Ser Gln Gly Asn
1               5                   10                  15

Thr Asn Gly Phe Pro Ala Thr Val Ser Asn Asp Leu Lys Ala Phe Thr

            20                  25                  30

Glu Gly Ala Val Leu Ser Phe His Asn Ile Cys Tyr Arg Val Lys Leu
        35                  40                  45

Lys Ser Gly Phe Leu Pro Cys Arg Lys Pro Val Glu Lys Glu Ile Leu
    50                  55                  60

Ser Asn Ile Asn Gly Ile Met Lys Pro Gly Leu Asn Ala Ile Leu Gly
65                  70                  75                  80

Pro Thr Gly Gly Gly Lys Ser Ser Leu Leu Asp Val Leu Ala Ala Arg
                85                  90                  95

Lys Asp Pro Ser Gly Leu Ser Gly Asp Val Leu Ile Asn Gly Ala Pro
            100                 105                 110

Arg Pro Ala Asn Phe Lys Cys Asn Ser Gly Tyr Val Val Gln Asp Asp
        115                 120                 125

Val Val Met Gly Thr Leu Thr Val Arg Glu Asn Leu Gln Phe Ser Ala
    130                 135                 140

Ala Leu Arg Leu Ala Thr Thr Met Thr Asn His Glu Lys Asn Glu Arg
145                 150                 155                 160

Ile Asn Arg Val Ile Glu Glu Leu Gly Leu Asp Lys Val Ala Asp Ser
                165                 170                 175

Lys Val Gly Thr Gln Phe Ile Arg Gly Val Ser Gly Gly Glu Arg Lys
            180                 185                 190

Arg Thr Ser Ile Gly Met Glu Leu Ile Thr Asp Pro Ser Ile Leu Ser
        195                 200                 205

Leu Asp Glu Pro Thr Thr Gly Leu Asp Ser Ser Thr Ala Asn Ala Val
    210                 215                 220

Leu Leu Leu Leu Lys Arg Met Ser Lys Gln Gly Arg Thr Ile Ile Phe
225                 230                 235                 240

Ser Ile His Gln Pro Arg Tyr Ser Ile Phe Lys Leu Phe Asp Ser Leu
                245                 250                 255

Thr Leu Leu Ala Ser Gly Arg Leu Met Phe His Gly Pro Ala Gln Glu
            260                 265                 270

Ala Leu Gly Tyr Phe Glu Ser Ala Gly Tyr His Cys Glu Ala Tyr Asn

275                 280                 285

Asn Pro Ala Asp Phe Phe Leu Asp Ile Ile Asn Gly Asp Ser Thr Ala
    290                 295                 300

Val Ala Leu Asn Arg Glu Glu Asp Phe Lys Ala Thr Glu Ile Ile Glu
305                 310                 315                 320

Pro Ser Lys Gln Asp Lys Pro Leu Ile Glu Lys Leu Ala Glu Ile Tyr
                325                 330                 335

Val Asn Ser Ser Phe Tyr Lys Glu Thr Lys Ala Glu Leu His Gln Leu
            340                 345                 350

Ser Gly Gly Glu Lys Lys Lys Lys Ile Thr Val Phe Lys Glu Ile Ser
        355                 360                 365

Tyr Thr Thr Ser Phe Cys His Gln Leu Arg Trp Val Ser Lys Arg Ser
    370                 375                 380

Phe Lys Asn Leu Leu Gly Asn Pro Gln Ala Ser Ile Ala Gln Ile Ile
385                 390                 395                 400

Val Thr Val Val Leu Gly Leu Val Ile Gly Ala Ile Tyr Phe Gly Leu
                405                 410                 415

Lys Asn Asp Ser Thr Gly Ile Gln Asn Arg Ala Gly Val Leu Phe Phe
            420                 425                 430

Leu Thr Thr Asn Gln Cys Phe Ser Ser Val Ser Ala Val Glu Leu Phe
        435                 440                 445

Val Val Glu Lys Lys Leu Phe Ile His Glu Tyr Ile Ser Gly Tyr Tyr
    450                 455                 460

Arg Val Ser Ser Tyr Phe Leu Gly Lys Leu Leu Ser Asp Leu Leu Pro
465                 470                 475                 480

Met Arg Met Leu Pro Ser Ile Ile Phe Thr Cys Ile Val Tyr Phe Met
                485                 490                 495

Leu Gly Leu Lys Pro Lys Ala Asp Ala Phe Phe Val Met Met Phe Thr
            500                 505                 510

Leu Met Met Val Ala Tyr Ser Ala Ser Ser Met Ala Leu Ala Ile Ala
        515                 520                 525

Ala Gly Gln Ser Val Val Ser Val Ala Thr Leu Leu Met Thr Ile Cys

530                 535                 540

Phe Val Phe Met Met Ile Phe Ser Gly Leu Leu Val Asn Leu Thr Thr
545                 550                 555                 560

Ile Ala Ser Trp Leu Ser Trp Leu Gln Tyr Phe Ser Ile Pro Arg Tyr
                565                 570                 575

Gly Phe Thr Ala Leu Gln His Asn Glu Phe Leu Gly Gln Asn Phe Cys
            580                 585                 590

Pro Gly Leu Asn Ala Thr Gly Asn Asn Pro Cys Asn Tyr Ala Thr Cys
        595                 600                 605

Thr Gly Glu Glu Tyr Leu Val Lys Gln Gly Ile Asp Leu Ser Pro Trp
    610                 615                 620

Gly Leu Trp Lys Asn His Val Ala Leu Ala Cys Met Ile Val Ile Phe
625                 630                 635                 640

Leu Thr Ile Ala Tyr Leu Lys Leu Leu Phe Leu Lys Lys Tyr Ser
                645                 650                 655

<210> 80
<211> 649
<212> PRT
<213> Homo sapiens

<400> 80
Met Gly Asp Leu Ser Ser Leu Thr Pro Gly Gly Ser Met Gly Leu Gln
1               5                   10                  15

Val Asn Arg Gly Ser Gln Ser Ser Leu Glu Gly Ala Pro Ala Thr Ala
            20                  25                  30

Pro Glu Pro His Ser Leu Gly Ile Leu His Ala Ser Tyr Ser Val Ser
        35                  40                  45

His Arg Val Arg Pro Trp Trp Asp Ile Thr Ser Cys Arg Gln Gln Trp
    50                  55                  60

Thr Arg Gln Ile Leu Lys Asp Val Ser Leu Tyr Val Glu Ser Gly Gln
65                  70                  75                  80

Ile Met Cys Ile Leu Gly Ser Ser Gly Ser Gly Lys Thr Thr Leu Leu
                85                  90                  95

Asp Ala Met Ser Gly Arg Leu Gly Arg Ala Gly Thr Phe Leu Gly Glu
            100                 105                 110

Val Tyr Val Asn Gly Arg Ala Leu Arg Arg Glu Gln Phe Gln Asp Cys
        115                 120                 125

Phe Ser Tyr Val Leu Gln Ser Asp Thr Leu Leu Ser Ser Leu Thr Val
    130                 135                 140

Arg Glu Thr Leu His Tyr Thr Ala Leu Leu Ala Ile Arg Arg Gly Asn
145                 150                 155                 160

Pro Gly Ser Phe Gln Lys Lys Val Glu Ala Val Met Ala Glu Leu Ser
                165                 170                 175

Leu Ser His Val Ala Asp Arg Leu Ile Gly Asn Tyr Ser Leu Gly Gly
            180                 185                 190

Ile Ser Thr Gly Glu Arg Arg Arg Val Ser Ile Ala Ala Gln Leu Leu
        195                 200                 205

Gln Asp Pro Lys Val Met Leu Phe Pro Thr Thr Gly Leu Asp Cys Met
    210                 215                 220

Thr Ala Asn Gln Ile Val Val Leu Leu Val Glu Leu Ala Arg Arg Asn
225                 230                 235                 240

Arg Ile Val Val Leu Thr Ile His Gln Pro Arg Ser Glu Leu Phe Gln
                245                 250                 255

Leu Phe Asp Lys Ile Ala Ile Leu Ser Phe Gly Glu Leu Ile Phe Cys
            260                 265                 270

Gly Thr Pro Ala Glu Met Leu Asp Phe Phe Asn Asp Cys Gly Tyr Pro
        275                 280                 285

Cys Pro Glu His Ser Asn Pro Phe Asp Phe Tyr Met Asp Leu Thr Ser
    290                 295                 300

Val Asp Thr Gln Ser Lys Glu Arg Glu Ile Glu Thr Ser Lys Arg Val
305                 310                 315                 320

Gln Met Ile Glu Ser Ala Tyr Lys Lys Ser Ala Ile Cys His Lys Thr
                325                 330                 335

Leu Lys Asn Ile Glu Arg Met Lys His Leu Lys Thr Leu Pro Met Val
            340                 345                 350

Pro Phe Lys Thr Lys Asp Ser Pro Gly Val Phe Ser Lys Leu Gly Val
        355                 360                 365

Leu Leu Arg Arg Val Thr Arg Asn Leu Val Arg Asn Lys Leu Ala Val
    370                 375                 380

Ile Thr Arg Leu Leu Gln Asn Leu Ile Met Gly Leu Phe Leu Leu Phe
385                 390                 395                 400

Phe Val Leu Arg Val Arg Ser Asn Val Leu Lys Gly Ala Ile Gln Asp
                405                 410                 415

Arg Val Gly Leu Leu Tyr Gln Phe Val Gly Ala Thr Pro Tyr Thr Gly
            420                 425                 430

Met Leu Asn Ala Val Asn Leu Phe Pro Val Leu Arg Ala Val Ser Asp
        435                 440                 445

Gln Glu Ser Gln Asp Gly Leu Tyr Gln Lys Trp Gln Met Met Leu Ala
    450                 455                 460

Tyr Ala Leu His Val Leu Pro Phe Ser Val Val Ala Thr Met Ile Phe
465                 470                 475                 480

Ser Ser Val Cys Tyr Trp Thr Leu Gly Leu His Pro Glu Val Ala Arg
                485                 490                 495

Phe Gly Tyr Phe Ser Ala Ala Leu Leu Ala Pro His Leu Ile Gly Glu
            500                 505                 510

Phe Leu Thr Leu Val Leu Leu Gly Ile Val Gln Asn Pro Asn Ile Val
        515                 520                 525

Asn Ser Val Val Ala Leu Leu Ser Ile Ala Gly Val Leu Val Gly Ser
    530                 535                 540

Gly Phe Leu Arg Asn Ile Gln Glu Met Pro Ile Pro Phe Lys Ile Ile
545                 550                 555                 560

Ser Tyr Phe Thr Phe Gln Lys Tyr Cys Ser Glu Ile Leu Val Val Asn
                565                 570                 575

Glu Phe Tyr Gly Leu Asn Phe Thr Cys Gly Ser Ser Asn Val Ser Val
            580                 585                 590

Thr Thr Asn Pro Met Cys Ala Phe Thr Gln Gly Ile Gln Phe Ile Glu
        595                 600                 605

Lys Thr Cys Pro Gly Ala Thr Ser Arg Phe Thr Met Asn Phe Leu Ile
    610                 615                 620

Leu Tyr Ser Phe Ile Pro Ala Leu Val Ile Leu Gly Ile Val Val Phe
625                 630                 635                 640

Lys Ile Arg Asp His Leu Ile Ser Arg
                645

<210> 81
<211> 673
<212> PRT
<213> Homo sapiens

<400> 81

<210> 82
<211> 590
<212> PRT
<213> Streptomyces lincolnensis

<400> 82
Met Glu Arg Gly Pro Gln Met Ala Asn Arg Ile Glu Gly Lys Ala Val
1               5                   10                  15

Asp Lys Thr Ser Ile Lys His Phe Val Lys Leu Ile Arg Ala Ala Lys
            20                  25                  30

Pro Arg Tyr Leu Phe Phe Val Ile Gly Ile Val Ala Gly Ile Ile Gly
        35                  40                  45

Thr Leu Ile Gln Leu Gln Val Pro Lys Met Val Gln Pro Leu Ile Asn
    50                  55                  60

Ser Phe Gly His Gly Val Asn Gly Gly Lys Val Ala Leu Val Ile Ala
65                  70                  75                  80

Leu Tyr Ile Gly Ser Ala Ala Val Ser Ala Ile Ala Ala Ile Val Leu
                85                  90                  95

Gly Ile Phe Gly Glu Ser Val Val Lys Asn Leu Arg Thr Arg Val Trp
            100                 105                 110

Asp Lys Met Ile His Leu Pro Val Lys Tyr Phe Asp Glu Val Lys Thr
        115                 120                 125

Gly Glu Met Ser Ser Arg Leu Ala Asn Asp Thr Thr Gln Val Lys Asn
    130                 135                 140

Leu Ile Ala Asn Ser Ile Pro Gln Ala Phe Thr Ser Ile Leu Leu Leu
145                 150                 155                 160

Val Gly Ser Ile Ile Phe Met Leu Gln Met Gln Trp Arg Leu Thr Leu
                165                 170                 175

Ala Met Ile Ile Ala Val Pro Ile Val Met Leu Ile Met Phe Pro Ile
            180                 185                 190

Met Thr Phe Gly Gln Lys Ile Gly Trp Thr Arg Gln Asp Ser Leu Ala

        195                 200                 205

Asn Phe Gln Gly Ile Ala Ser Glu Ser Leu Ser Glu Ile Arg Leu Val
    210                 215                 220

Lys Ser Ser Asn Ala Glu Lys Gln Ala Ser Lys Lys Ala Glu Asn Asp
225                 230                 235                 240

Val Asn Ala Leu Tyr Lys Ile Gly Val Lys Glu Ala Val Phe Asp Gly
                245                 250                 255

Leu Met Ser Pro Val Met Met Leu Ser Met Met Leu Met Ile Phe Gly
            260                 265                 270

Leu Leu Ala Tyr Gly Ile Tyr Leu Ile Ser Thr Gly Val Met Ser Leu
        275                 280                 285

Gly Thr Leu Leu Gly Met Met Met Tyr Leu Met Asn Leu Ile Gly Val
    290                 295                 300

Val Pro Thr Val Ala Thr Phe Phe Thr Glu Leu Ala Lys Ala Ser Gly
305                 310                 315                 320

Ser Thr Gly Arg Leu Thr Glu Leu Leu Asp Glu Glu Gln Glu Val Leu
                325                 330                 335

His Gln Gly Asp Ser Leu Asp Leu Glu Gly Lys Thr Leu Ser Ala His
            340                 345                 350

His Val Asp Phe Ala Tyr Asp Asp Ser Glu Gln Ile Leu His Asp Ile
        355                 360                 365

Ser Phe Glu Ala Gln Pro Asn Ser Ile Ile Ala Phe Ala Gly Pro Ser
    370                 375                 380

Gly Gly Gly Lys Ser Thr Ile Phe Ser Leu Leu Glu Arg Phe Tyr Gln
385                 390                 395                 400

Pro Thr Ala Gly Glu Ile Thr Ile Gly Gly Gln Pro Ile Asp Ser Val
                405                 410                 415

Ser Leu Glu Asn Trp Arg Ser Gln Ile Gly Phe Val Ser Gln Asp Ser
            420                 425                 430

Ala Ile Met Ala Gly Thr Ile Arg Glu Asn Leu Thr Tyr Gly Leu Glu
        435                 440                 445

Gly Asn Phe Thr Asp Glu Asp Leu Trp Gln Val Leu Asp Leu Ala Phe

450                 455                 460

Ala Arg Ser Phe Val Glu Asn Met Pro Asp Gln Leu Asn Thr Glu Val
465                 470                 475                 480

Gly Glu Arg Gly Val Lys Ile Ser Gly Gly Gln Arg Gln Arg Leu Ala
                485                 490                 495

Ile Ala Arg Ala Phe Leu Arg Asn Pro Lys Ile Leu Met Leu Asp Glu
            500                 505                 510

Ala Thr Ala Ser Leu Asp Ser Glu Ser Glu Ser Met Val Gln Arg Ala
        515                 520                 525

Leu Asp Ser Leu Met Lys Gly Arg Thr Thr Leu Val Ile Ala His Arg
    530                 535                 540

Leu Ser Thr Ile Val Asp Ala Asp Lys Ile Tyr Phe Ile Glu Lys Gly
545                 550                 555                 560

Glu Ile Thr Gly Ser Gly Lys His Asn Glu Leu Val Ala Thr His Pro
                565                 570                 575

Leu Tyr Ala Lys Tyr Val Ser Glu Gln Leu Thr Val Gly Gln
            580                 585                 590

<210> 83
<211> 330
<212> PRT
<213> Streptomyces peucetius

<400> 83
Met Asn Thr Gln Pro Thr Arg Ala Ile Glu Thr Ser Gly Leu Val Lys
1               5                   10                  15

Val Tyr Asn Gly Thr Arg Ala Val Asp Gly Leu Asp Leu Asn Val Pro
            20                  25                  30

Ala Gly Leu Val Tyr Gly Ile Leu Gly Pro Asn Gly Ala Gly Lys Ser
        35                  40                  45

Thr Thr Ile Arg Met Leu Ala Thr Leu Leu Arg Pro Asp Gly Gly Thr
    50                  55                  60

Ala Arg Val Phe Gly His Asp Val Thr Ser Glu Pro Asp Thr Val Arg
65                  70                  75                  80

Arg Arg Ile Ser Val Thr Gly Gln Tyr Ala Ser Val Asp Glu Gly Leu
                85                  90                  95

Thr Gly Thr Glu Asn Leu Val Met Met Gly Arg Leu Gln Gly Tyr Ser
            100                 105                 110

Trp Ala Arg Ala Arg Glu Arg Ala Ala Glu Leu Ile Asp Gly Phe Gly
        115                 120                 125

Leu Gly Asp Ala Arg Asp Arg Leu Leu Lys Thr Tyr Ser Gly Gly Met
    130                 135                 140

Arg Arg Arg Leu Asp Ile Ala Ala Ser Ile Val Val Thr Pro Asp Leu
145                 150                 155                 160

Leu Phe Leu Asp Glu Pro Thr Thr Gly Leu Asp Pro Arg Ser Arg Asn
                165                 170                 175

Gln Val Trp Asp Ile Val Arg Ala Leu Val Asp Ala Gly Thr Thr Val
            180                 185                 190

Leu Leu Thr Thr Gln Tyr Leu Asp Glu Ala Asp Gln Leu Ala Asp Arg
        195                 200                 205

Ile Ala Val Ile Asp His Gly Arg Val Ile Ala Glu Gly Thr Thr Gly
    210                 215                 220

Glu Leu Lys Ser Ser Leu Gly Ser Asn Val Leu Arg Leu Arg Leu His
225                 230                 235                 240

Asp Ala Gln Ser Arg Ala Glu Ala Glu Arg Leu Leu Ser Ala Glu Leu
                245                 250                 255

Gly Val Thr Ile His Arg Asp Ser Asp Pro Thr Ala Leu Ser Ala Arg
            260                 265                 270

Ile Asp Asp Pro Arg Gln Gly Met Arg Ala Leu Ala Glu Leu Ser Arg
        275                 280                 285

Thr His Leu Glu Val Arg Ser Phe Ser Leu Gly Gln Ser Ser Leu Asp
    290                 295                 300

Glu Val Phe Leu Ala Leu Thr Gly His Pro Ala Asp Asp Arg Ser Thr
305                 310                 315                 320

Glu Glu Ala Ala Glu Glu Glu Lys Val Ala
                325                 330

<210> 84
<211> 569
<212> PRT
<213> Streptomyces coelicolor

<400> 84
Met Gln Asn Ala His Arg Ser Asp Thr Gly Ala Ala Ala Leu Thr Gly
1               5                   10                  15

Thr Pro Glu Lys Leu Leu Pro Thr Gln Pro Glu Thr Gly Ser Phe Gln
            20                  25                  30

Val Val Leu Asp Asp Val Val Arg Ala Pro Gly Gly Arg Pro Leu Leu
        35                  40                  45

Asp Gly Val Asn Gln Ser Val Ala Leu Gly Glu Arg Val Gly Ile Ile
    50                  55                  60

Gly Glu Asn Gly Ser Gly Lys Ser Thr Leu Leu Arg Met Leu Ala Gly
65                  70                  75                  80

Val Asp Arg Pro Asp Gly Gly Gln Val Leu Val Arg Ala Pro Gly Gly
                85                  90                  95

Cys Gly Tyr Leu Pro Gln Thr Pro Asp Leu Pro Pro Glu Asp Thr Val
            100                 105                 110

Gln Asp Ala Ile Asp His Ala Leu Ala Glu Leu Arg Ser Leu Glu Arg
        115                 120                 125

Gly Leu Arg Glu Ala Glu Gln Ala Leu Ala Gly Ala Glu Pro Glu Glu
    130                 135                 140

Leu Glu Gly Leu Leu Gly Ala Tyr Gly Asp Leu Leu Glu Ala Phe Glu
145                 150                 155                 160

Ala Arg Asp Gly Tyr Ala Ala Asp Ala Arg Val Asp Ala Ala Met His
                165                 170                 175

Gly Leu Gly Leu Ala Gly Ile Thr Gly Asp Arg Arg Leu Gly Ser Leu
            180                 185                 190

Ser Gly Gly Glu Gln Ala Arg Leu Asn Leu Ala Cys Leu Leu Ala Ala
        195                 200                 205

Ser Pro Gln Leu Met Leu Leu Asp Glu Pro Thr Asn His Leu Asp Val
    210                 215                 220

Gly Ala Leu Glu Trp Leu Glu Glu Arg Leu Arg Ala His Arg Gly Ser
225                 230                 235                 240

Val Leu Val Val Ser His Asp Arg Val Phe Leu Glu Arg Val Ala Thr
                245                 250                 255

Ala Leu Trp Glu Val Asp Gly Glu Arg Arg Thr Val Asn Arg His Gly
            260                 265                 270

Gly Gly Tyr Ala Gly Tyr Leu Gln Ala Lys Ala Ala Ala Arg Arg Arg
        275                 280                 285

Trp Glu Gln Ala Tyr Gln Asp Trp Leu Glu Asp Leu Ala Arg Gln Arg
    290                 295                 300

Glu Leu Ala Arg Ser Ala Ala Asp His Leu Ala Thr Gly Pro Arg Arg
305                 310                 315                 320

Asn Thr Glu Arg Ser Asn Gln Arg His Gln Arg Asn Val Glu Lys Gln
                325                 330                 335

Ile Ser Ala Arg Val Arg Asn Ala Lys Glu Arg Val Arg Arg Leu Glu
            340                 345                 350

Glu Asn Pro Val Pro Arg Pro Pro Gln Pro Met Arg Phe Arg Ala Arg
        355                 360                 365

Val Glu Gly Gly Gly Thr Val Gly Arg Gly Gly Ala Leu Ala Glu Leu
    370                 375                 380

Tyr Lys Val Thr Val Gly Thr Arg Leu Asp Val Pro Ser Phe Thr Val
385                 390                 395                 400

Asp Pro Gly Glu Arg Ile Leu Ile Thr Gly His Asn Gly Ala Gly Lys
                405                 410                 415

Ser Thr Leu Leu Arg Val Leu Ala Gly Asp Leu Ala Pro Asp Gln Gly
            420                 425                 430

Glu Cys Glu Arg Pro Glu Arg Ile Gly Trp Leu Pro Gln Glu Thr Glu
        435                 440                 445

Ile Thr Asp Arg Gln Gln Ser Leu Leu Ala Ala Phe Ala Ala Gly Leu
    450                 455                 460

Pro Gly Ile Ala Glu Glu His Arg Gly Ala Leu Leu Gly Phe Gly Leu
465                 470                 475                 480

Phe Arg Pro Ser Ala Leu Gly Thr Ala Val Gly Asp Leu Ser Thr Gly
                485                 490                 495

Gln Leu Arg Arg Leu Ala Leu Ala Arg Leu Leu Arg Asp Pro Ala Asp
            500                 505                 510

Leu Leu Leu Leu Asp Glu Pro Thr Asn His Leu Ser Pro Ala Leu Val
        515                 520                 525

Glu Asp Leu Glu Glu Ala Leu Ala His Tyr Arg Gly Ala Leu Val Val
    530                 535                 540

Val Ser His Asp Arg Met Phe Ala Gln Arg Phe Thr Gly Arg Arg Met
545                 550                 555                 560

His Met Glu Gly Gly Arg Phe Val Glu
                565

<210> 85
<211> 1025
<212> PRT
<213> Plasmodium falciparum

<400> 85
Met Asp Val Ser Asn Tyr Glu Tyr Leu Arg Ser Tyr Gly Ile Lys Asn
  1               5                  10                  15

Glu Leu Lys Arg Lys Arg Thr His Lys Lys Ile Ile Ile Tyr His Leu
             20                  25                  30

Leu Asp Ile Ile Ile Phe Phe Leu Leu Phe Phe Ser Cys Tyr Asn Phe
         35                  40                  45

Asn Leu Glu Leu Cys Tyr Lys Tyr Glu Lys Ala Ile Phe Tyr Asn Phe
     50                  55                  60

Phe Lys Ser Ser Val Asp Leu Phe Leu Leu Asn Val Ile Arg Ile Ile
 65                  70                  75                  80

Tyr Thr Val Ile Leu Phe Arg Leu His Lys Lys Leu Thr Glu Leu Asn
                 85                  90                  95

Thr Leu Gly Lys Val Tyr Val Leu Ser Arg His Ile Thr Gly Ile Leu

            100                 105                 110

Val Ile Leu Asn Val Ile Lys Met Ile Asn Tyr Ser Tyr Val Ile Lys
        115                 120                 125

Ser Glu Asn Pro Leu Tyr Asn Thr Asn Met Tyr Leu Ile Thr Leu Lys
    130                 135                 140

Val Leu Phe Met Val Tyr Ser Met Ile Ser Ser Ile Tyr Tyr Tyr Phe
145                 150                 155                 160

Ile Gln Phe Lys Leu Tyr Asn Ile Lys Lys Lys Tyr Ile Ile Ala Arg
                165                 170                 175

Val Glu Leu Glu Lys Ile Leu Ile Asn Asp Ile Lys Ser Lys Lys Tyr
            180                 185                 190

Asn Ile Tyr Lys Ser Asp Glu Asn Ser Gly Leu Leu Gly Thr Asp Asn
        195                 200                 205

Asn Ser Thr Ile Met Asn Asn Glu Tyr Leu Asn Leu Asp Tyr Lys Asn
    210                 215                 220

Leu Leu Asp Met Asn Ile Ser Tyr Asn Lys Leu Asn Glu Lys Ile Asn
225                 230                 235                 240

Asn Asp Ile Ile Asn Asn Thr Ser Asp Val Gln Glu Lys Asn Met Asp
                245                 250                 255

Tyr Asn Asp Ile His Asn Phe Gln Lys Lys Lys Lys Ser Ser Asn Phe
260 265 / 270

Ala Tyr Leu Asn Phe Phe His Lys Glu Ser Lys Asp Asn Lys Ile Asp
275 280 285

Val Lys Glu Ser Phe Leu Asn Lys Arg Tyr Gly Ser Asn Lys Arg Ser
290 295 300

Ser Lys Ile Tyr Asp Asn Asn Asn Asn Asn Asn Asn Asn Asn Asn Ile
305 310 315 320

Asn Ser Lys Ile Asp Tyr Leu Glu Asn Asn Ile Thr Tyr Thr Glu Phe
325 330 335

Lys Lys Ile Leu Leu Pro Tyr Leu Trp Pro Ser Lys Arg Ile Asp Met
340 345 350

Lys Gly Asn Ser Ser Ile Leu Arg Thr Tyr Ile Val Leu Ile Phe Leu

355 360 365

Phe Ile Leu Val Ser Lys Val Phe Ser Val Ile Ser Pro Ile Tyr Leu
370 375 380

Gly Trp Ala Ser Asn Glu Val Leu Lys Lys Ser Leu Ser Ser Ser Val
385 390 395 400

Tyr Tyr Leu Gly Leu Tyr Val Thr Phe Phe Phe Ile Ser Lys Phe Leu
405 410 415

Lys Glu Val Cys Gly Val Leu Phe Ser Gln Val Gln Gln Ser Ala Phe
420 425 430

Ile Glu Leu Gln Glu Ser Ile Phe Gln Thr Phe His Asn Leu Ser Tyr
435 440 445

Glu Trp Tyr Ser Ser Lys Asn Ser Gly Gly Ile Met Arg Ile Val Asp
450 455 460

Arg Gly Thr Glu Ser Ala Asn Asn Leu Met Ser Ser Val Leu Met Tyr
465 470 475 480

Ile Ile Pro Ala Thr Ile Glu Gly Leu Ile Thr Cys Ile Ile Phe Ile
485 490 495

Phe Lys Tyr Lys Asn Ser Leu Leu Gly Ser Val Leu Phe Ile Gly Leu
500 505 510

Thr Leu Tyr Ile Tyr Ser Thr Ile Lys Ile Thr Lys Trp Arg Lys Lys
515 520 525

Ile Arg Thr Lys Ala Asn Glu Met Asp Asn Val Tyr His Asp Ile Ala
530 535 540

His Asp Ser Leu Thr Asn Tyr Glu Asn Val Lys Tyr Phe Ser Asn Glu
545 550 555 560

Lys Phe Glu Ile Lys Lys Phe Cys Asn Ala Leu Ser Asn Tyr His Arg
565 570 575

Tyr Asn Leu Lys Ile Leu Asn Ser Leu Gly Ile Leu Asn Thr Val Gln
580 585 590

Gln Phe Ile Leu Asn Gly Thr Leu Phe Phe Thr Leu Leu Cys Val Ile
595 600 605

Tyr Met Ile Val Lys Glu Gly Ser Asp Pro Gly Thr Phe Ile Ser Val

610                 615                 620

Val Val Tyr Thr Ser Asn Val Phe Ala Pro Leu Ser Ile Leu Gly Thr
625                 630                 635                 640

Leu Tyr Ala Thr Ile Ile Lys Ser Phe Thr Asp Ile Ser Asp Leu Ile
                645                 650                 655

Asp Ile Leu Arg Asp Lys Ile Asp Ile Ser Asn Asp Lys Asn Leu Lys
            660                 665                 670

Asn Phe Asp Leu Thr Ser Gln Glu Lys Lys Phe Gly Val Ser Ile Glu
        675                 680                 685

Phe Asn Asn Val His Phe Asn Tyr Pro Thr Gln Pro Leu His Thr Ser
    690                 695                 700

Leu Lys Asp Ile Asn Ile Tyr Ile Lys Pro Gly Thr Thr Cys Ala Leu
705                 710                 715                 720

Val Gly His Thr Gly Ser Gly Lys Thr Thr Ile Ser Lys Leu Leu Tyr
                725                 730                 735

Arg Phe Tyr Asp Ser Lys Gly Glu Ile Lys Ile Gly Gly Arg Asn Ile
            740                 745                 750

Asn Glu Tyr Thr Arg Asn Ser Ile Arg Asn Ile Ile Gly Ile Val Pro
        755                 760                 765

Gln Asp Thr Ile Leu Phe Asn Glu Ser Ile Lys Tyr Asn Ile Leu Tyr
    770                 775                 780

Gly Lys Leu Asp Ala Thr Glu Glu Glu Leu Ile Gln Ala Val Lys Ser
785                 790                 795                 800

Ala Gln Leu Tyr Asp Phe Ile Gln Ser Leu Pro Lys Lys Trp Asp Thr
                805                 810                 815

Leu Val Gly Asp Lys Gly Val Lys Leu Ser Gly Gly Glu Arg Gln Arg
            820                 825                 830

Ile Ser Ile Ala Arg Cys Leu Leu Lys Asp Pro Lys Ile Val Ile Phe
        835                 840                 845

Asp Glu Ala Thr Ser Ser Leu Asp Ser Arg Thr Glu Tyr Leu Phe Gln
    850                 855                 860

Lys Ala Val Glu Asp Leu Arg Lys Asn Arg Thr Ile Ile Ile Ile Ala

865                 870                 875                 880

His Lys Leu Cys Thr Ile Thr Thr Ala Glu Leu Ile Ile Leu Leu Asn
                885                 890                 895

Lys Gly Lys Ile Ile Glu Arg Gly Thr His Leu Asp Leu Leu Lys Cys
            900                 905                 910

Asn Gly Glu Tyr Thr Glu Met Trp Asn Met Gln Ser Lys Ser Asn Glu
        915                 920                 925

Pro His Thr Glu Thr Asn Ser Ser Ile Asp Lys Asp Asp Val Asn Lys
    930                 935                 940

Asn Asn Asn Lys Asn Asn Asp Val Ile Leu Asn Thr Cys Lys Asn Asp
945                 950                 955                 960

Ile Thr Thr Ser Phe Arg Ser Asn Ser Glu Lys Ser Ser Gln Glu Phe
                965                 970                 975

Ser Asp Ala Ser Asn His Ile Lys Gln Ser Lys Thr Ser Asn Asp His
            980                 985                 990

Asn Asn Asn Ile Asn Val His Lys Lys Asn Glu Gln Glu Gln Leu Phe
        995                 1000                1005

Leu Thr Asn Asp Lys Thr Asp Met Asp Asp Asn Met Asn Asn Lys Lys
    1010                1015                1020

Lys
1025

<210> 86
<211> 1419
<212> PRT
<213> Plasmodium falciparum

<400> 86
Met Gly Lys Glu Gln Lys Glu Lys Lys Asp Gly Asn Leu Ser Ile Lys
1               5                   10                  15

Glu Glu Val Glu Lys Glu Leu Asn Lys Lys Ser Thr Ala Glu Leu Phe
            20                  25                  30

Arg Lys Ile Lys Asn Glu Lys Ile Ser Phe Phe Leu Pro Phe Lys Cys
        35                  40                  45

Leu Pro Ala Gln His Arg Lys Leu Leu Phe Ile Ser Phe Val Cys Ala
    50                  55                  60

Val Leu Ser Gly Gly Thr Leu Pro Phe Phe Ile Ser Val Phe Gly Val
65                  70                  75                  80

Ile Leu Lys Asn Met Asn Leu Gly Asp Asp Ile Asn Pro Ile Ile Leu
                85                  90                  95

Ser Leu Val Ser Ile Gly Leu Val Gln Phe Ile Leu Ser Met Ile Ser
            100                 105                 110

Ser Tyr Cys Met Asp Val Ile Thr Ser Lys Ile Leu Lys Thr Leu Lys
        115                 120                 125

Leu Glu Tyr Leu Arg Ser Val Phe Tyr Gln Asp Gly Gln Phe His Asp
    130                 135                 140

Asn Asn Pro Gly Ser Lys Leu Arg Ser Asp Leu Asp Phe Tyr Leu Glu
145                 150                 155                 160

Gln Val Ser Ser Gly Ile Gly Thr Lys Phe Ile Thr Ile Phe Thr Tyr
                165                 170                 175

Ala Ser Ser Phe Leu Gly Leu Tyr Ile Trp Ser Leu Ile Lys Asn Ala
            180                 185                 190

Arg Leu Thr Leu Cys Ile Thr Cys Val Phe Pro Leu Ile Tyr Val Cys
        195                 200                 205

Gly Val Ile Cys Asn Lys Lys Val Lys Leu Asn Lys Lys Thr Ser Leu
    210                 215                 220

Leu Tyr Asn Asn Asn Thr Met Ser Ile Ile Glu Glu Ala Leu Met Gly
225                 230                 235                 240

Ile Arg Thr Val Ala Ser Tyr Cys Gly Glu Lys Thr Ile Leu Asn Lys
                245                 250                 255

Phe Asn Leu Ser Glu Thr Phe Tyr Ser Lys Tyr Ile Leu Lys Ala Asn
            260                 265                 270

Phe Val Glu Ala Leu His Ile Gly Leu Ile Asn Gly Leu Ile Leu Val
        275                 280                 285

Ser Tyr Ala Phe Gly Phe Trp Tyr Gly Thr Arg Ile Ile Ile Asn Ser
    290                 295                 300

Ala Thr Asn Gln Tyr Pro Asn Asn Asp Phe Asn Gly Ala Ser Val Ile
305                 310                 315                 320

Ser Ile Leu Leu Gly Val Leu Ile Ser Met Phe Met Leu Thr Ile Ile
                325                 330                 335

Leu Pro Asn Ile Thr Glu Tyr Met Lys Ala Leu Glu Ala Thr Asn Ser
            340                 345                 350

Leu Tyr Glu Ile Ile Asn Arg Lys Pro Leu Val Glu Asn Asn Asp Asp
        355                 360                 365

Gly Glu Thr Leu Pro Asn Ile Lys Lys Ile Glu Phe Lys Asn Val Arg
    370                 375                 380

Phe His Tyr Asp Thr Arg Lys Asp Val Glu Ile Tyr Lys Asp Leu Ser
385                 390                 395                 400

Phe Thr Leu Lys Glu Gly Lys Thr Tyr Ala Phe Val Gly Glu Ser Gly
                405                 410                 415

Cys Gly Lys Ser Thr Ile Leu Lys Leu Ile Glu Arg Leu Tyr Asp Pro
            420                 425                 430

Thr Glu Gly Asp Ile Ile Val Asn Asp Ser His Asn Leu Lys Asp Ile
        435                 440                 445

Asn Leu Lys Trp Trp Arg Ser Lys Ile Gly Val Val Ser Gln Asp Pro
    450                 455                 460

Leu Leu Phe Ser Asn Ser Ile Lys Asn Asn Ile Lys Tyr Ser Leu Tyr
465                 470                 475                 480

Ser Leu Lys Asp Leu Glu Ala Met Glu Asn Tyr Tyr Glu Glu Asn Thr
                485                 490                 495

Asn Asp Thr Tyr Glu Asn Lys Asn Phe Ser Leu Ile Ser Asn Ser Met
            500                 505                 510

Thr Ser Asn Glu Leu Leu Glu Met Lys Lys Glu Tyr Gln Thr Ile Lys
        515                 520                 525

Asp Ser Asp Val Val Asp Val Ser Lys Lys Val Leu Ile His Asp Phe
    530                 535                 540

Val Ser Ser Leu Pro Asp Lys Tyr Asp Thr Leu Val Gly Ser Asn Ala
545                 550                 555                 560

Ser Lys Leu Ser Gly Gly Gln Lys Gln Arg Ile Ser Ile Ala Arg Ala
                565                 570                 575

Ile Met Arg Asn Pro Lys Ile Leu Ile Leu Asp Glu Ala Thr Ser Ser
            580                 585                 590

Leu Asp Asn Lys Ser Glu Tyr Leu Val Gln Lys Thr Ile Asn Asn Leu
        595                 600                 605

Lys Gly Asn Glu Asn Arg Ile Thr Ile Ile Ile Ala His Arg Leu Ser
    610                 615                 620

Thr Ile Arg Tyr Ala Asn Thr Ile Phe Val Leu Ser Asn Arg Glu Arg
625                 630                 635                 640

Ser Asp Asn Asn Asn Asn Asn Asn Asn Asp Asp Asn Asn Asn Asn Asn
                645                 650                 655

Asn Asn Asn Asn Asn Lys Ile Asn Asn Glu Gly Ser Tyr Ile Ile Glu
            660                 665                 670

Gln Gly Thr His Asp Ser Leu Met Lys Asn Lys Asn Gly Ile Tyr His
        675                 680                 685

Leu Met Ile Asn Asn Gln Lys Ile Ser Ser Asn Lys Ser Ser Asn Asn
    690                 695                 700

Gly Asn Asp Asn Gly Ser Asp Asn Lys Ser Ser Ala Tyr Lys Asp Ser
705                 710                 715                 720

Asp Thr Gly Asn Asp Ala Asp Asn Met Asn Ser Leu Ser Ile His Glu
                725                 730                 735

Asn Glu Asn Ile Ser Asn Asn Arg Asn Cys Lys Asn Thr Ala Glu Asn
            740                 745                 750

Glu Lys Glu Glu Lys Val Pro Phe Phe Lys Arg Met Phe Arg Arg Lys
        755                 760                 765

Lys Lys Ala Pro Asn Asn Leu Arg Ile Ile Tyr Lys Glu Ile Phe Ser
    770                 775                 780

Tyr Lys Lys Asp Val Thr Ile Ile Phe Phe Ser Ile Leu Val Ala Gly
785                 790                 795                 800

Gly Leu Tyr Pro Val Phe Ala Leu Leu Tyr Ala Arg Tyr Val Ser Thr
                805                 810                 815

Leu Phe Asp Phe Ala Asn Leu Glu Tyr Asn Ser Asn Lys Tyr Ser Ile
            820                 825                 830

Tyr Ile Leu Leu Ile Ala Ile Ala Met Phe Ile Ser Glu Thr Leu Lys
        835                 840                 845

Asn Tyr Tyr Asn Asn Lys Ile Gly Glu Lys Val Glu Lys Thr Met Lys
    850                 855                 860

Arg Arg Leu Phe Glu Asn Ile Leu Tyr Gln Glu Met Ser Phe Phe Asp
865                 870                 875                 880

Gln Asp Lys Asn Thr Pro Gly Val Leu Ser Ala His Ile Asn Arg Asp
                885                 890                 895

Val His Leu Leu Lys Thr Gly Leu Val Asn Asn Ile Val Ile Phe Ser
            900                 905                 910

His Phe Ile Met Leu Phe Leu Val Ser Met Val Met Ser Phe Tyr Phe
        915                 920                 925

Cys Pro Ile Val Ala Ala Val Leu Thr Phe Ile Tyr Phe Ile Asn Met
    930                 935                 940

Arg Val Phe Ala Val Arg Ala Arg Leu Thr Lys Ser Lys Glu Ile Glu
945                 950                 955                 960

Lys Lys Glu Asn Met Ser Ser Gly Val Phe Ala Phe Ser Ser Asp Asp
                965                 970                 975

Glu Met Phe Lys Asp Pro Ser Phe Leu Ile Gln Glu Ala Phe Tyr Asn
            980                 985                 990

Met His Thr Val Ile Asn Tyr Gly Leu Glu Asp Tyr Phe Cys Asn Leu
        995                 1000                1005

Ile Glu Lys Ala Ile Asp Tyr Lys Asn Lys Gly Gln Lys Arg Arg Ile
    1010                1015                1020

Ile Val Asn Ala Ala Leu Trp Gly Phe Ser Gln Ser Ala Gln Leu Phe
1025                1030                1035                1040

Ile Asn Ser Phe Ala Tyr Trp Phe Gly Ser Phe Leu Ile Lys Arg Gly
                1045                1050                1055

Thr Ile Leu Val Asp Asp Phe Met Lys Ser Leu Phe Thr Phe Ile Phe
            1060                1065                1070

Thr Gly Ser Tyr Ala Gly Lys Leu Met Ser Leu Lys Gly Asp Ser Glu
        1075                1080                1085

Asn Ala Lys Leu Ser Phe Glu Lys Tyr Tyr Pro Leu Met Ile Arg Lys
    1090                1095                1100

Ser Asn Ile Asp Val Arg Asp Asp Gly Gly Ile Arg Ile Asn Lys Asn
1105                1110                1115                1120

Leu Ile Lys Gly Lys Val Asp Ile Lys Asp Val Asn Phe Arg Tyr Ile
                1125                1130                1135

Ser Arg Pro Asn Val Pro Ile Tyr Lys Asn Leu Ser Phe Thr Cys Asp
            1140                1145                1150

Ser Lys Lys Thr Thr Ala Ile Val Gly Glu Thr Gly Ser Gly Lys Ser
        1155                1160                1165

Thr Phe Met Asn Leu Leu Leu Arg Phe Tyr Asp Leu Lys Asn Asp His
    1170                1175                1180

Ile Ile Leu Lys Asn Asp Met Thr Asn Phe Gln Asp Tyr Gln Asn Asn
1185                1190                1195                1200

Asn Asn Asn Ser Leu Val Leu Lys Asn Val Asn Glu Phe Ser Asn Gln
                1205                1210                1215

Ser Gly Ser Ala Glu Asp Tyr Thr Val Phe Asn Asn Asn Gly Glu Ile
            1220                1225                1230

Leu Leu Asp Asp Ile Asn Ile Cys Asp Tyr Asn Leu Arg Asp Leu Arg
        1235                1240                1245

Asn Leu Phe Ser Ile Val Ser Gln Glu Pro Met Leu Phe Asn Met Ser
    1250                1255                1260

Ile Tyr Glu Asn Ile Lys Phe Gly Arg Glu Asp Ala Thr Leu Glu Asp
1265                1270                1275                1280

Val Lys Arg Val Ser Lys Phe Ala Ala Ile Asp Glu Phe Ile Glu Ser
                1285                1290                1295

Leu Pro Asn Lys Tyr Asp Thr Asn Val Gly Pro Tyr Gly Lys Ser Leu
            1300                1305                1310

Ser Gly Gly Gln Lys Gln Arg Ile Ala Ile Ala Arg Ala Leu Leu Arg
        1315                1320                1325

Glu Pro Lys Ile Leu Leu Leu Asp Glu Ala Thr Ser Ser Leu Asp Ser
    1330                1335                1340

Asn Ser Glu Lys Leu Ile Glu Lys Thr Ile Val Asp Ile Lys Asp Lys
1345                1350                1355                1360

Ala Asp Lys Thr Ile Ile Thr Ile Ala His Arg Ile Ala Ser Ile Lys
                1365                1370                1375

Arg Ser Asp Lys Ile Val Val Phe Asn Asn Pro Asp Arg Asn Gly Thr
            1380                1385                1390

Phe Val Gln Ser His Gly Thr His Asp Glu Leu Leu Ser Ala Gln Asp
        1395                1400                1405

Gly Ile Tyr Lys Lys Tyr Val Lys Leu Ala Lys
    1410                1415

<210> 87
<211> 1548
<212> PRT
<213> Leishmania tarentolae

<400> 87
Met Val Asp Asn Gly His Val Thr Ile Ala Met Ala Asp Leu Gly Thr
1               5                   10                  15

Val Val Glu Ile Ala Gln Val Arg Cys Gln Gln Glu Ala Gln Arg Lys
            20                  25                  30

Phe Ala Glu Gln Leu Asp Glu Leu Trp Gly Gly Glu Pro Ala Tyr Thr
        35                  40                  45

Pro Thr Val Glu Asp Gln Ala Ser Trp Phe Gln Gln Leu Tyr Tyr Gly
    50                  55                  60

Trp Ile Gly Asp Tyr Ile Tyr Lys Ala Ala Ala Gly Asn Ile Thr Glu
65                  70                  75                  80

Ala Asp Leu Pro Pro Pro Thr Arg Ser Thr Arg Thr Tyr His Ile Gly
                85                  90                  95

Arg Lys Leu Ser Arg Gln Ala His Ala Asp Ile Asp Ala Ser Arg Arg
            100                 105                 110

Trp Gln Gly Tyr Ile Gly Cys Glu Val Val Tyr Lys Ser Glu Ala Glu
        115                 120                 125

Ala Lys Gly Val Leu Arg Trp Val Gly His Leu Gln Gln Ser Asp Tyr
    130                 135                 140

Pro Arg Ser Leu Val Ala Gly Val Glu Trp Arg Met Pro Pro Arg His
145                 150                 155                 160

Arg Arg Leu Ala Val Leu Gly Ser Ala Ala Ala Leu His Asn Gly Val
                165                 170                 175

Val His Gly Glu Arg Leu Phe Trp Pro His Glu Asp Asn Tyr Leu Cys
            180                 185                 190

Ser Cys Glu Pro Val Glu Gln Leu Tyr Val Lys Ser Lys Tyr Asn Leu
        195                 200                 205

Ile Pro Pro Arg Pro Pro Pro Ser Pro Asp Leu Leu Arg Thr Leu Phe
    210                 215                 220

Lys Val His Trp Tyr His Val Trp Ala Gln Ile Leu Pro Lys Leu Leu
225                 230                 235                 240

Ser Asp Val Thr Ala Leu Met Leu Pro Val Leu Leu Glu Tyr Phe Val
                245                 250                 255

Lys Tyr Leu Asn Ala Asp Asn Ala Thr Trp Gly Trp Gly Leu Gly Leu
            260                 265                 270

Ala Leu Thr Ile Phe Leu Thr Asn Val Ile Gln Ser Cys Ser Ala His
        275                 280                 285

Lys Tyr Asp His Ile Ser Ile Arg Thr Ala Ala Leu Phe Glu Thr Ser
    290                 295                 300

Ser Met Ala Leu Leu Phe Glu Lys Cys Phe Thr Val Ser Arg Arg Ser
305                 310                 315                 320

Leu Gln Arg Pro Asp Met Ser Val Gly Arg Ile Met Asn Met Val Gly
                325                 330                 335

Asn Asp Val Asp Asn Ile Gly Ser Leu Asn Trp Tyr Val Met Tyr Phe
            340                 345                 350

Trp Ser Ala Pro Leu Gln Leu Val Leu Cys Leu Leu Leu Leu Ile Arg
        355                 360                 365

Leu Val Gly Trp Leu Arg Val Pro Gly Met Ala Val Leu Phe Val Thr
    370                 375                 380

Leu Pro Leu Gln Ala Val Ile Ser Lys His Val Gln Asp Val Ser Glu
385                 390                 395                 400

Arg Met Ala Ser Val Val Asp Leu Arg Ile Lys Arg Thr Asn Glu Leu
                405                 410                 415

Leu Ser Gly Val Arg Ile Val Lys Phe Met Gly Trp Glu Pro Val Phe
            420                 425                 430

Leu Ala Arg Ile Gln Asp Ala Arg Ser Arg Glu Leu Arg Cys Leu Arg
        435                 440                 445

Asp Val His Val Ala Asn Val Phe Phe Met Phe Val Asn Asp Ala Thr
    450                 455                 460

Pro Thr Leu Val Ile Ala Val Val Phe Ile Leu Tyr His Val Ser Gly
465                 470                 475                 480

Lys Val Leu Lys Pro Glu Val Val Phe Pro Thr Ile Ala Leu Leu Asn
                485                 490                 495

Thr Met Arg Val Ser Phe Phe Met Ile Pro Ile Ile Ile Ser Ser Ile
            500                 505                 510

Leu Gln Cys Phe Val Ser Ala Lys Arg Val Thr Ala Phe Ile Glu Cys
        515                 520                 525

Pro Asp Thr His Ser Gln Val Gln Asp Ile Ala Ser Ile Asp Val Pro
    530                 535                 540

Asp Ala Ala Ala Ile Phe Lys Gly Ala Ser Ile His Thr Tyr Leu Pro
545                 550                 555                 560

Val Lys Leu Pro Arg Cys Lys Ser Arg Leu Thr Ala Met Gln Arg Ser
                565                 570                 575

Thr Leu Trp Phe Arg Arg Arg Gly Val Pro Glu Thr Glu Trp Tyr Glu
            580                 585                 590

Val Asp Ser Pro Asp Ala Ser Ala Ser Ser Leu Ala Val His Ser Thr
        595                 600                 605

Thr Val His Met Gly Ser Thr Gln Thr Val Ile Thr Asp Ser Asp Gly
    610                 615                 620

Ala Ala Gly Glu Asp Glu Lys Gly Glu Val Glu Glu Gly Asp Arg Glu
625                 630                 635                 640

Tyr Tyr Gln Leu Val Ser Lys Glu Leu Leu Arg Asn Val Ser Leu Thr
                645                 650                 655

Ile Pro Lys Gly Lys Leu Thr Met Val Ile Gly Ser Thr Gly Ser Gly
            660                 665                 670

Lys Ser Thr Leu Leu Gly Ala Leu Met Gly Glu Tyr Ser Val Glu Ser
        675                 680                 685

Gly Glu Leu Trp Ala Glu Arg Ser Ile Ala Tyr Val Pro Gln Gln Ala
    690                 695                 700

Trp Ile Met Asn Ala Thr Leu Arg Gly Asn Ile Leu Phe Phe Asp Glu
705                 710                 715                 720

Glu Arg Ala Glu Asp Leu Gln Asp Val Ile Arg Cys Cys Gln Leu Glu
                725                 730                 735

Ala Asp Leu Ala Gln Phe Cys Gly Gly Leu Asp Thr Glu Ile Gly Glu
            740                 745                 750

Met Gly Val Asn Leu Ser Gly Gly Gln Lys Ala Arg Val Ser Leu Ala
        755                 760                 765

Arg Ala Val Tyr Ala Asn Arg Asp Val Tyr Leu Leu Asp Asp Pro Leu
    770                 775                 780

Ser Ala Leu Asp Ala His Val Gly Gln Arg Ile Val Gln Asp Val Ile
785                 790                 795                 800

Leu Gly Arg Leu Arg Gly Lys Thr Arg Val Leu Ala Thr His Gln Ile
                805                 810                 815

His Leu Leu Pro Leu Ala Asp Tyr Ile Val Val Leu Gln His Gly Ser
            820                 825                 830

Ile Val Phe Ala Gly Asp Phe Ala Ala Phe Ser Ala Thr Ala Leu Glu
        835                 840                 845

Glu Thr Leu Arg Gly Glu Leu Lys Gly Ser Lys Asp Val Glu Ser Cys
    850                 855                 860

Ser Ser Asp Val Asp Thr Glu Ser Ala Thr Ala Glu Thr Ala Pro Tyr
865                 870                 875                 880

Val Ala Lys Ala Lys Gly Leu Asn Ala Glu Gln Glu Thr Ser Leu Ala
                885                 890                 895

Gly Gly Glu Asp Pro Leu Arg Ser Asp Val Glu Ala Gly Arg Leu Met
            900                 905                 910

Thr Thr Glu Glu Lys Ala Thr Gly Lys Val Pro Trp Ser Thr Tyr Val
        915                 920                 925

Ala Tyr Leu Lys Ser Cys Gly Gly Leu Glu Ala Trp Gly Cys Leu Leu
    930                 935                 940

Ala Thr Phe Ala Leu Thr Glu Cys Val Thr Ala Ala Ser Ser Val Trp
945                 950                 955                 960

Leu Ser Ile Trp Ser Thr Gly Ser Leu Met Trp Ser Ala Asp Thr Tyr
                965                 970                 975

Leu Tyr Val Tyr Leu Phe Ile Val Phe Leu Glu Ile Phe Gly Ser Pro
            980                 985                 990

Leu Arg Phe Phe Leu Cys Tyr Tyr Leu Ile Arg Ile Gly Ser Arg Asn
        995                 1000                1005

Met His Arg Asp Leu Leu Glu Ser Ile Gly Val Ala Arg Met Ser Phe
    1010                1015                1020

Phe Asp Thr Thr Pro Val Gly Arg Val Leu Asn Arg Phe Thr Lys Asp
1025                1030                1035                1040

Met Ser Ile Leu Asp Asn Thr Leu Asn Asp Gly Tyr Leu Tyr Leu Leu
                1045                1050                1055

Glu Tyr Phe Phe Ser Met Cys Ser Thr Val Ile Ile Met Val Val Val
            1060                1065                1070

Gln Pro Phe Val Leu Val Ala Ile Val Pro Cys Val Tyr Ser Tyr Tyr
        1075                1080                1085

Lys Leu Met Gln Val Tyr Asn Ala Ser Asn Arg Glu Thr Arg Arg Ile
    1090                1095                1100

Lys Ser Ile Ala His Ser Pro Val Phe Thr Leu Leu Glu Glu Ser Leu
1105                1110                1115                1120

Gln Gly Gln Arg Thr Ile Ala Thr Tyr Gly Lys Leu His Leu Val Leu
                1125                1130                1135

Gln Glu Ala Leu Gly Arg Leu Asp Val Val Tyr Ser Ala Leu Tyr Met
            1140                1145                1150

Gln Asn Val Ser Asn Arg Trp Leu Gly Val Arg Leu Glu Phe Leu Ser
        1155                1160                1165

Cys Val Val Thr Phe Met Val Ala Phe Ile Gly Val Ile Gly Lys Met
    1170                1175                1180

Glu Gly Ala Ser Ser Gln Asn Ile Gly Leu Ile Ser Leu Ser Leu Thr
1185                1190                1195                1200

Met Ser Met Thr Leu Thr Glu Thr Leu Asn Trp Leu Val Arg Gln Val
                1205                1210                1215

Ala Met Val Glu Ala Asn Met Asn Ser Val Glu Arg Val Leu His Tyr
            1220                1225                1230

Thr Gln Glu Val Glu His Glu His Val Pro Glu Met Gly Glu Leu Val
        1235                1240                1245

Ala Gln Leu Val Arg Ser Glu Ser Gly Arg Gly Ala Asn Val Thr Glu
    1250                1255                1260

Thr Val Val Ile Glu Ser Ala Gly Ala Ala Ser Ser Ala Leu His Pro
1265                1270                1275                1280

Val Gln Ala Gly Ser Leu Val Leu Glu Gly Val Gln Met Arg Tyr Arg
                1285                1290                1295

Glu Gly Leu Pro Leu Val Leu Arg Gly Val Ser Phe Gln Ile Ala Pro
            1300                1305                1310

Arg Glu Lys Val Gly Ile Val Gly Arg Thr Gly Ser Gly Lys Ser Thr
        1315                1320                1325

Leu Leu Leu Thr Phe Met Arg Met Val Glu Val Cys Gly Gly Val Ile
    1330                1335                1340

His Val Asn Gly Arg Glu Met Ser Ala Tyr Gly Leu Arg Glu Leu Arg
1345                1350                1355                1360

Arg His Phe Ser Met Ile Pro Gln Asp Pro Val Leu Phe Asp Gly Thr
                1365                1370                1375

Val Arg Gln Asn Val Asp Pro Phe Leu Glu Ala Ser Ser Ala Glu Val
            1380                1385                1390

Trp Ala Ala Leu Glu Leu Val Gly Leu Arg Glu Arg Val Ala Ser Glu
        1395                1400                1405

Ser Glu Gly Ile Asp Ser Arg Val Leu Glu Gly Gly Ser Asn Tyr Ser
    1410                1415                1420

Val Gly Gln Arg Gln Leu Met Cys Met Ala Arg Ala Leu Leu Lys Arg
1425                1430                1435                1440

Gly Ser Gly Phe Ile Leu Met Asp Glu Ala Thr Ala Asn Ile Asp Pro
                1445                1450                1455

Ala Leu Asp Arg Gln Ile Gln Ala Thr Val Met Ser Ala Phe Ser Ala
            1460                1465                1470

Tyr Thr Val Ile Thr Ile Ala His Arg Leu His Thr Val Ala Gln Tyr
        1475                1480                1485

Asp Lys Ile Ile Val Met Asp His Gly Val Val Ala Glu Met Gly Ser
    1490                1495                1500

Pro Arg Glu Leu Val Met Asn His Gln Ser Met Phe His Ser Met Val
1505                1510                1515                1520

Glu Ser Leu Gly Ser Arg Gly Ser Lys Asp Phe Tyr Glu Leu Leu Met
                1525                1530                1535

Gly Arg Arg Ile Val Gln Pro Ala Val Leu Ser Asp
            1540                1545

<210> 88
<211> 1530
<212> PRT
<213> Schizosaccharomyces pombe

<400> 88
Met Asn Gln Asn Ser Asp Thr Thr His Gly Gln Ala Leu Gly Ser Thr
1               5                   10                  15

Leu Asn His Thr Thr Glu Val Thr Arg Ile Ser Asn Ser Ser Asp His
            20                  25                  30

Phe Glu Asp Ser Ser Ser Asn Val Asp Glu Ser Leu Asp Ser Ser Asn
        35                  40                  45

Pro Ser Ser Asn Glu Lys Ala Ser His Thr Asn Glu Glu Tyr Arg Ser

    50                  55                  60

Lys Gly Asn Gln Ser Tyr Val Pro Ser Ser Ser Asn Glu Pro Ser Pro
65                  70                  75                  80

Glu Ser Ser Ser Asn Ser Asp Ser Ser Ser Ser Asp Asp Ser Ser Val
                85                  90                  95

Asp Arg Leu Ala Gly Asp Pro Phe Glu Leu Gly Glu Asn Phe Asn Leu
            100                 105                 110

Lys His Tyr Leu Arg Ala Tyr Lys Asp Ser Leu Gln Arg Asp Asp Ile
        115                 120                 125

Ile Thr Arg Ser Ser Gly Val Cys Met Arg Asp His Ser Val Tyr Gly
    130                 135                 140

Val Gly Ser Gly Tyr Glu Phe Leu Lys Thr Phe Pro Asp Ile Phe Leu
145                 150                 155                 160

Gln Pro Tyr Arg Ala Ile Thr Glu Lys Gln Val Val Glu Lys Ala Ile
                165                 170                 175

Leu Ser His Cys His Ala Leu Ala Asn Ala Gly Glu Leu Val Met Val
            180                 185                 190

Leu Gly Gln Pro Gly Ser Gly Cys Ser Thr Phe Leu Arg Ser Val Thr
        195                 200                 205

Ser Asp Thr Val His Tyr Lys Arg Val Glu Gly Thr Thr His Tyr Asp
    210                 215                 220

Gly Ile Asp Lys Ala Asp Met Lys Lys Phe Phe Pro Gly Asp Leu Leu
225                 230                 235                 240

Tyr Ser Gly Glu Asn Asp Val His Phe Pro Ser Leu Thr Thr Ala Glu
                245                 250                 255

Thr Leu Asp Phe Ala Ala Lys Cys Arg Thr Pro Asn Asn Arg Pro Cys
            260                 265                 270

Asn Leu Thr Arg Gln Glu Tyr Val Ser Arg Glu Arg His Leu Ile Ala
        275                 280                 285

Thr Ala Phe Gly Leu Thr His Thr Phe Asn Thr Lys Val Gly Asn Asp
    290                 295                 300

Phe Val Arg Gly Val Ser Gly Gly Glu Arg Lys Arg Val Thr Ile Ser

305                 310                 315                 320

Glu Gly Phe Ala Thr Arg Pro Thr Ile Ala Cys Trp Asp Asn Ser Thr
                325                 330                 335

Arg Gly Leu Asp Ser Ser Thr Ala Phe Glu Phe Val Asn Val Leu Arg
            340                 345                 350

Thr Cys Ala Asn Glu Leu Lys Met Thr Ser Phe Val Thr Ala Tyr Gln
        355                 360                 365

Ala Ser Glu Lys Ile Tyr Lys Leu Phe Asp Arg Ile Cys Val Leu Tyr
    370                 375                 380

Ala Gly Arg Gln Ile Tyr Tyr Gly Pro Ala Asp Lys Ala Lys Gln Tyr
385                 390                 395                 400

Phe Leu Asp Met Gly Phe Asp Cys His Pro Arg Glu Thr Thr Pro Asp
                405                 410                 415

Phe Leu Thr Ala Ile Ser Asp Pro Lys Ala Arg Phe Pro Arg Lys Gly
            420                 425                 430

Phe Glu Asn Arg Val Pro Arg Thr Pro Asp Glu Phe Glu Gln Met Trp
        435                 440                 445

Arg Asn Ser Ser Val Tyr Ala Asp Leu Met Ala Glu Met Glu Ser Tyr
    450                 455                 460

Asp Lys Arg Trp Thr Glu Thr Thr Pro Ala Ser Ser Glu Ala Pro Glu
465                 470                 475                 480

Lys Asp Asn Phe Gly Ser Asp Ile Ser Ala Thr Thr Lys His Glu Leu
                485                 490                 495

Tyr Arg Gln Ser Ala Val Ala Glu Lys Ser Lys Arg Val Lys Asp Thr
            500                 505                 510

Ser Pro Tyr Thr Val Thr Phe Ser Gln Gln Leu Trp Tyr Cys Leu Ala
        515                 520                 525

Arg Ser Trp Glu Arg Tyr Ile Asn Asp Pro Ala Tyr Ile Gly Ser Met
    530                 535                 540

Ala Phe Ala Phe Leu Phe Gln Ser Leu Ile Ile Gly Ser Ile Phe Tyr
545                 550                 555                 560

Asp Met Lys Leu Asn Thr Val Asp Val Phe Ser Arg Gly Gly Val Leu

                565                 570                 575

Phe Phe Ser Ile Leu Phe Cys Ala Leu Gln Ser Leu Ser Glu Ile Ala
            580                 585                 590

Asn Met Phe Ser Gln Arg Pro Ile Ile Ala Lys His Arg Ala Ser Ala
        595                 600                 605

Leu Tyr His Pro Ala Ala Asp Val Ile Ser Ser Leu Ile Val Asp Leu
    610                 615                 620

Pro Phe Arg Phe Ile Asn Ile Ser Val Phe Ser Ile Val Leu Tyr Phe
625                 630                 635                 640

Leu Thr Asn Leu Lys Arg Thr Ala Gly Gly Phe Trp Thr Tyr Phe Leu
                645                 650                 655

Phe Leu Phe Ile Gly Ala Thr Cys Met Ser Ala Phe Phe Arg Ser Leu
            660                 665                 670

Ala Gly Ile Met Pro Asn Val Glu Ser Ala Ser Ala Leu Gly Gly Ile
        675                 680                 685

Gly Val Leu Ala Ile Ala Ile Tyr Thr Gly Tyr Ala Ile Pro Asn Ile
    690                 695                 700

Asp Val Gly Trp Trp Phe Arg Trp Ile Ala Tyr Leu Asp Pro Leu Gln
705                 710                 715                 720

Phe Gly Phe Glu Ser Leu Met Ile Asn Glu Phe Lys Ala Arg Gln Phe
                725                 730                 735

Glu Cys Ser Gln Leu Ile Pro Tyr Gly Ser Gly Tyr Asp Asn Tyr Pro
            740                 745                 750

Val Ala Asn Lys Ile Cys Pro Val Thr Ser Ala Glu Pro Gly Thr Asp
        755                 760                 765

Tyr Val Asp Gly Ser Thr Tyr Leu Tyr Ile Ser Phe Asn Tyr Lys Thr
    770                 775                 780

Arg Gln Leu Trp Arg Asn Leu Ala Ile Ile Ile Gly Tyr Tyr Ala Phe
785                 790                 795                 800

Leu Val Phe Val Asn Ile Val Ala Ser Glu Thr Leu Asn Phe Asn Asp
                805                 810                 815

Leu Lys Gly Glu Tyr Leu Val Phe Arg Arg Gly His Ala Pro Asp Ala

820                 825                 830

Val Lys Ala Ala Val Asn Glu Gly Gly Lys Pro Leu Asp Leu Glu Thr
        835                 840                 845

Gly Gln Asp Thr Gln Gly Gly Asp Val Val Lys Glu Ser Pro Asp Asn
    850                 855                 860

Glu Glu Glu Leu Asn Lys Glu Tyr Glu Gly Ile Glu Lys Gly His Asp
865                 870                 875                 880

Ile Phe Ser Trp Arg Asn Leu Asn Tyr Asp Ile Gln Ile Lys Gly Glu
                885                 890                 895

His Arg Arg Leu Leu Asn Gly Val Gln Gly Phe Val Val Pro Gly Lys
            900                 905                 910

Leu Thr Ala Leu Met Gly Glu Ser Gly Ala Gly Lys Thr Thr Leu Leu
        915                 920                 925

Asn Val Leu Ala Gln Arg Val Asp Thr Gly Val Val Thr Gly Asp Met
    930                 935                 940

Leu Val Asn Gly Arg Gly Leu Asp Ser Thr Phe Gln Arg Arg Thr Gly
945                 950                 955                 960

Tyr Val Gln Gln Gln Asp Val His Ile Gly Glu Ser Thr Val Arg Glu
                965                 970                 975

Ala Leu Arg Phe Ser Ala Ala Leu Arg Gln Pro Ala Ser Val Pro Leu
            980                 985                 990

Ser Glu Lys Tyr Glu Tyr Val Glu Ser Val Ile Lys Leu Leu Glu Met
        995                 1000                1005

Glu Ser Tyr Ala Glu Ala Ile Ile Gly Thr Pro Gly Ser Gly Leu Asn
    1010                1015                1020

Val Glu Gln Arg Lys Arg Ala Thr Ile Gly Val Glu Leu Ala Ala Lys
1025                1030                1035                1040

Pro Ala Leu Leu Leu Phe Leu Asp Glu Pro Thr Ser Gly Leu Asp Ser
                1045                1050                1055

Gln Ser Ala Trp Ser Ile Val Cys Phe Leu Arg Lys Leu Ala Asp Ala
            1060                1065                1070

Gly Gln Ala Ile Leu Cys Thr Ile His Gln Pro Ser Ala Val Leu Phe

1075                1080                1085

Asp Gln Phe Asp Arg Leu Leu Leu Leu Gln Lys Gly Gly Lys Thr Val
    1090                1095                1100

Tyr Phe Gly Asp Ile Gly Glu His Ser Lys Thr Leu Leu Asn Tyr Phe
1105                1110                1115                1120

Glu Ser His Gly Ala Val His Cys Pro Asp Asp Gly Asn Pro Ala Glu
                1125                1130                1135

Tyr Ile Leu Asp Val Ile Gly Ala Gly Ala Thr Ala Thr Thr Asn Arg
            1140                1145                1150

Asp Trp His Glu Val Trp Asn Asn Ser Glu Glu Arg Lys Ala Ile Ser
        1155                1160                1165

Ala Glu Leu Asp Lys Ile Asn Ala Ser Phe Ser Asn Ser Glu Asp Lys
    1170                1175                1180

Lys Thr Leu Ser Lys Glu Asp Arg Ser Thr Tyr Ala Met Pro Leu Trp
1185                1190                1195                1200

Phe Gln Val Lys Met Val Met Thr Arg Asn Phe Gln Ser Tyr Trp Arg
                1205                1210                1215

Glu Pro Ser Ile Leu Met Ser Lys Leu Ala Leu Asp Ile Phe Ala Gly
            1220                1225                1230

Leu Phe Ile Gly Phe Thr Phe Tyr Asn Gln Gly Leu Gly Val Gln Asn
        1235                1240                1245

Ile Gln Asn Lys Leu Phe Ala Val Phe Met Ala Thr Val Leu Ala Val
    1250                1255                1260

Pro Leu Ile Asn Gly Leu Gln Pro Lys Phe Ile Glu Leu Arg Asn Val
1265                1270                1275                1280

Phe Glu Val Arg Glu Lys Pro Ser Asn Ile Tyr Ser Trp Val Ala Phe
                1285                1290                1295

Val Phe Ser Ala Ile Ile Val Glu Ile Pro Phe Asn Leu Val Phe Gly
            1300                1305                1310

Thr Leu Phe Phe Leu Cys Trp Phe Tyr Pro Ile Lys Phe Tyr Lys His
        1315                1320                1325

Ile His His Pro Gly Asp Lys Thr Gly Tyr Ala Trp Leu Leu Tyr Met

1330                1335                1340

Phe Phe Gln Met Tyr Phe Ser Thr Phe Gly Gln Ala Val Ala Ser Ala
1345                1350                1355                1360

Cys Pro Asn Ala Gln Thr Ala Ser Val Val Asn Ser Leu Leu Phe Thr
                1365                1370                1375

Phe Val Ile Thr Phe Asn Gly Val Leu Gln Pro Asn Ser Asn Leu Val
            1380                1385                1390

Gly Phe Trp His Trp Met His Ser Leu Thr Pro Phe Thr Tyr Leu Ile
        1395                1400                1405

Glu Gly Leu Leu Ser Asp Leu Val His Gly Leu Pro Val Glu Cys Lys
    1410                1415                1420

Ser His Glu Met Leu Thr Ile Asn Pro Pro Ser Gly Gln Thr Cys Gly
1425                1430                1435                1440

Glu Tyr Met Ser Ala Phe Leu Thr Asn Asn Thr Ala Ala Gly Asn Leu
                1445                1450                1455

Leu Asn Pro Asn Ala Thr Thr Ser Cys Ser Tyr Cys Pro Tyr Gln Thr
            1460                1465                1470

Ala Asp Gln Phe Leu Glu Arg Phe Ser Met Arg Tyr Thr His Arg Trp
        1475                1480                1485

Arg Asn Leu Gly Ile Phe Val Gly Tyr Val Phe Phe Asn Ile Phe Ala
    1490                1495                1500

Val Leu Leu Leu Phe Tyr Val Phe Arg Val Met Lys Leu Arg Ser Thr
1505                1510                1515                1520

Trp Leu Gly Lys Lys Ile Thr Gly Thr Gly
                1525                1530

<210> 89
<211> 1501
<212> PRT
<213> Candida albicans

<400> 89
Met Ser Asp Ser Lys Met Ser Ser Gln Asp Glu Ser Lys Leu Glu Lys
  1               5                  10                  15

Ala Ile Ser Gln Asp Ser Ser Ser Glu Asn His Ser Ile Asn Glu Tyr
            20                  25                  30

His Gly Phe Asp Ala His Thr Ser Glu Asn Ile Gln Asn Leu Ala Arg
        35                  40                  45

Thr Phe Thr His Asp Ser Phe Lys Asp Asp Ser Ser Ala Gly Leu Leu
    50                  55                  60

Lys Tyr Leu Thr His Met Ser Glu Val Pro Gly Val Asn Pro Tyr Glu
65                  70                  75                  80

His Glu Glu Ile Asn Asn Asp Gln Leu Asn Pro Asp Ser Glu Asn Phe
                85                  90                  95

Asn Ala Lys Phe Trp Val Lys Asn Leu Arg Lys Leu Phe Glu Ser Asp
            100                 105                 110

Pro Glu Tyr Tyr Lys Pro Ser Lys Leu Gly Ile Gly Tyr Arg Asn Leu
        115                 120                 125

Arg Ala Tyr Gly Val Ala Asn Asp Ser Asp Tyr Gln Pro Thr Val Thr
    130                 135                 140

Asn Ala Leu Trp Lys Leu Ala Thr Glu Gly Phe Arg His Phe Gln Lys
145                 150                 155                 160

Asp Asp Asp Ser Arg Tyr Phe Asp Ile Leu Lys Ser Met Asp Ala Ile
                165                 170                 175

Met Arg Pro Gly Glu Leu Thr Val Val Leu Gly Arg Pro Gly Ala Gly
            180                 185                 190

Cys Ser Thr Leu Leu Lys Thr Ile Ala Val Asn Thr Tyr Gly Phe His
        195                 200                 205

Ile Gly Lys Glu Ser Gln Ile Thr Tyr Asp Gly Leu Ser Pro His Asp
    210                 215                 220

Ile Glu Arg His Tyr Arg Gly Asp Val Ile Tyr Ser Ala Glu Thr Asp
225                 230                 235                 240

Val His Phe Pro His Leu Ser Val Gly Asp Thr Leu Glu Phe Ala Ala
                245                 250                 255

Arg Leu Arg Thr Pro Gln Asn Arg Gly Glu Gly Ile Asp Arg Glu Thr
            260                 265                 270

Tyr Ala Lys His Met Ala Ser Val Tyr Met Ala Thr Tyr Gly Leu Ser
        275                 280                 285

His Thr Arg Asn Thr Asn Val Gly Asn Asp Phe Val Arg Gly Val Ser
    290                 295                 300

Gly Gly Glu Arg Lys Arg Val Ser Ile Ala Glu Ala Ser Leu Ser Gly
305                 310                 315                 320

Ala Asn Ile Gln Cys Trp Asp Asn Ala Thr Arg Gly Leu Asp Ser Ala
                325                 330                 335

Thr Ala Leu Glu Phe Ile Arg Ala Leu Lys Thr Ser Ala Val Ile Leu
            340                 345                 350

Asp Thr Thr Pro Leu Ile Ala Ile Tyr Gln Cys Ser Gln Asp Ala Tyr
        355                 360                 365

Asp Leu Phe Asp Lys Val Val Val Leu Tyr Glu Gly Tyr Gln Ile Phe
    370                 375                 380

Phe Gly Lys Ala Thr Lys Ala Lys Glu Tyr Phe Glu Lys Met Gly Trp
385                 390                 395                 400

Lys Cys Pro Gln Arg Gln Thr Thr Ala Asp Phe Leu Thr Ser Leu Thr
                405                 410                 415

Asn Pro Ala Glu Arg Glu Pro Leu Pro Gly Tyr Glu Asp Lys Val Pro
            420                 425                 430

Arg Thr Ala Gln Glu Phe Glu Thr Tyr Trp Lys Asn Ser Pro Glu Tyr
        435                 440                 445

Ala Glu Leu Thr Lys Glu Ile Asp Glu Tyr Phe Val Glu Cys Glu Arg
    450                 455                 460

Ser Asn Thr Arg Glu Thr Tyr Arg Glu Ser His Val Ala Lys Gln Ser
465                 470                 475                 480

Asn Asn Thr Arg Pro Ala Ser Pro Tyr Thr Val Ser Phe Phe Met Gln
                485                 490                 495

Val Arg Tyr Gly Val Ala Arg Asn Phe Leu Arg Met Lys Gly Asp Pro
            500                 505                 510

Ser Ile Pro Ile Phe Ser Val Phe Gly Gln Leu Val Met Gly Leu Ile
        515                 520                 525

Leu Ser Ser Val Phe Tyr Asn Leu Ser Gln Thr Thr Gly Ser Phe Tyr
    530                 535                 540

Tyr Arg Gly Ala Ala Met Phe Phe Ala Val Leu Phe Asn Ala Phe Ser
545                 550                 555                 560

Ser Leu Leu Glu Ile Met Ser Leu Phe Glu Ala Arg Pro Ile Val Glu
                565                 570                 575

Lys His Lys Lys Tyr Ala Leu Tyr Arg Pro Ser Ala Asp Ala Leu Ala
            580                 585                 590

Ser Ile Ile Ser Glu Leu Pro Val Lys Leu Ala Met Ser Met Ser Phe
        595                 600                 605

Asn Phe Val Phe Tyr Phe Met Val Asn Phe Arg Arg Asn Pro Gly Arg
    610                 615                 620

Phe Phe Phe Tyr Trp Leu Met Cys Ile Trp Cys Thr Phe Val Met Ser
625                 630                 635                 640

His Leu Phe Arg Ser Ile Gly Ala Val Ser Thr Ser Ile Ser Gly Ala
                645                 650                 655

Met Thr Pro Ala Thr Val Leu Leu Leu Ala Met Val Ile Tyr Thr Gly
            660                 665                 670

Phe Val Ile Pro Thr Pro Ser Met Leu Gly Trp Ser Arg Trp Ile Asn
        675                 680                 685

Tyr Ile Asn Pro Val Gly Tyr Val Phe Glu Ser Leu Met Val Asn Glu
    690                 695                 700

Phe His Gly Arg Glu Phe Gln Cys Ala Gln Tyr Val Pro Ser Gly Pro
705                 710                 715                 720

Gly Tyr Glu Asn Ile Ser Arg Ser Asn Gln Val Cys Thr Ala Val Gly
                725                 730                 735

Ser Val Pro Gly Asn Glu Met Val Ser Gly Thr Asn Tyr Leu Ala Gly
            740                 745                 750

Ala Tyr Gln Tyr Tyr Asn Ser His Lys Trp Arg Asn Leu Gly Ile Thr
        755                 760                 765

Ile Gly Phe Ala Val Phe Phe Leu Ala Ile Tyr Ile Ala Leu Thr Glu
    770                 775                 780

Phe Asn Lys Gly Ala Met Gln Lys Gly Glu Ile Val Leu Phe Leu Lys
785                 790                 795                 800

Gly Ser Leu Lys Lys His Lys Arg Lys Thr Ala Ala Ser Asn Lys Gly
                805                 810                 815

Asp Ile Glu Ala Gly Pro Val Ala Gly Lys Leu Asp Tyr Gln Asp Glu
            820                 825                 830

Ala Glu Ala Val Asn Asn Glu Lys Phe Thr Glu Lys Gly Ser Thr Gly
        835                 840                 845

Ser Val Asp Phe Pro Glu Asn Arg Glu Ile Phe Phe Trp Arg Asp Leu
    850                 855                 860

Thr Tyr Gln Val Lys Ile Lys Lys Glu Asp Arg Val Ile Leu Asp His
865                 870                 875                 880

Val Asp Gly Trp Val Lys Pro Gly Gln Ile Thr Ala Leu Met Gly Ala
                885                 890                 895

Ser Gly Ala Gly Lys Thr Thr Leu Leu Asn Cys Leu Ser Glu Arg Val
            900                 905                 910

Thr Thr Gly Ile Ile Thr Asp Gly Glu Arg Leu Val Asn Gly His Ala
        915                 920                 925

Leu Asp Ser Ser Phe Gln Arg Ser Ile Gly Tyr Val Gln Gln Gln Asp
    930                 935                 940

Val His Leu Pro Thr Ser Thr Val Arg Glu Ala Leu Gln Phe Ser Ala
945                 950                 955                 960

Tyr Leu Arg Gln Ser Asn Lys Ile Ser Lys Lys Glu Lys Asp Asp Tyr
                965                 970                 975

Val Asp Tyr Val Ile Asp Leu Leu Glu Met Thr Asp Tyr Ala Asp Ala
            980                 985                 990

Leu Val Gly Val Ala Gly Glu Gly Leu Asn Val Glu Gln Arg Lys Arg
        995                 1000                1005

Leu Thr Ile Gly Val Glu Leu Val Ala Lys Pro Lys Leu Leu Leu Phe
    1010                1015                1020

Leu Asp Glu Pro Thr Ser Gly Leu Asp Ser Gln Thr Ala Trp Ser Ile
1025                1030                1035                1040

Cys Lys Leu Met Arg Lys Leu Ala Asp His Gly Gln Ala Ile Leu Cys
            1045                1050                1055

Thr Ile His Gln Pro Ser Ala Leu Ile Met Ala Glu Phe Asp Arg Leu
        1060                1065                1070

Leu Phe Leu Gln Lys Gly Gly Arg Thr Ala Tyr Phe Gly Glu Leu Gly
    1075                1080                1085

Glu Asn Cys Gln Thr Met Ile Asn Tyr Phe Glu Lys Tyr Gly Ala Asp
  1090                1095                1100

Pro Cys Pro Lys Glu Ala Asn Pro Ala Glu Trp Met Leu Gln Val Val
1105                1110                1115                1120

Gly Ala Ala Pro Gly Ser His Ala Lys Gln Asp Tyr Phe Glu Val Trp
            1125                1130                1135

Arg Asn Ser Ser Glu Tyr Gln Ala Val Arg Glu Glu Ile Asn Arg Met
        1140                1145                1150

Glu Ala Glu Leu Ser Lys Leu Pro Arg Asp Asn Asp Pro Glu Ala Leu
    1155                1160                1165

Leu Lys Tyr Ala Ala Pro Leu Trp Lys Gln Tyr Leu Leu Val Ser Trp
  1170                1175                1180

Arg Thr Ile Val Gln Asp Trp Arg Ser Pro Gly Tyr Ile Tyr Ser Lys
1185                1190                1195                1200

Ile Phe Leu Val Val Ser Ala Ala Leu Phe Asn Gly Phe Ser Phe Phe
            1205                1210                1215

Lys Ala Lys Asn Asn Met Gln Gly Leu Gln Asn Gln Met Phe Ser Val
        1220                1225                1230

Phe Met Phe Phe Ile Pro Phe Asn Thr Leu Val Gln Gln Met Leu Pro
    1235                1240                1245

Tyr Phe Val Lys Gln Arg Asp Val Tyr Glu Val Arg Glu Ala Pro Ser
  1250                1255                1260

Arg Thr Phe Ser Trp Phe Ala Phe Ile Ala Gly Gln Ile Thr Ser Glu
1265                1270                1275                1280

Ile Pro Tyr Gln Val Ala Val Gly Thr Ile Ala Phe Phe Cys Trp Tyr
            1285                1290                1295

Tyr Pro Leu Gly Leu Tyr Asn Asn Ala Thr Pro Thr Asp Ser Val Asn
            1300                1305                1310

Pro Arg Gly Val Leu Met Trp Met Leu Val Thr Ala Phe Tyr Val Tyr
        1315                1320                1325

Thr Ala Thr Met Gly Gln Leu Cys Met Ser Phe Ser Glu Leu Ala Asp
    1330                1335                1340

Asn Ala Ala Asn Leu Ala Thr Leu Leu Phe Thr Met Cys Leu Asn Phe
1345                1350                1355                1360

Cys Gly Val Leu Ala Gly Pro Asp Val Leu Pro Gly Phe Trp Ile Phe
                1365                1370                1375

Met Tyr Arg Cys Asn Pro Phe Thr Tyr Leu Val Gln Ala Met Leu Ser
            1380                1385                1390

Thr Gly Leu Ala Asn Thr Phe Val Lys Cys Ala Glu Arg Glu Tyr Val
        1395                1400                1405

Ser Val Lys Pro Pro Asn Gly Glu Ser Cys Ser Thr Tyr Leu Asp Pro
    1410                1415                1420

Tyr Ile Lys Phe Ala Gly Gly Tyr Phe Glu Thr Arg Asn Asp Gly Ser
1425                1430                1435                1440

Cys Ala Phe Cys Gln Met Ser Ser Thr Asn Thr Phe Leu Lys Ser Val
                1445                1450                1455

Asn Ser Leu Tyr Ser Glu Arg Trp Arg Asn Phe Gly Ile Phe Ile Ala
            1460                1465                1470

Phe Ile Ala Ile Asn Ile Ile Leu Thr Val Ile Phe Tyr Trp Leu Ala
        1475                1480                1485

Arg Val Pro Lys Gly Asn Arg Glu Lys Lys Asn Lys Lys
    1490                1495                1500

<210> 90
<211> 1499
<212> PRT
<213> Candida albicans

<400> 90

Met Ser Thr Ala Asn Thr Ser Leu Ser Gln Gln Leu Asp Glu Asn Pro
1               5                   10                  15

Trp Val Asp Ala Ser Asp Asn Ser Ser Val Gln Glu Tyr Gln Gly Phe
            20                  25                  30

Asp Ala Thr Ala Ser His Asn Ile Gln Asp Leu Ala Arg Lys Leu Thr
        35                  40                  45

His Gly Ser Thr Asn Gly Asp His His Ser Ala Asn Asp Leu Ala Arg
    50                  55                  60

Tyr Leu Ser His Met Ser Asp Ile Pro Gly Val Ser Pro Phe Asn Gly
65                  70                  75                  80

Asn Ile Ser His Glu Gln Leu Asp Pro Asp Ser Glu Asn Phe Asn Ala
                85                  90                  95

Lys Tyr Trp Val Lys Asn Leu Lys Lys Leu Phe Glu Ser Asp Ser Asp
            100                 105                 110

Tyr Tyr Lys Pro Ser Lys Leu Gly Val Ala Tyr Arg Asn Leu Arg Ala
        115                 120                 125

Tyr Gly Ile Ala Asn Asp Ser Asp Tyr Gln Pro Thr Val Thr Asn Ala
    130                 135                 140

Leu Trp Lys Phe Thr Thr Glu Ala Ile Asn Lys Leu Lys Lys Pro Asp
145                 150                 155                 160

Asp Ser Lys Tyr Phe Asp Ile Leu Lys Ser Met Asp Ala Ile Met Arg
                165                 170                 175

Pro Gly Glu Leu Thr Val Val Leu Gly Arg Pro Gly Ala Gly Cys Ser
            180                 185                 190

Thr Leu Leu Lys Thr Ile Ala Val Asn Thr Tyr Gly Phe His Ile Gly
        195                 200                 205

Lys Glu Ser Gln Ile Thr Tyr Asp Gly Leu Ser Pro His Asp Ile Glu
    210                 215                 220

Arg His Tyr Arg Gly Asp Val Ile Tyr Ser Ala Glu Thr Asp Val His
225                 230                 235                 240

Phe Pro His Leu Ser Val Gly Asp Thr Leu Glu Phe Ala Ala Arg Leu
                245                 250                 255

Arg Thr Pro Gln Asn Arg Gly Glu Gly Ile Asp Arg Glu Thr Tyr Ala
            260                 265                 270

Lys His Met Ala Ser Val Tyr Met Ala Thr Tyr Gly Leu Ser His Thr
        275                 280                 285

Arg Asn Thr Asn Val Gly Asn Asp Phe Val Arg Gly Val Ser Gly Gly
    290                 295                 300

Glu Arg Lys Arg Val Ser Ile Ala Glu Ala Ser Leu Ser Gly Ala Asn
305                 310                 315                 320

Ile Gln Cys Trp Asp Asn Ala Thr Arg Gly Leu Asp Ser Ala Thr Ala
                325                 330                 335

Leu Glu Phe Ile Arg Ala Leu Lys Thr Ser Ala Thr Ile Leu Asp Thr
            340                 345                 350

Thr Pro Leu Ile Ala Ile Tyr Gln Cys Ser Gln Asp Ala Tyr Glu Leu
        355                 360                 365

Phe Asp Asn Val Val Val Leu Tyr Glu Gly Tyr Gln Ile Phe Phe Gly
    370                 375                 380

Lys Ala Ser Lys Ala Lys Glu Tyr Phe Glu Asn Met Gly Trp Lys Cys
385                 390                 395                 400

Pro Gln Arg Gln Thr Thr Ala Asp Phe Leu Thr Ser Leu Thr Asn Pro
                405                 410                 415

Ala Glu Arg Glu Pro Leu Pro Gly Tyr Glu Asp Lys Val Pro Arg Thr
            420                 425                 430

Ala Gln Glu Phe Glu Thr Phe Trp Lys Asn Ser Pro Glu Tyr Ala Glu
        435                 440                 445

Leu Thr Lys Glu Ile Asp Glu Tyr Phe Val Glu Cys Glu Arg Ser Asn
    450                 455                 460

Thr Gly Glu Thr Tyr Arg Glu Ser His Val Gly Lys Gln Ser Asn Asn
465                 470                 475                 480

Thr Arg Pro Ser Ser Pro Tyr Thr Val Ser Phe Phe Met Gln Val Arg
                485                 490                 495

Tyr Val Ile Ala Arg Asn Phe Leu Arg Met Lys Gly Asp Pro Ser Ile
            500                 505                 510

Pro Leu Ile Ser Ile Leu Ser Gln Leu Val Met Gly Leu Ile Leu Ala
        515                 520                 525

Ser Val Phe Phe Asn Leu Arg Lys Ser Thr Asp Thr Phe Tyr Phe Arg
    530                 535                 540

Gly Gly Ala Leu Phe Phe Ser Val Leu Phe Asn Ala Phe Ser Ser Leu
545                 550                 555                 560

Leu Glu Ile Leu Ser Leu Tyr Glu Ala Arg Pro Ile Val Glu Lys His
                565                 570                 575

Arg Lys Tyr Ala Leu Tyr Arg Pro Ser Ala Asp Ala Leu Ala Ser Ile
            580                 585                 590

Ile Ser Glu Leu Pro Val Lys Leu Leu Met Thr Met Ser Phe Asn Ile
        595                 600                 605

Val Tyr Tyr Phe Met Val Asn Leu Arg Arg Thr Ala Gly Asn Phe Phe
    610                 615                 620

Phe Tyr Trp Leu Met Cys Ala Ser Cys Thr Leu Val Met Ser His Met
625                 630                 635                 640

Phe Arg Ser Ile Gly Ala Val Thr Thr Thr Ile Ala Thr Ala Met Ser
                645                 650                 655

Leu Ser Thr Val Phe Leu Leu Ala Met Ile Ile Tyr Ala Gly Phe Val
            660                 665                 670

Leu Pro Ile Pro Tyr Ile Leu Gly Trp Ser Arg Trp Ile Arg Tyr Ile
        675                 680                 685

Asn Pro Val Thr Tyr Ile Phe Glu Ser Leu Met Val Asn Glu Phe His
    690                 695                 700

Gly Arg Glu Phe Glu Cys Gly Gln Tyr Ile Pro Ser Gly Pro Gly Phe
705                 710                 715                 720

Glu Asn Leu Pro Val Glu Asn Lys Val Cys Thr Thr Val Gly Ser Thr
                725                 730                 735

Pro Gly Ser Thr Val Val Gln Gly Thr Glu Tyr Ile Lys Leu Ala Tyr
            740                 745                 750

Gln Phe Tyr Ser Ser His Lys Trp Arg Asn Phe Gly Ile Thr Val Ala
        755                 760                 765

Phe Ala Val Phe Phe Leu Gly Val Tyr Val Ala Leu Thr Glu Phe Asn
    770                 775                 780

Lys Gly Ala Ser Gln Lys Gly Glu Ile Val Leu Phe Leu Lys Gly Ser
785                 790                 795                 800

Leu Lys Lys His Lys Arg Lys Thr Ala Ala Ser Asn Lys Gly Asp Ile
                805                 810                 815

Glu Ala Gly Pro Val Ala Gly Lys Leu Asp Tyr Gln Asp Glu Ala Glu
            820                 825                 830

Ala Val Asn Asn Glu Lys Phe Thr Glu Lys Gly Ser Thr Gly Ser Val
        835                 840                 845

Asp Phe Pro Glu Asn Arg Glu Ile Phe Phe Trp Arg Asp Leu Thr Tyr
    850                 855                 860

Gln Val Lys Ile Lys Lys Glu Asp Arg Val Ile Leu Asp His Val Asp
865                 870                 875                 880

Gly Trp Val Lys Pro Gly Gln Ile Thr Ala Leu Met Gly Ala Ser Gly
                885                 890                 895

Ala Gly Lys Thr Thr Leu Leu Asn Cys Leu Ser Glu Arg Val Thr Thr
            900                 905                 910

Gly Ile Ile Thr Asp Gly Glu Arg Leu Val Asn Gly His Ala Leu Asp
        915                 920                 925

Ser Ser Phe Gln Arg Ser Ile Gly Tyr Val Gln Gln Gln Asp Val His
    930                 935                 940

Leu Glu Thr Thr Thr Val Arg Glu Ala Leu Gln Phe Ser Ala Tyr Leu
945                 950                 955                 960

Arg Gln Ser Asn Lys Ile Ser Lys Lys Glu Lys Asp Asp Tyr Val Asp
                965                 970                 975

Tyr Val Ile Asp Leu Leu Glu Met Thr Asp Tyr Ala Asp Ala Leu Val
            980                 985                 990

Gly Val Ala Gly Glu Gly Leu Asn Val Glu Gln Arg Lys Arg Leu Thr
        995                 1000                1005

Ile Gly Val Glu Leu Val Ala Lys Pro Lys Leu Leu Leu Phe Leu Asp
    1010                1015                1020

Glu Pro Thr Ser Gly Leu Asp Ser Gln Thr Ala Trp Ser Ile Cys Lys
1025                1030                1035                1040

Leu Met Arg Lys Leu Ala Asp His Gly Gln Ala Ile Leu Cys Thr Ile
                1045                1050                1055

His Gln Pro Ser Ala Leu Ile Met Ala Glu Phe Asp Lys Leu Leu Phe
            1060                1065                1070

Leu Gln Lys Gly Gly Arg Thr Ala Tyr Phe Gly Glu Leu Gly Glu Asn
        1075                1080                1085

Cys Gln Thr Met Ile Asn Tyr Phe Glu Lys Tyr Gly Ala Asp Pro Cys
    1090                1095                1100

Pro Lys Glu Ala Asn Pro Ala Glu Trp Met Leu Gln Val Val Gly Ala
1105                1110                1115                1120

Ala Pro Gly Ser His Ala Lys Gln Asp Tyr Phe Glu Val Trp Arg Asn
                1125                1130                1135

Ser Ser Glu Tyr Gln Ala Val Arg Glu Glu Ile Asn Arg Met Glu Ala
            1140                1145                1150

Glu Leu Ser Lys Leu Pro Arg Asp Asn Asp Pro Glu Ala Leu Leu Lys
        1155                1160                1165

Tyr Ala Ala Pro Leu Trp Lys Gln Tyr Leu Leu Val Ser Trp Arg Thr
    1170                1175                1180

Ile Val Gln Asp Trp Arg Ser Pro Gly Tyr Ile Tyr Ser Lys Leu Ile
1185                1190                1195                1200

Leu Val Ile Ser Ser Ser Leu Phe Ile Gly Phe Ser Phe Phe Lys Ser
                1205                1210                1215

Lys Asn Asn Leu Gln Gly Leu Gln Ser Gln Met Leu Ala Val Phe Met
            1220                1225                1230

Phe Phe Val Pro Phe Thr Thr Phe Ile Asp Gln Met Leu Pro Tyr Phe
        1235                1240                1245

Val Lys His Arg Ala Val Tyr Glu Val Arg Glu Ala Pro Ser Arg Thr
    1250                1255                1260

Phe Ser Trp Phe Ala Phe Ile Ala Gly Gln Ile Thr Ser Glu Ile Pro
1265                1270                1275                1280

Phe Gln Ile Val Val Gly Thr Ile Ser Tyr Phe Cys Trp Tyr Tyr Pro
                1285                1290                1295

Val Gly Leu Tyr Ala Asn Ala Glu Pro Thr Asp Ser Val Asn Ser Arg
            1300                1305                1310

Gly Val Leu Met Trp Met Leu Leu Thr Ala Phe Tyr Val Tyr Thr Ser
        1315                1320                1325

Thr Met Gly Gln Leu Ala Ile Ser Leu Asn Glu Leu Ile Asp Asn Ala
    1330                1335                1340

Ala Asn Leu Ala Thr Thr Leu Phe Thr Leu Cys Leu Met Phe Cys Gly
1345                1350                1355                1360

Val Leu Ala Gly Pro Asn Val Ile Pro Gly Phe Trp Ile Phe Met Tyr
                1365                1370                1375

Arg Cys Asn Pro Phe Thr Tyr Leu Ile Gln Ala Ile Leu Ser Thr Gly
            1380                1385                1390

Leu Ala Asn Ala Lys Val Thr Cys Ala Pro Arg Glu Leu Val Thr Leu
        1395                1400                1405

Lys Pro Pro Met Gly Glu Thr Cys Ser Ser Phe Ile Gly Pro Tyr Thr
    1410                1415                1420

Glu Ala Ala Gly Gly Tyr Phe Ser Thr Asn Ser Asp Gly Thr Cys Ser
1425                1430                1435                1440

Val Cys Arg Ile Asp Ser Thr Asn Gln Phe Leu Glu Ser Ile Asn Ala
                1445                1450                1455

Leu Phe Ser Gln Arg Trp Arg Asn Phe Gly Ile Phe Val Ala Phe Ile
            1460                1465                1470

Gly Ile Asn Ile Ile Leu Thr Ile Phe Phe Tyr Trp Leu Ala Arg Val
        1475                1480                1485

Pro Lys Gly Asn Arg Glu Lys Lys Met Lys Lys
    1490                1495

<210> 91
<211> 1511
<212> PRT
<213> Saccharomyces cerevisiae

<400> 91
Met Pro Glu Ala Lys Leu Asn Asn Asn Val Asn Asp Val Thr Ser Tyr
1               5                   10                  15

Ser Ser Ala Ser Ser Ser Thr Glu Asn Ala Ala Asp Leu His Asn Tyr
            20                  25                  30

Asn Gly Phe Asp Glu His Thr Glu Ala Arg Ile Gln Lys Leu Ala Arg
        35                  40                  45

Thr Leu Thr Ala Gln Ser Met Gln Asn Ser Thr Gln Ser Ala Pro Asn
    50                  55                  60

Lys Ser Asp Ala Gln Ser Ile Phe Ser Ser Gly Val Glu Gly Val Asn
65                  70                  75                  80

Pro Ile Phe Ser Asp Pro Glu Ala Pro Gly Tyr Asp Pro Lys Leu Asp
                85                  90                  95

Pro Asn Ser Glu Asn Phe Ser Ser Ala Ala Trp Val Lys Asn Met Ala
            100                 105                 110

His Leu Ser Ala Ala Asp Pro Asp Phe Tyr Lys Pro Tyr Ser Leu Gly
        115                 120                 125

Cys Ala Trp Lys Asn Leu Ser Ala Ser Gly Ala Ser Ala Asp Val Ala
    130                 135                 140

Tyr Gln Ser Thr Val Val Asn Ile Pro Tyr Lys Ile Leu Lys Ser Gly
145                 150                 155                 160

Leu Arg Lys Phe Gln Arg Ser Lys Glu Thr Asn Thr Phe Gln Ile Leu
                165                 170                 175

Lys Pro Met Asp Gly Cys Leu Asn Pro Gly Glu Leu Leu Val Val Leu
            180                 185                 190

Gly Arg Pro Gly Ser Gly Cys Thr Thr Leu Leu Lys Ser Ile Ser Ser
        195                 200                 205

Asn Thr His Gly Phe Asp Leu Gly Ala Asp Thr Lys Ile Ser Tyr Ser
    210                 215                 220

Gly Tyr Ser Gly Asp Asp Ile Lys Lys His Phe Arg Gly Glu Val Val
225                 230                 235                 240

Tyr Asn Ala Glu Ala Asp Val His Leu Pro His Leu Thr Val Phe Glu

245                 250                 255

Thr Leu Val Thr Val Ala Arg Leu Lys Thr Pro Gln Asn Arg Ile Lys
            260                 265                 270

Gly Val Asp Arg Glu Ser Tyr Ala Asn His Leu Ala Glu Val Ala Met
        275                 280                 285

Ala Thr Tyr Gly Leu Ser His Thr Arg Asn Thr Lys Val Gly Asn Asp
    290                 295                 300

Ile Val Arg Gly Val Ser Gly Gly Glu Arg Lys Arg Val Ser Ile Ala
305                 310                 315                 320

Glu Val Ser Ile Cys Gly Ser Lys Phe Gln Cys Trp Asp Asn Ala Thr
                325                 330                 335

Arg Gly Leu Asp Ser Ala Thr Ala Leu Glu Phe Ile Arg Ala Leu Lys
            340                 345                 350

Thr Gln Ala Asp Ile Ser Asn Thr Ser Ala Thr Val Ala Ile Tyr Gln
        355                 360                 365

Cys Ser Gln Asp Ala Tyr Asp Leu Phe Asn Lys Val Cys Val Leu Asp
    370                 375                 380

Asp Gly Tyr Gln Ile Tyr Tyr Gly Pro Ala Asp Lys Ala Lys Lys Tyr
385                 390                 395                 400

Phe Glu Asp Met Gly Tyr Val Cys Pro Ser Arg Gln Thr Thr Ala Asp
                405                 410                 415

Phe Leu Thr Ser Val Thr Ser Pro Ser Glu Arg Thr Leu Asn Lys Asp
            420                 425                 430

Met Leu Lys Lys Gly Ile His Ile Pro Gln Thr Pro Lys Glu Met Asn
        435                 440                 445

Asp Tyr Trp Val Lys Ser Pro Asn Tyr Lys Glu Leu Met Lys Glu Val
    450                 455                 460

Asp Gln Arg Leu Leu Asn Asp Asp Glu Ala Ser Arg Glu Ala Ile Lys
465                 470                 475                 480

Glu Ala His Ile Ala Lys Gln Ser Lys Arg Ala Arg Pro Ser Ser Pro
                485                 490                 495

Tyr Thr Val Ser Tyr Met Met Gln Val Lys Tyr Leu Leu Ile Arg Asn

            500                 505                 510

Met Trp Arg Leu Arg Asn Asn Ile Gly Phe Thr Leu Phe Met Ile Leu
        515                 520                 525

Gly Asn Cys Ser Met Ala Leu Ile Leu Gly Ser Met Phe Phe Lys Ile
    530                 535                 540

Met Lys Lys Gly Asp Thr Ser Thr Phe Tyr Phe Arg Gly Ser Ala Met
545                 550                 555                 560

Phe Phe Ala Ile Leu Phe Asn Ala Phe Ser Ser Leu Leu Glu Ile Phe
                565                 570                 575

Ser Leu Tyr Glu Ala Arg Pro Ile Thr Glu Lys His Arg Thr Tyr Ser
            580                 585                 590

Leu Tyr His Pro Ser Ala Asp Ala Phe Ala Ser Val Leu Ser Glu Ile
        595                 600                 605

Pro Ser Lys Leu Ile Ile Ala Val Cys Phe Asn Ile Ile Phe Tyr Phe
    610                 615                 620

Leu Val Asp Phe Arg Arg Asn Gly Gly Val Phe Phe Phe Tyr Leu Leu
625                 630                 635                 640

Ile Asn Ile Val Ala Val Phe Ser Met Ser His Leu Phe Arg Cys Val
                645                 650                 655

Gly Ser Leu Thr Lys Thr Leu Ser Glu Ala Met Val Pro Ala Ser Met
            660                 665                 670

Leu Leu Leu Ala Leu Ser Met Tyr Thr Gly Phe Ala Ile Pro Lys Lys
        675                 680                 685

Lys Ile Leu Arg Trp Ser Lys Trp Ile Trp Tyr Ile Asn Pro Leu Ala
    690                 695                 700

Tyr Leu Phe Glu Ser Leu Leu Ile Asn Glu Phe His Gly Ile Lys Phe
705                 710                 715                 720

Pro Cys Ala Glu Tyr Val Pro Arg Gly Pro Ala Tyr Ala Asn Ile Ser
                725                 730                 735

Ser Thr Glu Ser Val Cys Thr Val Val Gly Ala Val Pro Gly Gln Asp
            740                 745                 750

Tyr Val Leu Gly Asp Asp Phe Ile Arg Gly Thr Tyr Gln Tyr Tyr His

        755                 760                 765

Lys Asp Lys Trp Arg Gly Phe Gly Ile Gly Met Ala Tyr Val Val Phe
    770                 775                 780

Phe Phe Phe Val Tyr Leu Phe Leu Cys Glu Tyr Asn Glu Gly Ala Lys
785                 790                 795                 800

Gln Lys Gly Glu Ile Leu Val Phe Pro Arg Ser Ile Val Lys Arg Met
                805                 810                 815

Lys Lys Arg Gly Val Leu Thr Glu Lys Asn Ala Asn Asp Pro Glu Asn
            820                 825                 830

Val Gly Glu Arg Ser Asp Leu Ser Ser Asp Arg Lys Met Leu Gln Glu
        835                 840                 845

Ser Ser Glu Glu Glu Ser Asp Thr Tyr Gly Glu Ile Gly Leu Ser Lys
    850                 855                 860

Ser Glu Ala Ile Phe His Trp Arg Asn Leu Cys Tyr Glu Val Gln Ile
865                 870                 875                 880

Lys Ala Glu Thr Arg Arg Ile Leu Asn Asn Val Asp Gly Trp Val Lys
                885                 890                 895

Pro Gly Thr Leu Thr Ala Leu Met Gly Ala Ser Gly Ala Gly Lys Thr
            900                 905                 910

Thr Leu Leu Asp Cys Leu Ala Glu Arg Val Thr Met Gly Val Ile Thr
        915                 920                 925

Gly Asp Ile Leu Val Asn Gly Ile Pro Arg Asp Lys Ser Phe Pro Arg
    930                 935                 940

Ser Ile Gly Tyr Cys Gln Gln Gln Asp Leu His Leu Lys Thr Ala Thr
945                 950                 955                 960

Val Arg Glu Ser Leu Arg Phe Ser Ala Tyr Leu Arg Gln Pro Ala Glu
                965                 970                 975

Val Ser Ile Glu Glu Lys Asn Arg Tyr Val Glu Glu Val Ile Lys Ile
            980                 985                 990

Leu Glu Met Glu Lys Tyr Ala Asp Ala Val Val Gly Val Ala Gly Glu
        995                 1000                1005

Gly Leu Asn Val Glu Gln Arg Lys Arg Leu Thr Ile Gly Val Glu Leu

1010                1015                1020

Thr Ala Lys Pro Lys Leu Leu Val Phe Leu Asp Glu Pro Thr Ser Gly
1025                1030                1035                1040

Leu Asp Ser Gln Thr Ala Trp Ser Ile Cys Gln Leu Met Lys Lys Leu
                1045                1050                1055

Ala Asn His Gly Gln Ala Ile Leu Cys Thr Ile His Gln Pro Ser Ala
            1060                1065                1070

Ile Leu Met Gln Glu Phe Asp Arg Leu Leu Phe Met Gln Arg Gly Gly
        1075                1080                1085

Lys Thr Val Tyr Phe Gly Asp Leu Gly Glu Gly Cys Lys Thr Met Ile
    1090                1095                1100

Asp Tyr Phe Glu Ser His Gly Ala His Lys Cys Pro Ala Asp Ala Asn
1105                1110                1115                1120

Pro Ala Glu Trp Met Leu Glu Val Val Gly Ala Ala Pro Gly Ser His
                1125                1130                1135

Ala Asn Gln Asp Tyr Tyr Glu Val Trp Arg Asn Ser Glu Glu Tyr Arg
            1140                1145                1150

Ala Val Gln Ser Glu Leu Asp Trp Met Glu Arg Glu Leu Pro Lys Lys
        1155                1160                1165

Gly Ser Ile Thr Ala Ala Glu Asp Lys His Glu Phe Ser Gln Ser Ile
    1170                1175                1180

Ile Tyr Gln Thr Lys Leu Val Ser Ile Arg Leu Phe Gln Gln Tyr Trp
1185                1190                1195                1200

Arg Ser Pro Asp Tyr Leu Trp Ser Lys Phe Ile Leu Thr Ile Phe Asn
                1205                1210                1215

Gln Leu Phe Ile Gly Phe Thr Phe Phe Lys Ala Gly Thr Ser Leu Gln
            1220                1225                1230

Gly Leu Gln Asn Gln Met Leu Ala Val Phe Met Phe Thr Val Ile Phe
        1235                1240                1245

Asn Pro Ile Leu Gln Gln Tyr Leu Pro Ser Phe Val Gln Gln Arg Asp
    1250                1255                1260

Leu Tyr Glu Ala Arg Glu Arg Pro Ser Arg Thr Phe Ser Trp Ile Ser

1265                1270                1275                1280

Phe Ile Phe Ala Gln Ile Phe Val Glu Val Pro Trp Asn Ile Leu Ala
                1285                1290                1295

Gly Thr Ile Ala Tyr Phe Ile Tyr Tyr Tyr Pro Ile Gly Phe Tyr Ser
            1300                1305                1310

Asn Ala Ser Ala Ala Gly Gln Leu His Glu Arg Gly Ala Leu Phe Trp
        1315                1320                1325

Leu Phe Ser Cys Ala Phe Tyr Val Tyr Val Gly Ser Met Gly Leu Leu
    1330                1335                1340

Val Ile Ser Phe Asn Gln Val Ala Glu Ser Ala Ala Asn Leu Ala Ser
1345                1350                1355                1360

Leu Leu Phe Thr Met Ser Leu Ser Phe Cys Gly Val Met Thr Thr Pro
                1365                1370                1375

Ser Ala Met Pro Arg Phe Trp Ile Phe Met Tyr Arg Val Ser Pro Leu
            1380                1385                1390

Thr Tyr Phe Ile Gln Ala Leu Leu Ala Val Gly Val Ala Asn Val Asp
        1395                1400                1405

Val Lys Cys Ala Asp Tyr Glu Leu Leu Glu Phe Thr Pro Pro Ser Gly
    1410                1415                1420

Met Thr Cys Gly Gln Tyr Met Glu Pro Tyr Leu Gln Leu Ala Lys Thr
1425                1430                1435                1440

Gly Tyr Leu Thr Asp Glu Asn Ala Thr Asp Thr Cys Ser Phe Cys Gln
                1445                1450                1455

Ile Ser Thr Thr Asn Asp Tyr Leu Ala Asn Val Asn Ser Phe Tyr Ser
            1460                1465                1470

Glu Arg Trp Arg Asn Tyr Gly Ile Phe Ile Cys Tyr Ile Ala Phe Asn
        1475                1480                1485

Tyr Ile Ala Gly Val Phe Phe Tyr Trp Leu Ala Arg Val Pro Lys Lys
    1490                1495                1500

Asn Gly Lys Leu Ser Lys Lys
1505                1510

<210> 92
<211> 1501
<212> PRT
<213> Saccharomyces cerevisiae

<400> 92
Met Ser Asn Ile Lys Ser Thr Gln Asp Ser Ser His Asn Ala Val Ala
1               5                   10                  15

Arg Ser Ser Ser Ala Ser Phe Ala Ala Ser Glu Glu Ser Phe Thr Gly
            20                  25                  30

Ile Thr His Asp Lys Asp Glu Gln Ser Asp Thr Pro Ala Asp Lys Leu
        35                  40                  45

Thr Lys Met Leu Thr Gly Pro Ala Arg Asp Thr Ala Ser Gln Ile Ser
    50                  55                  60

Ala Thr Val Ser Glu Met Ala Pro Asp Val Val Ser Lys Val Glu Ser
65                  70                  75                  80

Phe Ala Asp Ala Leu Ser Arg His Thr Thr Arg Ser Gly Ala Phe Asn
                85                  90                  95

Met Asp Ser Asp Ser Asp Asp Gly Phe Asp Ala His Ala Ile Phe Glu
            100                 105                 110

Ser Phe Val Arg Asp Ala Asp Glu Gln Gly Ile His Ile Arg Lys Ala
        115                 120                 125

Gly Val Thr Ile Glu Asp Val Ser Ala Lys Gly Val Asp Ala Ser Ala
    130                 135                 140

Leu Glu Gly Ala Thr Phe Gly Asn Ile Leu Cys Leu Pro Leu Thr Ile
145                 150                 155                 160

Phe Lys Gly Ile Lys Ala Lys Arg His Gln Lys Met Arg Gln Ile Ile
                165                 170                 175

Ser Asn Val Asn Ala Leu Ala Glu Ala Gly Glu Met Ile Leu Val Leu
            180                 185                 190

Gly Arg Pro Gly Ala Gly Cys Ser Ser Phe Leu Lys Val Thr Ala Gly
        195                 200                 205

Glu Ile Asp Gln Phe Ala Gly Gly Val Ser Gly Glu Val Ala Tyr Asp
    210                 215                 220

Gly Ile Pro Gln Glu Glu Met Met Lys Arg Tyr Lys Ala Asp Val Ile
225                 230                 235                 240

Tyr Asn Gly Glu Leu Asp Val His Phe Pro Tyr Leu Thr Val Lys Gln
                245                 250                 255

Thr Leu Asp Phe Ala Ile Ala Cys Lys Thr Pro Ala Leu Arg Val Asn
            260                 265                 270

Asn Val Ser Lys Lys Glu Tyr Ile Ala Ser Arg Arg Asp Leu Tyr Ala
        275                 280                 285

Thr Ile Phe Gly Leu Arg His Thr Tyr Asn Thr Lys Val Gly Asn Asp
    290                 295                 300

Phe Val Arg Gly Val Ser Gly Gly Glu Arg Lys Arg Val Ser Ile Ala
305                 310                 315                 320

Glu Ala Leu Ala Ala Lys Gly Ser Ile Tyr Cys Trp Asp Asn Ala Thr
                325                 330                 335

Arg Gly Leu Asp Ala Ser Thr Ala Leu Glu Tyr Ala Lys Ala Ile Arg
            340                 345                 350

Ile Met Thr Asn Leu Leu Lys Ser Thr Ala Phe Val Thr Ile Tyr Gln
        355                 360                 365

Ala Ser Glu Asn Ile Tyr Glu Thr Phe Asp Lys Val Thr Val Leu Tyr
    370                 375                 380

Ser Gly Lys Gln Ile Tyr Phe Gly Leu Ile His Glu Ala Lys Pro Tyr
385                 390                 395                 400

Phe Ala Lys Met Gly Tyr Leu Cys Pro Pro Arg Gln Ala Thr Ala Glu
                405                 410                 415

Phe Leu Thr Ala Leu Thr Asp Pro Asn Gly Phe His Leu Ile Lys Pro
            420                 425                 430

Gly Tyr Glu Asn Lys Val Pro Arg Thr Ala Glu Glu Phe Glu Thr Tyr
        435                 440                 445

Trp Leu Asn Ser Pro Glu Phe Ala Gln Met Lys Lys Asp Ile Ala Ala
    450                 455                 460

Tyr Lys Glu Lys Val Asn Thr Glu Lys Thr Lys Glu Val Tyr Asp Glu
465                 470                 475                 480

Ser Met Ala Gln Glu Lys Ser Lys Tyr Thr Arg Lys Lys Ser Tyr Tyr
                485                 490                 495

Thr Val Ser Tyr Trp Glu Gln Val Lys Leu Cys Thr Gln Arg Gly Phe
            500                 505                 510

Gln Arg Ile Tyr Gly Asn Lys Ser Tyr Thr Val Ile Asn Val Cys Ser
        515                 520                 525

Ala Ile Ile Gln Ser Phe Ile Thr Gly Ser Leu Phe Tyr Asn Thr Pro
    530                 535                 540

Ser Ser Thr Ser Gly Ala Phe Ser Arg Gly Gly Val Leu Tyr Phe Ala
545                 550                 555                 560

Leu Leu Tyr Tyr Ser Leu Met Gly Leu Ala Asn Ile Ser Phe Glu His
                565                 570                 575

Arg Pro Ile Leu Gln Lys His Lys Gly Tyr Ser Leu Tyr His Pro Ser
            580                 585                 590

Ala Glu Ala Ile Gly Ser Thr Leu Ala Ser Phe Pro Phe Arg Met Ile
        595                 600                 605

Gly Leu Thr Cys Phe Phe Ile Ile Leu Phe Phe Leu Ser Gly Leu His
    610                 615                 620

Arg Thr Ala Gly Ser Phe Phe Thr Ile Tyr Leu Phe Leu Thr Met Cys
625                 630                 635                 640

Ser Glu Ala Ile Asn Gly Leu Phe Glu Met Val Ser Ser Val Cys Asp
                645                 650                 655

Thr Leu Ser Gln Ala Asn Ser Ile Ser Gly Ile Leu Met Met Ser Ile
            660                 665                 670

Ser Met Tyr Ser Thr Tyr Met Ile Gln Leu Pro Ser Met His Pro Trp
        675                 680                 685

Phe Lys Trp Ile Ser Tyr Val Leu Pro Ile Arg Tyr Ala Phe Glu Ser
    690                 695                 700

Met Leu Asn Ala Glu Phe His Gly Arg His Met Asp Cys Ala Asn Thr
705                 710                 715                 720

Leu Val Pro Ser Gly Gly Asp Tyr Asp Asn Leu Ser Asp Asp Tyr Lys
                725                 730                 735

Val Cys Ala Phe Val Gly Ser Lys Pro Gly Gln Ser Tyr Val Leu Gly
            740                 745                 750

Asp Asp Tyr Leu Lys Asn Gln Phe Gln Tyr Val Tyr Lys His Thr Trp
        755                 760                 765

Arg Asn Phe Gly Ile Leu Trp Cys Phe Leu Leu Gly Tyr Val Val Leu
    770                 775                 780

Lys Val Ile Phe Thr Glu Tyr Lys Arg Pro Val Lys Gly Gly Gly Asp
785                 790                 795                 800

Ala Leu Ile Phe Lys Lys Gly Ser Lys Arg Phe Ile Ala His Ala Asp
                805                 810                 815

Glu Glu Ser Pro Asp Asn Val Asn Asp Ile Asp Ala Lys Glu Gln Phe
            820                 825                 830

Ser Ser Glu Ser Ser Gly Ala Asn Asp Glu Val Phe Asp Asp Leu Glu
        835                 840                 845

Ala Lys Gly Val Phe Ile Trp Lys Asp Val Cys Phe Thr Ile Pro Tyr
    850                 855                 860

Glu Gly Gly Lys Arg Met Leu Leu Asp Asn Val Ser Gly Tyr Cys Ile
865                 870                 875                 880

Pro Gly Thr Met Thr Ala Leu Met Gly Glu Ser Gly Ala Gly Lys Thr
                885                 890                 895

Thr Leu Leu Asn Thr Leu Ala Gln Arg Asn Val Gly Ile Ile Thr Gly
            900                 905                 910

Asp Met Leu Val Asn Gly Arg Pro Ile Asp Ala Ser Phe Glu Arg Arg
        915                 920                 925

Thr Gly Tyr Val Gln Gln Gln Asp Ile His Ile Ala Glu Leu Thr Val
    930                 935                 940

Arg Glu Ser Leu Gln Phe Ser Ala Arg Met Arg Arg Pro Gln His Leu
945                 950                 955                 960

Pro Asp Ser Glu Lys Met Asp Tyr Val Glu Lys Ile Ile Arg Val Leu
                965                 970                 975

Gly Met Glu Glu Tyr Ala Glu Ala Leu Val Gly Glu Val Gly Cys Gly
            980                 985                 990

Leu Asn Val Glu Gln Arg Lys Lys Leu Ser Ile Gly Val Glu Leu Val
        995                 1000                1005

Ala Lys Pro Asp Leu Leu Leu Phe Leu Asp Glu Pro Thr Ser Gly Leu
    1010                1015                1020

Asp Ser Gln Ser Ser Trp Ala Ile Ile Gln Leu Leu Arg Lys Leu Ser
1025                1030                1035                1040

Lys Ala Gly Gln Ser Ile Leu Cys Thr Ile His Gln Pro Ser Ala Thr
                1045                1050                1055

Leu Phe Glu Glu Phe Asp Arg Leu Leu Leu Leu Arg Lys Gly Gly Gln
            1060                1065                1070

Thr Val Tyr Phe Gly Asp Ile Gly Lys Asn Ser Ala Thr Ile Leu Asn
        1075                1080                1085

Tyr Phe Glu Arg Asn Gly Ala Arg Lys Cys Asp Ser Ser Glu Asn Pro
    1090                1095                1100

Ala Glu Tyr Ile Leu Glu Ala Ile Gly Ala Gly Ala Thr Ala Ser Val
1105                1110                1115                1120

Lys Glu Asp Trp His Glu Lys Trp Leu Asn Ser Val Glu Phe Glu Gln
                1125                1130                1135

Thr Lys Glu Lys Val Gln Asp Leu Ile Asn Asp Leu Ser Lys Gln Glu
            1140                1145                1150

Thr Lys Ser Glu Val Gly Asp Lys Pro Ser Lys Tyr Ala Thr Ser Tyr
        1155                1160                1165

Ala Tyr Gln Phe Arg Tyr Val Leu Ile Arg Thr Ser Thr Ser Phe Trp
    1170                1175                1180

Arg Ser Leu Asn Tyr Ile Met Ser Lys Met Met Leu Met Leu Val Gly
1185                1190                1195                1200

Gly Leu Tyr Ile Gly Phe Thr Phe Phe Asn Val Gly Lys Ser Tyr Val
                1205                1210                1215

Gly Leu Gln Asn Ala Met Phe Ala Ala Phe Ile Ser Ile Ile Leu Ser
            1220                1225                1230

Ala Pro Ala Met Asn Gln Ile Gln Gly Arg Ala Ile Ala Ser Arg Glu
        1235                1240                1245

Leu Phe Glu Val Arg Glu Ser Gln Ser Asn Met Phe His Trp Ser Leu
    1250                1255                1260

Val Leu Ile Thr Gln Tyr Leu Ser Glu Leu Pro Tyr His Leu Phe Phe
1265                1270                1275                1280

Ser Thr Ile Phe Phe Val Ser Ser Tyr Phe Pro Leu Arg Ile Phe Phe
                1285                1290                1295

Glu Ala Ser Arg Ser Ala Val Tyr Phe Leu Asn Tyr Cys Ile Met Phe
            1300                1305                1310

Gln Leu Tyr Tyr Val Gly Leu Gly Leu Met Ile Leu Tyr Met Ser Pro
        1315                1320                1325

Asn Leu Pro Ser Ala Asn Val Ile Leu Gly Leu Cys Leu Ser Phe Met
    1330                1335                1340

Leu Ser Phe Cys Gly Val Thr Gln Pro Val Ser Leu Met Pro Gly Phe
1345                1350                1355                1360

Trp Thr Phe Met Trp Lys Ala Ser Pro Tyr Thr Tyr Phe Val Gln Asn
                1365                1370                1375

Leu Val Gly Ile Met Leu His Lys Lys Pro Val Val Cys Lys Lys Lys
            1380                1385                1390

Glu Leu Asn Tyr Phe Asn Pro Pro Asn Gly Ser Thr Cys Gly Glu Tyr
        1395                1400                1405

Met Lys Pro Phe Leu Glu Lys Ala Thr Gly Tyr Ile Glu Asn Pro Asp
    1410                1415                1420

Ala Thr Ser Asp Cys Ala Tyr Cys Ile Tyr Glu Val Gly Asp Asn Tyr
1425                1430                1435                1440

Leu Thr His Ile Ser Ser Lys Tyr Ser Tyr Leu Trp Arg Asn Phe Gly
                1445                1450                1455

Ile Phe Trp Ile Tyr Ile Phe Phe Asn Ile Ile Ala Met Val Cys Val
            1460                1465                1470

Tyr Tyr Leu Phe His Val Arg Gln Ser Ser Phe Leu Ser Pro Val Ser
        1475                1480                1485

Ile Leu Asn Lys Ile Lys Asn Ile Arg Lys Lys Lys Gln
    1490                1495                1500